# EUROPEAN PATENT APPLICATION

(11) **EP 4 796 554 A1**
(43) Date of publication of application: **26.08.2026**
(21) Application number: 24879131.1
(22) Date of filing: 18.10.2024
(51) Int. Cl.: C07H 15/04, A61K 31/7088, A61K 47/54, A61K 31/713, C12N 15/113

(54) **TARGETED DELIVERY LIGAND**

(30) Priority: 20.10.2023 CN 202311369031
(71) Applicant: Ibridge US Inc., Lewes, Delaware 19958 (US)
(72) Inventor: CHEN, Bo, Chengdu, Sichuan 610219 (CN); XU, Jianyan, Chengdu, Sichuan 610219 (CN); MIAO, Yu, Chengdu, Sichuan 610219 (CN); ZHAN, Qingming, Chengdu, Sichuan 610219 (CN); TENG, Gang, Chengdu, Sichuan 610219 (CN); ZHANG, Haifan, Chengdu, Sichuan 610219 (CN); WANG, Lei, Chengdu, Sichuan 610219 (CN)
(74) Representative: Maiwald GmbH
(86) International application number: PCT/CN2024/125816
(87) International publication number: WO 2025/082491

(57) **Abstract**

The present invention provides a targeted delivery ligand comprising a conjugated group as shown in formula (X), or an isotopic variant, tautomer, stereoisomer thereof, or a mixture thereof, and a nucleic acid molecule containing the targeted delivery ligand.

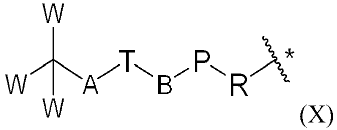

## Description

The present invention claims the priority to Chinese invention patent application CN 202311369031.X, filed on October 20, 2023, the entire contents of which are incorporated herein by reference as a part of the present invention.

### Technical Field

The present invention belongs to the field of medicine, and specifically relates to a targeted delivery ligand and a nucleic acid molecule containing the targeted delivery ligand.

### Background Art

The liver is the largest metabolic organ in the human body, participating in the synthesis and metabolism of various chemical substances in the body. Abnormal liver function can lead to a series of liver-related diseases, including cancer, metabolic diseases, and the like. Liver-targeted therapies have always been a research hotspot in the treatment of liver-related diseases. Since its discovery in 1998, siRNA interference (RNAi) therapy has become a promising candidate therapy for a variety of diseases. However, since siRNAs are relatively polyanionic molecules, these molecules are difficult to enter cells through passive diffusion mechanisms. Furthermore, the targeted delivery of siRNA to tissues and cells in vivo remains the biggest challenge in its clinical application, due to its rapid degradation in plasma, clearance in the kidneys, limited permeability across capillary endothelial barriers, and low cellular uptake efficiency.

N-acetylgalactosamine (GalNAc) is a high-affinity targeting ligand for an asialoglycoprotein receptor (ASGPR). ASGPR is an ideal receptor for active targeting. It is specifically and highly expressed on the surface of hepatocytes. After binding to GalNAc, it enters the cell via endocytosis to form endosomes, thereby bringing a sufficient amount of nucleic acid drugs into the cell and achieving liver-targeted delivery of the nucleic acid drugs. N-acetylgalactosamine (GalNAc) can specifically bind to the asialoglycoprotein receptor (ASGPR) on the surface of hepatocytes.

Accordingly, there is a demand in the art for GalNAc-containing targeted delivery ligands.

### Summary of the Invention

The present invention provides a novel targeted delivery ligand containing N-acetylgalactosamine.

In one aspect, the present invention provides a delivery ligand, wherein the delivery ligand comprises a conjugated group as shown in formula (X), or an isotopic variant, tautomer, or stereoisomer thereof, or a mixture thereof: 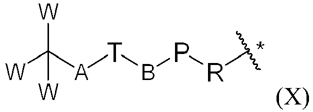 wherein
the R is attached to a biomolecule via 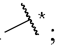
each W is independently selected from H, D, 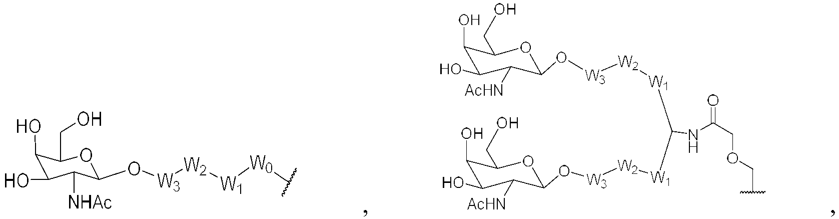 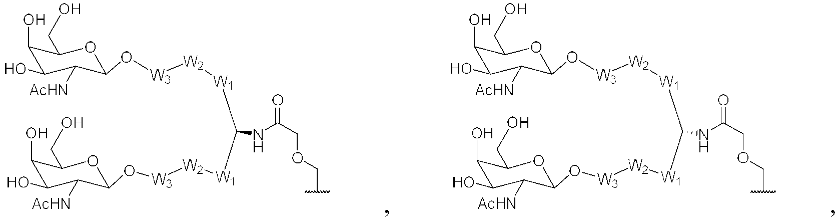 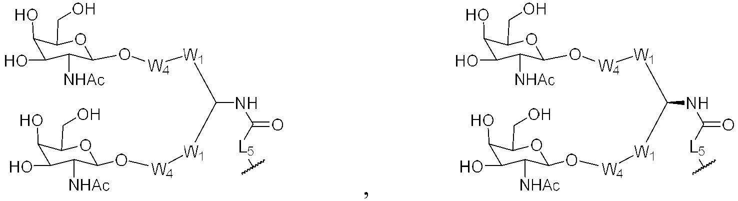 or 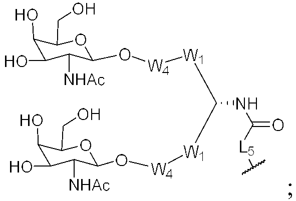
W₀ is selected from a chemical bond, -OCH₂-, -OCH₂CH₂-, - OCH₂OCH₂-, -OCH₂CH₂CH₂-, -OCH₂CH₂CH₂CH₂-, -CH₂OCH₂CH₂CH₂-, - CH₂CH₂OCH₂CH₂-, -CH₂CH₂CH₂OCH₂-, -CH₂CH₂OCH₂CH₂O-, C₁₋₆ alkylene, C₁₋₆ haloalkylene, C₁₋₆ alkenylene or C₁₋₆ alkynylene, which is optionally deuterated, up to fully deuterated;
W₁ is selected from a chemical bond, -O-, -O-L₁-, -C(O)-, -C(O)-L₁-, - C(O)O- or -C(O)O-L₁-, which is optionally deuterated, up to fully deuterated;
L₁ is -(CRₐR_{b})ₘ-, and 1, 2, 3, 4 or 5 non-adjacent CRₐR_{b} in the L₁ may be replaced by a heteroatom selected from -O-, -S- and -NRₐ-;
Rₐ and R_{b} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₐ and R_{b} on any one or more carbon atoms in L₁, together with the same or different carbon atoms to which they are attached, form C₃₋₁₀ cycloalkyl, 3- to 10-membered heterocyclyl, C₆₋₁₀ aryl or 5- to 10-membered heteroaryl;
W₂ is selected from -L₂-NH-, -L₂-O-, -L₂-C(O)- or -L₂-OC(O)-, which is optionally deuterated, up to fully deuterated;
L₂ is -(CR_{c}R_{d})ₙ-, and 1, 2, 3, 4 or 5 non-adjacent CR_{c}R_{d} in the L₂ may be replaced by a heteroatom selected from -O-, -S- and -NR_{c}-;
R_{c} and R_{d} are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{c} and R_{d} on any one or more carbon atoms in L₂, together with the same or different carbon atoms to which they are attached, form C₃₋₁₀ cycloalkyl, 3- to 10-membered heterocyclyl, C₆₋₁₀ aryl or 5- to 10-membered heteroaryl;
W₃ is selected from -L₃-C(O)NH-, -L₃-NHC(O)-, -L₃-OC(O)NH- or - L₃-NHC(O)O-, which is optionally deuterated, up to fully deuterated;
L₃ is -(CRₑR_{f})ₕ-, and 1, 2, 3, 4 or 5 non-adjacent CRₑR_{f} in the L₃ may be replaced by a heteroatom selected from -O-, -S- and -NRₑ-;
Rₑ and R_{f} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₑ and R_{f} on any one or more carbon atoms in L₃, together with the same or different carbon atoms to which they are attached, form C₃₋₁₀ cycloalkyl, 3- to 10-membered heterocyclyl, C₆₋₁₀ aryl or 5- to 10-membered heteroaryl;
W₄ is selected from -L₄-NHC(O)-, -L₄-NHC(O)-(CH₂)₁₋₆-, -L₄-NHC(O)O-, -L₄-NHC(O)O-(CH₂)₁₋₆-, -L₄-C(O)NH-, -L₄-C(O)NH-(CH₂)₁₋₆-, -L₄-OC(O)NH- or -L₄-OC(O)NH-(CH₂)₁₋₆-, which is optionally deuterated, up to fully deuterated;
L₄ is selected from -(CR_{g}Rₕ)ₖ-, -(CR_{g}Rₕ)₁₋₆-(OCR_{g}Rₕ-CR_{g}Rₕ)ₖ- or - (CR_{g}Rₕ)₁₋₆-(NH-CR_{g}Rₕ-CR_{g}Rₕ)ₖ-, and 1, 2, 3, 4 or 5 non-adjacent CR_{g}Rₕ in the L₄ may be replaced by a heteroatom selected from -O-, -S- and -NR_{g}-;
R_{g} and Rₕ are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{g} and Rₕ on any one or more carbon atoms in L₄, together with the same or different carbon atoms to which they are attached, form C₃₋₁₀ cycloalkyl, 3- to 10-membered heterocyclyl, C₆₋₁₀ aryl or 5- to 10-membered heteroaryl;
m, n, h and k are independently selected from 0, 1, 2, 3, 4, 5, 6, 7, 8, 9 or 10;
L₅ is selected from a chemical bond, C₁₋₆ alkylene, C₁₋₆ alkenylene or - C₁₋₆ alkylene-O-C₁₋₆ alkylene-, which is optionally deuterated, up to fully deuterated;
A is selected from a chemical bond, -C(O)- or -NHC(O)-;
T is -(CRᵢRⱼ)_{q}-, and 1, 2, 3, 4 or 5 non-adjacent CRᵢRⱼ in the T may be replaced by a heteroatom selected from -O-, -S- and -NRᵢ-; T is optionally deuterated, up to fully deuterated;
Rᵢ and Rⱼ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rᵢ and Rⱼ on any one or more carbon atoms in T, together with the same or different carbon atoms to which they are attached, form C₃₋₁₀ cycloalkyl, 3- to 10-membered heterocyclyl, C₆₋₁₀ aryl or 5- to 10-membered heteroaryl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-, and 1, 2 or 3 non-adjacent CRₖRₘ in the B may be replaced by a heteroatom selected from -O-, -Sand -NRₖ-; the B is optionally deuterated, up to fully deuterated;
Rₖ and Rₘ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₖ and Rₘ on any one or more carbon atoms in B, together with the same or different carbon atoms to which they are attached, form C₃₋₁₀ cycloalkyl, 3- to 10-membered heterocyclyl, C₆₋₁₀ aryl or 5- to 10-membered heteroaryl;
P is selected from a chemical bond or 1, 2 or 3 amino acid residues;
R is selected from 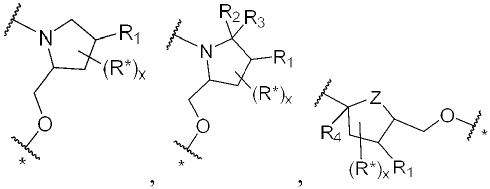 or 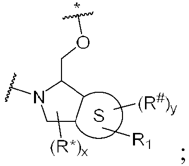
ring S is selected from C₃₋₁₀ cycloalkyl, 3- to 10-membered heterocyclyl, C₆₋₁₀ aryl or 5- to 10-membered heteroaryl;
R₁ is selected from a hydroxyl-containing group, such as -OR₀, -C₁₋₆ alkylene-OR₀, -C₁₋₆ alkenylene-OR₀ or -C₁₋₆ alkynylene-OR₀, wherein R₀ is H, D, a hydroxyl protecting group or a solid-phase carrier;
the hydroxyl protecting group is selected from trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), - C(O)CH₂CH₂C(O)OH or 4,4'-dimethoxytrityl (DMTr), preferably - C(O)CH₂CH₂C(O)OH or 4,4'-dimethoxytrityl (DMTr);
R₂ and R₃ are independently selected from H, D, halogen, C₁₋₆ alkyl, C₁₋₆ alkoxy, C₁₋₆ haloalkyl, C₁₋₆ alkenyl or C₁₋₆ alkynyl; alternatively, R₂ and R₃, together with the same carbon atom to which they are attached, form C₃₋₁₀ cycloalkyl, 3- to 10-membered heterocyclyl, C₆₋₁₀ aryl or 5- to 10-membered heteroaryl; R₂, R₃ and the ring formed thereby are optionally deuterated, up to fully deuterated;
R₄ is selected from H, D, halogen, C₁₋₆ alkyl, C₁₋₆ alkoxy, C₁₋₆ haloalkyl, C₁₋₆ alkenyl or C₁₋₆ alkynyl, and R₄ is optionally deuterated, up to fully deuterated;
Z is selected from O, NH or CH₂;
R* and R^{#} are independently selected from H, D, halogen, C₁₋₆ alkyl, C₁₋₆ haloalkyl, C₁₋₆ alkoxy, C₃₋₁₀ cycloalkyl, 3- to 10-membered heterocyclyl, C₆₋₁₀ aryl or 5- to 10-membered heteroaryl;
x and y are independently selected from 0, 1, 2, 3, 4 or 5;
the above-mentioned W, A, T, B, P and R are each optionally further substituted with 1, 2, 3, 4, 5, 6, 7, 8 or more substituents selected from the following: H, D, OH, CN, NH₂, NO₂, oxo, thio, halogen, C₁₋₆ alkyl, C₁₋₆ haloalkyl, C₁₋₆ alkoxy, C₃₋₁₀ cycloalkyl, 3- to 10-membered heterocyclyl, C₆₋₁₀ aryl or 5- to 10-membered heteroaryl;
provided that, when R is 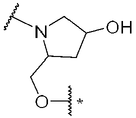 (including a stereoisomeric form thereof, such as 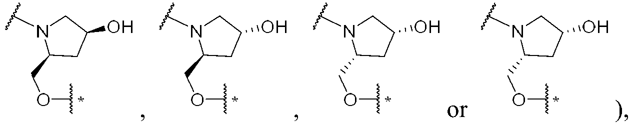 at least one pair among Rₐ and R_{b}, R_{c} and R_{d}, Rₑ and R_{f}, R_{g} and Rₕ, Rᵢ and Rⱼ, and Rₖ and Rₘ, together with the same or different carbon atoms to which they are attached, forms C₃₋₁₀ cycloalkyl, 3- to 10-membered heterocyclyl, C₆₋₁₀ aryl or 5- to 10-membered heteroaryl.

In another aspect, the present invention relates to a nucleic acid molecule, comprising a nucleic acid and the delivery ligand described herein attached thereto.

In another aspect, the present invention relates to a double-stranded RNA molecule, comprising a sense strand and an antisense strand, wherein the double-stranded RNA molecule contains one or more delivery ligands described herein.

In another aspect, the present invention relates to a pharmaceutical composition, comprising the nucleic acid molecule described herein or the double-stranded RNA molecule described herein, and a pharmaceutically acceptable carrier or excipient.

In another aspect, the present invention relates to the nucleic acid molecule described herein or the double-stranded RNA molecule described herein.

In another aspect, the present invention relates to a compound of formula (X'), or a pharmaceutically acceptable salt, stereoisomer, tautomer or isotopic variant thereof, or a mixture thereof: 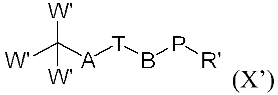 wherein
R' is selected from 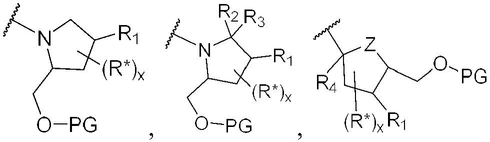 or 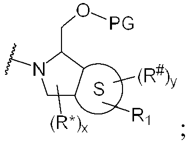
PG is a hydroxyl protecting group, such as trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), - C(O)CH₂CH₂C(O)OH or 4,4'-dimethoxytrityl (DMTr);
preferably, PG is selected from 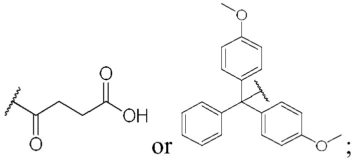
each W' is independently selected from H, D, 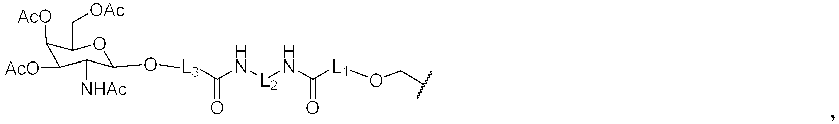 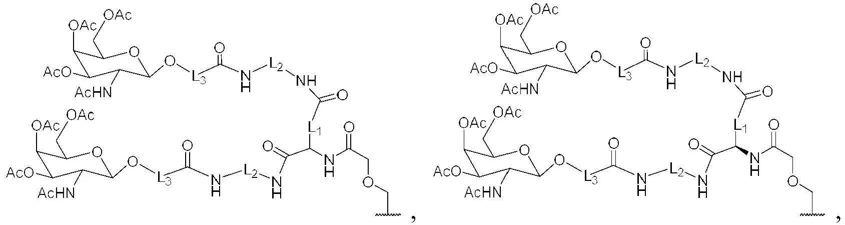 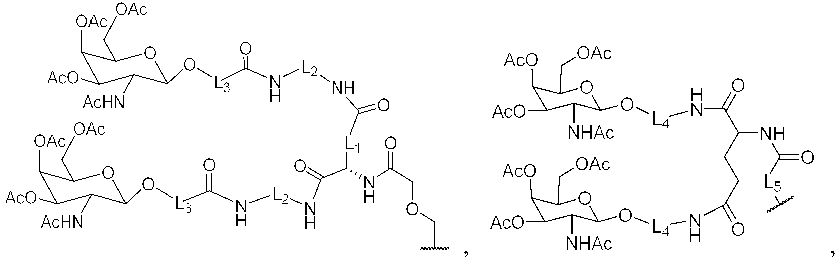 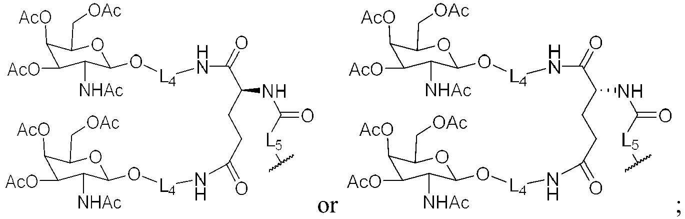
provided that, when R' is 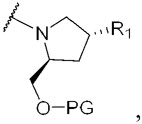 at least one pair among Rₐ and R_{b}, R_{c} and R_{d}, Rₑ and R_{f}, R_{g} and Rₕ, Rᵢ and Rⱼ, and Rₖ and Rₘ in W', together with the carbon atom to which they are attached, forms C₃₋₇ cycloalkyl or 3- to 7-membered heterocyclyl;
each of the other variables is as defined herein.

In another aspect, the present invention relates to a compound of formula (XI), or a pharmaceutically acceptable salt, isotopic variant, tautomer, stereoisomer, polymorph, hydrate or solvate thereof: 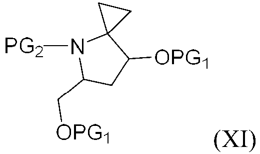 wherein
each PG₁ is independently selected from H or a hydroxyl protecting group;
PG₂ is H or an amino protecting group.

In another aspect, the present invention relates to a compound of formula (XII) or formula (XIII), or a pharmaceutically acceptable salt, isotopic variant, tautomer, stereoisomer, polymorph, hydrate or solvate thereof: 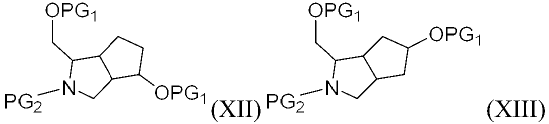 wherein
each PG₁ is independently selected from H or a hydroxyl protecting group;
PG₂ is H or an amino protecting group.

### Brief Description of the Drawings

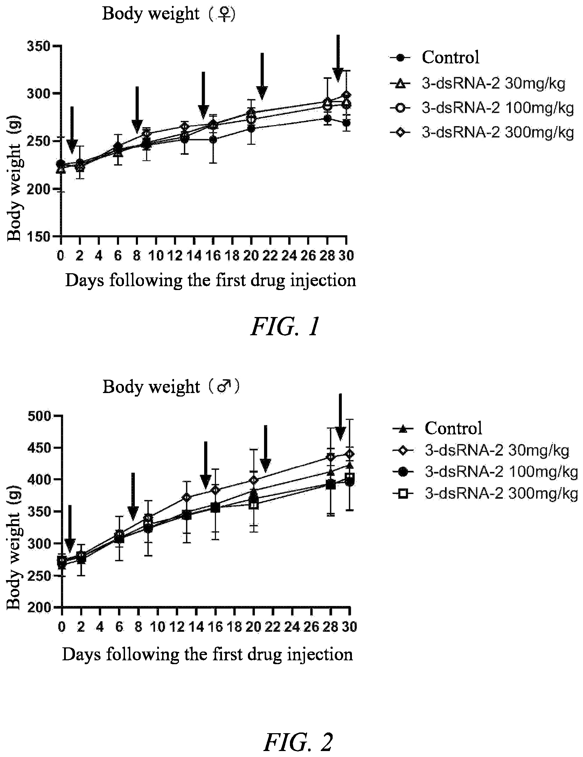

FIG. 1 and FIG. 2 show the body weight change curves of female and male rats, respectively, after administration of the siRNA conjugate 3-dsRNA-2 of the present invention.

### Detailed Description of the Invention

### Definitions

### Chemical definitions

Definitions of specific functional groups and chemical terms are described in more detail below.

When a numerical range is listed, every value and sub-range within the stated range are intended to be included. For example, "C₁₋₆ alkyl" includes C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

"C₁₋₆ alkyl" refers to a linear or branched saturated hydrocarbon group having 1 to 6 carbon atoms. In some embodiments, C₁₋₄ alkyl and C₁₋₂ alkyl are preferred. Examples of C₁₋₆ alkyl include: methyl (C₁), ethyl (C₂), n-propyl (C₃), isopropyl (C₃), n-butyl (C₄), tert-butyl (C₄), sec-butyl (C₄), isobutyl (C₄), n-pentyl (C₅), 3- pentyl (C₅), pentyl (C₅), neopentyl (C₅), 3-methyl-2-butyl (C₅), tert-pentyl (C₅) and n-hexyl (C₆). The term "C₁₋₆ alkyl" further includes a heteroalkyl group in which one or more (e.g., 1, 2, 3, or 4) carbon atoms are replaced by heteroatoms (e.g., oxygen, sulphur, nitrogen, boron, silicon, and phosphorus). The alkyl group may be optionally substituted with one or more substituents, for example, with 1 to 5 substituents, 1 to 3 substituents, or 1 substituent. Common alkyl abbreviations include: Me(-CH₃), Et(-CH₂CH₃), iPr(-CH(CH₃)₂), nPr(-CH₂CH₂CH₃), n-Bu(-CH₂CH₂CH₂CH₃) or i-Bu(-CH₂CH(CH₃)₂).

"C₂₋₆ alkenyl" refers to a linear or branched hydrocarbon group having 2 to 6 carbon atoms and at least one carbon-carbon double bond. In some embodiments, C₂₋₄ alkenyl is preferred. Examples of C₂₋₆ alkenyl include: ethenyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. The term "C₂₋₆ alkenyl" further includes a heteroalkenyl group in which one or more (e.g., 1, 2, 3, or 4) carbon atoms are replaced by heteroatoms (e.g., oxygen, sulphur, nitrogen, boron, silicon, and phosphorus). The alkenyl group may be optionally substituted with one or more substituents, for example, with 1 to 5 substituents, 1 to 3 substituents, or 1 substituent.

"C₂₋₆ alkynyl" refers to a linear or branched hydrocarbon group having 2 to 6 carbon atoms, at least one carbon-carbon triple bond, and optionally one or more carbon-carbon double bonds. In some embodiments, C₂₋₄ alkynyl is preferred. Examples of C₂₋₆ alkynyl include, but are not limited to: ethynyl (C₂), 1-propynyl (C₃), 2-propynyl (C₃), 1-butynyl (C₄), 2-butynyl (C₄), pentynyl (C₅), hexynyl (C₆), etc. The term "C₂₋₆ alkynyl" further includes a heteroalkynyl group in which one or more (e.g., 1, 2, 3, or 4) carbon atoms are replaced by heteroatoms (e.g., oxygen, sulphur, nitrogen, boron, silicon, and phosphorus). The alkynyl group may be optionally substituted with one or more substituents, for example, with 1 to 5 substituents, 1 to 3 substituents, or 1 substituent.

"Halo" or "halogen" refers to fluorine (F), chlorine (Cl), bromine (Br) and iodine (I).

Therefore, "C₁₋₆ haloalkyl" refers to the above-mentioned "C₁₋₆ alkyl" which is substituted with one or more halogen groups. In some embodiments, C₁₋₄ haloalkyl is particularly preferred, and C₁₋₂ haloalkyl is more preferred. Exemplary haloalkyl groups include, but are not limited to: -CF₃, -CH₂F, -CHF₂, -CHFCH₂F, - CH₂CHF₂, -CF₂CF₃, -CCl₃, -CH₂Cl, -CHCl₂, 2,2,2-trifluoro-1,1-dimethyl-ethyl, etc. The haloalkyl group may be substituted at any available point of attachment, for example, with 1 to 5 substituents, 1 to 3 substituents, or 1 substituent.

"C₁₋₆ alkoxy" refers to an -OR group, wherein R is the C₁₋₆ alkyl as defined above. C₁₋₄ alkoxy is preferred.

"C₁₋₆ alkylene" refers to a divalent group formed by removing another hydrogen from C₁₋₆ alkyl, and may be substituted or unsubstituted. In some embodiments, C₁₋₄ alkylene, C₂₋₄ alkylene and C₁₋₃ alkylene are preferred. The unsubstituted alkylene groups include, but are not limited to: methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Exemplary substituted alkylene groups, for example, the alkylene groups substituted with one or more alkyl (methyl) groups, include, but are not limited to: substituted methylene (-CH(CH₃)- or -C(CH₃)₂-), substituted ethylene (-CH(CH₃)CH₂-, - CH₂CH(CH₃)-, -C(CH₃)₂CH₂-, or -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, - CH₂C(CH₃)₂CH₂-, or -CH₂CH₂C(CH₃)₂-), etc.

"C₂₋₆ alkenylene" refers to a divalent group formed by removing another hydrogen from C₂₋₆ alkenyl, and may be substituted or unsubstituted. In some embodiments, C₄₋₆ alkenylene, C₁₋₄ alkenylene and C₂₋₄ alkenylene are preferred.

"C₂₋₆ alkynylene" refers to a divalent group formed by removing another hydrogen from C₂₋₆ alkynyl, and may be substituted or unsubstituted. In some embodiments, C₄₋₆ alkynylene, C₁₋₄ alkynylene and C₂₋₄ alkynylene are preferred.

"C₃₋₁₀ cycloalkyl" refers to a non-aromatic cyclic hydrocarbon group having 3 to 10 ring carbon atoms and zero heteroatoms. In some embodiments, C₅₋₇ cycloalkyl, C₃₋₇ cycloalkyl and C₃₋₅ cycloalkyl are particularly preferred, and C₅₋₆ cycloalkyl is more preferred. The cycloalkyl group further includes a ring system in which the above-mentioned cycloalkyl ring is fused with one or more aryl or heteroaryl groups, wherein the point of attachment is on the cycloalkyl ring; and in such cases, the number of carbons continues to be indicative of the number of carbons in the cycloalkyl system. Exemplary cycloalkyl groups include, but are not limited to: cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), etc. The cycloalkyl group may be optionally substituted with one or more substituents, for example, with 1 to 5 substituents, 1 to 3 substituents, or 1 substituent.

"3- to 10-membered heterocyclyl" refers to a group of a 3- to 10-membered non-aromatic ring system having ring carbon atoms and 1 to 5 ring heteroatoms, wherein each heteroatom is independently selected from nitrogen, oxygen, sulphur, boron, phosphorus and silicon. In heterocyclyl groups containing one or more nitrogen atoms, the point of attachment may be a carbon or nitrogen atom, as valency permits. In some embodiments, 4- to 9-membered heterocyclyl is preferred, which is a 4- to 9-membered non-aromatic ring system having ring carbon atoms and 1 to 5 ring heteroatoms; in some embodiments, 5- to 8-membered heterocyclyl is preferred, which is a 5- to 8-membered non-aromatic ring system having ring carbon atoms and 1 to 5 ring heteroatoms; in some embodiments, 3- to 8-membered heterocyclyl is preferred, which is a 3- to 8-membered non-aromatic ring system having ring carbon atoms and 1 to 4 ring heteroatoms; 3- to 7-membered heterocyclyl is preferred, which is a 3- to 7-membered non-aromatic ring system having ring carbon atoms and 1 to 3 ring heteroatoms; 3- to 5-membered heterocyclyl is preferred, which is a 3- to 5-membered non-aromatic ring system having ring carbon atoms and 1 to 2 ring heteroatoms; 4- to 7-membered heterocyclyl is preferred, which is a 4- to 7-membered non-aromatic ring system having ring carbon atoms and 1 to 3 ring heteroatoms; 4- to 6-membered heterocyclyl is preferred, which is a 4- to 6-membered non-aromatic ring system having ring carbon atoms and 1 to 3 ring heteroatoms; 3- to 5-membered heterocyclyl is preferred, which is a 3- to 5-membered non-aromatic ring system having ring carbon atoms and 1 to 3 ring heteroatoms; and 5- to 6-membered heterocyclyl is more preferred, which is a 5- to 6-membered non-aromatic ring system having ring carbon atoms and 1 to 3 ring heteroatoms. The heterocyclyl group further includes a ring system in which the above-mentioned heterocyclyl ring is fused with one or more cycloalkyl groups, wherein the point of attachment is on the cycloalkyl ring, or a ring system in which the above-mentioned heterocyclyl ring is fused with one or more aryl or heteroaryl groups, wherein the point of attachment is on the heterocyclyl ring; and in such cases, the number of ring members continues to be indicative of the number of ring members in the heterocyclyl ring system. Exemplary 3-membered heterocyclyl groups containing one heteroatom include, but are not limited to: aziridinyl, oxiranyl and thiorenyl. Exemplary 4-membered heterocyclyl groups containing one heteroatom include, but are not limited to: azetidinyl, oxetanyl and thietanyl. Exemplary 5-membered heterocyclyl groups containing one heteroatom include, but are not limited to: tetrahydrofuryl, dihydrofuryl, tetrahydrothienyl, dihydrothienyl, pyrrolidyl, dihydropyrrolyl and pyrrolyl-2,5-dione. Exemplary 5-membered heterocyclyl groups containing two heteroatoms include, but are not limited to: dioxolanyl, oxasulphuranyl, disulphuranyl and oxazolidin-2-one. Exemplary 5-membered heterocyclyl groups containing three heteroatoms include, but are not limited to: triazolinyl, oxadiazolinyl and thiadiazolinyl. Exemplary 6-membered heterocyclyl groups containing one heteroatom include, but are not limited to: piperidyl, tetrahydropyranyl, dihydropyridyl and thianyl. Exemplary 6-membered heterocyclyl groups containing two heteroatoms include, but are not limited to: piperazinyl, morpholinyl, dithianyl and dioxanyl. Exemplary 6-membered heterocyclyl groups containing three heteroatoms include, but are not limited to: triazinanyl. Exemplary 7-membered heterocyclyl groups containing one heteroatom include, but are not limited to: azepanyl, oxepanyl and thiepanyl. Exemplary 5-membered heterocyclyl groups fused with a C₆ aryl ring (also referred to herein as 5,6-bicyclic heterocyclyl) include, but are not limited to: indolinyl, isoindolinyl, dihydrobenzofuryl, dihydrobenzothienyl, benzoxazolinonyl, etc. Exemplary 6-membered heterocyclyl groups fused with a C₆ aryl ring (also referred to herein as 6,6-bicyclic heterocyclyl) include, but are not limited to: tetrahydroquinolyl, tetrahydroisoquinolyl, etc. The heterocyclyl group may be optionally substituted with one or more substituents, for example, with 1 to 5 substituents, 1 to 3 substituents, or 1 substituent.

"C₆₋₁₀ aryl" refers to a group of a monocyclic or polycyclic (e.g., bicyclic) 4n+2 aromatic ring system (e.g., having 6 or 10 π electrons shared in a cyclic arrangement) having 6-10 ring carbon atoms and zero heteroatoms. In some embodiments, the aryl group has six ring carbon atoms ("C₆ aryl"; e.g., phenyl). In some embodiments, the aryl group has ten ring carbon atoms ("C₁₀ aryl"; e.g., naphthyl, such as 1-naphthyl and 2-naphthyl). The aryl group further includes a ring system in which the above-mentioned aryl ring is fused with one or more cycloalkyl or heterocyclyl groups, and the point of attachment is on the aryl ring; and in such cases, the number of carbon atoms continues to be indicative of the number of carbon atoms in the aryl ring system. The aryl group may be optionally substituted with one or more substituents, for example, with 1 to 5 substituents, 1 to 3 substituents, or 1 substituent.

"5- to 10-membered heteroaryl" refers to a group of a 5- to 10-membered monocyclic or bicyclic 4n+2 aromatic ring system (e.g., having 6, 10 or 14 π electrons shared in a cyclic arrangement) having ring carbon atoms and 1-4 ring heteroatoms, wherein each heteroatom is independently selected from nitrogen, oxygen and sulphur. In heteroaryl groups containing one or more nitrogen atoms, the point of attachment may be a carbon or nitrogen atom, as valency permits. The heteroaryl bicyclic ring system may include one or more heteroatoms in one or both rings. The heteroaryl group further includes a ring system in which the above-mentioned heteroaryl ring is fused with one or more cycloalkyl or heterocyclyl groups, and the point of attachment is on the heteroaryl ring; and in such cases, the number of carbon atoms continues to be indicative of the number of carbon atoms in the heteroaryl ring system. In some embodiments, 5- to 9-membered heteroaryl is preferred, which is a 5- to 9-membered monocyclic or bicyclic 4n+2 aromatic ring system having ring carbon atoms and 1-4 ring heteroatoms. In some other embodiments, 5- to 6-membered heteroaryl is particularly preferred, which is a 5- to 6-membered monocyclic or bicyclic 4n+2 aromatic ring system having ring carbon atoms and 1-4 ring heteroatoms. Exemplary 5-membered heteroaryl groups containing one heteroatom include, but are not limited to: pyrrolyl, furyl and thienyl. Exemplary 5-membered heteroaryl groups containing two heteroatoms include, but are not limited to: imidazolyl, pyrazolyl, oxazolyl, isoxazolyl, thiazolyl and isothiazolyl. Exemplary 5-membered heteroaryl groups containing three heteroatoms include, but are not limited to: triazolyl, oxadiazolyl (e.g., 1,2,4-oxadiazolyl)and thiadiazolyl. Exemplary 5-membered heteroaryl groups containing four heteroatoms include, but are not limited to: tetrazolyl. Exemplary 6-membered heteroaryl groups containing one heteroatom include, but are not limited to: pyridyl. Exemplary 6-membered heteroaryl groups containing two heteroatoms include, but are not limited to: pyridazinyl, pyrimidyl and pyrazinyl. Exemplary 6-membered heteroaryl groups containing three or four heteroatoms respectively include, but are not limited to: triazinyl and tetrazinyl. Exemplary 7-membered heteroaryl groups containing one heteroatom include, but are not limited to: azacycloheptatrienyl, oxacycloheptatrienyl and thiacycloheptatrienyl. Exemplary 5,6-bicyclic heteroaryl groups include, but are not limited to: indolyl, isoindolyl, indazolyl, benzotriazolyl, benzothienyl, isobenzothienyl, benzofuryl, benzisofuryl, benzimidazolyl, benzoxazolyl, benzisoxazolyl, benzoxadiazolyl, benzothiazolyl, benzisothiazolyl, benzothiadiazolyl, indazinyl and purinyl. Exemplary 6,6-bicyclic heteroaryl groups include, but are not limited to: naphthyridinyl, pteridinyl, quinolyl, isoquinolyl, cinnolinyl, quinoxalinyl, phthalazinyl and quinazolinyl. The heteroaryl group may be optionally substituted with one or more substituents, for example, with 1 to 5 substituents, 1 to 3 substituents, or 1 substituent.

"Carbonyl", whether used alone or in combination with other terms (e.g., aminocarbonyl), represents -C(O)-.

"Oxo" represents =O.

"Thio" represents =S.

The alkyl, alkenyl, alkynyl, alkylene, alkenylene, alkynylene, cycloalkyl, heterocyclyl, aryl and heteroaryl groups and the like as defined herein are optionally substituted groups.

Exemplary substituents on carbon atoms include, but are not limited to: halogen, -CN, -NO₂, -N₃, -SO₂H, -SO₃H, -OH, -OR^{aa}, -ON(R^{bb})₂, -N(R^{bb})₂, - N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂,-NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, - NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, - SO₂OR^{aa}, -OSO₂R^{aa}, -S(=O)R^{aa}, -OS(=O)R^{aa}, -Si(R^{aa})₃, -OSi(R^{aa})3, -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, - OP(=O)(OR^{cc})₂, -P(=O)₂N(R^{bb})₂, -OP(=O)₂N(R^{bb})₂, -P(=O)(NR^{bb})₂, - OP(=O)(NR^{bb})₂, -NR^{bb}P(=O)(OR^{cc})₂, -NR^{bb}P(=O)(NR^{bb})₂, -P(R^{cc})₂, -P(R^{cc})₃,-OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl and heteroaryl, wherein each of alkyl, alkenyl, alkynyl, cycloalkyl, heterocyclyl, aryl and heteroaryl is independently substituted with 0, 1, 2, 3, 4 or 5 R^{dd} groups;
alternatively, two geminal hydrogens on a carbon atom are substituted with group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc};
each R^{aa} is independently selected from alkyl, haloalkyl, alkenyl, alkynyl, cycloalkyl, heterocyclyl, aryl and heteroaryl; alternatively, two R^{aa} groups combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 R^{dd} groups;
each R^{bb} is independently selected from hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, - C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl; alternatively, two R^{bb} groups combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 R^{dd} groups;
each R^{cc} is independently selected from hydrogen, alkyl, haloalkyl, alkenyl, alkynyl, cycloalkyl, heterocyclyl, aryl and heteroaryl; alternatively, two R^{cc} groups combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 R^{dd} groups;
each R^{dd} is independently selected from: halogen, -CN, -NO₂, -N₃, - SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})2, -NR^{ff}SO₂R^{ee}, -SO₂N(R^{ff})₂, -SO₂R^{ee}, -SO₂OR^{ee}, -OSO₂R^{ee}, - S(=O)R^{ee}, -Si(R^{ee})₃, -OSi(R^{ee})₃, -C(=S)N(R^{ff})₂, -C(=O)SR^{ee}, -C(=S)SR^{ee}, - SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl or heteroaryl, wherein each of alkyl, alkenyl, alkynyl, cycloalkyl, heterocyclyl, aryl and heteroaryl is independently substituted with 0, 1, 2, 3, 4 or 5 R^{gg} groups; alternatively, two geminal R^{dd} substituents may combine to form =O or =S;
each R^{ee} is independently selected from alkyl, haloalkyl, alkenyl, alkynyl, cycloalkyl, aryl, heterocyclyl and heteroaryl, wherein each of alkyl, alkenyl, alkynyl, cycloalkyl, heterocyclyl, aryl and heteroaryl is independently substituted with 0, 1, 2, 3, 4 or 5 R^{gg} groups;
each R^{ff} is independently selected from hydrogen, alkyl, haloalkyl, alkenyl, alkynyl, cycloalkyl, heterocyclyl, aryl and heteroaryl; alternatively, two R^{ff} groups combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 R^{gg} groups;
each R^{gg} is independently: halogen, -CN, -NO₂, -N₃, -SO₂H, -SO₃H, - OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, - CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -NHC(=O)NH(C₁₋₆ alkyl), - NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, - OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, - Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ cycloalkyl, C₆-C₁₀ aryl, C₃-C₇ heterocyclyl, or C₅-C₁₀ heteroaryl; alternatively, two geminal R^{gg} substituents may combine to form =O or =S; wherein X⁻ is a counter ion.

Exemplary substituents on nitrogen atoms include, but are not limited to: hydrogen, -OH, -OR^{aa}, -N(R^{cc})₂, -CN, -C(=O)R^{aa}, -C(=O)N(R^{cc})₂, -CO₂R^{aa}, - SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, - SO₂OR^{cc}, -SOR^{aa}, -C(=S)N(R^{cc})₂, -C(=O)SR^{cc}, -C(=S)SR^{cc}, -P(=O)₂R^{aa}, - P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl; alternatively, two R^{cc} groups attached to the nitrogen atom combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described above.

### Other definitions

The term "biomolecule" as used herein includes, but is not limited to, functional proteins, enzymes, antigens, antibodies, peptides, nucleic acids (e.g., mononucleotides or nucleosides, oligonucleotides, polynucleotides, and single-stranded and higher-stranded nucleic acids), lectins, receptors, or combinations thereof. The biomolecule is preferably a nucleic acid.

As used herein, unless otherwise stated, a divalent structure can be attached to the remainder of the compound in either a left-to-right or right-to-left orientation. In one embodiment, the divalent structure is preferably attached to the remainder of the compound in a left-to-right orientation. For example, in general formula (X), 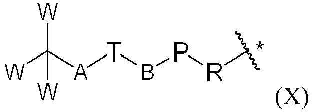 when A is defined as -C(O)NH-, it represents the following two modes of attachment:

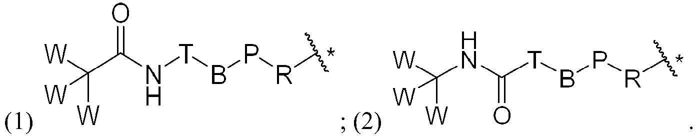

Other divalent structures shall be understood in a similar manner.

The term "amino acid residue" refers to and encompasses any natural or synthetic amino acid residue, and is not limited to amino acid residues within the group consisting of 20 naturally occurring amino acids. The 20 naturally occurring amino acid residues are selected from the group consisting of the following amino acid residues: alanine (Ala or A), cysteine (Cys or C), aspartic acid (Asp or D), glutamic acid (Glu or E), phenylalanine (Phe or F), glycine (Gly or G), histidine (His or H), isoleucine (Ile or I), lysine (Lys or K), leucine (Leu or L), methionine (Met or M), asparagine (Asn or N), proline (Pro or P), glutamine (Gln or Q), arginine (Arg or R), serine (Ser or S), threonine (Thr or T), valine (Val or V), tryptophan (Trp or W), and tyrosine (Tyr or Y) residues.

The term "siRNA" refers to a class of double-stranded RNA molecules that can mediate the silencing of their complementary target RNAs (e.g., mRNAs, such as transcripts of protein-coding genes). The siRNA is typically double-stranded, consisting of an antisense strand complementary to the target RNA and a sense strand complementary to that antisense strand. For the sake of convenience, such mRNAs are also referred to herein as mRNAs to be silenced. Such genes are also termed target genes. Typically, the RNAs to be silenced are endogenous genes or pathogen-derived genes. In addition, RNAs other than mRNAs (e.g., tRNAs) and viral RNAs may also be targeted.

The term "antisense strand" refers to a strand of siRNA that comprises a region which is fully, sufficiently, or substantially complementary to the target sequence. The term "sense strand" refers to a strand of siRNA that comprises a region which is fully, sufficiently, or substantially complementary to the region of the antisense strand as defined herein.

The term "complementary region" refers to a region on the antisense strand which is fully, sufficiently, or substantially complementary to the target mRNA sequence. In cases where the complementary region is not fully complementary to the target sequence, mismatches can be located in the internal or terminal regions of the molecule. Typically, the most tolerated mismatches are located in the terminal regions, for example, within 5, 4, 3, 2 or 1 nucleotide(s) at the 5' and/or 3' ends. The segment of the antisense strand that is most sensitive to mismatches is referred to as the "seed region". For example, in an siRNA with a strand of 19 nt, some mismatches can be tolerated at position 19 (counting from the 5' end to the 3' end).

The term "complementary" refers to the ability of a first polynucleotide to hybridise with a second polynucleotide under certain conditions, such as stringent conditions. For example, stringent conditions may include 400 mM NaCl, 40 mM PIPES pH 6.4, 1 mM EDTA, at 50°C or 70°C for 12-16 hours. In terms of meeting the aforementioned requirements regarding their ability to hybridise, "complementary" sequences may also comprise or entirely consist of non-Watson-Crick base pairs and/or base pairs formed from non-natural and modified nucleotides. Such non-Watson-Crick base pairs include, but are not limited to, G:U wobble base pairs or Hoogsteen base pairs.

A polynucleotide that is "at least partially complementary", "sufficiently complementary", or "substantially complementary" to a messenger RNA (mRNA) refers to one that is substantially complementary to a contiguous segment of the mRNA of interest. For example, a polynucleotide is at least partially complementary to PCSK9 mRNA if its sequence is substantially complementary to an uninterrupted segment of the mRNA encoding PCSK9. Herein, the terms "complementary", "fully complementary", "sufficiently complementary", and "substantially complementary" may be employed with respect to the base pairing between the sense strand and the antisense strand of an siRNA, or between the antisense strand of an siRNA reagent and a target sequence.

"Sufficiently complementary" means that the sense strand only needs to be complementary to the antisense strand to the extent necessary to maintain the overall double-stranded character of the molecule. In other words, while perfect complementarity is generally required, one or more mismatches (relative to the target mRNA), such as 6, 5, 4, 3, 2 or 1 mismatch(es), may be included in some cases, particularly within the antisense strand, without compromising the maintenance of the overall double-stranded character of the molecule between the sense strand and the antisense strand.

"shRNA" refers to short hairpin RNA. shRNA comprises two short inverted repeats. The shRNA cloned into an shRNA expression vector comprises two short inverted repeats separated by a stem-loop sequence, forming a hairpin structure under the control of a pol III promoter. This is then followed by the addition of 5-6 Ts to serve as the transcription terminator for RNA polymerase III.

A "protecting group", also known as a "protective group", refers to any atom or atomic group that is added to a molecule to prevent the existing groups within the molecule from undergoing undesired chemical reactions. A "protecting group" may be a labile chemical moiety known in the art, which is used to protect reactive groups such as hydroxyl, amino and thiol groups, preventing undesired or untimely reactions occurring during chemical synthesis. Protecting groups are typically used selectively and/or orthogonally to protect sites during the reactions at other reactive sites, and can then be removed to leave the unprotected groups either intact or available for further reactions.

A non-limiting list of protective groups includes benzyl; substituted benzyl; alkyl carbonyl and alkoxy carbonyl (e.g., tert-butyloxycarbonyl (BOC), acetyl or isobutyryl); arylalkyl carbonyl and arylalkoxy carbonyl (e.g., benzyloxy carbonyl); substituted methyl ether (e.g., methoxymethyl ether); substituted diethyl ether; substituted benzyl ether; tetrahydropyranyl ether; silyl (e.g., trimethylsilyl, triethylsilyl, triisopropylsilyl, tert-butyldimethylsilyl, tri-isopropylsilyloxymethyl, [2-(trimethylsilyl)ethoxy]methyl or tert-butyldiphenylsilyl); esters (e.g., benzoate); carbonates (e.g., methoxymethyl carbonate); sulphonates (e.g., toluenesulphonate or methanesulphonate); acyclic ketals (e.g., dimethyl acetal); cyclic ketals (e.g., 1,3-dioxane, 1,3-dioxolane, and those described herein); acyclic acetals; cyclic acetals (e.g., those described herein); acyclic hemiacetals; cyclic hemiacetals; cyclic dithioketals (e.g., 1,3-dithiane or 1,3-dithiolane); orthoesters (e.g., those described herein); and triarylmethyl groups (e.g., trityl; monomethoxytrityl (MMTr); 4,4'-dimethoxytrityl (DMTr); 4,4',4"-trimethoxytrityl (TMTr); and those described herein). Preferred protective groups are selected from acetyl (Ac), benzoyl (Bzl), benzyl (Bn), isobutyryl (iBu), phenylacetyl, benzyloxymethyl acetal (BOM), β-methoxyethoxymethyl ether (MEM), methoxymethyl ether (MOM), p-methoxybenzyl ether (PMB), methylthiomethyl ether, pivaloyl (Piv), tetrahydropyranyl (THP), trityl (Trt), methoxytrityl[(4-methoxyphenyl)diphenylmethyl] (MMT), dimethoxytrityl, [bis-(4-methoxyphenyl)phenylmethyl (DMT), trimethylsilyl ether (TMS), tert-butyldimethylsilyl ether (TBDMS), tri-iso-propylsilyloxymethyl ether (TOM), triisopropylsilyl ether (TIPS), methyl ether, ethoxyethyl ether (EE), N,N-dimethylformamidine and 2-cyanoethyl (CE).

A "hydroxyl protecting group" refers to a group that can prevent a hydroxyl group from undergoing chemical reactions and can be removed under specific conditions to regenerate the hydroxyl group. These mainly include silane-type protecting groups, acyl-type protecting groups, or ether-type protecting groups, with the following being preferred:
trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), di-p-methoxytrityl (DMTr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), -C(O)CH₂CH₂C(O)OH or 4,4'-dimethoxytrityl. Among these, -C(O)CH₂CH₂C(O)OH or 4,4'-dimethoxytrityl is preferred, and -C(O)CH₂CH₂C(O)OH is more preferred.

An "amino protecting group" is well understood by those skilled in the art of synthetic organic chemistry; it refers to a moiety that can be selectively attached to and removed from a suitable amine functional group. Amino protecting groups, along with the methods for their use, are described in the authoritative monograph Greene's Protective Groups in Organic Synthesis, Fourth Edition (Wiley, 2006) by P.G.M.Wuts and T.W.Greene. In some embodiments, the amino protecting group is selected from carbamates, amides, and sulphonamides. In some embodiments, the amino protecting group is benzyl or a Schiff base.

Non-limiting examples of carbamate-based amino protecting groups (formed with the protected amino group) include: methyl carbamate, 9-fluorenylmethyl carbamate (FMOC), 2,2,2-trichloroethyl carbamate, 2-trimethylsilylethyl carbamate, 1,1-dimethylpropynyl carbamate, 1-methyl-1-phenylethyl carbamate, 1-methyl-1-(4-biphenyl)ethyl carbamate, 1,1-dimethyl-2-haloethyl carbamate, 1,1-dimethyl-2-cyanoethyl carbamate, tert-butyl carbamate, cyclobutyl carbamate, 1-methylcyclobutyl carbamate, 1-adamantyl carbamate, vinyl carbamate, allyl carbamate, cinnamyl carbamate, 8-quinolyl carbamate, N-hydroxypiperidyl carbamate, 4,5-diphenyl-3-oxazolin-2-one, benzyl carbamate, p-nitrobenzyl carbamate, 3,4-dimethoxy-6-nitrobenzyl carbamate, 2,4-dichlorobenzyl carbamate, 5-benzisoxazolylmethyl carbamate, 9-anthrylmethyl carbamate, diphenylmethyl carbamate, isonicotinoyl carbamate, S-benzyl carbamate, and N-(N'-phenylaminothiocarbonyl) derivatives. In one embodiment, the amino protecting group (formed with the protected amino group) includes: methyl carbamate, tert-butyl carbamate, vinyl carbamate, and allyl carbamate.

Non-limiting examples of amide-based amino protecting groups include N-formyl, N-acetyl, N-chloroacetyl, N-trichloroacetyl, N-trifluoroacetyl, No-nitrophenylacetyl, N-o-nitrophenoxyacetyl, N-acetoacetyl, N-3-phenylpropionyl, N-3-(p-hydroxyphenyl)propionyl, N-2-methyl-2-(o-nitrophenoxy)propionyl, N-4-chlorobutyryl, N-o-nitrocinnamoyl, N-picolinoyl, N-(N'-acetylmethionyl), N-benzoyl, N-phthaloyl and N-dithiosuccinyl. In one embodiment, the amino protecting group is selected from N-formyl, N-acetyl, N-chloroacetyl, N-trichloroacetyl, N-trifluoroacetyl and N-acetoacetyl.

Other non-limiting examples of amino protecting groups include N-allyl, N-phenacyl, N-3-acetoxypropyl, quaternary ammonium salts, N-methoxymethyl, N-benzyloxymethyl, N-pivaloyloxymethyl, N-tetrahydropyranyl, N-2,4-dinitrophenyl, N-benzyl, N-o-nitrobenzyl, N-bis(p-methoxyphenyl)methyl, N-trityl, N-(p-methoxyphenyl)diphenylmethyl, N-diphenyl-4-pyridylmethyl, 2-picoline N'-oxide, N,N'-isopropylidene, N-salicylidene, N-(5,-dimethyl-3-oxo-1-cyclohexenyl), N-nitro, N-oxide, N-diphenylphosphinyl, N-dimethylthiophosphinyl, N-dimethylthiophosphinyl, N-benzenesulphinyl, N-o-nitrobenzenesulphinyl, N-2,4,6-trimethylbenzenesulphonyl, N-tosyl, N-benzylsulphonyl, N-trifluoromethanesulphonyl and N-phenylacylsulphonyl. In one embodiment, the amino protecting group is selected from N-allyl, N-phenacyl, N-3-acetoxypropyl, quaternary ammonium salts, N-methoxymethyl, N-benzyloxymethyl, N-pivaloyloxymethyl and N-tetrahydropyranyl.

As used herein, the term "pharmaceutically acceptable salt" refers to those carboxylate salts and amino acid addition salts of the compounds of the present invention which, within the scope of sound medical judgment, are suitable for use in contact with the tissues of patients without undue toxicity, irritation, allergic response, etc., are commensurate with a reasonable benefit/risk ratio, and are effective for their intended use, including (where possible) zwitterionic forms of the compounds of the present invention.

The present invention encompasses tautomers, which are functional group isomers formed by the rapid migration of an atom between two positions within a molecule. For compounds that exist in different tautomeric forms, a compound as described is not limited to any specific tautomer but is intended to encompass all tautomeric forms.

The compounds of the present invention may contain one or more asymmetric centres and may therefore exist in various stereoisomeric forms, for example, enantiomeric and/or diastereomeric forms. For example, the compounds of the present invention can be individual enantiomers, diastereomers or geometric isomers (such as cis and trans isomers), or can be in the form of mixtures of stereoisomers, including racemic mixtures and mixtures enriched in one or more stereoisomers. The present invention contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present invention. Herein, "*" denotes a chiral centre, which may have an (S) absolute configuration, an (R) absolute configuration, or a mixture of both, i.e., a racemic form. Isomers may be separated from mixtures by methods known to those skilled in the art, including: chiral high pressure liquid chromatography (HPLC), as well as the formation and crystallisation of chiral salts; alternatively, preferred isomers may be prepared by asymmetric synthesis. Unless otherwise stated, the term "enantiomer" or "optical isomer" refers to stereoisomers that are mirror images of each other.

Unless otherwise stated, the term "cis-trans isomer" or "geometric isomer" is caused by the fact that double bonds or single bonds of ring-forming carbon atoms cannot rotate freely.

Unless otherwise stated, the term "diastereomer" refers to stereoisomers in which molecules have two or more chiral centres and are not mirror images of each other.

Unless otherwise stated, the wedge-shaped solid bond ( 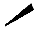 ) and the wedge-shaped dotted bond ( 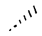 ) represent the absolute configuration of a stereoscopic centre.

Those skilled in the art will appreciate that an organic compound may form a complex with a solvent in which it reacts or from which it precipitates or crystallises. These complexes are called "solvates". When the solvent is water, the complex is called a "hydrate". The present invention encompasses all solvates of the compounds of the present invention.

The term "solvate" refers to the form of a compound or a salt thereof in association with a solvent, usually formed by a solvolysis reaction. This physical association may involve hydrogen bonding. Conventional solvents include water, methanol, ethanol, acetic acid, DMSO, THF, diethyl ether, etc. The compounds described herein can be prepared, for example, in a crystalline form, and can be solvated. Suitable solvates include pharmaceutically acceptable solvates and further include stoichiometric solvates and non-stoichiometric solvates. In some cases, the solvate will be capable of isolation, for example, when one or more solvent molecules are incorporated in the crystal lattice of the crystalline solid. The "solvate" includes both solution-phase and isolatable solvates. Representative solvates include hydrates, ethanolates and methanolates.

The term "hydrate" refers to a compound in combination with water. Usually, the ratio of the number of water molecules contained in a hydrate of a compound to the number of molecules of the compound in the hydrate is determined. Therefore, the hydrate of a compound can be represented, for example, by general formula R•x H₂O, wherein R is the compound, and x is a number greater than 0. A given compound may form more than one type of hydrate, including, for example, a monohydrate (x is 1), a lower hydrate (x is a number greater than 0 and less than 1, for example, a hemihydrate (R•0.5 H₂O)) and a polyhydrate (x is a number greater than 1, for example, a dihydrate (R•2 H₂O) and a hexahydrate (R•6 H₂O)).

The compounds of the present invention may be in amorphous or crystalline forms (polymorphs). Furthermore, the compounds of the present invention may exist in one or more crystalline forms. Therefore, the present invention includes within its scope all amorphous or crystalline forms of the compounds of the present invention. The term "polymorph" refers to a crystalline form of a compound (or a salt, hydrate, or solvate thereof) in a specific crystal packing arrangement. All polymorphs have the same elemental composition. Different crystalline forms usually have different X-ray diffraction patterns, infrared spectra, melting points, density, hardness, crystal shape, optoelectronic properties, stability and solubility. The recrystallisation solvent, crystallisation rate, storage temperature and other factors may cause one crystalline form to predominate. Various polymorphs of a compound can be prepared by crystallisation under different conditions.

The present invention further includes isotopically labelled compounds (isotopic variants) which are identical to those described in formula (I), but have one or more atoms replaced by an atom having an atomic mass or mass number different from the atomic mass or mass number usually found in nature. Examples of isotopes that can be incorporated into the compounds of the present invention include isotopes of hydrogen, carbon, nitrogen, oxygen, phosphorus, sulphur, fluorine and chlorine , such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. The compounds of the present invention, the prodrugs thereof, and the pharmaceutically acceptable salts of the compounds or the prodrugs containing the above-mentioned isotopes and/or other isotopes of other atoms all fall within the scope of the present invention. Certain isotopically labelled compounds of the present invention, for example those into which radioactive isotopes such as ³H and ¹⁴C are incorporated, are useful in drug and/or substrate tissue distribution assays. Tritium (i.e., ³H) and carbon-14 (i.e., ¹⁴C) isotopes are particularly preferred for their ease of preparation and detectability. Further, substitution with heavier isotopes, such as deuterium (i.e., ²H), can provide therapeutic benefits due to higher metabolic stability, such as an extended in vivo half-life or a reduced dosage requirement, and may thus be preferred in some cases. The isotopically labelled compounds of formula (I) of the present invention and the prodrugs thereof can generally be prepared by replacing non-isotopically labelled reagents with readily available isotopically labelled reagents when carrying out the processes disclosed in the following procedures and/or examples and preparation examples.

### Detailed Description of Embodiments

The technical solutions of the present invention will be further described in detail below with reference to specific examples. It should be understood that the following examples are merely illustrative and explanatory of the present invention and should not be construed as limiting the scope of protection of the present invention. All technologies implemented on the basis of the above-mentioned content of the present invention are encompassed within the scope of protection of the present invention.

Herein, the "compounds of the present invention" refer to conjugated groups as shown in formula (X) and the like (and sub-general formulas thereof, such as formula (I), formula (I-1), formula (I-2), formula (I'), formula (I'-1), formula (I'-2), formula (II), formula (II-1), formula (II-2), formula (II'), formula (II'-1), formula (II'-2), formula (III), formula (III-1), formula (III-2), formula (III-3), formula (III-4), formula (III'), formula (III'-1), formula (III'-2), formula (III'-3), formula (III'-4), formula (III'-5), formula (III'-6), formula (IV), formula (IV-1), formula (IV-2), formula (IV'), formula (IV'-1), formula (IV'-2), formula (V), formula (V-1), formula (V-2), formula (V-3), formula (V-4), formula (V'), formula (V'-1), formula (V'-2), formula (V'-3), formula (V'-4), formula (VI), formula (VI-1), formula (VI-2), formula (VI'), formula (VI'-1), formula (VI'-2), formula (VII), formula (VII-1), formula (VII-2), formula (VII'), formula (VII'-1), formula (VII'-2), formula (VIII), formula (VIII-1), formula (VIII-2), formula (VIII-3), formula (VIII-4), formula (VIII'), formula (VIII'-1), formula (VIII'-2), formula (VIII'-3), formula (VIII'-4), formula (IX), formula (IX-1), formula (IX-2), formula (IX'), formula (IX'-1) or formula (IX'-2)), compounds of formula (X') (and sub-general formulas thereof), compounds of formula (XI) (and sub-general formulas thereof), compounds of formula (XII) (and sub-general formulas thereof), compounds of formula (XIII) (and sub-general formulas thereof), and pharmaceutically acceptable salts, isotopic variants, tautomers, stereoisomers, prodrugs, polymorphs, hydrates or solvates thereof.

Herein, the compounds are named using the standard nomenclature. For compounds having asymmetric centres, it should be understood that all optical isomers and mixtures thereof are intended to be encompassed, unless otherwise stated. Furthermore, unless otherwise specified, all isomeric compounds encompassed by the present invention may occur in both the Z and E forms of carbon-carbon double bonds. For compounds that exist in different tautomeric forms, a compound as described is not limited to any specific tautomer but is intended to encompass all tautomeric forms.

In one embodiment, the present invention relates to a delivery ligand, wherein the delivery ligand comprises a conjugated group as shown in formula (X), or an isotopic variant, tautomer, or stereoisomer thereof, or a mixture thereof: 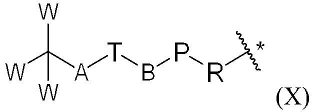 wherein
the R is attached to a biomolecule via 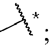
each W is independently selected from H, D, 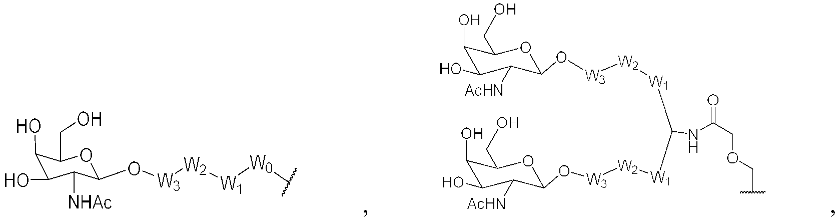 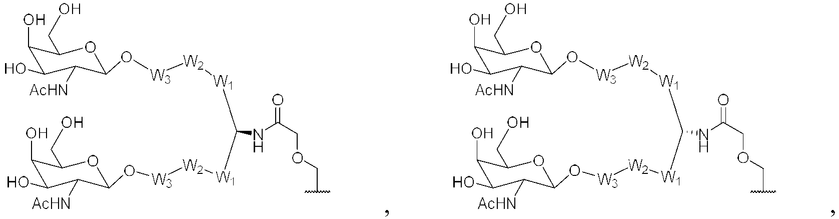 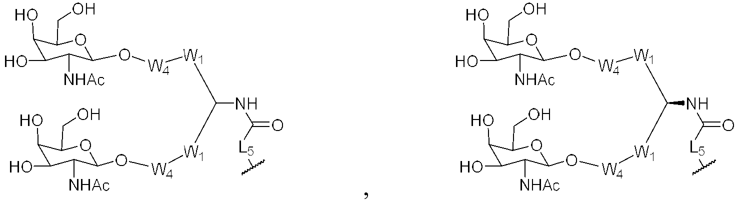 or 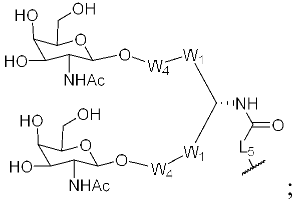
W₀ is selected from a chemical bond, -OCH₂-, -OCH₂CH₂-, - OCH₂OCH₂-, -OCH₂CH₂CH₂-, -OCH₂CH₂CH₂CH₂-, -CH₂OCH₂CH₂CH₂-, - CH₂CH₂OCH₂CH₂-, -CH₂CH₂CH₂OCH₂-, -CH₂CH₂OCH₂CH₂O-, C₁₋₆ alkylene, C₁₋₆ haloalkylene, C₁₋₆ alkenylene or C₁₋₆ alkynylene, which is optionally deuterated, up to fully deuterated;
W₁ is selected from a chemical bond, -O-, -O-L₁-, -C(O)-, -C(O)-L₁-, - C(O)O- or -C(O)O-L₁-, preferably -O-, -O-L₁-, -C(O)-, -C(O)-L₁-, -C(O)O- or - C(O)O-L₁-, which is optionally deuterated, up to fully deuterated;
L₁ is -(CRₐR_{b})ₘ-, and 1, 2, 3, 4 or 5 non-adjacent CRₐR_{b} in the L₁ may be replaced by a heteroatom selected from -O-, -S- and -NRₐ-;
Rₐ and R_{b} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₐ and R_{b} on any one or more carbon atoms in L₁, together with the same or different carbon atoms to which they are attached, form C₃₋₁₀ cycloalkyl, 3- to 10-membered heterocyclyl, C₆₋₁₀ aryl or 5- to 10-membered heteroaryl;
W₂ is selected from -L₂-NH-, -L₂-O-, -L₂-C(O)- or -L₂-OC(O)-, which is optionally deuterated, up to fully deuterated;
L₂ is -(CR_{c}R_{d})ₙ-, and 1, 2, 3, 4 or 5 non-adjacent CR_{c}R_{d} in the L₂ may be replaced by a heteroatom selected from -O-, -S- and -NR_{c}-;
R_{c} and R_{d} are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{c} and R_{d} on any one or more carbon atoms in L₂, together with the same or different carbon atoms to which they are attached, form C₃₋₁₀ cycloalkyl, 3- to 10-membered heterocyclyl, C₆₋₁₀ aryl or 5- to 10-membered heteroaryl;
W₃ is selected from -L₃-C(O)NH-, -L₃-NHC(O)-, -L₃-OC(O)NH- or - L₃-NHC(O)O-, which is optionally deuterated, up to fully deuterated;
L₃ is -(CRₑR_{f})ₕ-, and 1, 2, 3, 4 or 5 non-adjacent CRₑR_{f} in the L₃ may be replaced by a heteroatom selected from -O-, -S- and -NRₑ-;
Rₑ and R_{f} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₑ and R_{f} on any one or more carbon atoms in L₃, together with the same or different carbon atoms to which they are attached, form C₃₋₁₀ cycloalkyl, 3- to 10-membered heterocyclyl, C₆₋₁₀ aryl or 5- to 10-membered heteroaryl;
W₄ is selected from -L₄-NHC(O)-, -L₄-NHC(O)-(CH₂)₁₋₆-, -L₄-NHC(O)O-, -L₄-NHC(O)O-(CH₂)₁₋₆-, -L₄-C(O)NH-, -L₄-C(O)NH-(CH₂)₁₋₆-, -L₄-OC(O)NH- or -L₄-OC(O)NH-(CH₂)₁₋₆-, which is optionally deuterated, up to fully deuterated;
L₄ is selected from -(CR_{g}Rₕ)ₖ-, -(CR_{g}Rₕ)₁₋₆-(OCR_{g}Rₕ-CR_{g}Rₕ)ₖ- or - (CR_{g}Rₕ)₁₋₆-(NH-CR_{g}Rₕ-CR_{g}Rₕ)ₖ-, and 1, 2, 3, 4 or 5 non-adjacent CR_{g}Rₕ in the L₄ may be replaced by a heteroatom selected from -O-, -S- and -NR_{g}-;
R_{g} and Rₕ are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{g} and Rₕ on any one or more carbon atoms in L₄, together with the same or different carbon atoms to which they are attached, form C₃₋₁₀ cycloalkyl, 3- to 10-membered heterocyclyl, C₆₋₁₀ aryl or 5- to 10-membered heteroaryl;
m, n, h and k are independently selected from 0, 1, 2, 3, 4, 5, 6, 7, 8, 9 or 10;
L₅ is selected from a chemical bond, C₁₋₆ alkylene, C₁₋₆ alkenylene or - C₁₋₆ alkylene-O-C₁₋₆ alkylene-, which is optionally deuterated, up to fully deuterated;
A is selected from a chemical bond, -C(O)- or -NHC(O)-;
T is -(CRᵢRⱼ)_{q}-, and 1, 2, 3, 4 or 5 non-adjacent CRᵢRⱼ in the T may be replaced by a heteroatom selected from -O-, -S- and -NRᵢ-; T is optionally deuterated, up to fully deuterated;
Rᵢ and Rⱼ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rᵢ and Rⱼ on any one or more carbon atoms in T, together with the same or different carbon atoms to which they are attached, form C₃₋₁₀ cycloalkyl, 3- to 10-membered heterocyclyl, C₆₋₁₀ aryl or 5- to 10-membered heteroaryl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-, and 1, 2 or 3 non-adjacent CRₖRₘ in the B may be replaced by a heteroatom selected from -O-, -Sand -NRₖ-; the B is optionally deuterated, up to fully deuterated;
Rₖ and Rₘ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₖ and Rₘ on any one or more carbon atoms in B, together with the same or different carbon atoms to which they are attached, form C₃₋₁₀ cycloalkyl, 3- to 10-membered heterocyclyl, C₆₋₁₀ aryl or 5- to 10-membered heteroaryl;
P is selected from a chemical bond or 1, 2 or 3 amino acid residues;
R is selected from 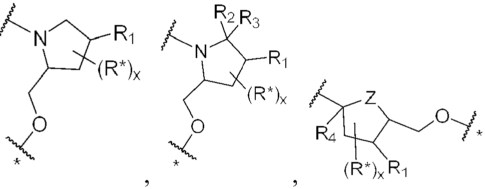 or 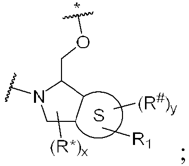
ring S is selected from C₃₋₁₀ cycloalkyl, 3- to 10-membered heterocyclyl, C₆₋₁₀ aryl or 5- to 10-membered heteroaryl;
R₁ is selected from a hydroxyl-containing group, such as -OR₀, -C₁₋₆ alkylene-OR₀, -C₁₋₆ alkenylene-OR₀ or -C₁₋₆ alkynylene-OR₀, wherein R₀ is H, D, a hydroxyl protecting group or a solid-phase carrier;
the hydroxyl protecting group is selected from trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), - C(O)CH₂CH₂C(O)OH or 4,4'-dimethoxytrityl (DMTr), preferably - C(O)CH₂CH₂C(O)OH or 4,4'-dimethoxytrityl (DMTr);
R₂ and R₃ are independently selected from H, D, halogen, C₁₋₆ alkyl, C₁₋₆ alkoxy, C₁₋₆ haloalkyl, C₁₋₆ alkenyl or C₁₋₆ alkynyl; alternatively, R₂ and R₃, together with the same carbon atom to which they are attached, form C₃₋₁₀ cycloalkyl, 3- to 10-membered heterocyclyl, C₆₋₁₀ aryl or 5- to 10-membered heteroaryl; R₂, R₃ and the ring formed thereby are optionally deuterated, up to fully deuterated;
R₄ is selected from H, D, halogen, C₁₋₆ alkyl, C₁₋₆ alkoxy, C₁₋₆ haloalkyl, C₁₋₆ alkenyl or C₁₋₆ alkynyl, and R₄ is optionally deuterated, up to fully deuterated;
Z is selected from O, NH or CH₂;
R* and R^{#} are independently selected from H, D, halogen, C₁₋₆ alkyl, C₁₋₆ haloalkyl, C₁₋₆ alkoxy, C₃₋₁₀ cycloalkyl, 3- to 10-membered heterocyclyl, C₆₋₁₀ aryl or 5- to 10-membered heteroaryl;
x and y are independently selected from 0, 1, 2, 3, 4 or 5;
the above-mentioned W, A, T, B, P and R are each optionally further substituted with 1, 2, 3, 4, 5, 6, 7, 8 or more substituents selected from the following: H, D, OH, CN, NH₂, NO₂, oxo, thio, halogen, C₁₋₆ alkyl, C₁₋₆ haloalkyl, C₁₋₆ alkoxy, C₃₋₁₀ cycloalkyl, 3- to 10-membered heterocyclyl, C₆₋₁₀ aryl or 5- to 10-membered heteroaryl;
provided that, when R is 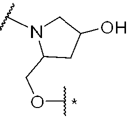 (including a stereoisomeric form thereof, such as 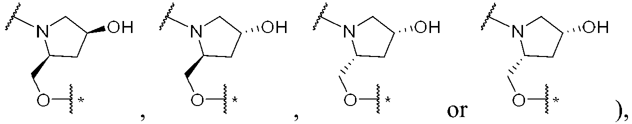 at least one pair among Rₐ and R_{b}, R_{c} and R_{d}, Rₑ and R_{f}, R_{g} and Rₕ, Rᵢ and Rⱼ, and Rₖ and Rₘ, together with the same or different carbon atoms to which they are attached, forms C₃₋₁₀ cycloalkyl, 3- to 10-membered heterocyclyl, C₆₋₁₀ aryl or 5- to 10-membered heteroaryl.
W

In one embodiment, W is H; in another embodiment, W is D; in another embodiment, W is 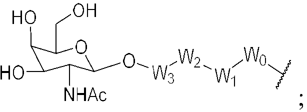 in another embodiment, W is 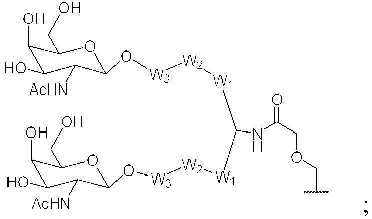 in another embodiment, W is 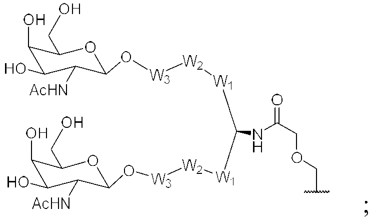 in another embodiment, W is 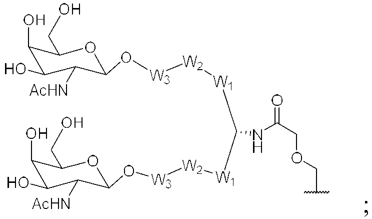 in another embodiment, W is 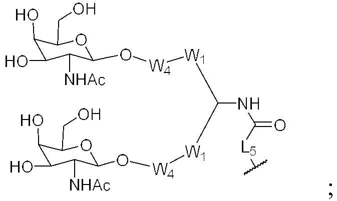 in another embodiment, W is 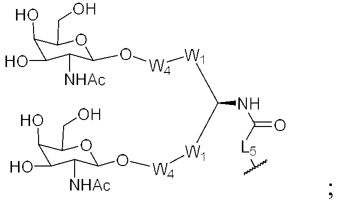 in another embodiment, W is

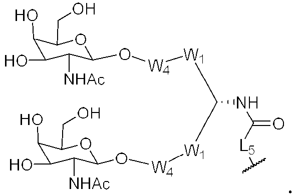

In one embodiment, W is H; in another embodiment, W is 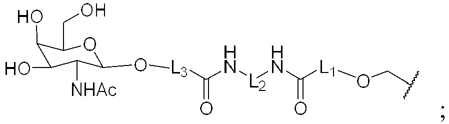 in another embodiment, W is 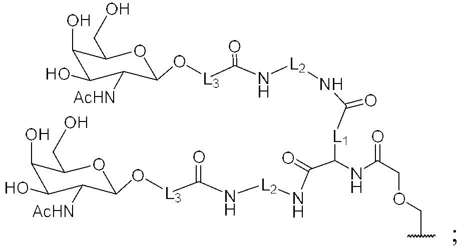 in another embodiment, W is 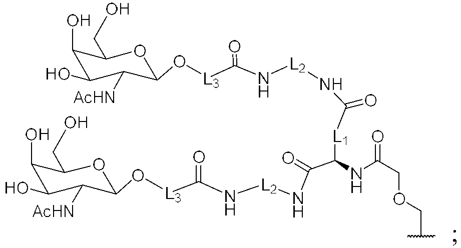 in another embodiment, W is 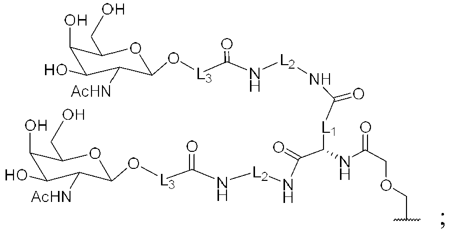 in another embodiment, W is 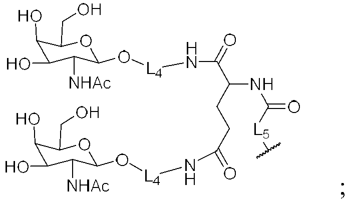 in another embodiment, W is 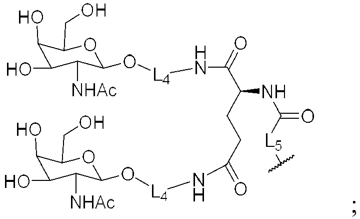 in another embodiment, W is

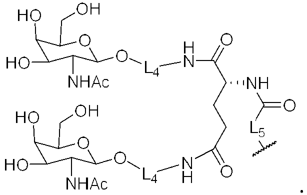

In a specific embodiment, W is 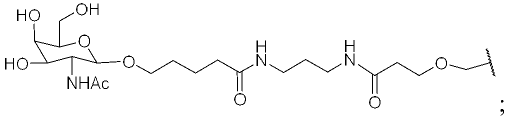 in another specific embodiment, W is 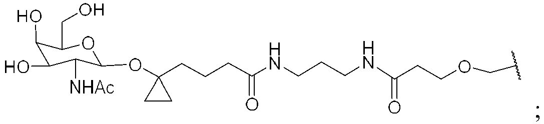 in another specific embodiment, W is 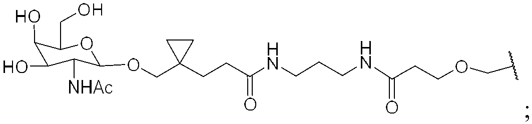 in another specific embodiment, W is 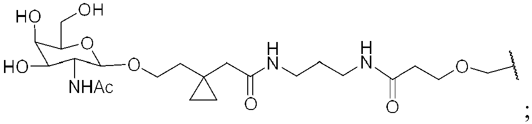 in another specific embodiment, W is 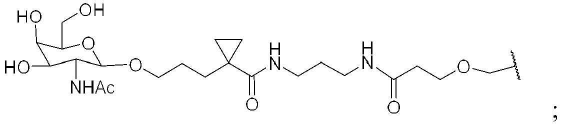 in another specific embodiment, W is 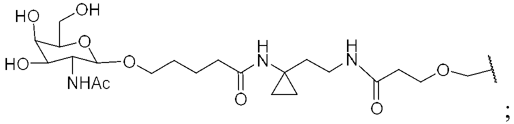 in another specific embodiment, W is 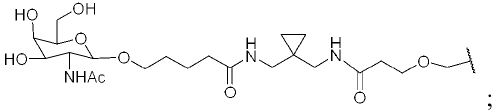 in another specific embodiment, W is 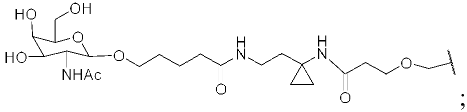 in another specific embodiment, W is 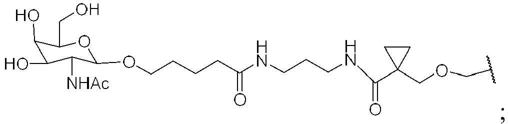 in another specific embodiment, W is 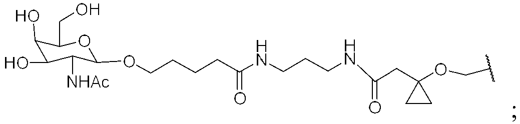 in another specific embodiment, W is 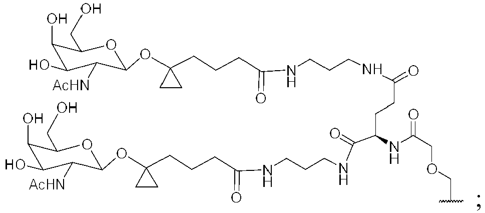 in another specific embodiment, W is 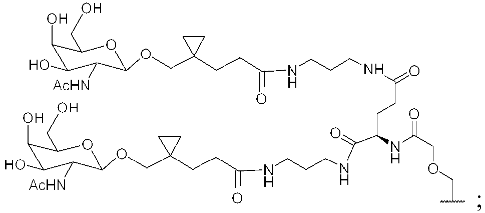 in another specific embodiment, W is 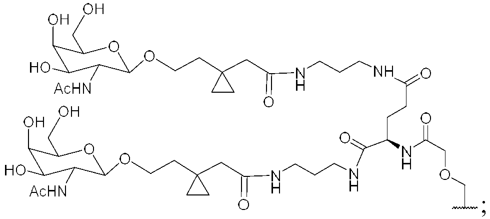 in another specific embodiment, W is 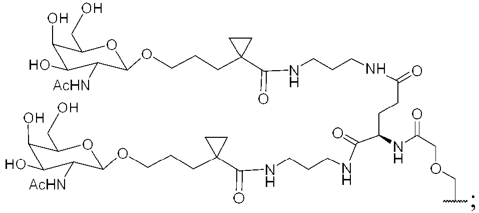 in another specific embodiment, W is 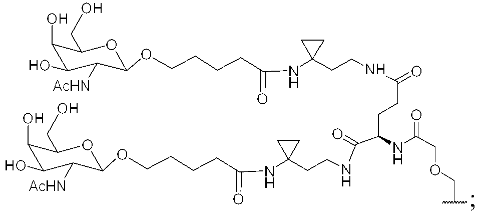 in another specific embodiment, W is 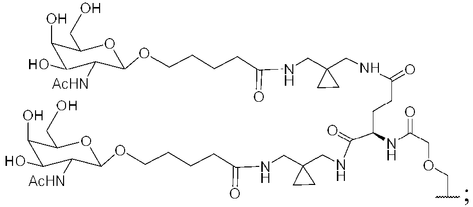 in another specific embodiment, W is 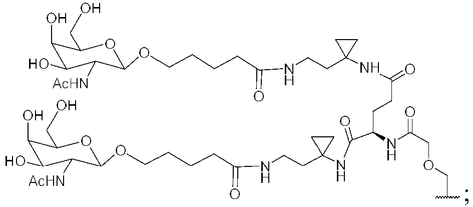 in another specific embodiment, W is 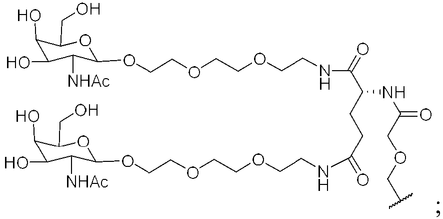 in another specific embodiment, W is 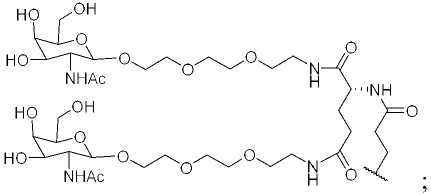 in another specific embodiment, W is 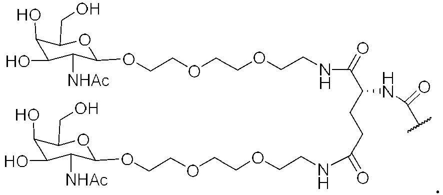 W'

In one embodiment, W' is H; in another embodiment, W' is D; in another embodiment, W' is 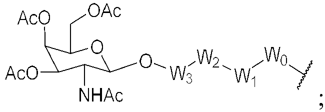 in another embodiment, W' is 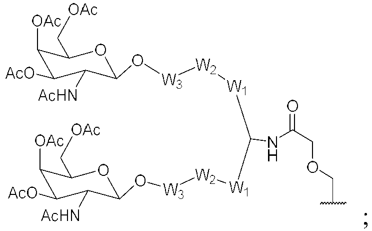 in another embodiment, W' is 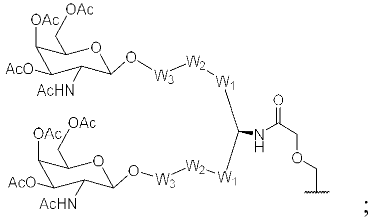 in another embodiment, W' is 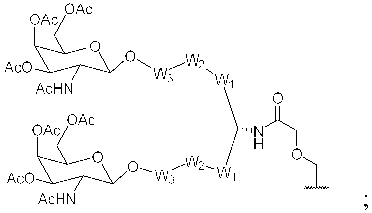 in another embodiment, W' is 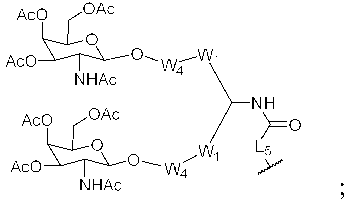 in another embodiment, W' is 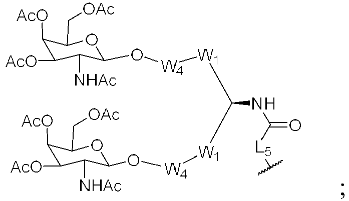 in another embodiment, W' is

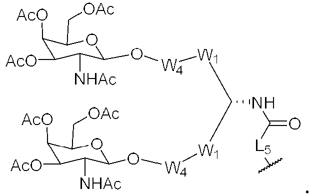

In one embodiment, W' is H; in another embodiment, W' is D; in another embodiment, W' is 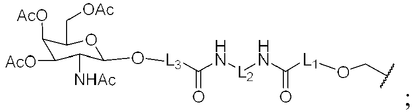 in another embodiment, W' is 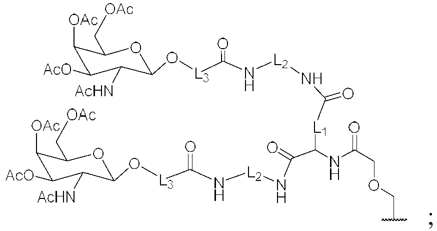 in another embodiment, W' is 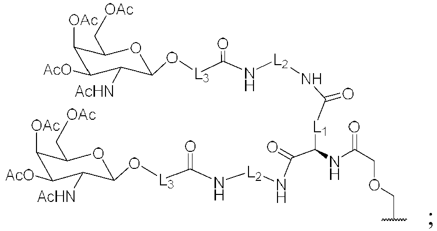 in another embodiment, W' is 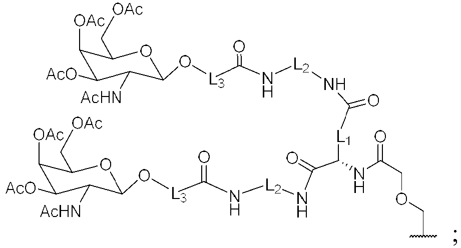 in another embodiment, W' is 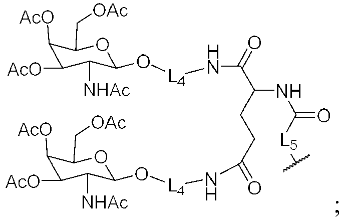 in another embodiment, W' is 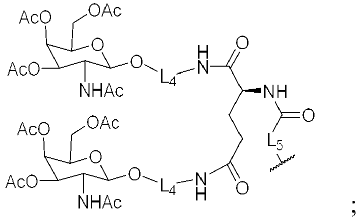 in another embodiment, W' is

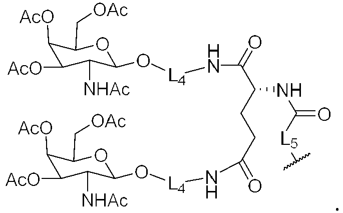

In a specific embodiment, W' is 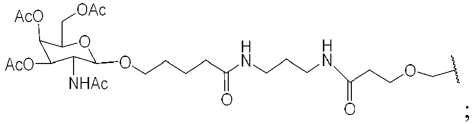 in another specific embodiment, W' is 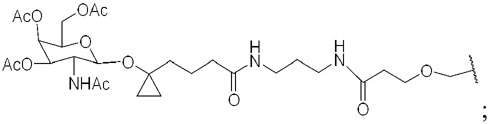 in another specific embodiment, W' is 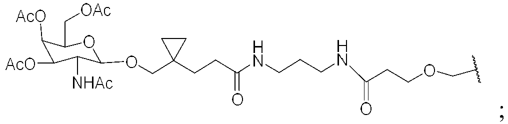 in another specific embodiment, W' is 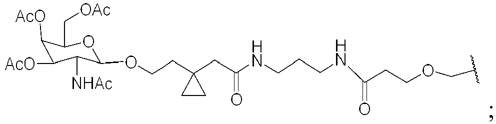 in another specific embodiment, W' is 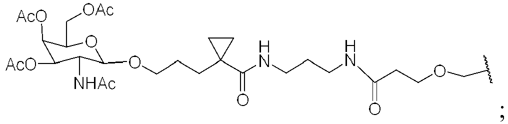 in another specific embodiment, W' is 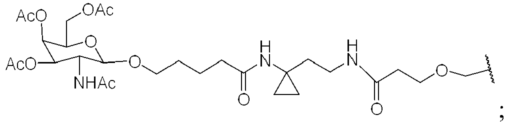 in another specific embodiment, W' is 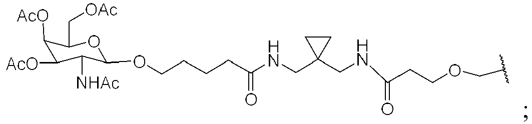 in another specific embodiment, W' is 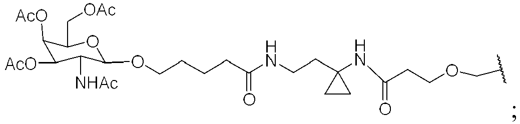 in another specific embodiment, W' is 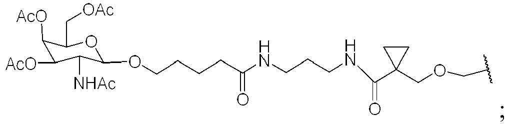 in another specific embodiment, W' is 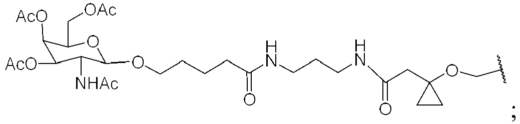 in another specific embodiment, W' is 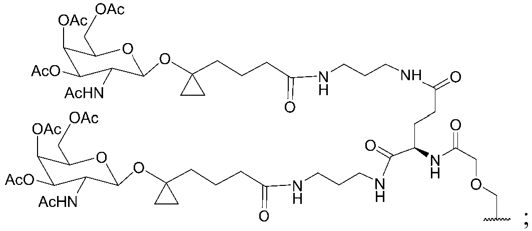 in another specific embodiment, W' is 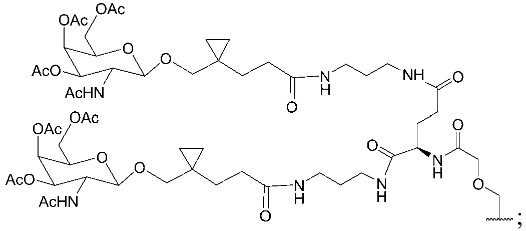 in another specific embodiment, W' is 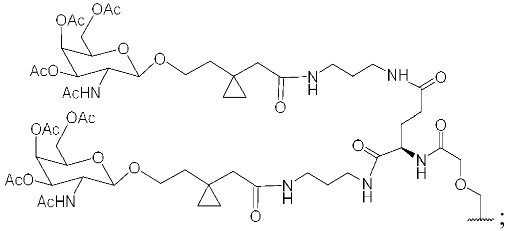 in another specific embodiment, W' is 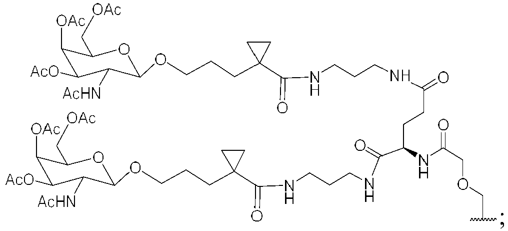 in another specific embodiment, W' is 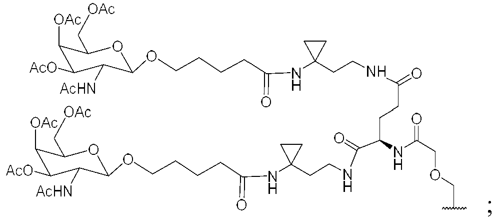 in another specific embodiment, W' is 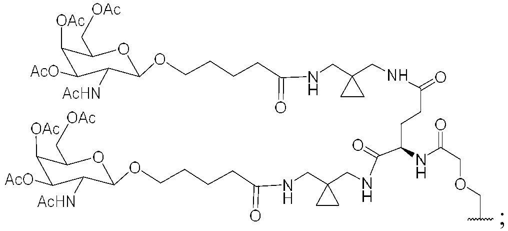 in another specific embodiment, W' is 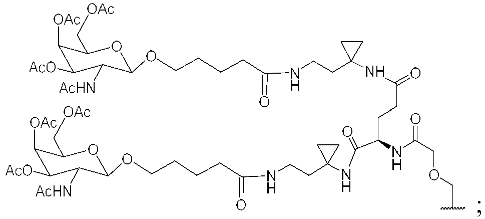 in another specific embodiment, W' is 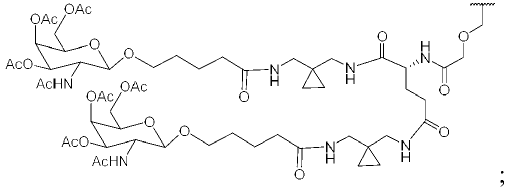 in another specific embodiment, W' is 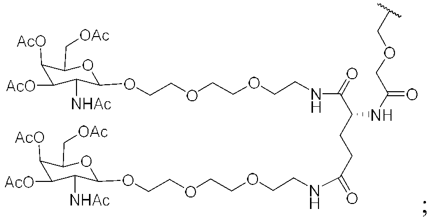 in another specific embodiment, W' is 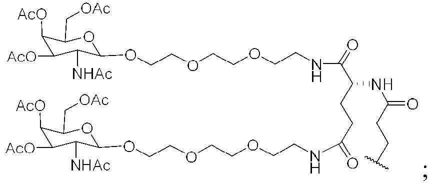 in another specific embodiment, W' is 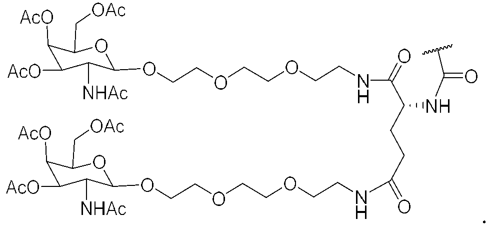 W₀

In one embodiment, W₀ is a chemical bond; in another embodiment, W₀ is C₁₋₆ alkylene; in another embodiment, W₀ is C₁₋₆ alkyleneoxy; in another embodiment, W₀ is -O-C₁₋₆ alkylene-O-C₁₋₆ alkylene-; in another embodiment, W₀ is -O-C₁₋₆ alkylene-C₁₋₆ alkylene-O-; in another embodiment, W₀ is -O-C₁₋₆ alkylene-O-C₁₋₆ alkylene-O-; in another embodiment, W₀ is C₁₋₆ haloalkylene; in another embodiment, W₀ is C₁₋₆ alkenylene; in another embodiment, W₀ is C₁₋₆ alkynylene.

In a specific embodiment, W₀ is a chemical bond; in another specific embodiment, W₀ is -OCH₂-; in another specific embodiment, W₀ is -OCH₂CH₂-; in another specific embodiment, W₀ is -OCH₂OCH₂-; in another specific embodiment, W₀ is -OCH₂CH₂CH₂-; in another specific embodiment, W₀ is -OCH₂CH₂CH₂CH₂-; in another specific embodiment, W₀ is -CH₂OCH₂CH₂CH₂-; in another specific embodiment, W₀ is -CH₂CH₂OCH₂CH₂-; in another specific embodiment, W₀ is - CH₂CH₂CH₂OCH₂-; in another specific embodiment, W₀ is -CH₂CH₂OCH₂CH₂O-.

In a preferred embodiment, W₀ is -OCH₂-.

Each of the above-mentioned W₀ is the same or different, and is optionally deuterated, up to fully deuterated.
W₁

Each W₀ is the same or different. In one embodiment, W₁ is a chemical bond; in another embodiment, W₁ is -O-; in another embodiment, W₁ is -O-L₁-; in another embodiment, W₁ is -C(O)-; in another embodiment, W₁ is -C(O)-L₁-; in another embodiment, W₁ is -C(O)O-; in another embodiment, W₁ is -C(O)O-L₁-.

Each of the above-mentioned W₀ is the same or different, and is optionally deuterated, up to fully deuterated.
W₂

In one embodiment, W₂ is -L₂-NH-; in another embodiment, W₂ is -L₂-O-; in another embodiment, W₂ is -L₂-C(O)-; in another embodiment, W₂ is -L₂-OC(O)-.

Each of the above-mentioned W₂ is the same or different, and is optionally deuterated, up to fully deuterated.
W₃

In one embodiment, W₃ is -L₃-C(O)NH-; in another embodiment, W₃ is -L₃-NHC(O)-; in another embodiment, W₃ is -L₃-OC(O)NH-; in another embodiment, W₃ is -L₃-NHC(O)O-.

Each of the above-mentioned W₃ is the same or different, and is optionally deuterated, up to fully deuterated.
W₄

In one embodiment, W₄ is -L₄-NHC(O)-; in another embodiment, W₄ is -L₄-NHC(O)-(CH₂)₁₋₆-; in another embodiment, W₄ is -L₄-NHC(O)O-; in another embodiment, W₄ is -L₄-NHC(O)O-(CH₂)₁₋₆-; in another embodiment, W₄ is -L₄-C(O)NH-; in another embodiment, W₄ is -L₄-C(O)NH-(CH₂)₁₋₆-; in another embodiment, W₄ is -L₄-OC(O)NH-; in another embodiment, W₄ is -L₄-OC(O)NH-(CH₂)₁₋₆-.

Each of the above-mentioned W₄ is the same or different, and is optionally deuterated, up to fully deuterated.
L₁

In one embodiment, L₁ is -(CRₐR_{b})ₘ-.

In one embodiment, CRₐR_{b} in the L₁ is not replaced by a heteroatom; in another embodiment, 1, 2, 3, 4 or 5 non-adjacent CRₐR_{b} in the L₁ may be replaced by a heteroatom selected from -O-, -S- and -NRₐ-.

In one embodiment, Rₐ is H; in another embodiment, Rₐ is D; in another embodiment, Rₐ is halogen; in another embodiment, Rₐ is C₁₋₆ alkyl; in another embodiment, Rₐ is C₁₋₆ alkoxy; in another embodiment, Rₐ is C₁₋₆ haloalkyl; in another embodiment, Rₐ is C₁₋₆ alkenyl; in another embodiment, Rₐ is C₁₋₆ alkynyl.

In one embodiment, R_{b} is H; in another embodiment, R_{b} is D; in another embodiment, R_{b} is halogen; in another embodiment, R_{b} is C₁₋₆ alkyl; in another embodiment, R_{b} is C₁₋₆ alkoxy; in another embodiment, R_{b} is C₁₋₆ haloalkyl; in another embodiment, R_{b} is C₁₋₆ alkenyl; in another embodiment, R_{b} is C₁₋₆ alkynyl.

In one embodiment, Rₐ and R_{b} on any one carbon atom in L₁, together with the same carbon atom to which they are attached, form a ring; in another embodiment, Rₐ and R_{b} on any more carbon atoms in L₁, together with the same carbon atom to which they are attached, form a ring; in another embodiment, Rₐ and R_{b} on any more carbon atoms in L₁, together with the different carbon atoms to which they are attached, form a ring; the above-mentioned rings are selected from C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl.

In a preferred embodiment, Rₐ and R_{b} on any one or more carbon atoms in L₁, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl, such as cyclopropyl.

In a specific embodiment, L₁ is -(CRₐR_{b})₂-.

In a more specific embodiment, L₁ is -CH₂CH₂-; in another more specific embodiment, L₁ is

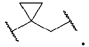

The above-mentioned L₁ is optionally deuterated, up to fully deuterated.
L₂

In one embodiment, L₂ is -(CR_{c}R_{d})ₙ-.

In one embodiment, CR_{c}R_{d} in the L₂ is not replaced by a heteroatom; in another embodiment, 1, 2, 3, 4 or 5 non-adjacent CR_{c}R_{d} in the L₂ may be replaced by a heteroatom selected from -O-, -S- and -NR_{c}-.

In one embodiment, R_{c} is H; in another embodiment, R_{c} is D; in another embodiment, R_{c} is halogen; in another embodiment, R_{c} is C₁₋₆ alkyl; in another embodiment, R_{c} is C₁₋₆ alkoxy; in another embodiment, R_{c} is C₁₋₆ haloalkyl; in another embodiment, R_{c} is C₁₋₆ alkenyl; in another embodiment, R_{c} is C₁₋₆ alkynyl.

In one embodiment, R_{d} is H; in another embodiment, R_{d} is D; in another embodiment, R_{d} is halogen; in another embodiment, R_{d} is C₁₋₆ alkyl; in another embodiment, R_{d} is C₁₋₆ alkoxy; in another embodiment, R_{d} is C₁₋₆ haloalkyl; in another embodiment, R_{d} is C₁₋₆ alkenyl; in another embodiment, R_{d} is C₁₋₆ alkynyl.

In one embodiment, R_{c} and R_{d} on any one carbon atom in L₂, together with the same carbon atom to which they are attached, form a ring; in another embodiment, R_{c} and R_{d} on any more carbon atoms in L₂, together with the same carbon atom to which they are attached, form a ring; in another embodiment, R_{c} and R_{d} on any more carbon atoms in L₂, together with the different carbon atoms to which they are attached, form a ring; the above-mentioned rings are selected from C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl.

In a preferred embodiment, R_{c} and R_{d} on any one or more carbon atoms in L₂, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl, such as cyclopropyl.

In a specific embodiment, L₂ is -(CR_{c}R_{d})₃-.

In a more specific embodiment, L₂ is -CH₂CH₂CH₂-; in another more specific embodiment, L₂ is 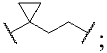 in another more specific embodiment, L₂ is 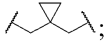 in another more specific embodiment, L₂ is

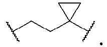

The above-mentioned L₂ is optionally deuterated, up to fully deuterated.
L₃

In one embodiment, L₃ is -(CRₑR_{f})ₕ-.

In one embodiment, CRₑR_{f} in the L₃ is not replaced by a heteroatom; in another embodiment, 1, 2, 3, 4 or 5 non-adjacent CRₑR_{f} in the L₃ may be replaced by a heteroatom selected from -O-, -S- and -NRₑ-.

In one embodiment, Rₑ is H; in another embodiment, Rₑ is D; in another embodiment, Rₑ is halogen; in another embodiment, Rₑ is C₁₋₆ alkyl; in another embodiment, Rₑ is C₁₋₆ alkoxy; in another embodiment, Rₑ is C₁₋₆ haloalkyl; in another embodiment, Rₑ is C₁₋₆ alkenyl; in another embodiment, Rₑ is C₁₋₆ alkynyl.

In one embodiment, R_{f} is H; in another embodiment, R_{f} is D; in another embodiment, R_{f} is halogen; in another embodiment, R_{f} is C₁₋₆ alkyl; in another embodiment, R_{f} is C₁₋₆ alkoxy; in another embodiment, R_{f} is C₁₋₆ haloalkyl; in another embodiment, R_{f} is C₁₋₆ alkenyl; in another embodiment, R_{f} is C₁₋₆ alkynyl.

In one embodiment, Rₑ and R_{f} on any one carbon atom in L₃, together with the same carbon atom to which they are attached, form a ring; in another embodiment, Rₑ and R_{f} on any more carbon atoms in L₃, together with the same carbon atom to which they are attached, form a ring; in another embodiment, Rₑ and R_{f} on any more carbon atoms in L₃, together with the different carbon atoms to which they are attached, form a ring; the above-mentioned rings are selected from C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl.

In a preferred embodiment, Rₑ and R_{f} on any one or more carbon atoms in L₃, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl, such as cyclopropyl.

In a specific embodiment, L₃ is -(CRₑR_{f})₄-.

In a more specific embodiment, L₃ is -CH₂CH₂CH₂CH₂-; in another more specific embodiment, L₃ is

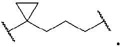

The above-mentioned L₃ is optionally deuterated, up to fully deuterated.
L₄

In one embodiment, L₄ is -(CR_{g}Rₕ)ₖ-; in another embodiment, L₄ is - (CR_{g}Rₕ)₁₋₆-(OCR_{g}Rₕ-CR_{g}Rₕ)ₖ-; in another embodiment, L₄ is -(CR_{g}Rₕ)₁₋₆-(NH-CR_{g}Rₕ-CR_{g}Rₕ)ₖ-.

In one embodiment, CR_{g}Rₕ in the L₄ is not replaced by a heteroatom; in another embodiment, 1, 2, 3, 4 or 5 non-adjacent CR_{g}Rₕ in the L₄ may be replaced by a heteroatom selected from -O-, -S- and -NR_{g}-.

In one embodiment, R_{g} is H; in another embodiment, R_{g} is D; in another embodiment, R_{g} is halogen; in another embodiment, R_{g} is C₁₋₆ alkyl; in another embodiment, R_{g} is C₁₋₆ alkoxy; in another embodiment, R_{g} is C₁₋₆ haloalkyl; in another embodiment, R_{g} is C₁₋₆ alkenyl; in another embodiment, R_{g} is C₁₋₆ alkynyl.

In one embodiment, Rₕ is H; in another embodiment, Rₕ is D; in another embodiment, Rₕ is halogen; in another embodiment, Rₕ is C₁₋₆ alkyl; in another embodiment, Rₕ is C₁₋₆ alkoxy; in another embodiment, Rₕ is C₁₋₆ haloalkyl; in another embodiment, Rₕ is C₁₋₆ alkenyl; in another embodiment, Rₕ is C₁₋₆ alkynyl.

In one embodiment, R_{g} and Rₕ on any one carbon atom in L₄, together with the same carbon atom to which they are attached, form a ring; in another embodiment, R_{g} and Rₕ on any more carbon atoms in L₄, together with the same carbon atom to which they are attached, form a ring; in another embodiment, R_{g} and Rₕ on any more carbon atoms in L₄, together with the different carbon atoms to which they are attached, form a ring; the above-mentioned rings are selected from C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl.

In a preferred embodiment, R_{g} and Rₕ on any one or more carbon atoms in L₄, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl, such as cyclopropyl.

In a specific embodiment, L₄ is -(CR_{g}Rₕ)₂-(OCR_{g}Rₕ-CR_{g}Rₕ)₂-.

In a more specific embodiment, L₄ is

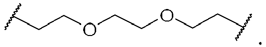

The above-mentioned L₄ is optionally deuterated, up to fully deuterated.
L₅

In one embodiment, L₅ is a chemical bond; in another embodiment, L₅ is C₁₋₆ alkylene; in another embodiment, L₅ is C₁₋₆ alkenylene; in another embodiment, L₅ is -C₁₋₆ alkylene-O-C₁₋₆ alkylene-.

In a specific embodiment, L₅ is a chemical bond; in another specific embodiment, L₅ is -CH₂CH₂-; in another specific embodiment, L₅ is -CH₂OCH₂-.

The above-mentioned L₅ is optionally deuterated, up to fully deuterated.
A

In one embodiment, A is a chemical bond; in another embodiment, A is -C(O)-; in another embodiment, A is -NHC(O)-.
T

In one embodiment, T is -(CRᵢRⱼ)_{q}-.

In one embodiment, CRᵢRⱼ in the T is not replaced by a heteroatom; in another embodiment, 1, 2, 3, 4 or 5 non-adjacent CRᵢRⱼ in the T may be replaced by a heteroatom selected from -O-, -S- and -NRᵢ-.

In one embodiment, Rᵢ is H; in another embodiment, Rᵢ is D; in another embodiment, Rᵢ is halogen; in another embodiment, Rᵢ is C₁₋₆ alkyl; in another embodiment, Rᵢ is C₁₋₆ alkoxy; in another embodiment, Rᵢ is C₁₋₆ haloalkyl; in another embodiment, Rᵢ is C₁₋₆ alkenyl; in another embodiment, Rᵢ is C₁₋₆ alkynyl.

In one embodiment, Rⱼ is H; in another embodiment, Rⱼ is D; in another embodiment, Rⱼ is halogen; in another embodiment, Rⱼ is C₁₋₆ alkyl; in another embodiment, Rⱼ is C₁₋₆ alkoxy; in another embodiment, Rⱼ is C₁₋₆ haloalkyl; in another embodiment, Rⱼ is C₁₋₆ alkenyl; in another embodiment, Rⱼ is C₁₋₆ alkynyl.

In one embodiment, Rᵢ and Rⱼ on any one carbon atom in T, together with the same carbon atom to which they are attached, form a ring; in another embodiment, Rᵢ and Rⱼ on any more carbon atoms in T, together with the same carbon atom to which they are attached, form a ring; in another embodiment, Rᵢ and Rⱼ on any more carbon atoms in T, together with the different carbon atoms to which they are attached, form a ring; the above-mentioned rings are selected from C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl.

In a specific embodiment, T is -(CRᵢRⱼ)₇₋₁₀-.

In a more specific embodiment, T is 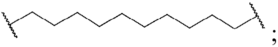 in another more specific embodiment, T is 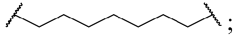 in another more specific embodiment, T is

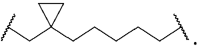

The above-mentioned T is optionally deuterated, up to fully deuterated.
m, n, h, k and q

In one embodiment, m is selected from 0, 1, 2, 3, 4, 5, 6, 7, 8, 9 or 10.

In one embodiment, n is selected from 0, 1, 2, 3, 4, 5, 6, 7, 8, 9 or 10.

In one embodiment, h is selected from 0, 1, 2, 3, 4, 5, 6, 7, 8, 9 or 10.

In one embodiment, k is selected from 0, 1, 2, 3, 4, 5, 6, 7, 8, 9 or 10.

In one embodiment, q is selected from 0, 1, 2, 3, 4, 5, 6, 7, 8, 9, 10, 11, 12, 13, 14 or 15.
B

In one embodiment, B is -C(O)-(CRₖRₘ)₀₋₆-; in another embodiment, B is -NH-(CRₖRₘ)₀₋₆-; in another embodiment, B is -OC(O)-(CRₖRₘ)₀₋₆-; in another embodiment, B is -NHC(O)-(CRₖRₘ)₀₋₆-; in another embodiment, B is -C(O)NH-(CRₖRₘ)₀₋₆-.

In one embodiment, CRₖRₘ in the B is not replaced by a heteroatom; in another embodiment, 1, 2, 3, 4 or 5 non-adjacent CRₖRₘ in the B may be replaced by a heteroatom selected from -O-, -S- and -NRₖ-.

In one embodiment, Rₖ is H; in another embodiment, Rₖ is D; in another embodiment, Rₖ is halogen; in another embodiment, Rₖ is C₁₋₆ alkyl; in another embodiment, Rₖ is C₁₋₆ alkoxy; in another embodiment, Rₖ is C₁₋₆ haloalkyl; in another embodiment, Rₖ is C₁₋₆ alkenyl; in another embodiment, Rₖ is C₁₋₆ alkynyl.

In one embodiment, Rₘ is H; in another embodiment, Rₘ is D; in another embodiment, Rₘ is halogen; in another embodiment, Rₘ is C₁₋₆ alkyl; in another embodiment, Rₘ is C₁₋₆ alkoxy; in another embodiment, Rₘ is C₁₋₆ haloalkyl; in another embodiment, Rₘ is C₁₋₆ alkenyl; in another embodiment, Rₘ is C₁₋₆ alkynyl.

In one embodiment, Rₖ and Rₘ on any one carbon atom in B, together with the same carbon atom to which they are attached, form a ring; in another embodiment, Rₖ and Rₘ on any more carbon atoms in B, together with the same carbon atom to which they are attached, form a ring; in another embodiment, Rₖ and Rₘ on any more carbon atoms in B, together with the different carbon atoms to which they are attached, form a ring; the above-mentioned rings are selected from C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl, more preferably C₃₋₅ cycloalkyl.

In a specific embodiment, B is -C(O)-; in another specific embodiment, B is -C(O)NH-; in another specific embodiment, B is -C(O)NH-CRₖRₘ-;
in a more specific embodiment, B is -C(O)-; in another specific embodiment, B is 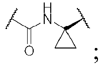 in another specific embodiment, B is

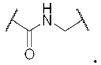

The B is optionally deuterated, up to fully deuterated.
P

In one embodiment, P is a chemical bond; in another embodiment, P is 1 amino acid residue; in another embodiment, P is 2 amino acid residues; in another embodiment, P is 3 amino acid residues.

In a specific embodiment, P is a chemical bond; in another embodiment, P is -Cit- ( 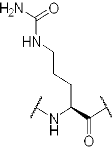 ); in another embodiment, P is -Arg-; in another embodiment, P is -Phe- ( 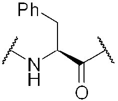 ).
R

In one embodiment, R is 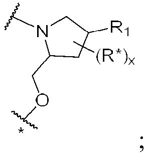 in another embodiment, R is 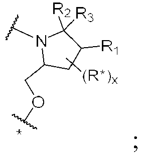 in another embodiment, R is 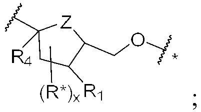 in another embodiment, R is

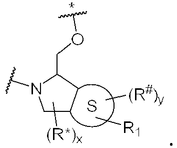

In a specific embodiment, R is 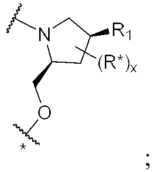 in another specific embodiment, R is 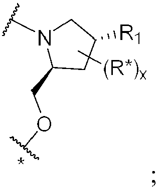
in another specific embodiment, R is 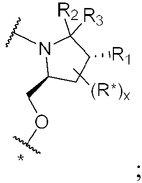 in another specific embodiment, R is 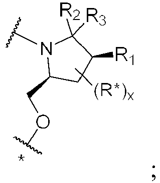 in another specific embodiment, R is 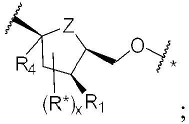 in another specific embodiment, R is 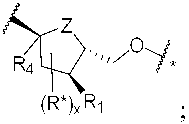
in another specific embodiment, R is 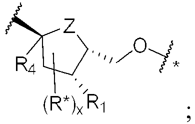 in another specific embodiment, R is 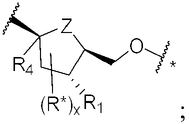
in another specific embodiment, R is 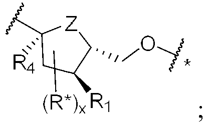 in another specific embodiment, R is 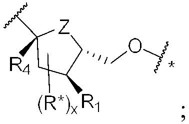 in another specific embodiment, R is

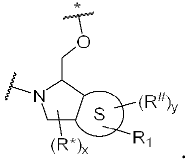

In a more specific embodiment, R is 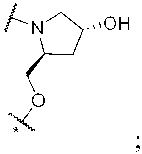 in another more specific embodiment, R is 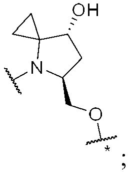
in another more specific embodiment, R is 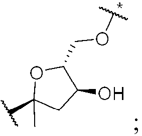 in another more specific embodiment, R is 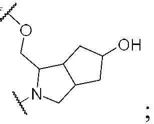
in another more specific embodiment, R is 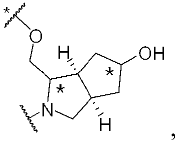 and * denotes a chiral centre, which is independently selected from (S)- or (R)-configuration, or a mixture thereof; in another more specific embodiment, R is 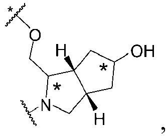 and * denotes a chiral centre, which is independently selected from (S)- or (R)-configuration, or a mixture thereof; in another more specific embodiment, R is 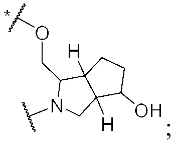 in another more specific embodiment, R is 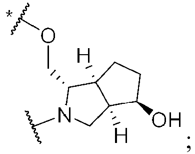 in another more specific embodiment, R is 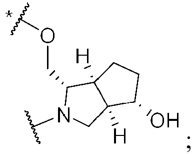 in another more specific embodiment, R is 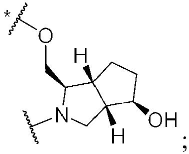 in another more specific embodiment, R is 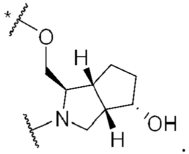
R'

In one embodiment, R' is 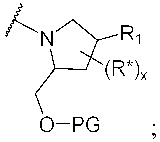 in another embodiment, R' is 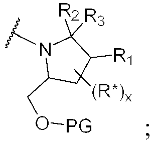 in another embodiment, R' is 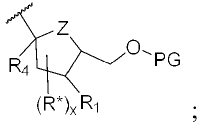 in another embodiment, R' is

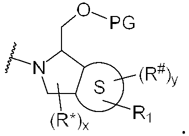

In a specific embodiment, R' is 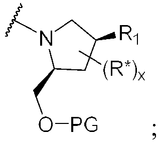 in another specific embodiment, R' is 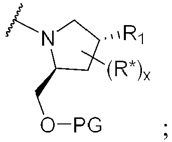 in another specific embodiment, R' is 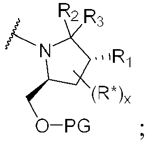 in another specific embodiment, R' is 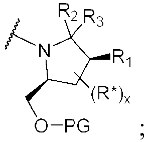 in another specific embodiment, R' is 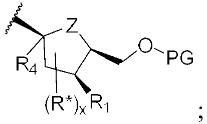 in another specific embodiment, R' is 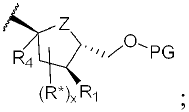 in another specific embodiment, R' is 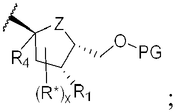 in another specific embodiment, R' is 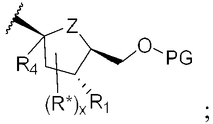 in another specific embodiment, R' is 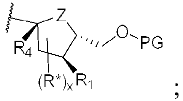 in another specific embodiment, R' is 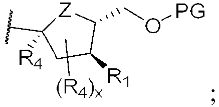 in another specific embodiment, R' is

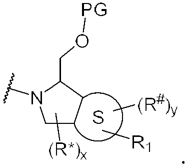

In a more specific embodiment, R' is 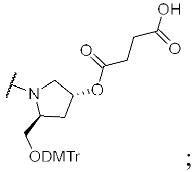 in another more specific embodiment, R' is 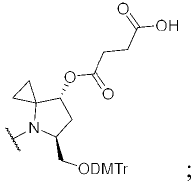 in another more specific embodiment, R' is 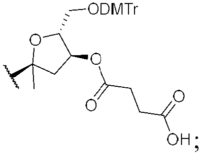 in another more specific embodiment, R' is 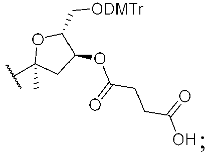 in another more specific embodiment, R' is 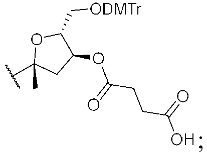 in another more specific embodiment, R' is 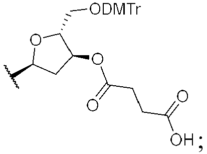 in another more specific embodiment, R' is 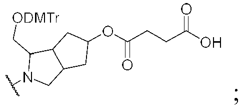 in another more specific embodiment, R' is 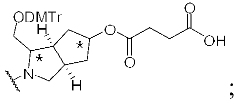 in another more specific embodiment, R' is 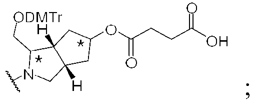 in another more specific embodiment, R' is 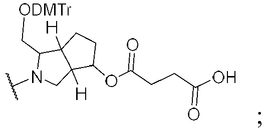 in another more specific embodiment, R' is 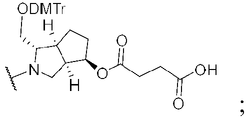 in another more specific embodiment, R' is 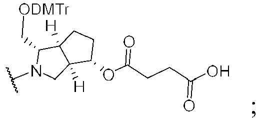 in another more specific embodiment, R' is 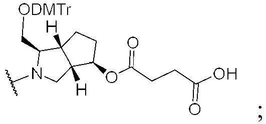 in another more specific embodiment, R' is 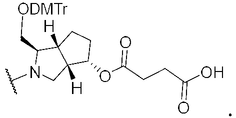 ring S

In one embodiment, ring S is C₃₋₁₀ cycloalkyl; in another embodiment, ring S is 3- to 10-membered heterocyclyl; in another embodiment, ring S is C₃₋₇ cycloalkyl; in another embodiment, ring S is 3- to 7-membered heterocyclyl; in another embodiment, ring S is C₅₋₆ cycloalkyl; in another embodiment, ring S is cyclopentyl; in another embodiment, ring S is C₆₋₁₀ aryl; in another embodiment, ring S is 5- to 10-membered heteroaryl.

In a preferred embodiment, ring S is cyclopentyl.
R₁

In one embodiment, R₁ is a hydroxyl-containing group; in another embodiment, R₁ is -OR₀; in another embodiment, R₁ is -C₁₋₆ alkylene-OR₀; in another embodiment, R₁ is -C₁₋₆ alkenylene-OR₀; in another embodiment, R₁ is -C₁-₆ alkynylene-OR₀; the above-mentioned R₀ is H, D, a hydroxyl protecting group or a solid-phase carrier.

In a specific embodiment, R₁ is OH.
R₂ and R₃

In one embodiment, R₂ is H; in another embodiment, R₂ is D; in another embodiment, R₂ is halogen; in another embodiment, R₂ is C₁₋₆ alkyl, such as C₁₋₄ alkyl; in another embodiment, R₂ is C₁₋₆ alkoxy; in another embodiment, R₂ is C₁₋₆ haloalkyl; in another embodiment, R₂ is C₁₋₆ alkenyl; in another embodiment, R₂ is C₁₋₆ alkynyl.

In one embodiment, R₃ is H; in another embodiment, R₃ is D; in another embodiment, R₃ is halogen; in another embodiment, R₃ is C₁₋₆ alkyl, such as C₁₋₄ alkyl; in another embodiment, R₃ is C₁₋₆ alkoxy; in another embodiment, R₃ is C₁₋₆ haloalkyl; in another embodiment, R₃ is C₁₋₆ alkenyl; in another embodiment, R₃ is C₁₋₆ alkynyl.

In one embodiment, R₂ and R₃, together with the same carbon atom to which they are attached, form C₃₋₁₀ cycloalkyl, 3- to 10-membered heterocyclyl, C₆₋₁₀ aryl or 5- to 10-membered heteroaryl; in another embodiment, R₂ and R₃, together with the same carbon atom to which they are attached, form C₃₋₅ cycloalkyl, such as cyclopropyl.

The above-mentioned R₂, R₃ and the ring formed thereby are optionally deuterated, up to fully deuterated.
R₄

In one embodiment, R₄ is H; in another embodiment, R₄ is D; in another embodiment, R₄ is halogen; in another embodiment, R₄ is C₁₋₆ alkyl, such as C₁₋₄ alkyl; in another embodiment, R₄ is C₁₋₆ alkoxy; in another embodiment, R₄ is C₁₋₆ haloalkyl; in another embodiment, R₄ is C₁₋₆ alkenyl; in another embodiment, R₄ is C₁₋₆ alkynyl.

In a preferred embodiment, R₄ is H or methyl.

The above-mentioned R₄ is optionally deuterated, up to fully deuterated.
Z

In one embodiment, Z is O; in another embodiment, Z is NH; in another embodiment, Z is CH₂.
R* and R^{#}

In one embodiment, R* is H; in another embodiment, R* is D; in another embodiment, R* is halogen; in another embodiment, R* is C₁₋₆ alkyl; in another embodiment, R* is C₁₋₆ haloalkyl; in another embodiment, R* is C₁₋₆ alkoxy; in another embodiment, R* is C₃₋₁₀ cycloalkyl; in another embodiment, R* is 3- to 10-membered heterocyclyl; in another embodiment, R* is C₆₋₁₀ aryl; in another embodiment, R* is 5- to 10-membered heteroaryl.

In one embodiment, R^{#} is H; in another embodiment, R^{#} is D; in another embodiment, R^{#} is halogen; in another embodiment, R^{#} is C₁₋₆ alkyl; in another embodiment, R^{#} is C₁₋₆ haloalkyl; in another embodiment, R^{#} is C₁₋₆ alkoxy; in another embodiment, R^{#} is C₃₋₁₀ cycloalkyl; in another embodiment, R^{#} is 3- to 10-membered heterocyclyl; in another embodiment, R^{#} is C₆₋₁₀ aryl; in another embodiment, R^{#} is 5- to 10-membered heteroaryl.
x and y

In one embodiment, x is selected from 0, 1, 2, 3, 4 or 5.

In one embodiment, y is selected from 0, 1, 2, 3, 4 or 5.

The above-mentioned W, A, T, B, P and R are each optionally further substituted with 1, 2, 3, 4, 5, 6, 7, 8 or more substituents selected from the following:

H, D, OH, CN, NH₂, NO₂, oxo, thio, halogen, C₁₋₆ alkyl, C₁₋₆ haloalkyl, C₁₋₆ alkoxy, C₃₋₁₀ cycloalkyl, 3- to 10-membered heterocyclyl, C₆₋₁₀ aryl or 5- to 10-membered heteroaryl.

Any technical solution in any of the above specific embodiments or any combination thereof may be combined with any technical solution in other specific embodiments or any combination thereof. For example, any technical solution for W or any combination thereof may be combined with any technical solution for A, T, B, P, R and the like, or any combination thereof. The present invention is intended to include combinations of all these technical solutions, which are not listed one by one due to space limitations.

In a more specific embodiment, the present invention provides the above-mentioned delivery ligand, wherein at least one pair among Rₐ and R_{b}, R_{c} and R_{d}, Rₑ and R_{f}, R_{g} and Rₕ, Rᵢ and Rⱼ, and Rₖ and Rₘ, together with the same or different carbon atoms to which they are attached, forms C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

In a more specific embodiment, the present invention provides the above-mentioned delivery ligand, wherein Rₐ and R_{b} on any one or more carbon atoms in L₁, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

In a more specific embodiment, the present invention provides the above-mentioned delivery ligand, wherein R_{c} and R_{d} on any one or more carbon atoms in L₂, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

In a more specific embodiment, the present invention provides the above-mentioned delivery ligand, wherein Rₑ and R_{f} on any one or more carbon atoms in L₃, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

In a more specific embodiment, the present invention provides the above-mentioned delivery ligand, wherein R_{g} and Rₕ on any one or more carbon atoms in L₄, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

In a more specific embodiment, the present invention provides the above-mentioned delivery ligand, wherein Rᵢ and Rⱼ on any one or more carbon atoms in T, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

In a more specific embodiment, the present invention provides the above-mentioned delivery ligand, wherein Rₖ and Rₘ on any one or more carbon atoms in B, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

In a more specific embodiment, the present invention provides the above-mentioned delivery ligand, wherein R is selected from 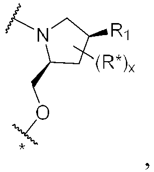 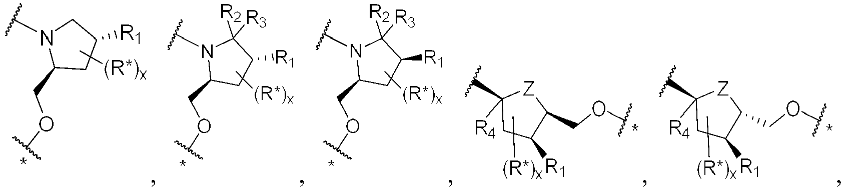 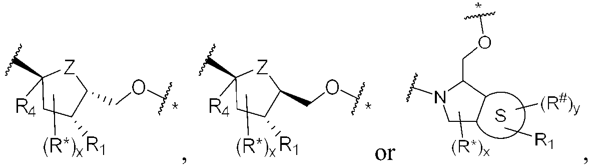 wherein ring S, R₁, R₂, R₃, R₄, Z, R*, R^{#}, x and y are as defined herein;
preferably, R is selected from 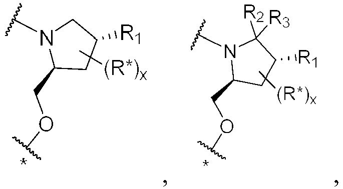 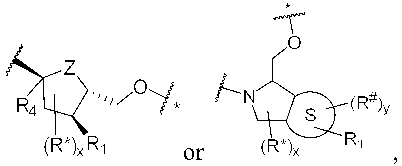 wherein ring S, R₁, R₂, R₃, R₄, Z, R*, R^{#}, x and y are as defined herein;
more preferably, R is selected from

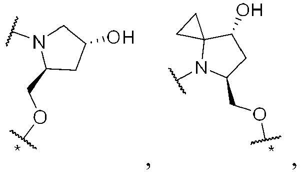

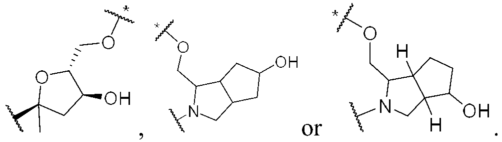

In a more specific embodiment, the present invention provides the above-mentioned delivery ligand, wherein R is selected from 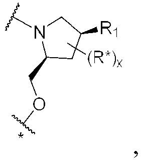 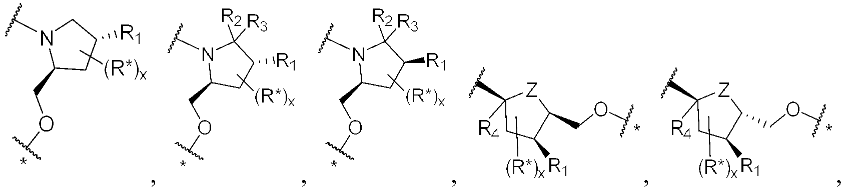 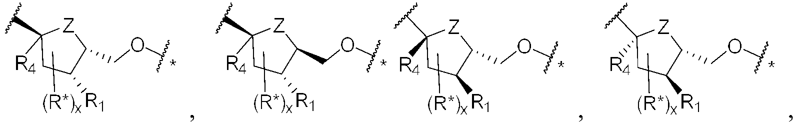 or 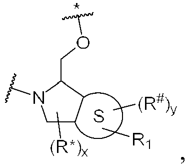 wherein ring S, R₁, R₂, R₃, R₄, Z, R*, R#, x and y are as defined herein;
preferably, R is selected from 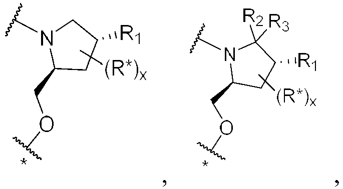 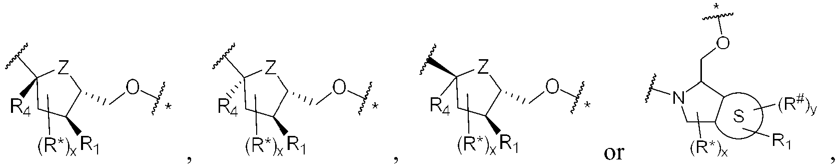 wherein ring S, R₁, R₂, R₃, R₄, Z, R*, R#, x and y are as defined in any one of claims 1-8;
more preferably, R is selected from 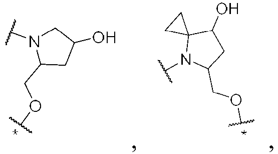 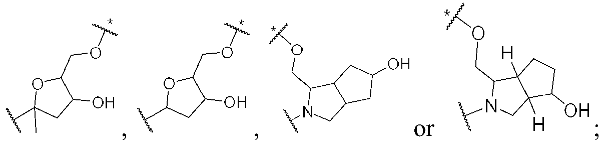
more preferably, R is selected from 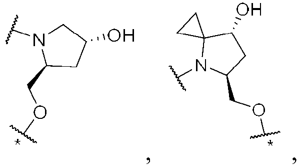 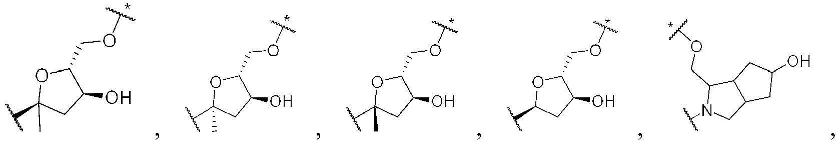 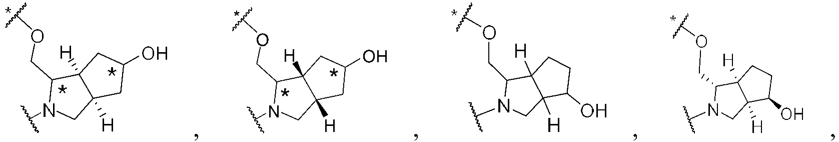 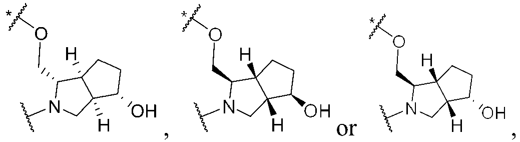 and * denotes a chiral centre, which is independently selected from (S)- or (R)-configuration, or a mixture thereof.

In a more specific embodiment, the present invention provides the above-mentioned delivery ligand, wherein
W₀ is selected from -OCH₂-, -OCH₂CH₂-, -OCH₂CH₂CH₂- or - OCH₂CH₂CH₂CH₂-;
W₁ is selected from a chemical bond, -O-, -O-L₁-, -C(O)-Li- or -C(O)-, preferably O--O-L₁-, -C(O)-Li- or -C(O)-;
W₂ is selected from -L₂-NH- or -L₂-O-;
W₃ is selected from -L₃-C(O)NH- or -L₃-NHC(O)-;
W₄ is selected from -L₄-NHC(O)-, -L₄-NHC(O)-(CH₂)₁₋₆-, -L₄-C(O)NH- or -L₄-C(O)NH-(CH₂)₁₋₆-;
preferably,
W₀ is -OCH₂-;
W₁ is selected from a chemical bond, -C(O)-Li- or -C(O)-, preferably - C(O)-Li- or -C(O)-;
W₂ is -L₂-NH-;
W₃ is -L₃-C(O)NH-;
W₄ is selected from -L₄-NHC(O)- or -L₄-NHC(O)-(CH₂)₁₋₆-.

In a more specific embodiment, the present invention provides the above-mentioned delivery ligand, wherein
the R is attached to a biomolecule via 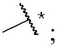
each W is independently selected from H, D, 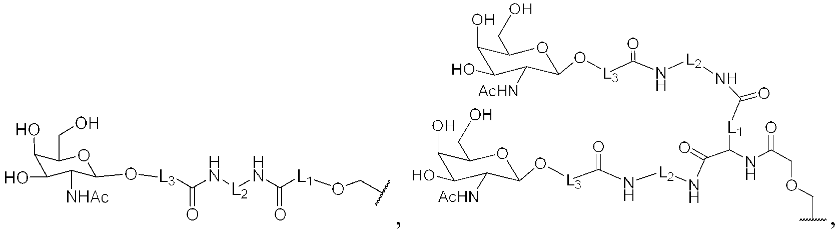 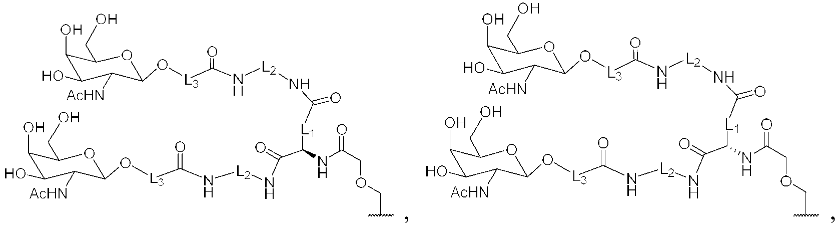 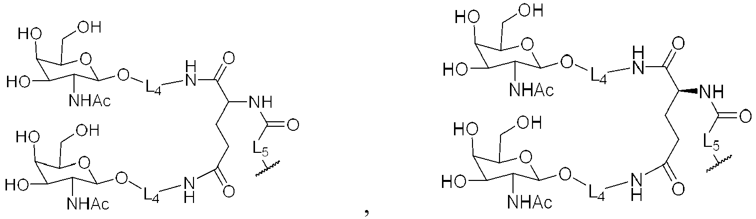 or 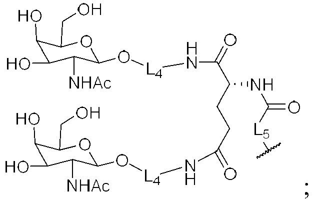 L₁ is -(CRₐR_{b})ₘ-;
Rₐ and R_{b} are each independently H, D, C₁₋₆ alkyl, C₁₋₆ alkoxy or C₁₋₆ haloalkyl; alternatively, Rₐ and R_{b} on any one or more carbon atoms in L₁, together with the carbon atom to which they are attached, form C₃₋₇ cycloalkyl or 3- to 7-membered heterocyclyl;
L₂ is -(CR_{c}R_{d})ₙ-;
R_{c} and R_{d} are each independently H, D, C₁₋₆ alkyl, C₁₋₆ alkoxy or C₁₋₆ haloalkyl; alternatively, R_{c} and R_{d} on any one or more carbon atoms in L₂, together with the carbon atom to which they are attached, form C₃₋₇ cycloalkyl or 3- to 7-membered heterocyclyl;
L₃ is -(CRₑR_{f})ₕ-;
Rₑ and R_{f} are each independently H, D, C₁₋₆ alkyl, C₁₋₆ alkoxy or C₁₋₆ haloalkyl; alternatively, Rₑ and R_{f} on any one or more carbon atoms in L₃, together with the carbon atom to which they are attached, form C₃₋₇ cycloalkyl or 3- to 7-membered heterocyclyl;
L₄ is -(CR_{g}Rₕ)₁₋₆-(OCR_{g}Rₕ-CR_{g}Rₕ)ₖ-;
R_{g} and Rₕ are each independently H, D, C₁₋₆ alkyl, C₁₋₆ alkoxy or C₁₋₆ haloalkyl; alternatively, R_{g} and Rₕ on any one or more carbon atoms in L₄, together with the carbon atom to which they are attached, form C₃₋₇ cycloalkyl or 3- to 7-membered heterocyclyl;
m, n, h and k are independently selected from 1, 2, 3, 4, 5, 6, 7 or 8;
L₅ is selected from a chemical bond, C₁₋₆ alkylene, C₁₋₆ alkenylene or - C₁₋₆ alkylene-O-C₁₋₆ alkylene-;
A is selected from -C(O)- or -NHC(O)-;
T is -(CRᵢRⱼ)_{q}-;
Rᵢ and Rⱼ are each independently H, D, C₁₋₆ alkyl, C₁₋₆ alkoxy or C₁₋₆ haloalkyl; alternatively, Rᵢ and Rⱼ on any one or more carbon atoms in T, together with the carbon atom to which they are attached, form C₃₋₇ cycloalkyl or 3- to 7-membered heterocyclyl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-;
Rₖ and Rₘ are each independently H, D, C₁₋₆ alkyl, C₁₋₆ alkoxy or C₁₋₆ haloalkyl; alternatively, Rₖ and Rₘ on any one or more carbon atoms in B, together with the carbon atom to which they are attached, form C₃₋₇ cycloalkyl or 3- to 7-membered heterocyclyl;
P is selected from a chemical bond or 1 to 3 amino acid residues;
R is selected from 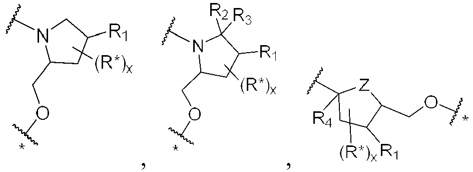 or 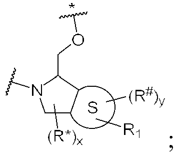
ring S is selected from C₃₋₁₀ cycloalkyl or 3- to 10-membered heterocyclyl;
R₁ is selected from -OR₀, -CH₂OR₀ or -CH₂CH₂OR₀, wherein R₀ is H, D, a hydroxyl protecting group or a solid-phase carrier;
the hydroxyl protecting group is selected from trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), - C(O)CH₂CH₂C(O)OH or 4,4'-dimethoxytrityl (DMTr), preferably - C(O)CH₂CH₂C(O)OH or 4,4'-dimethoxytrityl (DMTr);
R₂ and R₃ are independently selected from H, D, C₁₋₆ alkyl, C₁₋₆ alkoxy or C₁₋₆ haloalkyl; alternatively, R₂ and R₃, together with the carbon atom to which they are attached, form C₃₋₇ cycloalkyl or 3- to 7-membered heterocyclyl;
R₄ is selected from H, D, C₁₋₆ alkyl, C₁₋₆ alkoxy or C₁₋₆ haloalkyl;
Z is selected from O, NH or CH₂;
R* and R^{#} are independently selected from H, D, C₁₋₆ alkyl or C₁₋₆ haloalkyl;
x and y are independently selected from 0, 1, 2, 3, 4 or 5;
provided that, when R is 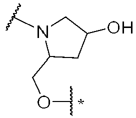 (including a stereoisomeric form thereof, such as 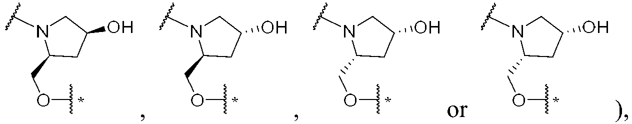 at least one pair among Rₐ and R_{b}, R_{c} and R_{d}, Rₑ and R_{f}, R_{g} and Rₕ, Rᵢ and Rⱼ, and Rₖ and Rₘ, together with the carbon atom to which they are attached, forms C₃₋₇ cycloalkyl or 3- to 7-membered heterocyclyl.

In a more specific embodiment, the present invention provides the above-mentioned delivery ligand, wherein the conjugated group is selected from a structure as shown in formula (I), formula (I-1), formula (I-2), formula (I'), formula (I'-1), formula (I'-2), formula (II), formula (II-1), formula (II-2), formula (II'), formula (II'-1), formula (II'-2), formula (III), formula (III-1), formula (III-2), formula (III-3), formula (III-4), formula (III-5), formula (III-6), formula (III'), formula (III'-1), formula (III'-2), formula (III'-3), formula (III'-4), formula (III'-5), formula (III'-6), formula (IIIc), formula (IIIc-1), formula (IIIc-2), formula (IIIc-3), formula (IIIc-4), formula (III'c), formula (III'c-1), formula (III'c-2), formula (III'c-3), formula (III'c-4), formula (IV), formula (IV-1), formula (IV-2), formula (IV'), formula (IV'-1), formula (IV'-2), formula (V), formula (V-1), formula (V-2), formula (V-3), formula (V-4), formula (V'), formula (V'-1), formula (V'-2), formula (V'-3) or formula (V'-4), or an isotopic variant, tautomer, or stereoisomer thereof, or a mixture thereof: 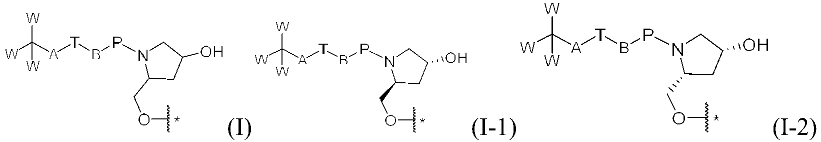 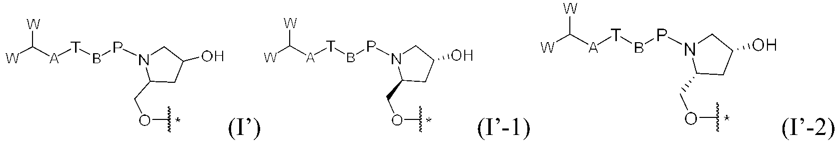 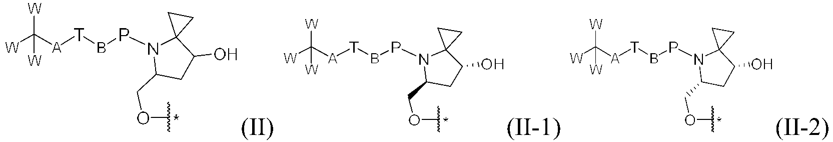 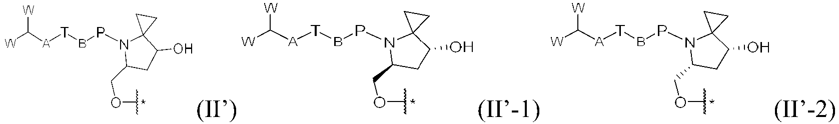 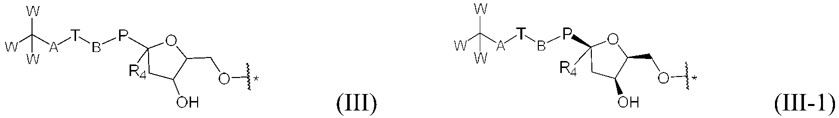 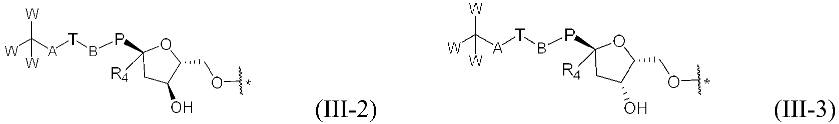 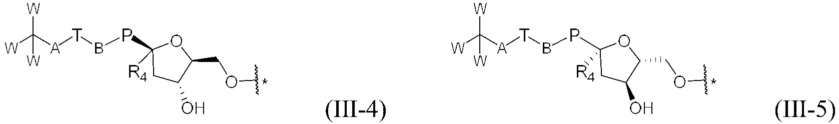 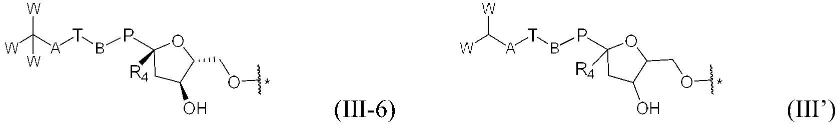 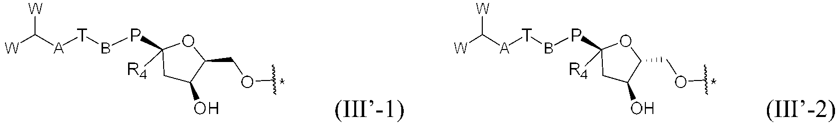 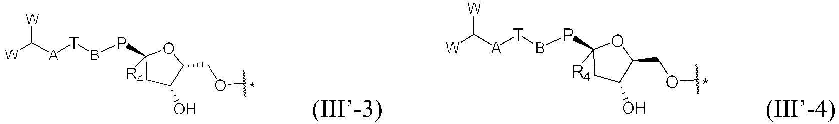 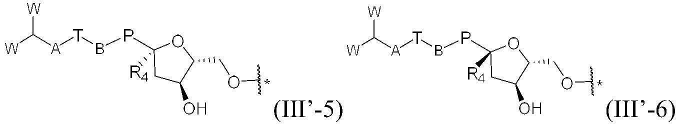 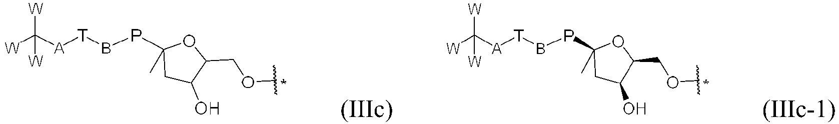 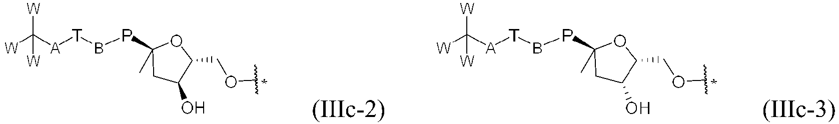 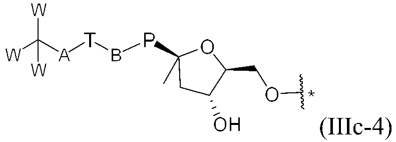 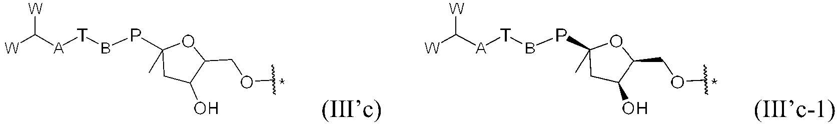 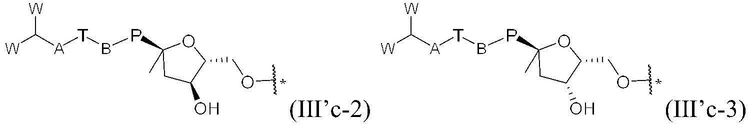 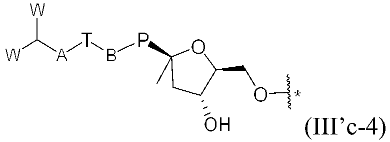 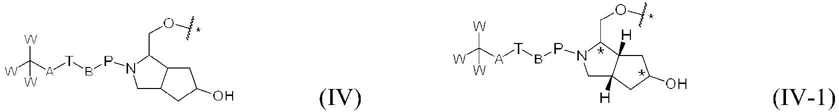 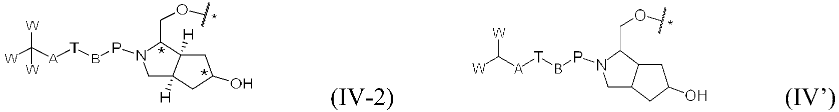 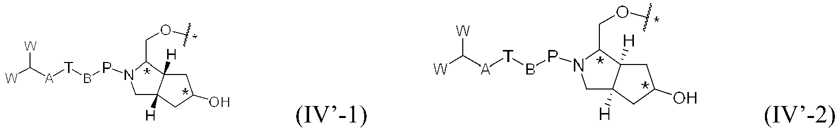 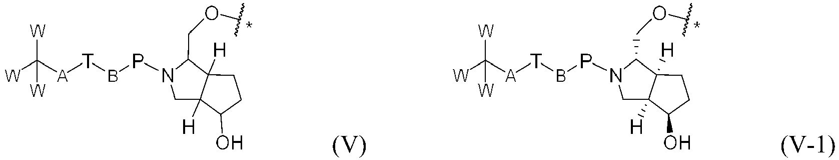 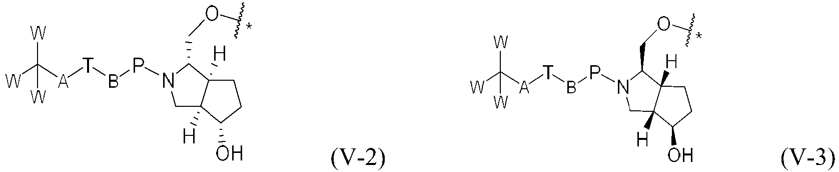 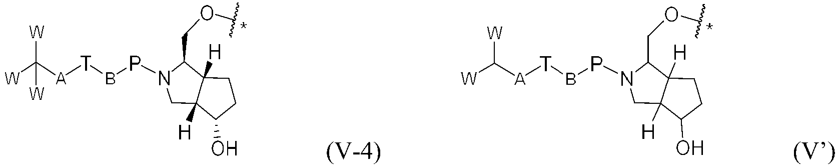 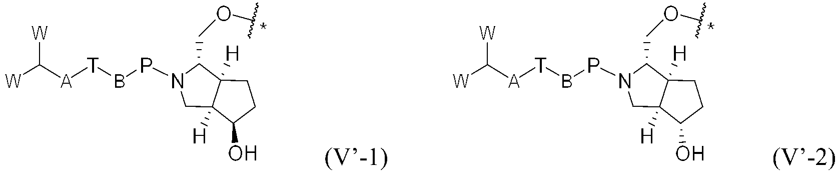 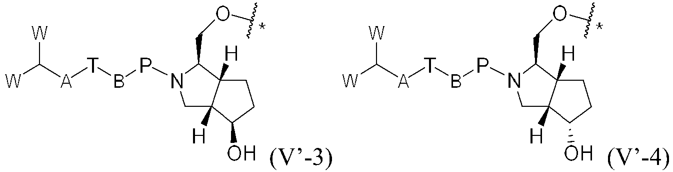 wherein * denotes a chiral centre, which is independently selected from (S)- or (R)-configuration, or a mixture thereof, and each of the other variables is as defined herein.

In a more specific embodiment, the present invention provides the above-mentioned delivery ligand, wherein the conjugated group is selected from a structure as shown in formula (I-1), formula (I'-1), formula (II-1), formula (II'-1), formula (III-2), formula (III-5), formula (III-6), formula (III'-2), formula (III'-5), formula (III'-6), formula (IV), formula (IV-1), formula (IV-2), formula (IV'), formula (IV'-1), formula (IV'-2), formula (V), formula (V-1), formula (V-4), formula (V'), formula (V'-1) or formula (V'-4), or an isotopic variant, tautomer, or stereoisomer thereof, or a mixture thereof: 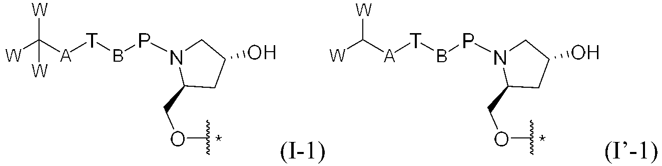 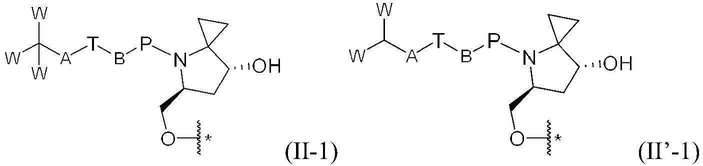 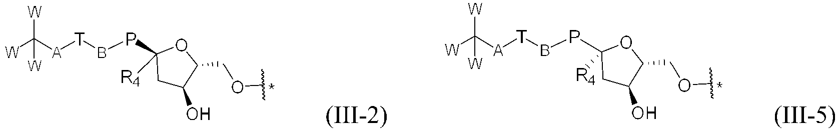 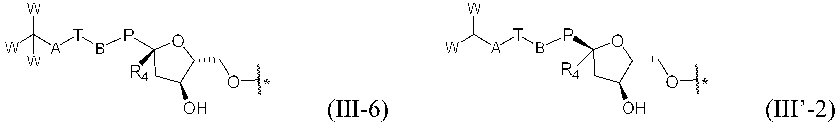 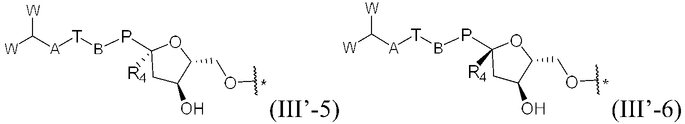 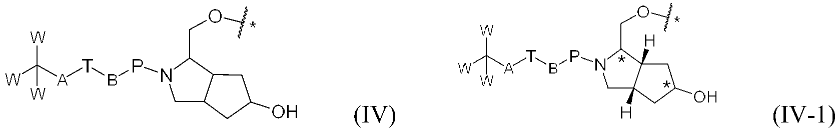 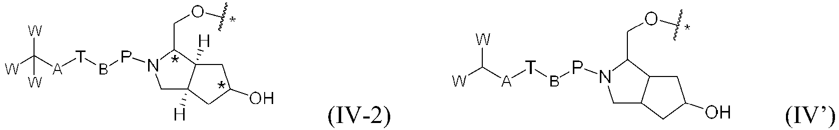 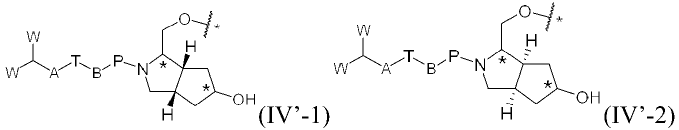 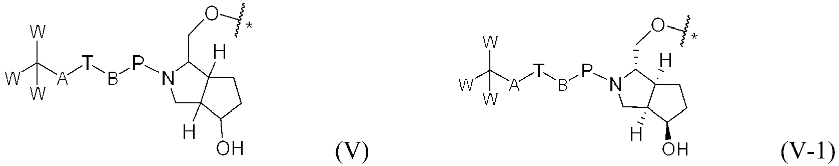 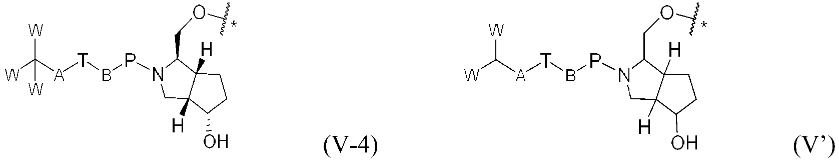 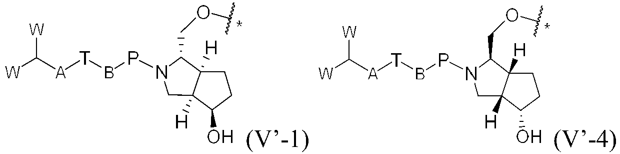 wherein
* denotes a chiral centre, which is independently selected from (S)- or (R)-configuration, or a mixture thereof;
each W is independently selected from H, 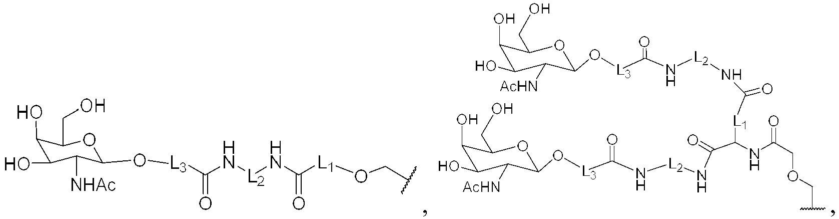 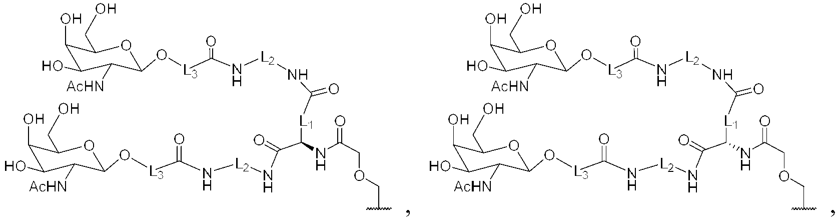 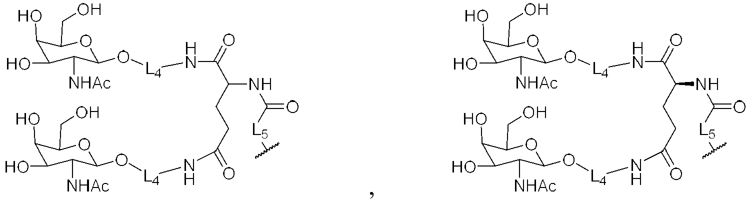 or 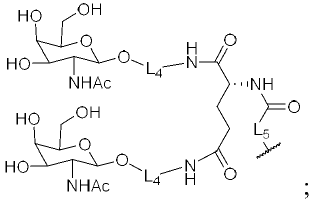
L₁ is -(CRₐR_{b})ₘ-, and 1, 2, 3, 4 or 5 non-adjacent CRₐR_{b} in the L₁ may be replaced by a heteroatom selected from -O-, -S- and -NRₐ-;
Rₐ and R_{b} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₐ and R_{b} on any one or more carbon atoms in L₁, together with the same or different carbon atoms to which they are attached, form C₃₋₁₀ cycloalkyl or 3- to 10-membered heterocyclyl;
L₂ is -(CR_{c}R_{d})ₙ-, and 1, 2, 3, 4 or 5 non-adjacent CR_{c}R_{d} in the L₂ may be replaced by a heteroatom selected from -O-, -S- and -NR_{c}-;
R_{c} and R_{d} are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{c} and R_{d} on any one or more carbon atoms in L₂, together with the same or different carbon atoms to which they are attached, form C₃₋₁₀ cycloalkyl or 3- to 10-membered heterocyclyl;
L₃ is -(CRₑR_{f})ₕ-, and 1, 2, 3, 4 or 5 non-adjacent CRₑR_{f} in the L₃ may be replaced by a heteroatom selected from -O-, -S- and -NRₑ-;
Rₑ and R_{f} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₑ and R_{f} on any one or more carbon atoms in L₃, together with the same or different carbon atoms to which they are attached, form C₃₋₁₀ cycloalkyl or 3- to 10-membered heterocyclyl;
L₄ is selected from -(CR_{g}Rₕ)ₖ-, -(CR_{g}Rₕ)₁₋₆-(OCR_{g}Rₕ-CR_{g}Rₕ)ₖ- or - (CR_{g}Rₕ)₁₋₆-(NH-CR_{g}Rₕ-CR_{g}Rₕ)ₖ-, and 1, 2, 3, 4 or 5 non-adjacent CR_{g}Rₕ in the L₄ may be replaced by a heteroatom selected from -O-, -S- and -NR_{g}-;
R_{g} and Rₕ are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{g} and Rₕ on any one or more carbon atoms in L₄, together with the same or different carbon atoms to which they are attached, form C₃₋₁₀ cycloalkyl or 3- to 10-membered heterocyclyl;
m, n, h and k are independently selected from 1, 2, 3, 4, 5, 6, 7, 8, 9 or 10;
L₅ is selected from a chemical bond, C₁₋₆ alkylene, C₁₋₆ alkenylene or - C₁₋₆ alkylene-O-C₁₋₆ alkylene-;
A is selected from -C(O)- or -NHC(O)-;
T is -(CRᵢRⱼ)_{q}-, and 1, 2, 3, 4 or 5 non-adjacent CRᵢRⱼ in the T may be replaced by a heteroatom selected from -O-, -S- and -NRᵢ-;
Rᵢ and Rⱼ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rᵢ and Rⱼ on any one or more carbon atoms in T, together with the same or different carbon atoms to which they are attached, form C₃₋₁₀ cycloalkyl or 3- to 10-membered heterocyclyl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-, and 1, 2, 3, 4 or 5 non-adjacent CRₖRₘ in the B may be replaced by a heteroatom selected from -O-, -Sand -NRᵢ-;
Rₖ and Rₘ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₖ and Rₘ on any one or more carbon atoms in B, together with the same or different carbon atoms to which they are attached, form C₃₋₁₀ cycloalkyl or 3- to 10-membered heterocyclyl;
P is selected from a chemical bond or 1 to 3 amino acid residues;
L₁-L₅, T and B are optionally deuterated, up to fully deuterated;
provided that, when the conjugated group has a structure of formula (I), at least one pair among Rₐ and R_{b}, R_{c} and R_{d}, Rₑ and R_{f}, R_{g} and Rₕ, Rᵢ and Rⱼ, and Rₖ and Rₘ, together with the carbon atom to which they are attached, forms C₃₋₁₀ cycloalkyl or 3- to 10-membered heterocyclyl.

In a more specific embodiment, the present invention provides the above-mentioned delivery ligand, wherein
L₁ is -(CRₐR_{b})ₘ-;
Rₐ and R_{b} are each independently H, D, C₁₋₆ alkyl, C₁₋₆ alkoxy or C₁₋₆ haloalkyl; alternatively, Rₐ and R_{b} on any one or more carbon atoms in L₁, together with the carbon atom to which they are attached, form C₃₋₇ cycloalkyl or 3- to 7-membered heterocyclyl;
L₂ is -(CR_{c}R_{d})ₙ-;
R_{c} and R_{d} are each independently H, D, C₁₋₆ alkyl, C₁₋₆ alkoxy or C₁₋₆ haloalkyl; alternatively, R_{c} and R_{d} on any one or more carbon atoms in L₂, together with the carbon atom to which they are attached, form C₃₋₇ cycloalkyl or 3- to 7-membered heterocyclyl;
L₃ is -(CRₑR_{f})ₕ-;
Rₑ and R_{f} are each independently H, D, C₁₋₆ alkyl, C₁₋₆ alkoxy or C₁₋₆ haloalkyl; alternatively, Rₑ and R_{f} on any one or more carbon atoms in L₃, together with the carbon atom to which they are attached, form C₃₋₇ cycloalkyl or 3- to 7-membered heterocyclyl;
L₄ is -(CR_{g}Rₕ)₁₋₆-(OCR_{g}Rₕ-CR_{g}Rₕ)ₖ-;
R_{g} and Rₕ are each independently H, D, C₁₋₆ alkyl, C₁₋₆ alkoxy or C₁₋₆ haloalkyl; alternatively, R_{g} and Rₕ on any one or more carbon atoms in L₄, together with the carbon atom to which they are attached, form C₃₋₇ cycloalkyl or 3- to 7-membered heterocyclyl;
m, n, h and k are independently selected from 1, 2, 3, 4 or 5;
L₅ is selected from a chemical bond, C₁₋₆ alkylene or -C₁₋₄ alkylene-O-C₁₋₄ alkylene-;
A is selected from -C(O)- or -NHC(O)-;
T is -(CRᵢRⱼ)_{q}-;
Rᵢ and Rⱼ are each independently H, D, C₁₋₆ alkyl, C₁₋₆ alkoxy or C₁₋₆ haloalkyl; alternatively, Rᵢ and Rⱼ on any one or more carbon atoms in T, together with the carbon atom to which they are attached, form C₃₋₇ cycloalkyl or 3- to 7-membered heterocyclyl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-;
Rₖ and Rₘ are each independently H, D, C₁₋₆ alkyl, C₁₋₆ alkoxy or C₁₋₆ haloalkyl; alternatively, Rₖ and Rₘ on any one or more carbon atoms in B, together with the carbon atom to which they are attached, form C₃₋₇ cycloalkyl or 3- to 7-membered heterocyclyl;
P is selected from a chemical bond or 1 amino acid residue.

In a more specific embodiment, the present invention provides the above-mentioned delivery ligand, wherein
L₁ is -(CRₐR_{b})ₘ-;
Rₐ and R_{b} are each independently H, D, C₁₋₆ alkyl or C₁₋₆ haloalkyl; alternatively, Rₐ and R_{b} on any one or more carbon atoms in L₁, together with the carbon atom to which they are attached, form C₃₋₇ cycloalkyl;
m is selected from 1, 2, 3 or 4;
L₂ is -(CR_{c}R_{d})ₙ-;
R_{c} and R_{d} are each independently H, D, C₁₋₆ alkyl or C₁₋₆ haloalkyl; alternatively, R_{c} and R_{d} on any one or more carbon atoms in L₂, together with the carbon atom to which they are attached, form C₃₋₇ cycloalkyl;
n is selected from 1, 2, 3 or 4;
L₃ is -(CRₑR_{f})ₕ-;
Rₑ and R_{f} are each independently H, D, C₁₋₆ alkyl or C₁₋₆ haloalkyl; alternatively, Rₑ and R_{f} on any one or more carbon atoms in L₃, together with the carbon atom to which they are attached, form C₃₋₇ cycloalkyl;
h is selected from 2, 3, 4 or 5;
L₄ is -(CR_{g}Rₕ)₁₋₆-(OCR_{g}Rₕ-CR_{g}Rₕ)ₖ-;
R_{g} and Rₕ are each independently H, D, C₁₋₆ alkyl or C₁₋₆ haloalkyl; alternatively, R_{g} and Rₕ on any one or more carbon atoms in L₄, together with the carbon atom to which they are attached, form C₃₋₇ cycloalkyl;
k is selected from 1, 2, 3 or 4;
L₅ is selected from a chemical bond, C₁₋₆ alkylene or -C₁₋₄ alkylene-O-C₁₋₄ alkylene-;
A is selected from -C(O)- or -NHC(O)-;
T is -(CRᵢRⱼ)_{q}-;
Rᵢ and Rⱼ are each independently H, D, C₁₋₆ alkyl or C₁₋₆ haloalkyl; alternatively, Rᵢ and Rⱼ on any one or more carbon atoms in T, together with the carbon atom to which they are attached, form C₃₋₇ cycloalkyl;
q is selected from 5, 6, 7, 8, 9, 10, 11 or 12;
B is selected from -C(O)-, -OC(O)-, -NH-, -NHC(O)-, -C(O)-(CRₖRₘ)₀-₄- or -C(O)NH-(CRₖRₘ)₀₋₄-;
Rₖ and Rₘ are each independently H, D, C₁₋₆ alkyl or C₁₋₆ haloalkyl; alternatively, Rₖ and Rₘ on any one or more carbon atoms in B, together with the carbon atom to which they are attached, form C₃₋₇ cycloalkyl;
P is selected from a chemical bond or 1 amino acid residue.

In a more specific embodiment, the present invention provides the above-mentioned delivery ligand, wherein
L₁ is -(CRₐR_{b})ₘ-;
Rₐ and R_{b} are each independently H or C₁₋₄ alkyl; alternatively, Rₐ and R_{b} on any one or more carbon atoms in L₁, together with the carbon atom to which they are attached, form C₃₋₅ cycloalkyl;
m is selected from 1, 2 or 3;
L₂ is -(CR_{c}R_{d})ₙ-;
R_{c} and R_{d} are each independently H or C₁₋₄ alkyl; alternatively, R_{c} and R_{d} on any one or more carbon atoms in L₂, together with the carbon atom to which they are attached, form C₃₋₅ cycloalkyl;
n is selected from 2, 3 or 4;
L₃ is -(CRₑR_{f})ₕ-;
Rₑ and R_{f} are each independently H or C₁₋₄ alkyl; alternatively, Rₑ and R_{f} on any one or more carbon atoms in L₃, together with the carbon atom to which they are attached, form C₃₋₅ cycloalkyl;
h is selected from 3, 4 or 5;
L₄ is -(CR_{g}Rₕ)₁₋₆-(OCR_{g}Rₕ-CR_{g}Rₕ)ₖ-;
R_{g} and Rₕ are each independently H or C₁₋₄ alkyl; alternatively, R_{g} and Rₕ on any one or more carbon atoms in L₄, together with the carbon atom to which they are attached, form C₃₋₅ cycloalkyl;
k is selected from 2, 3 or 4;
L₅ is selected from a chemical bond, -(CH₂)₁₋₄- or -(CH₂)₁₋₂O(CH₂)₁₋₂-;
A is selected from -C(O)- or -NHC(O)-;
T is -(CRᵢRⱼ)_{q}-;
Rᵢ and Rⱼ are each independently H or C₁₋₄ alkyl; alternatively, Rᵢ and Rⱼ on any one or more carbon atoms in T, together with the carbon atom to which they are attached, form C₃₋₅ cycloalkyl;
q is selected from 5, 6, 7, 8, 9 or 10;
B is selected from -C(O)-, -C(O)NH- or -C(O)NH-(CRₖRₘ)₁₋₃-;
Rₖ and Rₘ are each independently H or C₁₋₄ alkyl; alternatively, Rₖ and Rₘ on any one or more carbon atoms in B, together with the carbon atom to which they are attached, form cyclopropyl;
P is selected from a chemical bond, -Cit-, -Arg- or -Phe-.

In a more specific embodiment, the present invention provides the above-mentioned delivery ligand, wherein
L₁ is -(CRₐR_{b})₂-, preferably -CH₂CH₂- or 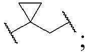
Rₐ and R_{b} are each independently H; alternatively, Rₐ and R_{b} on any one or more carbon atoms in L₁, together with the carbon atom to which they are attached, form cyclopropyl;
L₂ is -(CR_{c}R_{d})₃-, preferably -CH₂CH₂CH₂-, 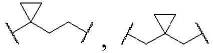 or 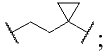
R_{c} and R_{d} are each independently H; alternatively, R_{c} and R_{d} on any one or more carbon atoms in L₂, together with the carbon atom to which they are attached, form cyclopropyl;
L₃ is -(CRₑR_{f})₄-, preferably -CH₂CH₂CH₂CH₂- or 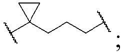
Rₑ and R_{f} are each independently H; alternatively, Rₑ and R_{f} on any one or more carbon atoms in L₃, together with the carbon atom to which they are attached, form cyclopropyl;
L₄ is -(CR_{g}Rₕ)₂-(OCR_{g}Rₕ-CR_{g}Rₕ)₂-, preferably 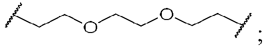
R_{g} and Rₕ are each independently H; alternatively, R_{g} and Rₕ on any one or more carbon atoms in L₄, together with the carbon atom to which they are attached, form cyclopropyl;
A is -C(O)- or -NHC(O)-;
L₅ is selected from a chemical bond, -CH₂CH₂- or -CH₂OCH₂-;
T is -(CRᵢRⱼ)₇₋₁₀-;
Rᵢ and Rⱼ are each independently H; alternatively, Rᵢ and Rⱼ on any one or more carbon atoms in T, together with the carbon atom to which they are attached, form cyclopropyl;
B is selected from -C(O)-, -C(O)NH- or -C(O)NH-CRₖRₘ-;
Rₖ and Rₘ are independently H; alternatively, Rₖ and Rₘ in CRₖRₘ, together with the carbon atom to which they are attached, form cyclopropyl;
P is selected from a chemical bond, -Cit- or -Phe-.

In a more specific embodiment, the present invention provides the above-mentioned delivery ligand, wherein
each W is the same or different, and is independently selected from H, 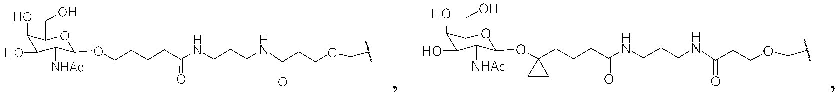 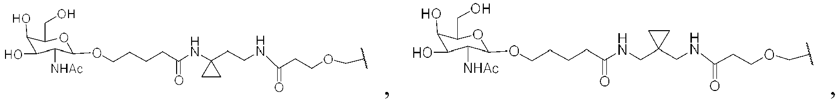 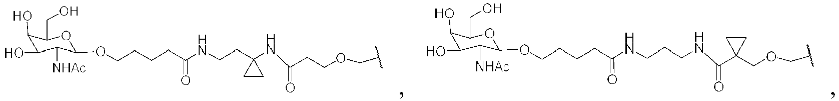 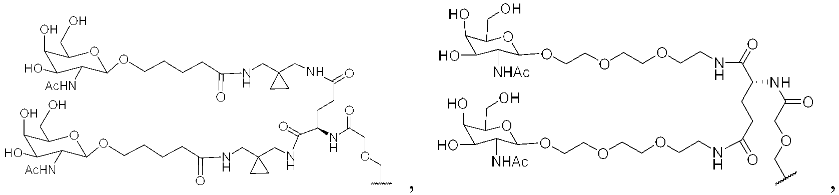 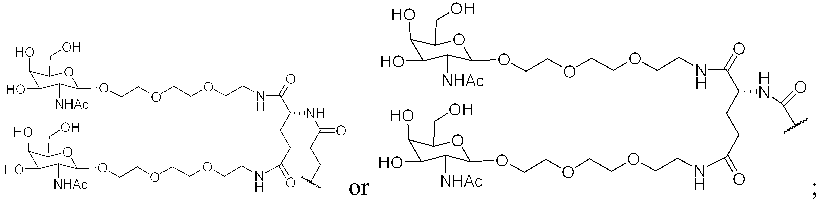
A is -NHC(O)-;
T is selected from 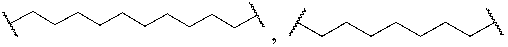 or 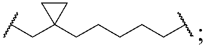
B is selected from -C(O)-, 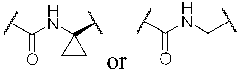
P is selected from a chemical bond,

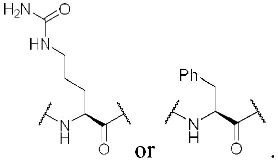

In a more specific embodiment, the present invention provides the above-mentioned delivery ligand, wherein the conjugated group is selected from a structure as shown in formula (VI), formula (VI-1), formula (VI-2), formula (VI'), formula (VI'-1), formula (VI'-2), formula (VII), formula (VII-1), formula (VII-2), formula (VII'), formula (VII'-1), formula (VII'-2), formula (VIII), formula (VIII-1), formula (VIII-2), formula (VIII-3), formula (VIII-4), formula (VIII-5), formula (VIII-6), formula (VIII'), formula (VIII'-1), formula (VIII'-2), formula (VIII'-3), formula (VIII'-4), formula (VIII'-5), formula (VIII'-6), formula (IX), formula (IX-1), formula (IX-2), formula (IX'), formula (IX'-1) or formula (IX'-2), or an isotopic variant, tautomer, or stereoisomer thereof, or a mixture thereof: 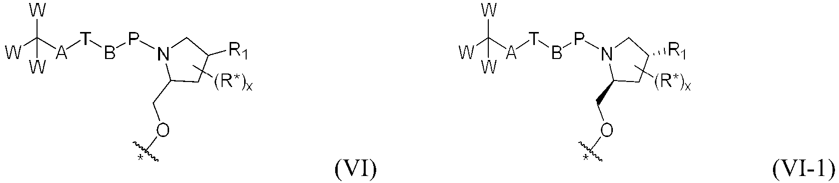 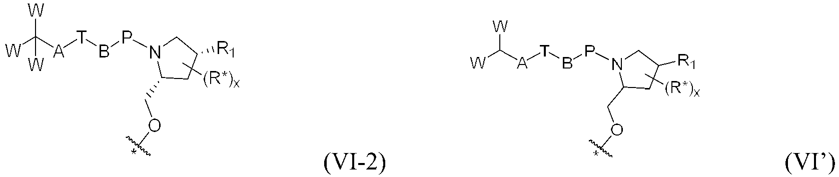 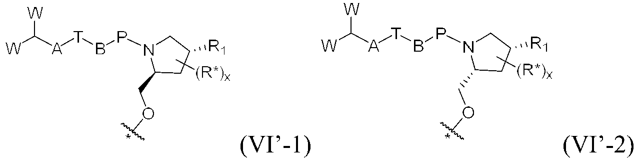 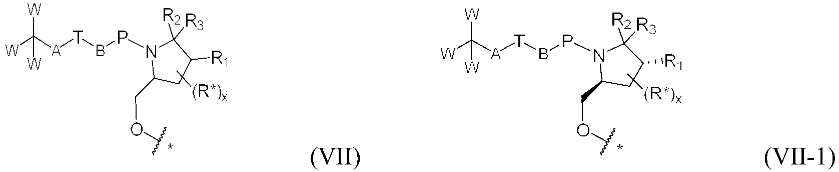 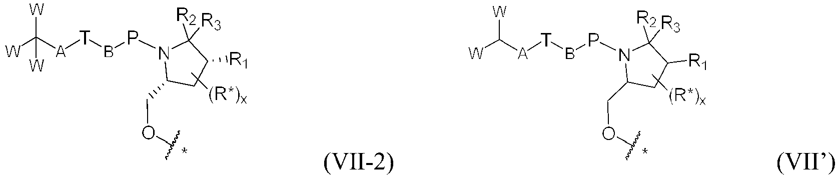 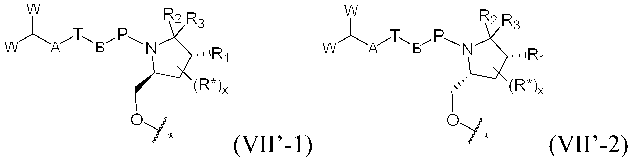 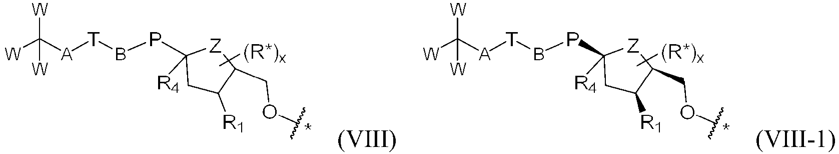 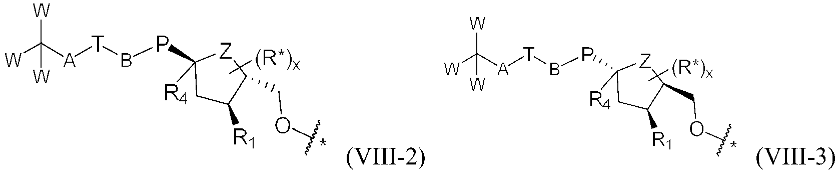 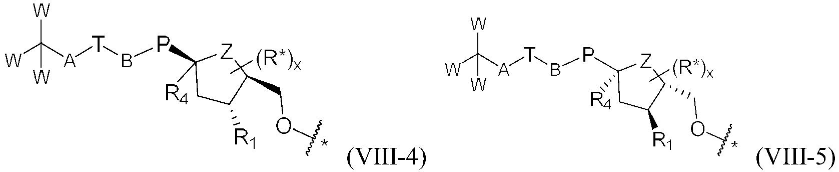 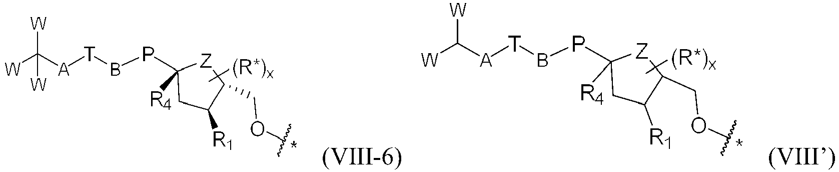 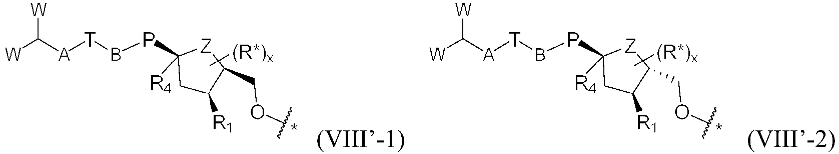 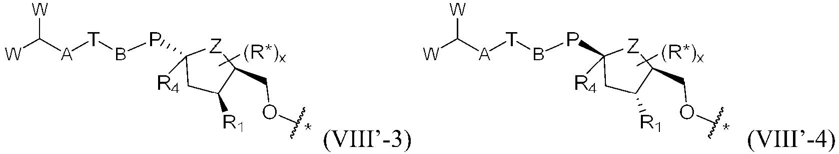 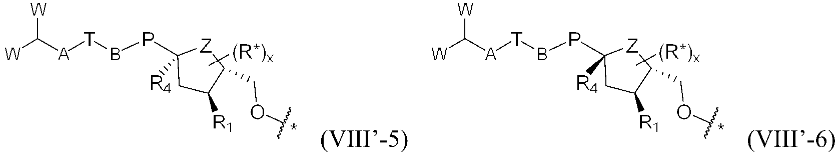 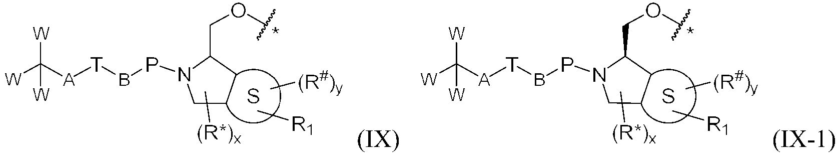 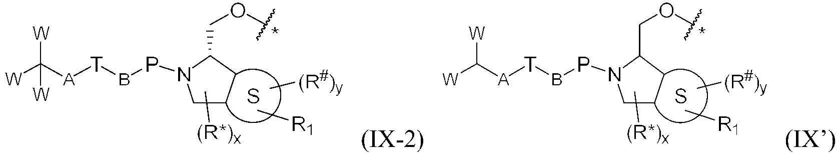 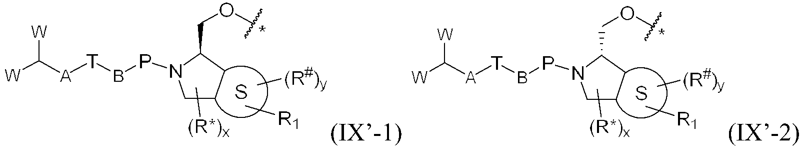 wherein each variable is as defined herein.

In a more specific embodiment, the present invention provides the above-mentioned delivery ligand, wherein the conjugated group is selected from a structure as shown in formula (VI-1) or formula (VI'-1), or an isotopic variant, tautomer, or stereoisomer thereof, or a mixture thereof: 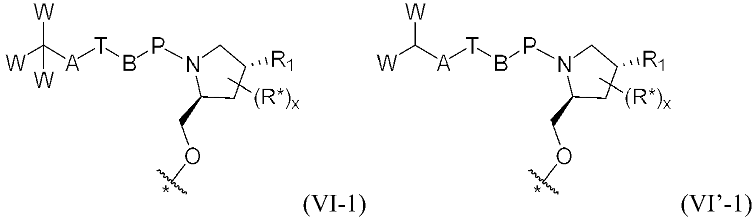
each W is independently selected from H, 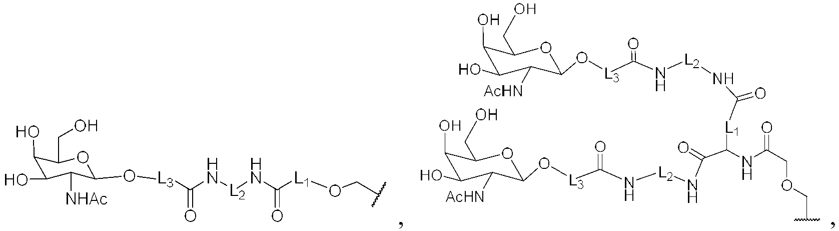 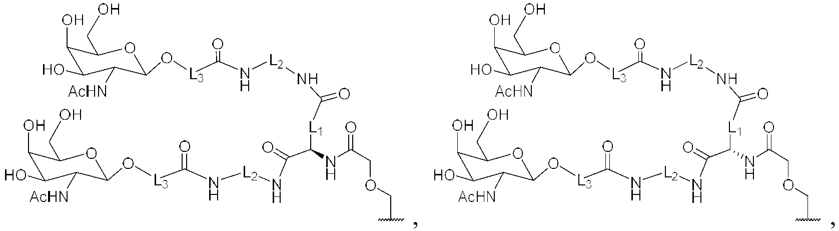 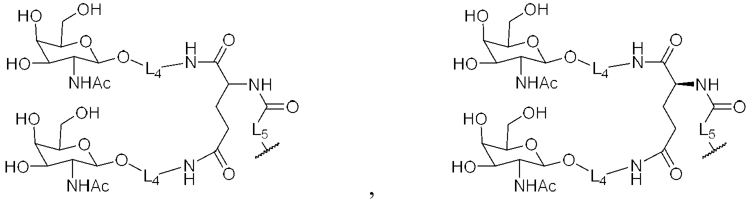 or 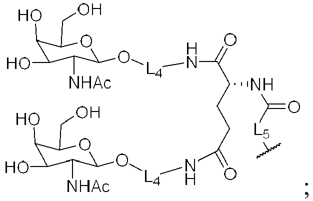
L₁, L₂, L₃, L₄, L₅, A, B, T and P are as defined herein;
provided that, at least one pair among Rₐ and R_{b}, R_{c} and R_{d}, Rₑ and R_{f}, R_{g} and Rₕ, Rᵢ and Rⱼ, and Rₖ and Rₘ, together with the carbon atom to which they are attached, forms a ring;
R₁ is selected from -OR₀, -CH₂OR₀ or -CH₂CH₂OR₀, wherein R₀ is H, D, a hydroxyl protecting group or a solid-phase carrier;
the hydroxyl protecting group is preferably -C(O)CH₂CH₂C(O)OH or 4,4'-dimethoxytrityl (DMTr);
R* is selected from H, D, C₁₋₆ alkyl or C₁₋₆ haloalkyl;
x is selected from 0, 1, 2, 3, 4, 5 or 6;
preferably,
R₁ is -OR₀, wherein R₀ is H or a hydroxyl protecting group, preferably H;
the hydroxyl protecting group is preferably -C(O)CH₂CH₂C(O)OH;
R* is selected from H or C₁₋₆ alkyl;
x is selected from 0, 1, 2, 3 or 4;
more preferably,
R₁ is OH;
R* is selected from H or C₁₋₄ alkyl, preferably H;
x is 0, 1, 2 or 3.

In a more specific embodiment, the present invention provides the above-mentioned delivery ligand, wherein the conjugated group is selected from a structure as shown in formula (VII-1) or formula (VII'-1), or an isotopic variant, tautomer, or stereoisomer thereof, or a mixture thereof: 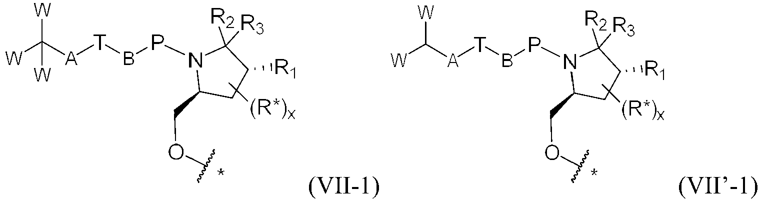 wherein
each W is independently selected from H, 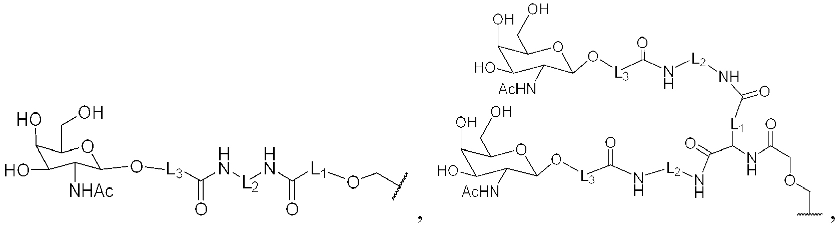 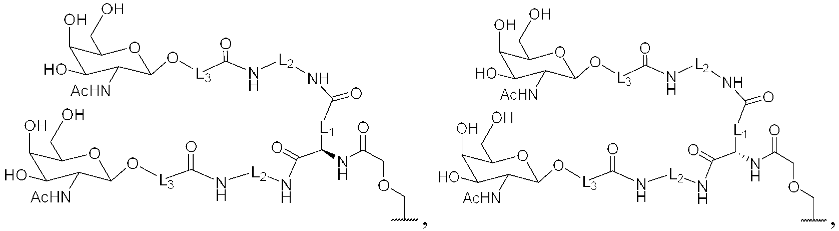 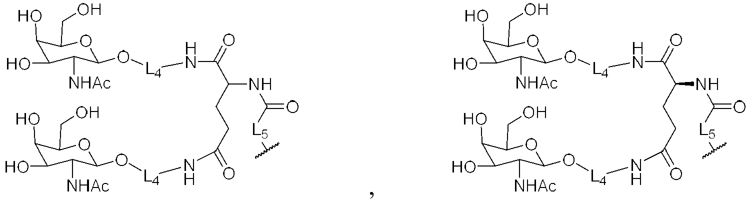 or 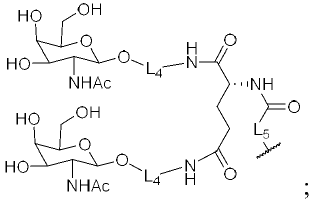
L₁, L₂, L₃, L₄, L₅, A, B, T and P are as defined herein;
R₁ is selected from -OR₀, -CH₂OR₀ or -CH₂CH₂OR₀, wherein R₀ is H, D, a hydroxyl protecting group or a solid-phase carrier;
the hydroxyl protecting group is preferably -C(O)CH₂CH₂C(O)OH or 4,4'-dimethoxytrityl (DMTr);
R₂ and R₃ are independently selected from H, D, C₁₋₆ alkyl, C₁₋₆ alkoxy or C₁₋₆ haloalkyl; alternatively, R₂ and R₃, together with the carbon atom to which they are attached, form C₃₋₇ cycloalkyl or 3- to 7-membered heterocyclyl;
R* is selected from H, D, C₁₋₆ alkyl or C₁₋₆ haloalkyl;
x is selected from 0, 1, 2, 3 or 4;
preferably,
R₁ is -OR₀, wherein R₀ is H or a hydroxyl protecting group, preferably H;
the hydroxyl protecting group is preferably -C(O)CH₂CH₂C(O)OH;
R₂ and R₃ are independently selected from H, D, C₁₋₆ alkyl or C₁₋₆ haloalkyl; alternatively, R₂ and R₃, together with the carbon atom to which they are attached, form C₃₋₅ cycloalkyl;
R* is selected from H or C₁₋₆ alkyl;
x is selected from 0, 1, 2, 3 or 4;
more preferably,
R₁ is OH;
R₂ and R₃ are independently selected from H or C₁₋₄ alkyl; alternatively, R₂ and R₃, together with the carbon atom to which they are attached, form C₃₋₅ cycloalkyl, such as cyclopropyl;
R* is selected from H or C₁₋₄ alkyl, preferably H;
x is 0, 1, 2 or 3.

In a more specific embodiment, the present invention provides the above-mentioned delivery ligand, wherein the conjugated group is selected from a structure as shown in formula (VIII-2), formula (VIII-5), formula (VIII-6), formula (VIII'-2), formula (VIII'-5) or formula (VIII'-6), or an isotopic variant, tautomer, or stereoisomer thereof, or a mixture thereof: 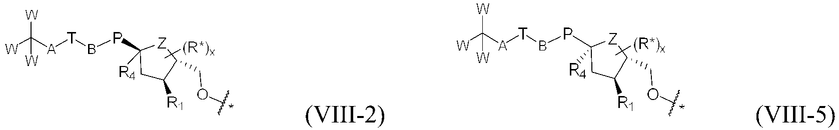 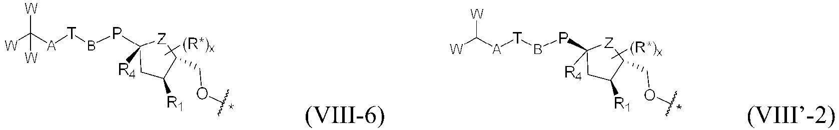 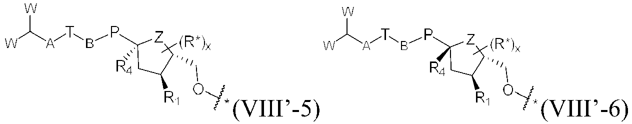 wherein
each W is independently selected from H, 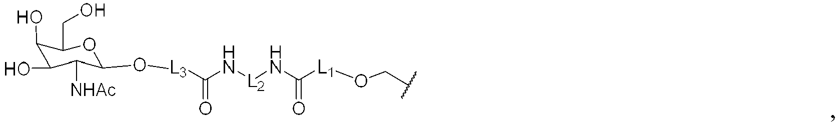 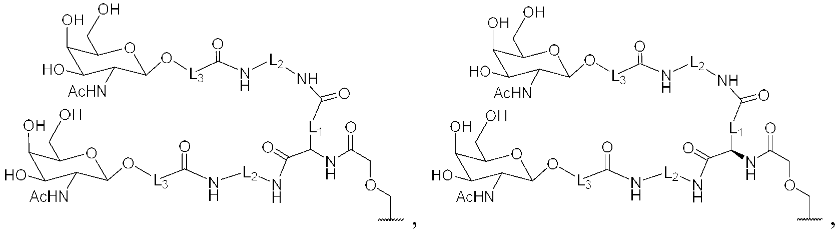 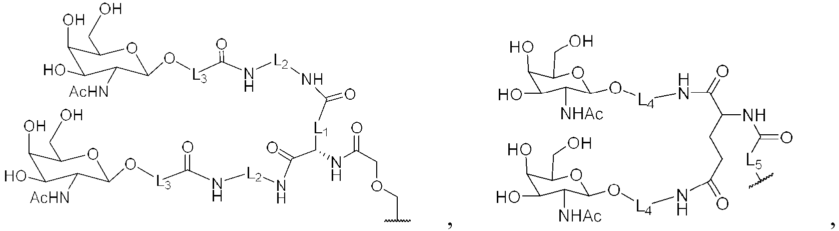 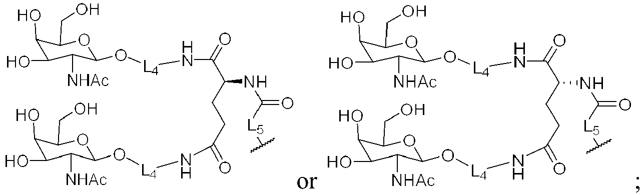
L₁, L₂, L₃, L₄, L₅, A, B, T and P are as defined herein;
Z is selected from O, NH or CH₂;
R₁ is selected from -OR₀, -CH₂OR₀ or -CH₂CH₂OR₀, wherein R₀ is H, D, a hydroxyl protecting group or a solid-phase carrier;
the hydroxyl protecting group is preferably -C(O)CH₂CH₂C(O)OH or 4,4'-dimethoxytrityl (DMTr);
R₄ is selected from H, D, C₁₋₆ alkyl or C₁₋₆ haloalkyl;
R* is selected from H, D, C₁₋₆ alkyl or C₁₋₆ haloalkyl;
x is selected from 0, 1, 2, 3 or 4;
preferably,
Z is selected from O, NH or CH₂;
R₁ is -OR₀, wherein R₀ is H or a hydroxyl protecting group, preferably H;
the hydroxyl protecting group is preferably -C(O)CH₂CH₂C(O)OH;
R₄ is selected from H or C₁₋₆ alkyl;
R* is selected from H or C₁₋₆ alkyl;
x is selected from 0, 1, 2, 3 or 4;
more preferably,
Z is selected from O or NH, preferably O;
R₁ is OH;
R₄ is selected from H or C₁₋₄ alkyl, preferably methyl;
R* is selected from H or C₁₋₄ alkyl, preferably H;
x is 0, 1, 2 or 3.

In a more specific embodiment, the present invention provides the above-mentioned delivery ligand, wherein the conjugated group is selected from a structure as shown in formula (IX) or formula (IX'), or an isotopic variant, tautomer, or stereoisomer thereof, or a mixture thereof: 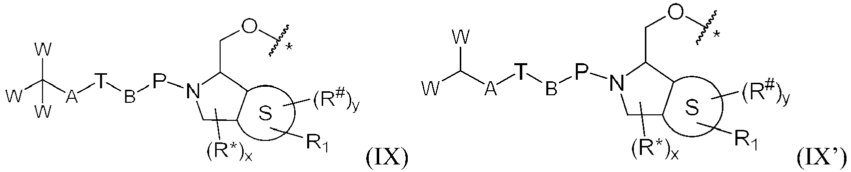
each W is independently selected from H, 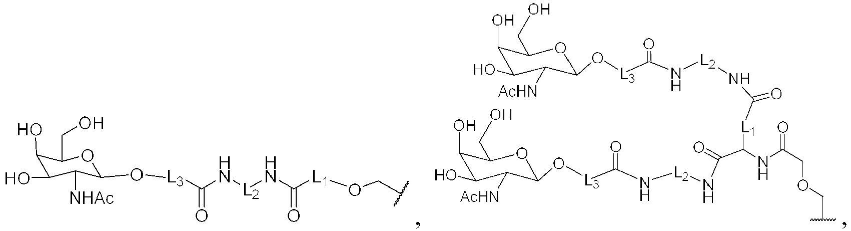 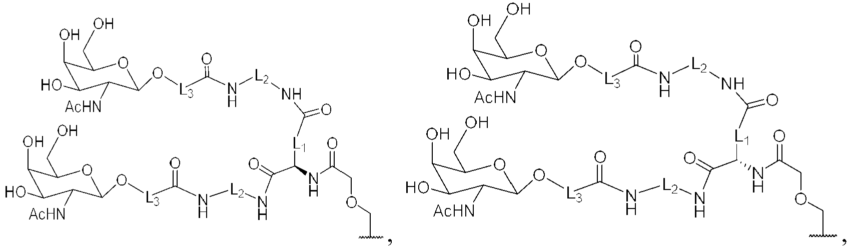 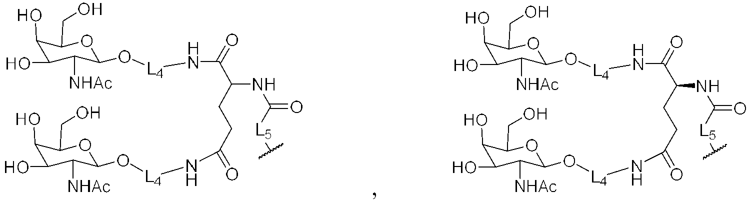 or 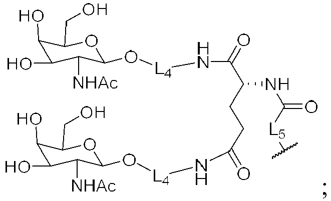
L₁, L₂, L₃, L₄, L₅, A, B, T and P are as defined herein;
ring S is selected from C₃₋₁₀ cycloalkyl or 3- to 10-membered heterocyclyl;
R₁ is selected from -OR₀, -CH₂OR₀ or -CH₂CH₂OR₀, wherein R₀ is H, D, a hydroxyl protecting group or a solid-phase carrier;
the hydroxyl protecting group is preferably -C(O)CH₂CH₂C(O)OH or 4,4'-dimethoxytrityl (DMTr);
R* and R^{#} are independently selected from H, D, C₁₋₆ alkyl or C₁₋₆ haloalkyl;
x and y are independently selected from 0, 1, 2, 3 or 4;
preferably,
ring S is selected from C₃₋₇ cycloalkyl or 3- to 7-membered heterocyclyl;
R₁ is -OR₀, wherein R₀ is H or a hydroxyl protecting group, preferably H;
the hydroxyl protecting group is preferably -C(O)CH₂CH₂C(O)OH;
R₄ is selected from H or C₁₋₆ alkyl;
R* and R^{#} are independently selected from H or C₁₋₆ alkyl;
x and y are independently selected from 0, 1, 2, 3 or 4;
more preferably,
ring S is C₅₋₆ cycloalkyl, such as cyclopentyl;
R₁ is OH;
R* and R^{#} are independently selected from H or C₁₋₄ alkyl, preferably H;
x and y are independently selected from 0, 1, 2 or 3.

In a more specific embodiment, the present invention provides the above-mentioned delivery ligand, wherein the conjugated group is selected from a structure as shown in formula (V-1) or formula (V-4), or an isotopic variant, tautomer, or stereoisomer thereof, or a mixture thereof: 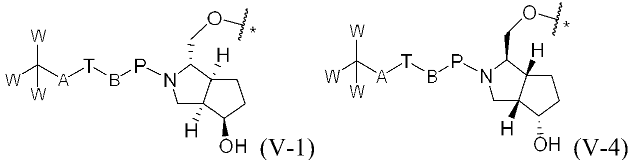
each W is independently selected from H, 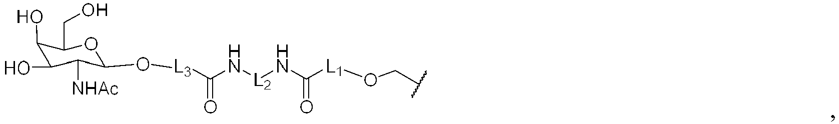 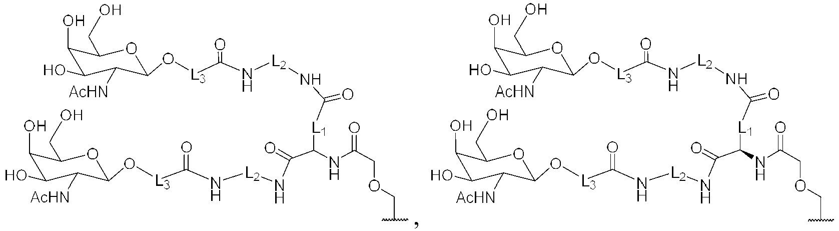 or 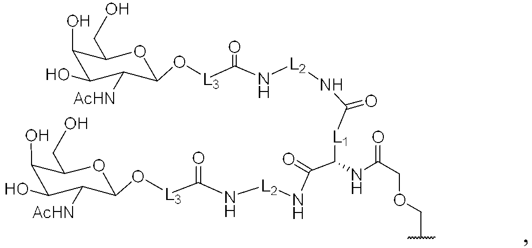 preferably 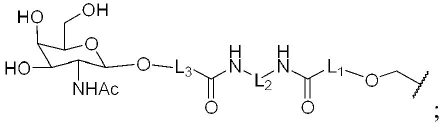
L₁ is -(CRₐR_{b})ₘ-;
Rₐ and R_{b} are each independently H, D, C₁₋₆ alkyl, C₁₋₆ alkoxy or C₁₋₆ haloalkyl; alternatively, Rₐ and R_{b} on any one or more carbon atoms in L₁, together with the carbon atom to which they are attached, form C₃₋₇ cycloalkyl or 3- to 7-membered heterocyclyl;
L₂ is -(CR_{c}R_{d})ₙ-;
R_{c} and R_{d} are each independently H, D, C₁₋₆ alkyl, C₁₋₆ alkoxy or C₁₋₆ haloalkyl; alternatively, R_{c} and R_{d} on any one or more carbon atoms in L₂, together with the carbon atom to which they are attached, form C₃₋₇ cycloalkyl or 3- to 7-membered heterocyclyl;
L₃ is -(CRₑR_{f})ₕ-;
Rₑ and R_{f} are each independently H, D, C₁₋₆ alkyl, C₁₋₆ alkoxy or C₁₋₆ haloalkyl; alternatively, Rₑ and R_{f} on any one or more carbon atoms in L₃, together with the carbon atom to which they are attached, form C₃₋₇ cycloalkyl or 3- to 7-membered heterocyclyl;
m, n and h are independently selected from 1, 2, 3, 4 or 5;
A is selected from -C(O)- or -NHC(O)-;
T is -(CRᵢRⱼ)_{q}-;
Rᵢ and Rⱼ are each independently H, D, C₁₋₆ alkyl, C₁₋₆ alkoxy or C₁₋₆ haloalkyl; alternatively, Rᵢ and Rⱼ on any one or more carbon atoms in T, together with the carbon atom to which they are attached, form C₃₋₇ cycloalkyl or 3- to 7-membered heterocyclyl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-;
Rₖ and Rₘ are each independently H, D, C₁₋₆ alkyl, C₁₋₆ alkoxy or C₁₋₆ haloalkyl; alternatively, Rₖ and Rₘ on any one or more carbon atoms in B, together with the carbon atom to which they are attached, form C₃₋₇ cycloalkyl or 3- to 7-membered heterocyclyl;
P is selected from a chemical bond or 1 amino acid residue;
preferably,
each W is independently 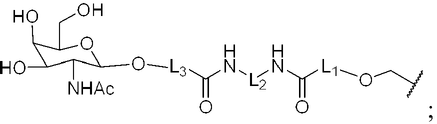
L₁ is -(CRₐR_{b})ₘ-;
Rₐ and R_{b} are each independently H, D, C₁₋₆ alkyl or C₁₋₆ alkoxy; alternatively, Rₐ and R_{b} on any one or more carbon atoms in L₁, together with the carbon atom to which they are attached, form C₃₋₇ cycloalkyl;
L₂ is -(CR_{c}R_{d})ₙ-;
R_{c} and R_{d} are each independently H, D, C₁₋₆ alkyl or C₁₋₆ alkoxy; alternatively, R_{c} and R_{d} on any one or more carbon atoms in L₂, together with the carbon atom to which they are attached, form C₃₋₇ cycloalkyl;
L₃ is -(CRₑR_{f})ₕ-;
Rₑ and R_{f} are each independently H, D, C₁₋₆ alkyl or C₁₋₆ alkoxy; alternatively, Rₑ and R_{f} on any one or more carbon atoms in L₃, together with the carbon atom to which they are attached, form C₃₋₇ cycloalkyl;
m, n and h are independently selected from 1, 2, 3, 4 or 5;
A is selected from -C(O)- or -NHC(O)-;
T is -(CRᵢRⱼ)_{q}-;
Rᵢ and Rⱼ are each independently H, D, C₁₋₆ alkyl or C₁₋₆ alkoxy;
q is selected from 4, 5, 6, 7, 8, 9, 10, 11 or 12;
B is selected from -C(O)-(CRₖRₘ)₀₋₆-, -NH-(CRₖRₘ)₀₋₆- or -OC(O)-(CRₖRₘ)₀₋₆-;
Rₖ and Rₘ are each independently H, D, C₁₋₆ alkyl or C₁₋₆ alkoxy;
P is selected from a chemical bond or 1 amino acid residue, preferably a chemical bond;
more preferably,
each W is independently 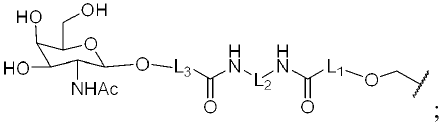
L₁ is -(CRₐR_{b})ₘ-;
Rₐ and R_{b} are each independently H, C₁₋₆ alkyl or C₁₋₆ alkoxy, and Rₐ and R_{b} on at least one carbon atom in L₁, together with the carbon atom to which they are attached, form C₃₋₅ cycloalkyl;
L₂ is -(CR_{c}R_{d})ₙ-;
R_{c} and R_{d} are each independently H, C₁₋₆ alkyl or C₁₋₆ alkoxy;
L₃ is -(CRₑR_{f})ₕ-;
Rₑ and R_{f} are each independently H, C₁₋₆ alkyl or C₁₋₆ alkoxy;
m, n and h are independently selected from 1, 2, 3, 4 or 5;
A is selected from -C(O)- or -NHC(O)-, preferably -NHC(O)-;
T is -(CRᵢRⱼ)_{q}-;
Rᵢ and Rⱼ are each independently H, C₁₋₆ alkyl or C₁₋₆ alkoxy;
q is selected from 4, 5, 6, 7, 8, 9, 10, 11 or 12;
B is -C(O)-(CRₖRₘ)₀₋₆-;
Rₖ and Rₘ are each independently H, C₁₋₆ alkyl or C₁₋₆ alkoxy;
P is a chemical bond.

In a more specific embodiment, the present invention provides the above-mentioned delivery ligand, wherein each W is the same or different, and is independently selected from: H; 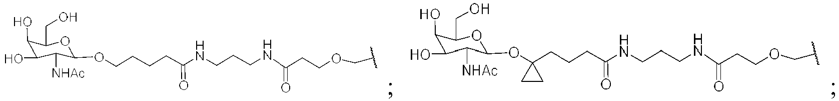 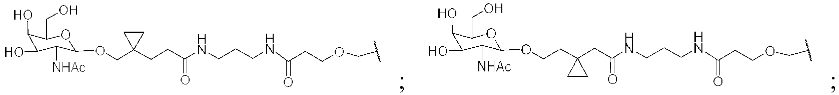 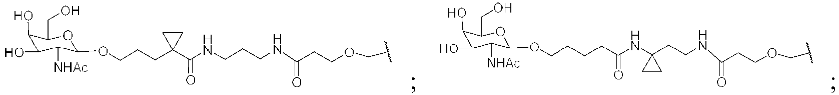 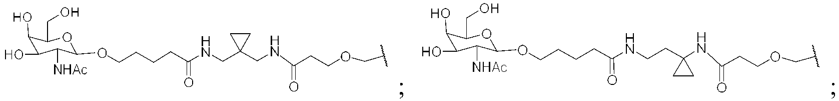 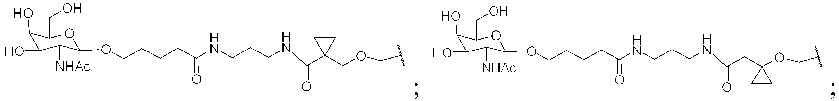 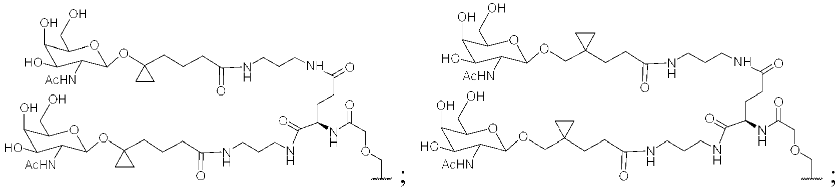 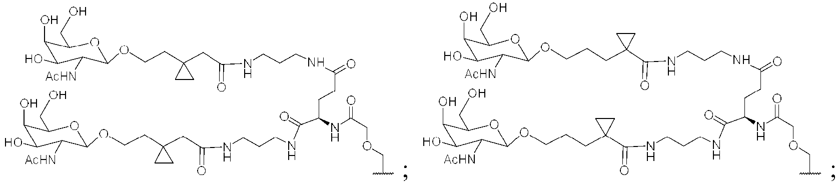 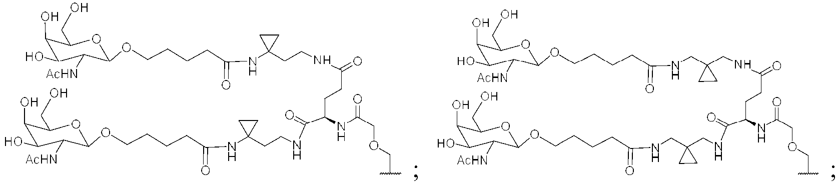 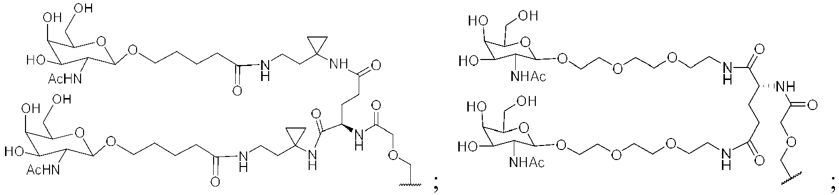 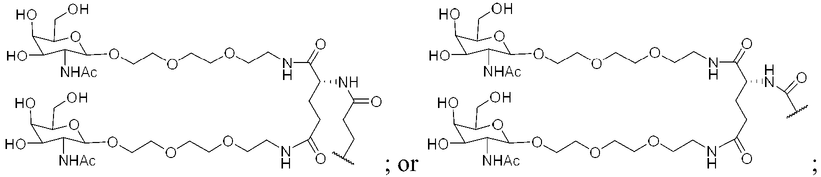 preferably, each W is independently selected from: H;

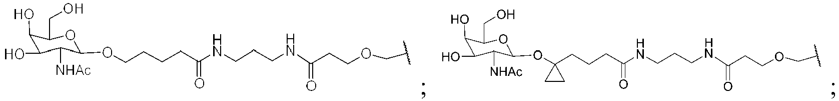

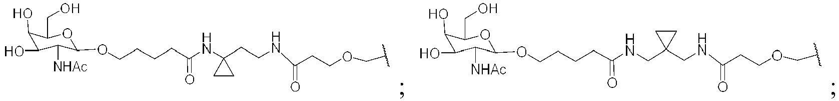

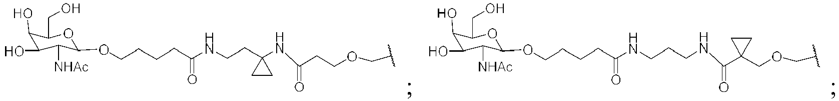

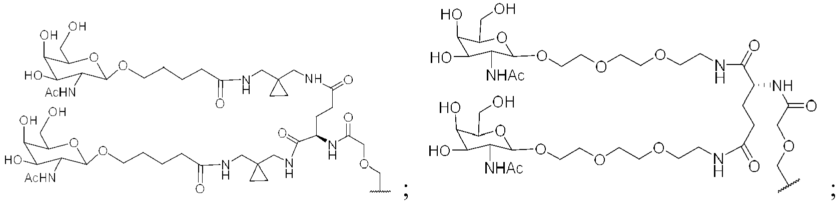

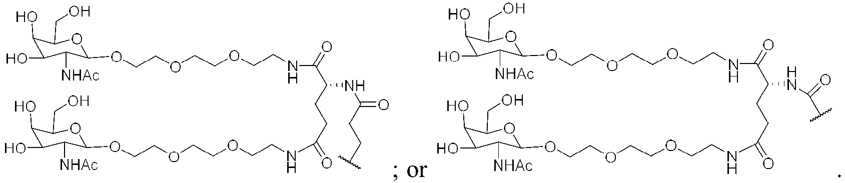

In a more specific embodiment, the present invention provides the above-mentioned delivery ligand, wherein 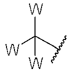 is selected from: H,

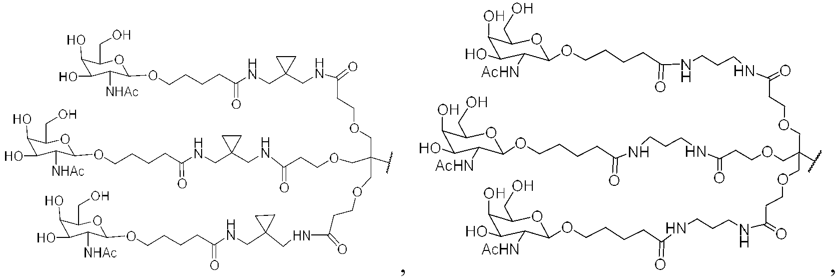

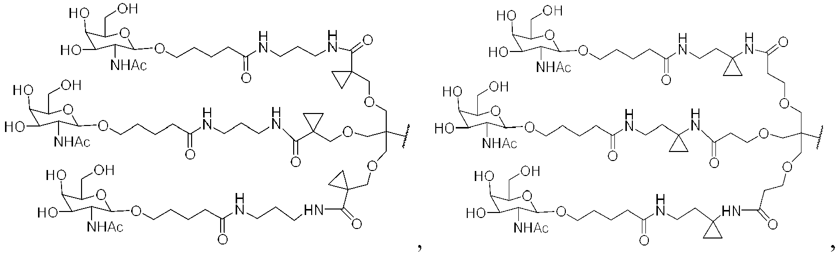

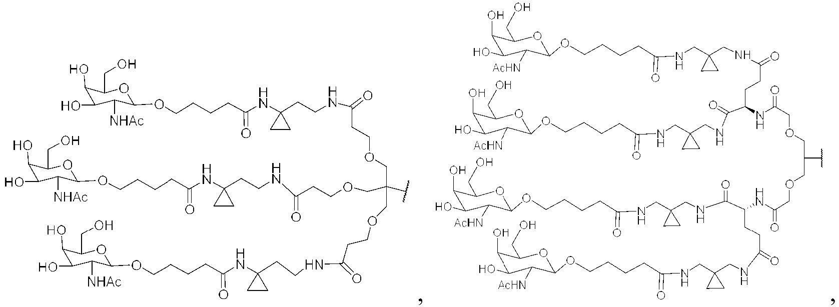

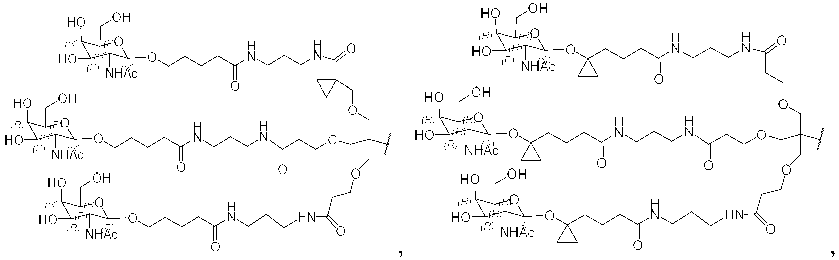

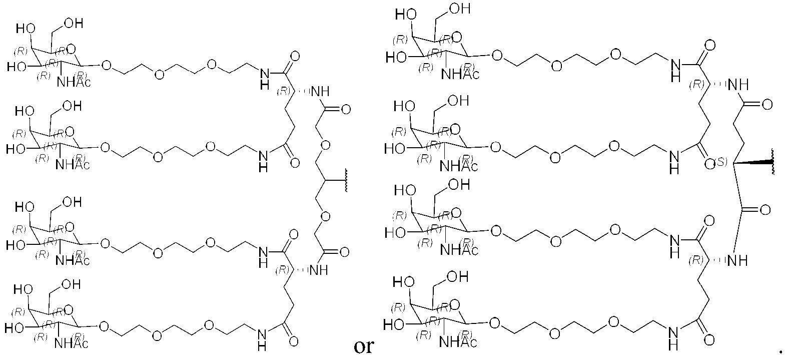

In a more specific embodiment, the present invention provides the above-mentioned delivery ligand, wherein the -A-T-B-P- is selected from the following: 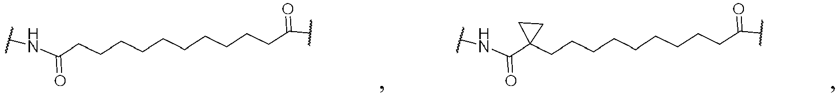 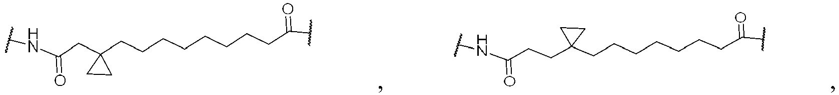 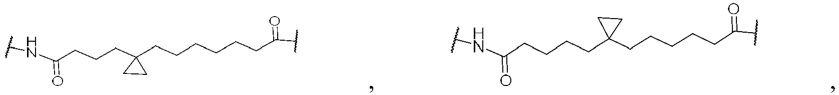 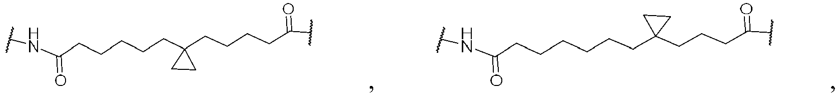 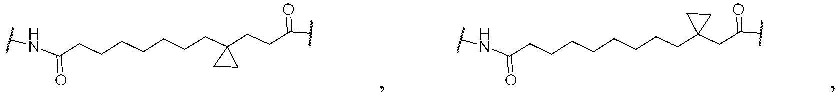 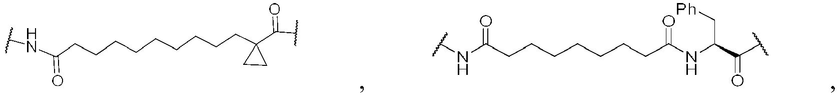 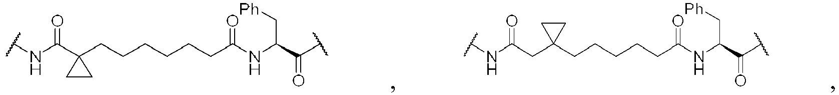 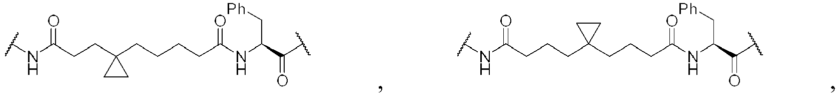 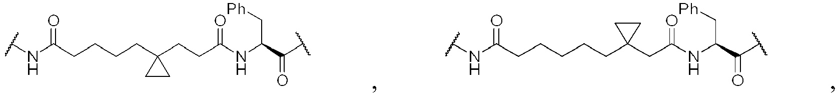 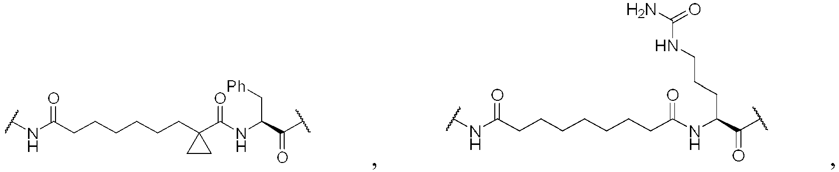 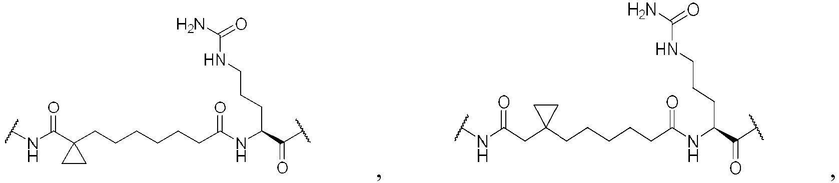 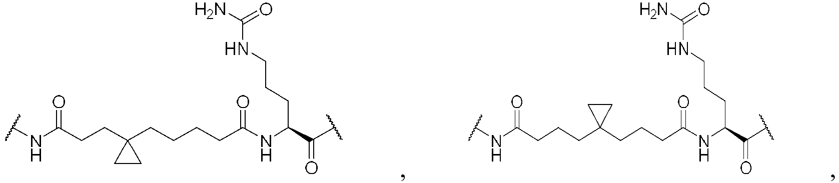 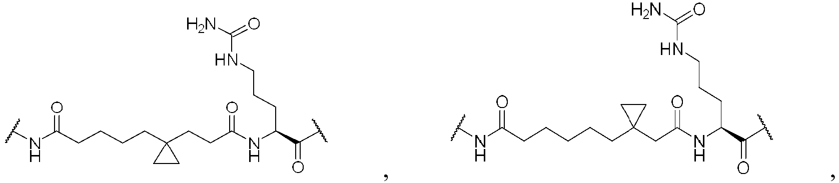 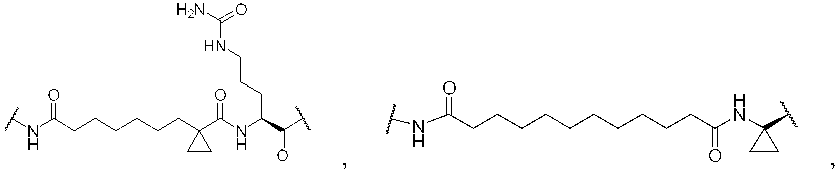 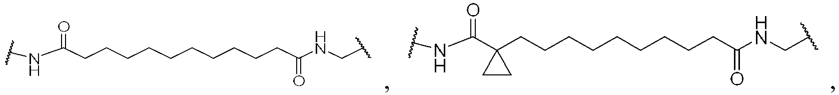 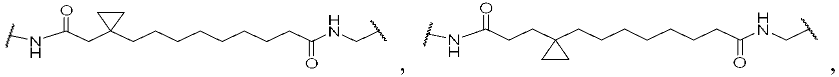 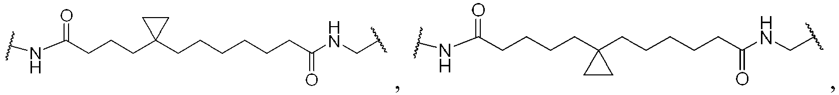 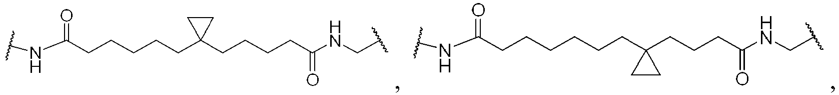 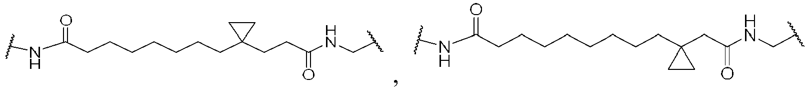 or 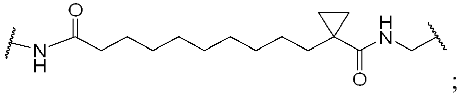 preferably, the -A-T-B-P- is selected from the following:

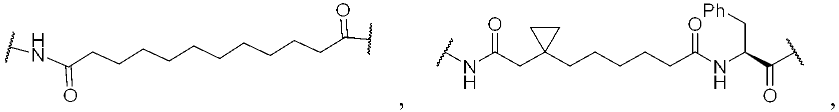

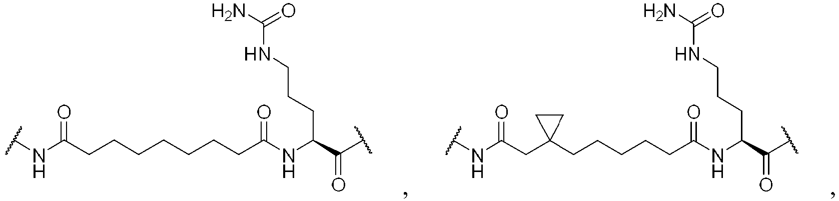

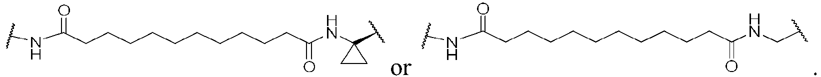

In a more specific embodiment, the present invention provides the above-mentioned delivery ligand, wherein the conjugated group is selected from a structure as shown in Table A, or an isotopic variant, tautomer, or stereoisomer thereof, or a mixture thereof:

**Table A. Representative conjugated groups of the present invention**

| No. | Structural formula |
|---|---|
| A1 | 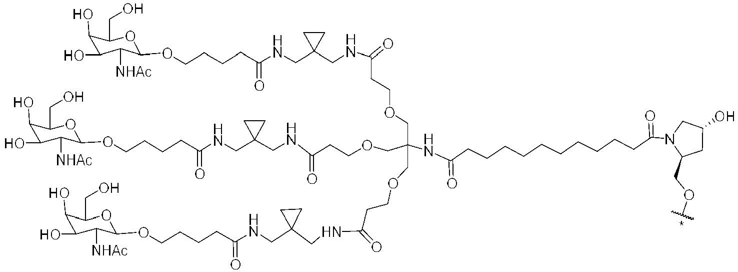 |
| A2 | 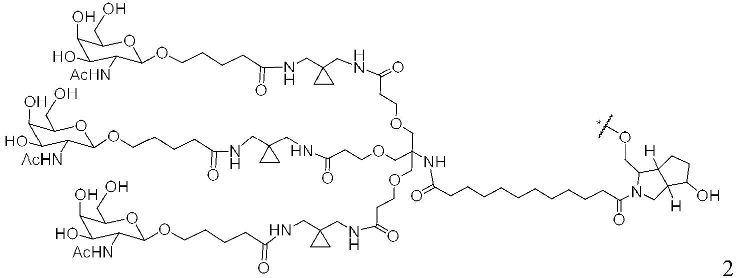 |
| | 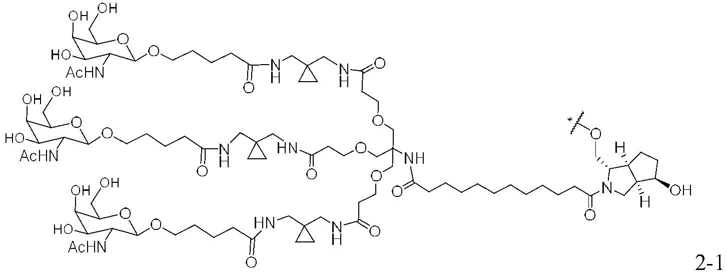 |
| | 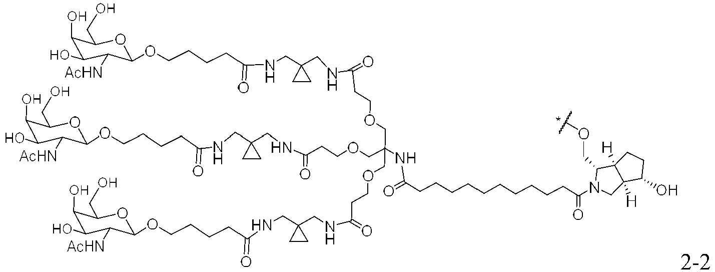 |
| | 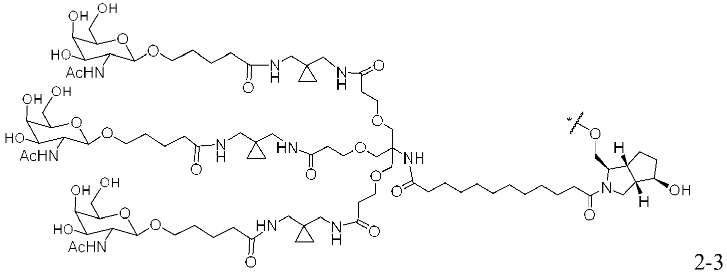 |
| | 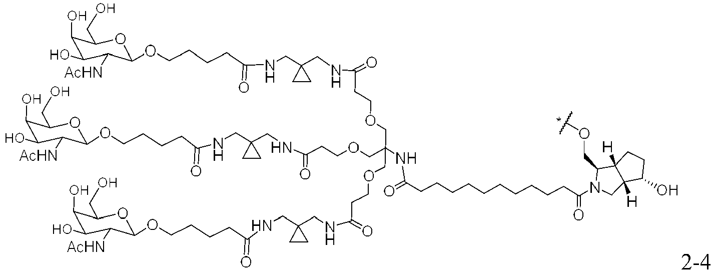 |
| A3 | 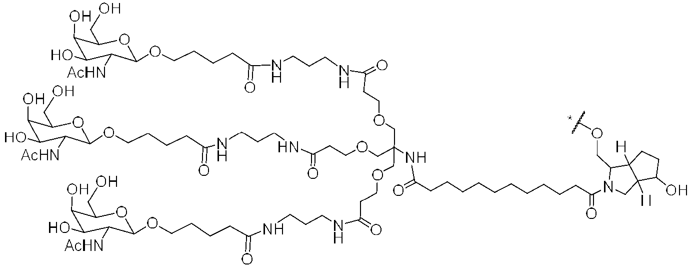 |
| A4 | 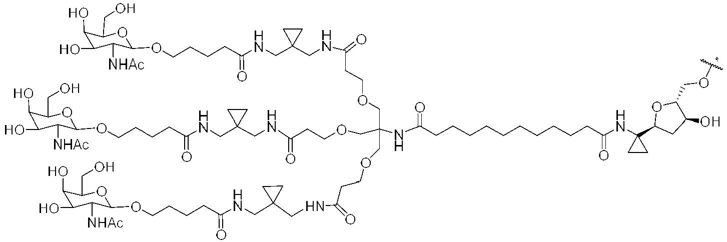 |
| A5 | 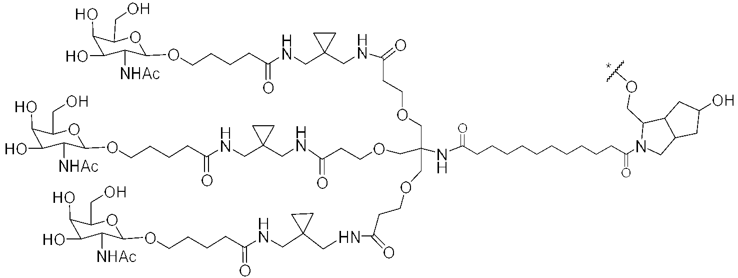 |
| | 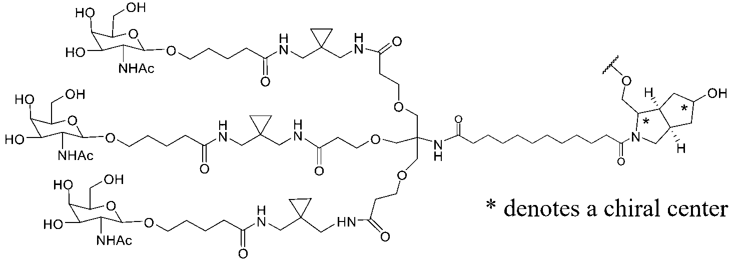 |
| | 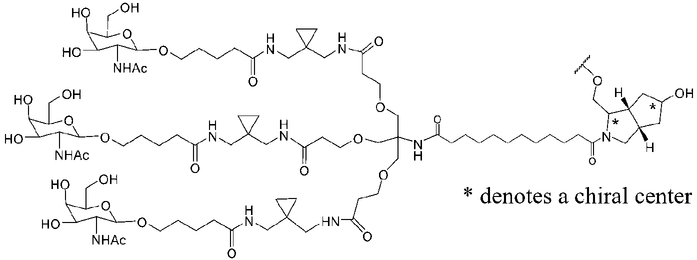 |
| A6 | 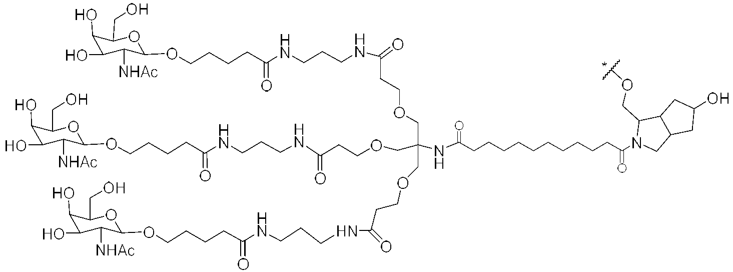 |
| A7 | 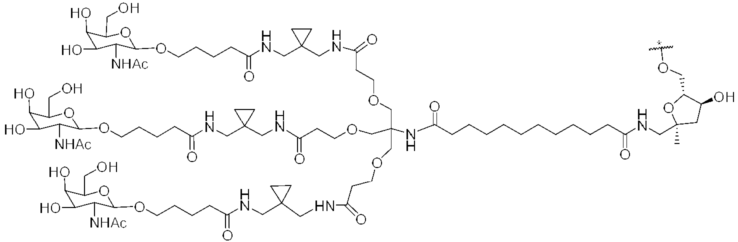 |
| A8 | 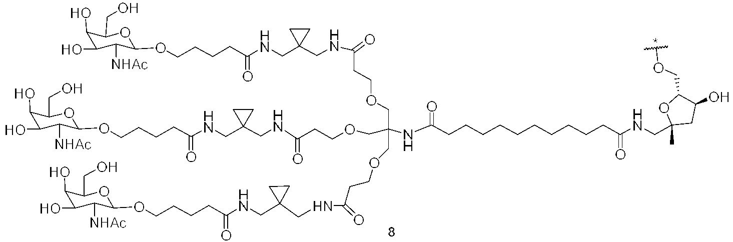 |
| A9 | 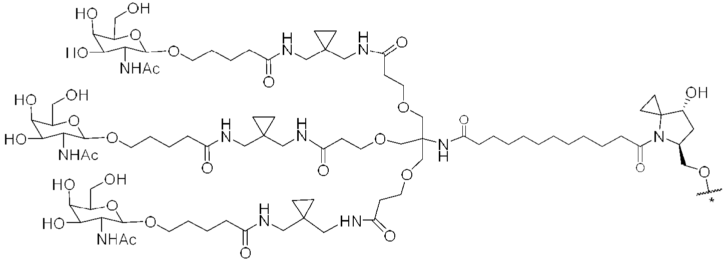 |
| A10 | 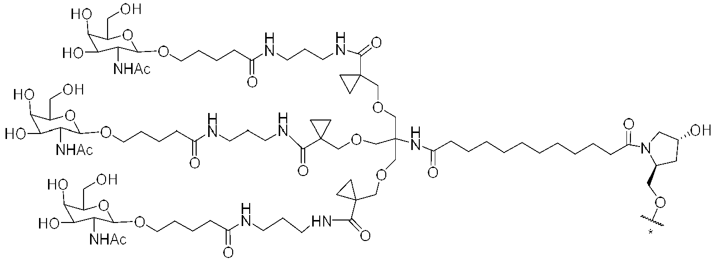 |
| A11 | 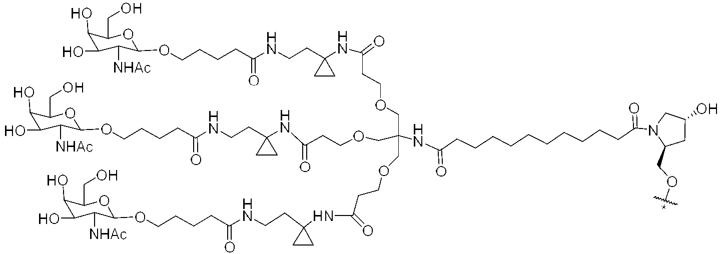 |
| A12 | 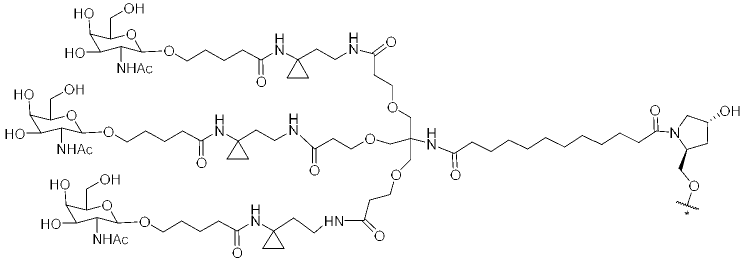 |
| A13 | 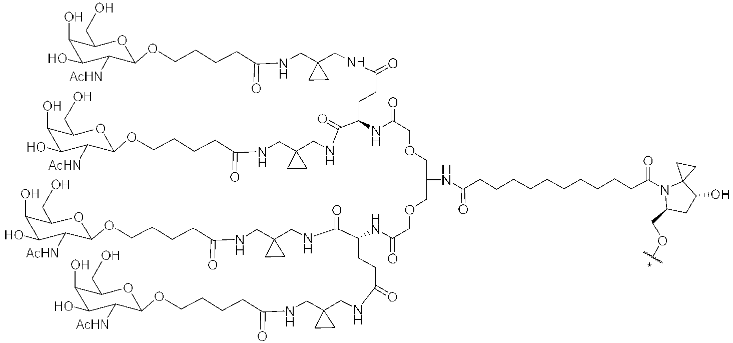 |
| A14 | 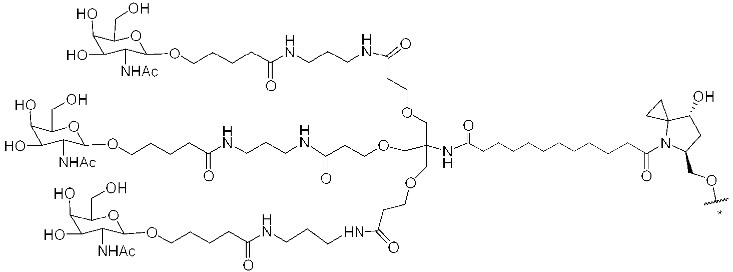 |
| A15 | 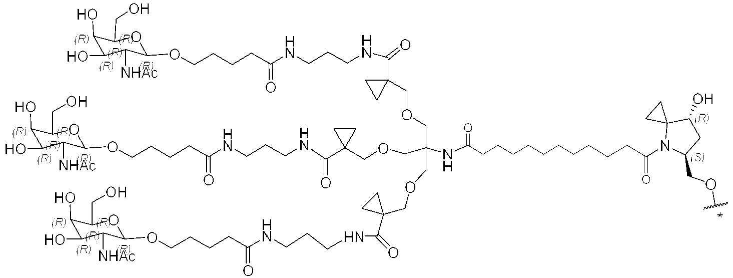 |
| A16 | 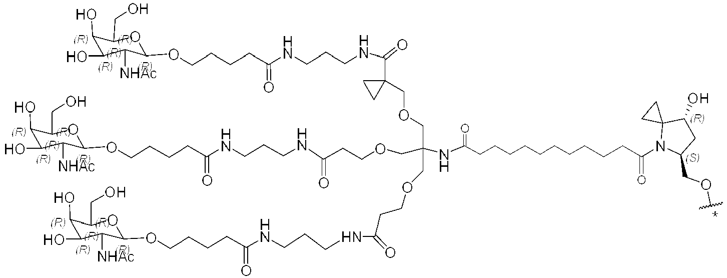 |
| A17 | 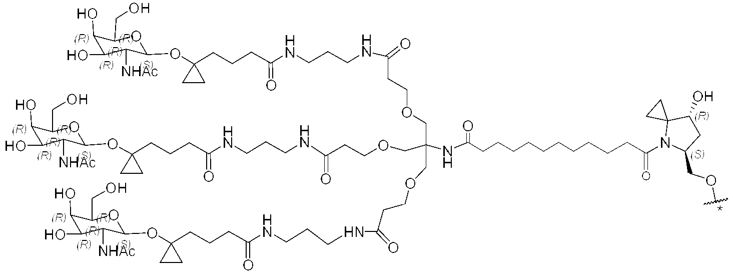 |
| A18 | 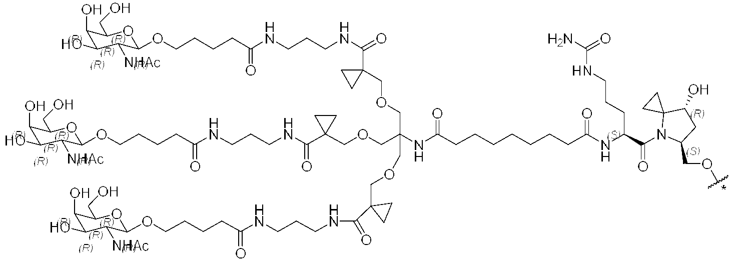 |
| A19 | 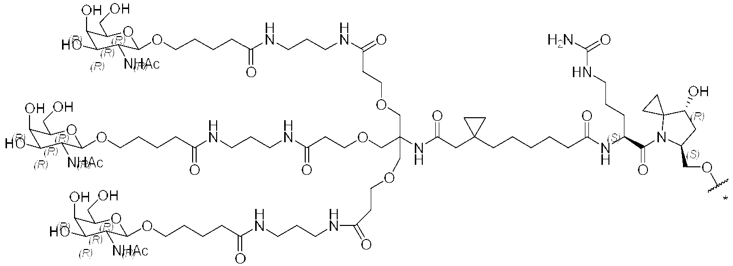 |
| A20 | 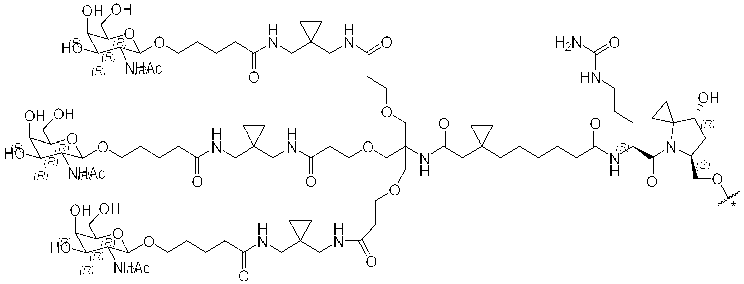 |
| A21 | 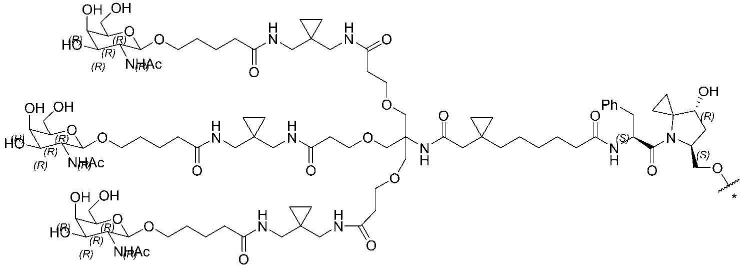 |
| A22 | 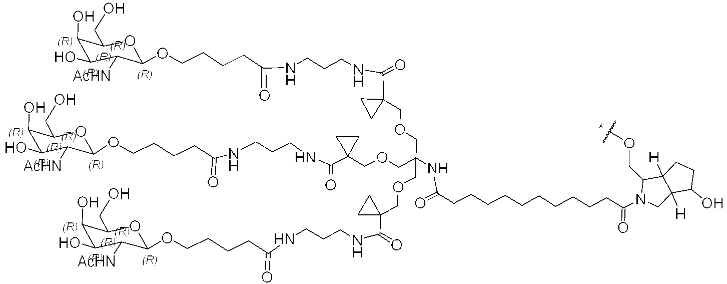 |
| A23 | 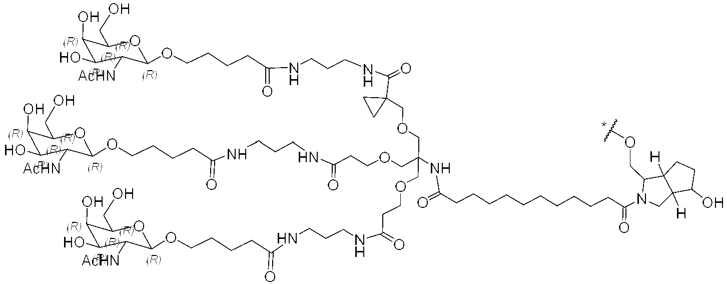 |
| A24 | 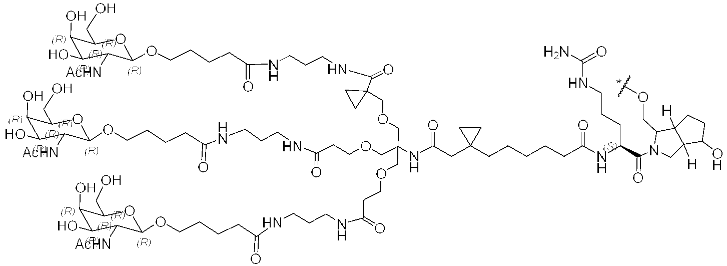 |
| A25 | 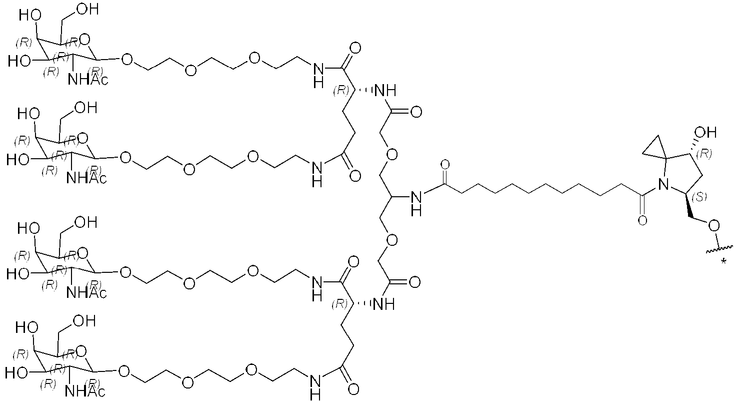 |
| A26 | 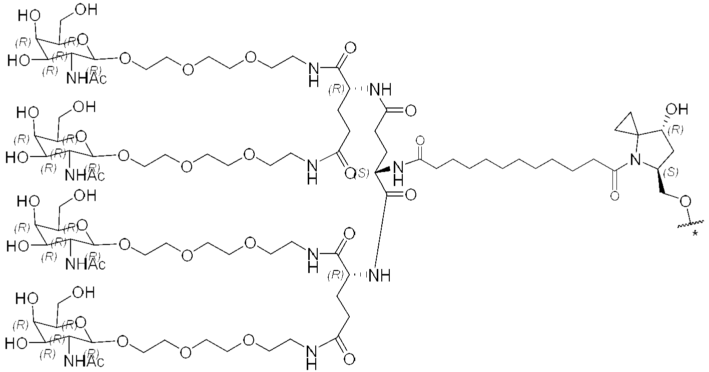 |
| A27 | 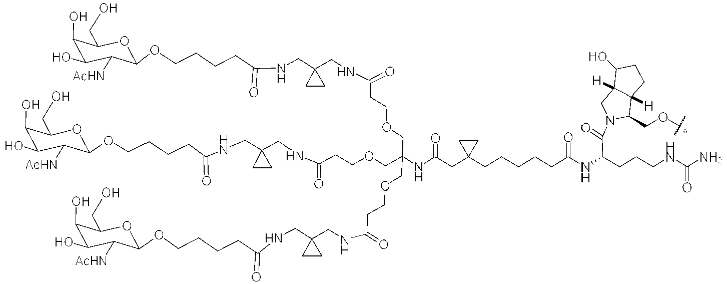 |
| A28 | 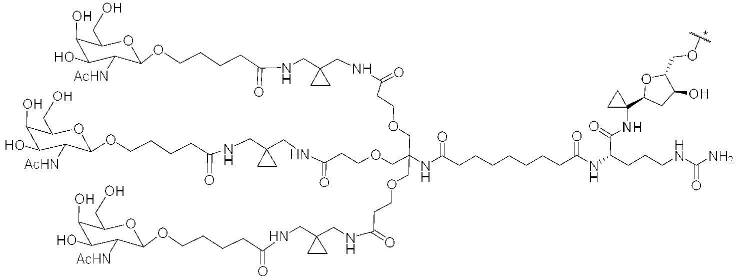 |

| | |
|---|---|
| wherein * denotes a chiral centre, which is independently selected from (S)- or (R)-configuration, or a mixture thereof, and the conjugated group is attached to a biomolecule via 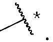 | |

In a more specific embodiment, the present invention provides the above-mentioned delivery ligand, wherein the biomolecule is a nucleic acid.

In a more specific embodiment, the present invention provides the above-mentioned delivery ligand, targeting an asialoglycoprotein receptor (ASGPR) on the surface of hepatocytes.

In a more specific embodiment, the present invention provides a nucleic acid molecule, comprising a nucleic acid and the above-mentioned delivery ligand attached thereto.

In a more specific embodiment, the present invention provides the above-mentioned nucleic acid molecule, wherein the nucleic acid is selected from DNA, RNA and a DNA/RNA hybrid.

In a more specific embodiment, the present invention provides the above-mentioned nucleic acid molecule, wherein the nucleic acid molecule is single-stranded or double-stranded.

In a more specific embodiment, the present invention provides the above-mentioned nucleic acid molecule, wherein the nucleic acid molecule is selected from small interfering RNA (siRNA) and short hairpin RNA (shRNA).

In a more specific embodiment, the present invention provides the above-mentioned nucleic acid molecule, specifically binding to the asialoglycoprotein receptor (ASGPR) on the surface of hepatocytes.

In a more specific embodiment, the present invention provides a double-stranded RNA molecule, comprising a sense strand and an antisense strand, wherein the double-stranded RNA molecule contains one or more of the above-mentioned delivery ligands.

In a more specific embodiment, the present invention provides the above-mentioned double-stranded RNA molecule, wherein the target of the double-stranded RNA is selected from CFB, TTR, or PCSK9.

In a more specific embodiment, the present invention provides the above-mentioned double-stranded RNA molecule, wherein in the double-stranded RNA, the sense strand comprises:
(1) a nucleotide sequence having at least 80%, at least 85%, at least 90%, at least 95%, at least 98%, at least 99%, or 100% sequence identity to SEQ ID NO: 1;
(2) a nucleotide sequence having at least 80%, at least 85%, at least 90%, at least 95%, at least 98%, at least 99%, or 100% sequence identity to SEQ ID NO: 5; or
(3) a nucleotide sequence having at least 80%, at least 85%, at least 90%, at least 95%, at least 98%, at least 99%, or 100% sequence identity to SEQ ID NO: 8.

In a more specific embodiment, the present invention provides the above-mentioned double-stranded RNA molecule, wherein in the double-stranded RNA, the antisense strand comprises:
(1) a nucleotide sequence having at least 80%, at least 85%, at least 90%, at least 95%, at least 98%, at least 99%, or 100% sequence identity to SEQ ID NO: 2;
(2) a nucleotide sequence having at least 80%, at least 85%, at least 90%, at least 95%, at least 98%, at least 99%, or 100% sequence identity to SEQ ID NO: 4; or
(3) a nucleotide sequence having at least 80%, at least 85%, at least 90%, at least 95%, at least 98%, at least 99%, or 100% sequence identity to SEQ ID NO: 7.

In a more specific embodiment, the present invention provides the above-mentioned double-stranded RNA molecule, wherein in the double-stranded RNA, the sense strand has:
(1) a nucleotide sequence as shown in SEQ ID NO: 1;
(2) a nucleotide sequence as shown in SEQ ID NO: 5; or
(3) a nucleotide sequence as shown in SEQ ID NO: 8.

In a more specific embodiment, the present invention provides the above-mentioned double-stranded RNA molecule, wherein in the double-stranded RNA, the antisense strand has:
(1) a nucleotide sequence as shown in SEQ ID NO: 2;
(2) a nucleotide sequence as shown in SEQ ID NO: 4; or
(3) a nucleotide sequence as shown in SEQ ID NO: 7.

In a more specific embodiment, the present invention provides the above-mentioned double-stranded RNA molecule, wherein the conjugated group is attached to the 3'-end of the sense strand of the double-stranded RNA molecule.

In a more specific embodiment, the present invention provides the above-mentioned double-stranded RNA molecule, wherein the conjugated group is attached to the 5'-end of the sense strand of the double-stranded RNA molecule.

In a more specific embodiment, the present invention provides the above-mentioned double-stranded RNA molecule, wherein the conjugated group is attached to the 3'-end and 5'-end of the sense strand of the double-stranded RNA molecule.

In a more specific embodiment, the present invention provides the above-mentioned double-stranded RNA molecule, wherein the conjugated group is attached to the 3'-end of the antisense strand of the double-stranded RNA molecule.

In a more specific embodiment, the present invention provides the above-mentioned double-stranded RNA molecule, wherein the conjugated group is attached to the 5'-end of the antisense strand of the double-stranded RNA molecule.

In a more specific embodiment, the present invention provides the above-mentioned double-stranded RNA molecule, wherein the conjugated group is attached to the 3'-end and 5'-end of the antisense strand of the double-stranded RNA molecule.

In a more specific embodiment, the present invention provides the above-mentioned double-stranded RNA molecule, wherein each of the sense strand and the antisense strand of the double-stranded RNA molecule has 10 to 30 nucleotides, preferably 15 to 25 nucleotides, more preferably 20 to 25 nucleotides.

In a more specific embodiment, the present invention provides the above-mentioned double-stranded RNA molecule, wherein one or more nucleotides in the sense strand and/or the antisense strand of the double-stranded RNA molecule comprise a chemical modification.

In a more specific embodiment, the present invention provides the above-mentioned double-stranded RNA molecule, which is used for inhibiting the expression or activity of a target gene.

In a more specific embodiment, the present invention provides a pharmaceutical composition, comprising the above-mentioned nucleic acid molecule or the above-mentioned double-stranded RNA molecule, and a pharmaceutically acceptable carrier or excipient.

In a more specific embodiment, the present invention provides a kit, comprising the above-mentioned nucleic acid molecule or the above-mentioned double-stranded RNA molecule.

In a more specific embodiment, the present invention provides a compound of formula (X'), or a pharmaceutically acceptable salt, stereoisomer, tautomer or isotopic variant thereof, or a mixture thereof: 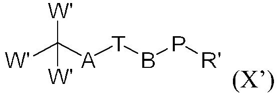 wherein
R' is selected from 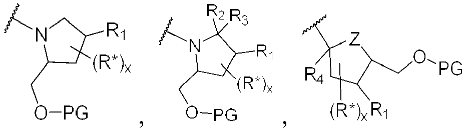 or 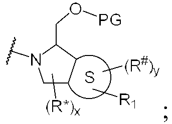
PG is a hydroxyl protecting group, such as trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), - C(O)CH₂CH₂C(O)OH or 4,4'-dimethoxytrityl (DMTr);
preferably, PG is selected from 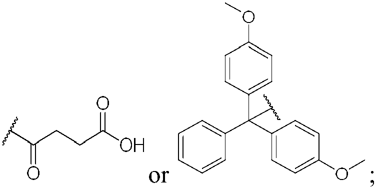
each W' is independently selected from H, D, 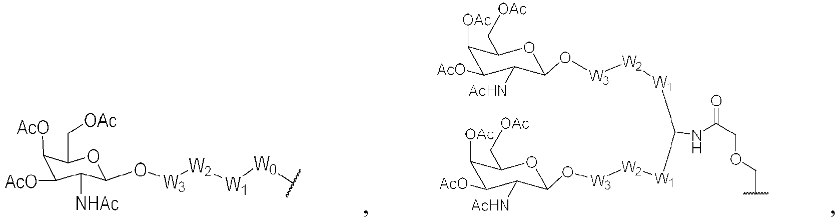 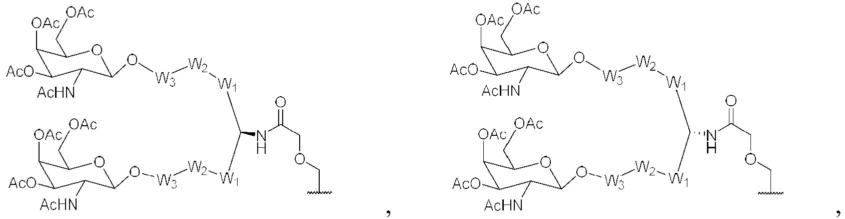 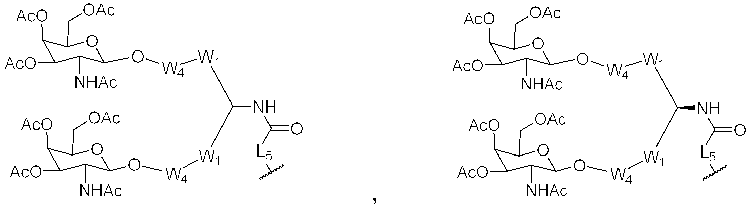 or 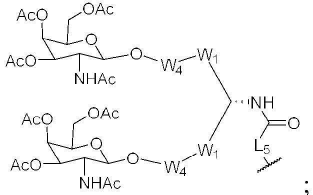
provided that, when R' is 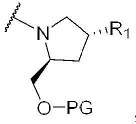 at least one pair among Rₐ and R_{b}, R_{c} and R_{d}, Rₑ and R_{f}, R_{g} and Rₕ, Rᵢ and Rⱼ, and Rₖ and Rₘ in W', together with the carbon atom to which they are attached, forms C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl;
each of the other variables is as defined herein.

In a more specific embodiment, the present invention provides the above-mentioned compound of formula (X'), which is selected from a structure as shown in formula (Ia), formula (Ia-1), formula (Ia-2), formula (Ia'), formula (Ia'-1), formula (Ia'-2), formula (IIa), formula (IIa-1), formula (IIa-2), formula (IIa'), formula (IIa'-1), formula (IIa'-2), formula (IIIa), formula (IIIa-1), formula (IIIa-2), formula (IIIa-3), formula (IIIa-4), formula (IIIa'), formula (IIIa'-1), formula (IIIa'-2), formula (IIIa'-3), formula (IIIa'-4), formula (IIIb), formula (IIIb-1), formula (IIIb-2), formula (IIIb-3), formula (IIIb-4), formula (IIIb-5), formula (IIIb-6), formula (IIIb'), formula (IIIb'-1), formula (IIIb'-2), formula (IIIb'-3), formula (IIIb'-4), formula (IIIb'-5), formula (IIIb'-6), formula (IVa), formula (IVa-1), formula (IVa-2), formula (IVa'), formula (IVa'-1), formula (IVa'-2), formula (Va), formula (Va-1), formula (Va-2), formula (Va-3), formula (Va-4), formula (Va'), formula (Va'-1), formula (Va'-2), formula (Va'-3) or formula (Va'-4): 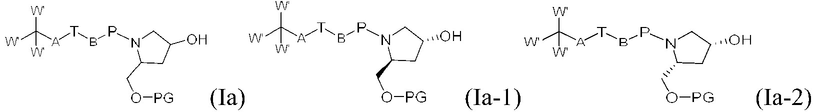 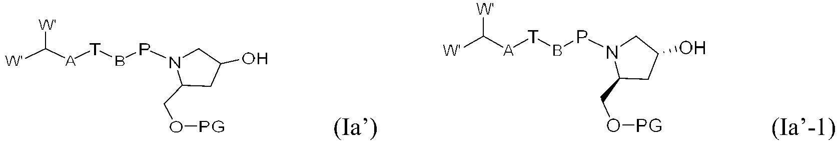 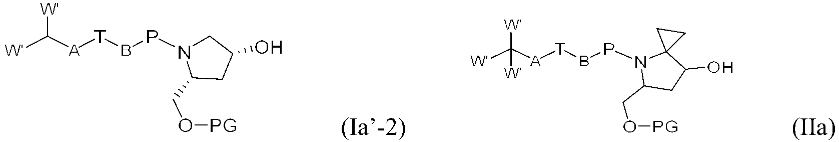 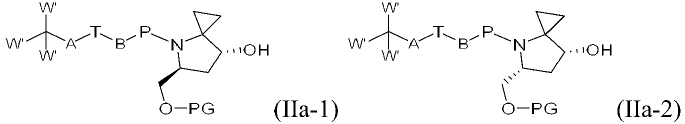 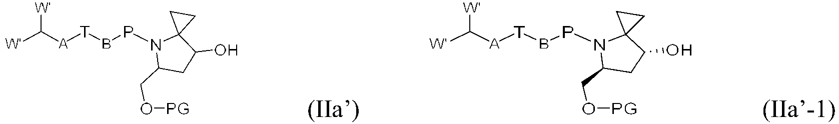 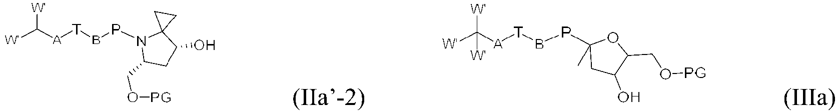 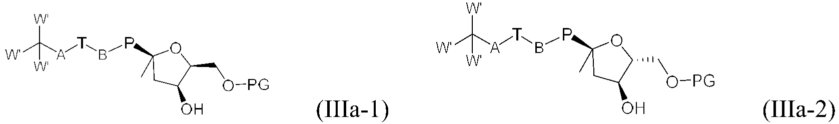 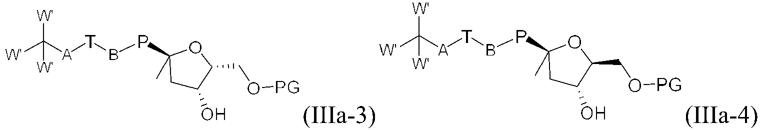 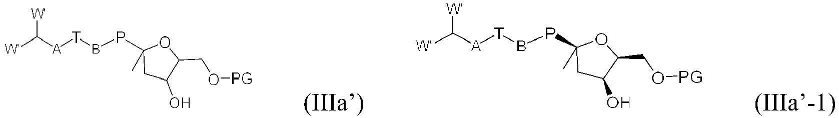 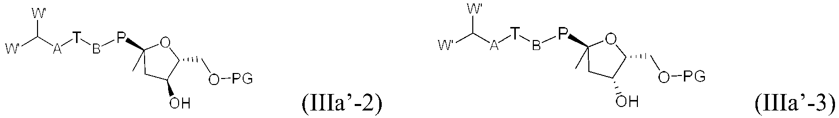 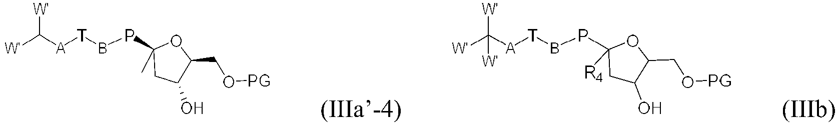 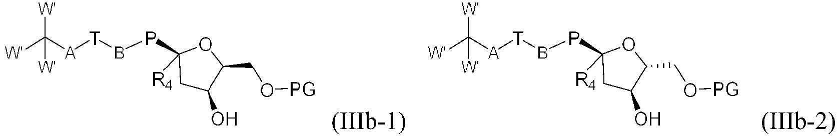 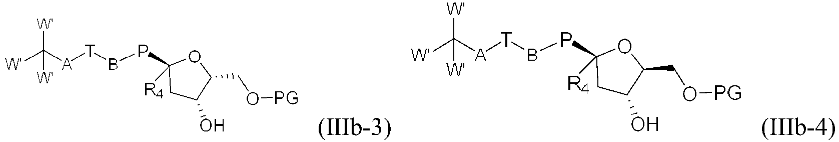 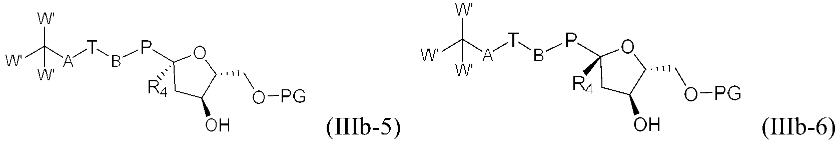 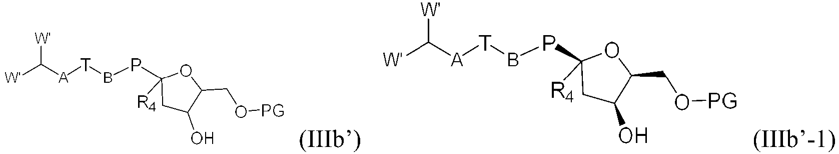 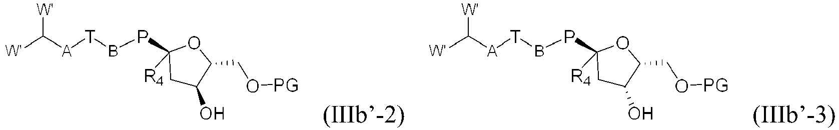 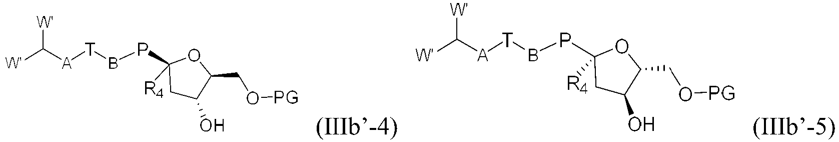 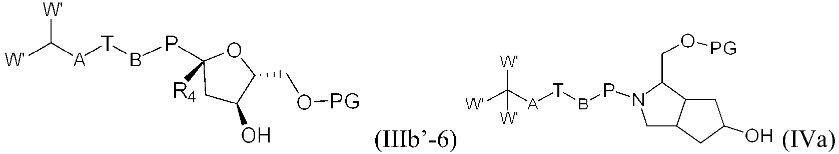 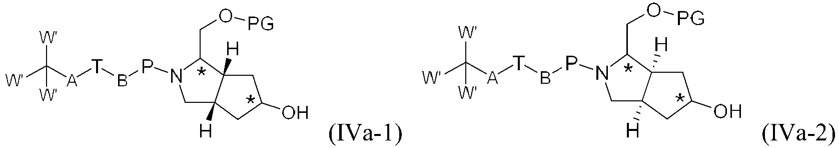 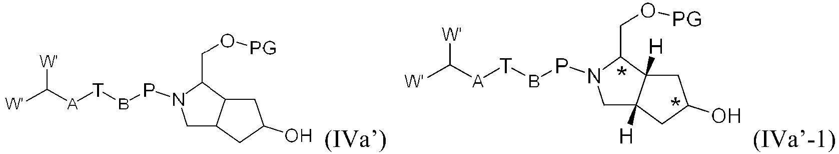 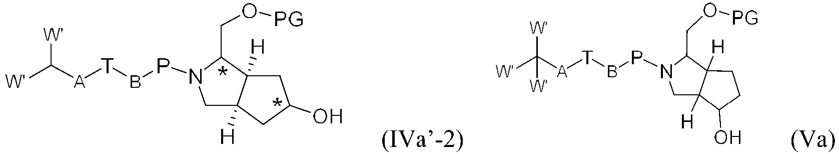 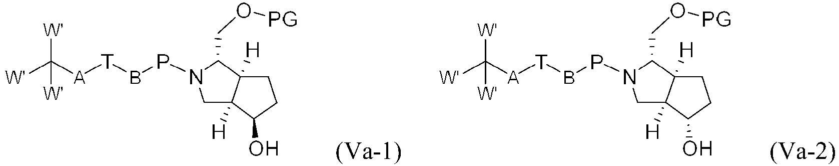 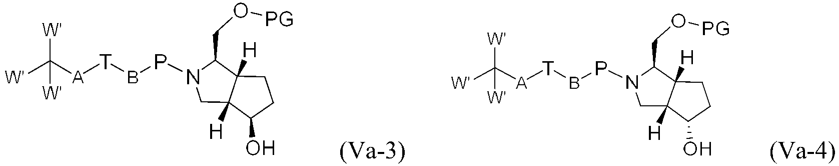 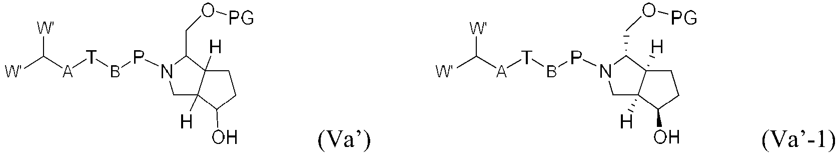 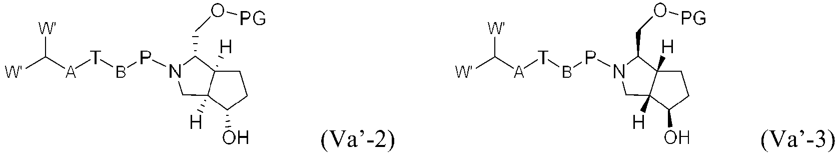 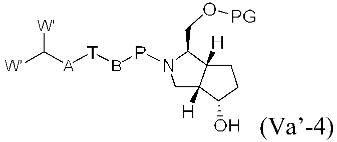 wherein * denotes a chiral centre, which is independently selected from (S)- or (R)-configuration, or a mixture thereof, and each of the other variables is as defined herein.

In a more specific embodiment, the present invention provides the above-mentioned compound of formula (X'), which is selected from a structure as shown in formula (VIa), formula (VIa-1), formula (VIa-2), formula (VIa'), formula (VIa'-1), formula (VIa'-2), formula (VIIa), formula (VIIa-1), formula (VIIa-2), formula (VIIa'), formula (VIIa'-1), formula (VIIa'-2), formula (VIIIa), formula (VIIIa-1), formula (VIIIa-2), formula (VIIIa-3), formula (VIIIa-4), formula (VIIIa-5), formula (VIIIa-6), formula (VIIIa'), formula (VIIIa'-1), formula (VIIIa'-2), formula (VIIIa'-3), formula (VIIIa'-4), formula (VIIIa'-5), formula (VIIIa'-6), formula (IXa), formula (IXa-1), formula (IXa-2), formula (IXa'), formula (IXa'-1) or formula (IXa'-2): 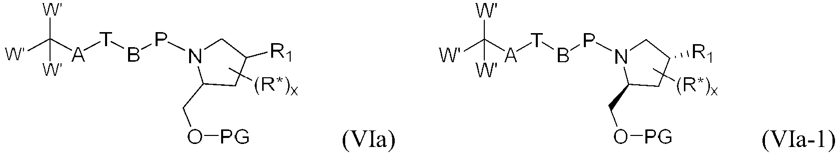 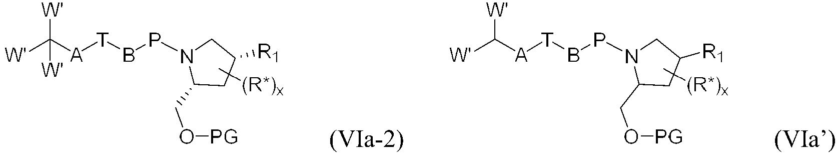 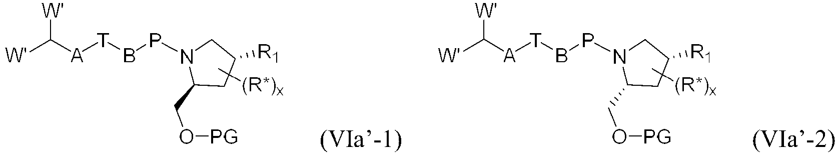 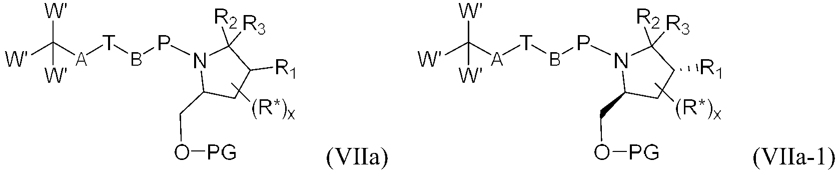 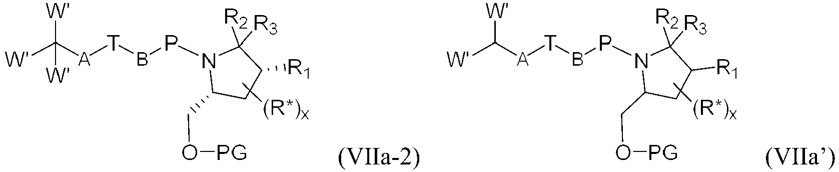 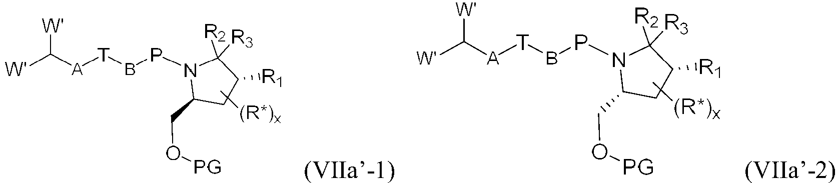 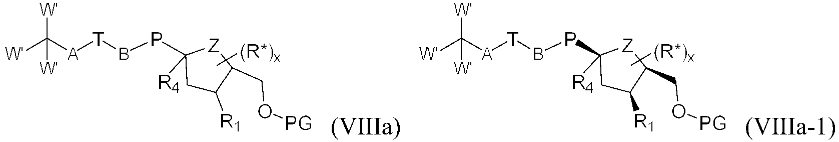 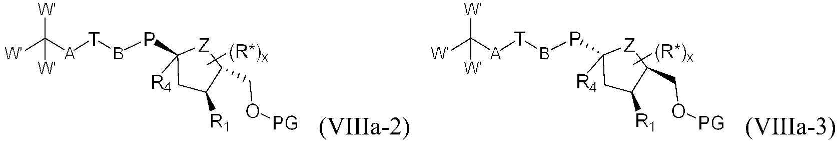 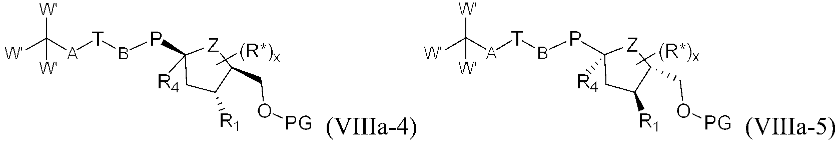 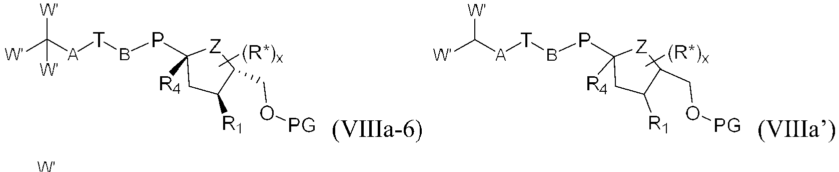 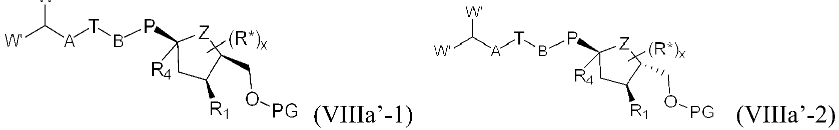 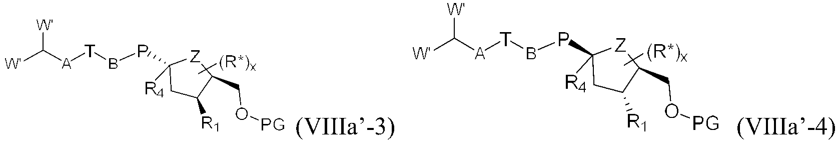 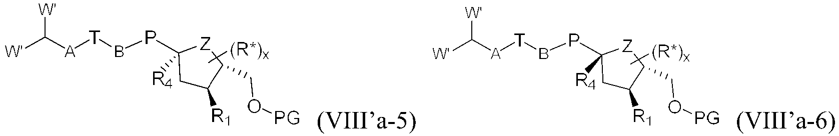 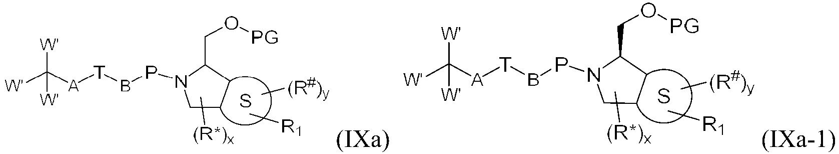 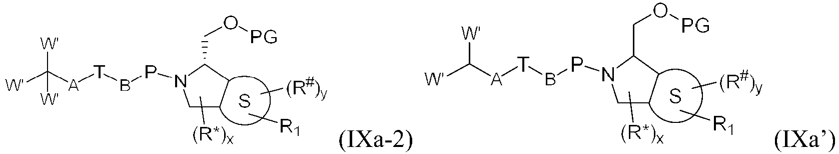 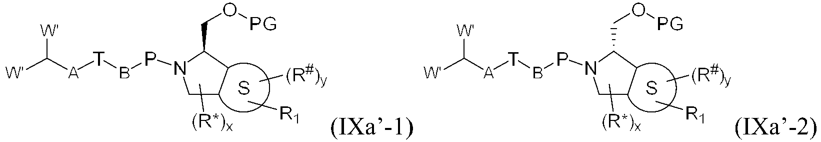 wherein each variable is as defined herein.

In a more specific embodiment, the present invention provides the above-mentioned compound of formula (X'), wherein each W' is independently selected from H, D,

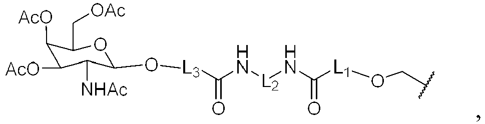

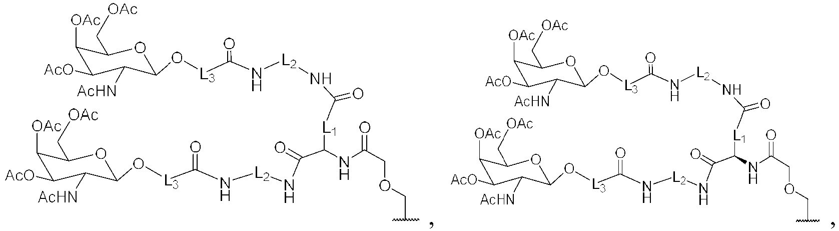

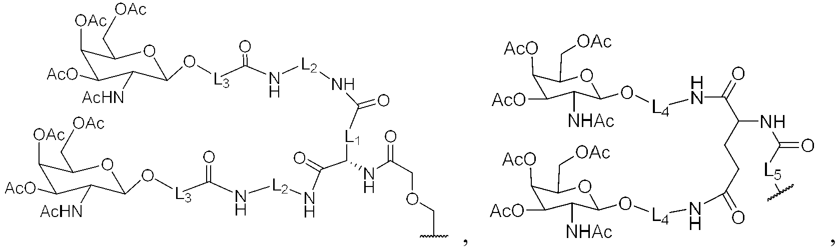

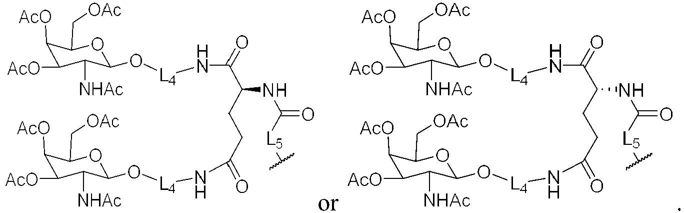

In a more specific embodiment, the present invention provides the above-mentioned compound of formula (X'), wherein each W' is the same or different, and is independently selected from: H; 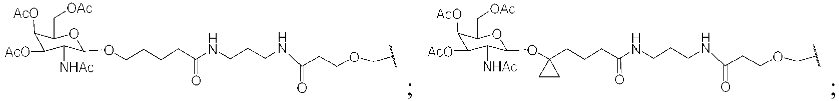 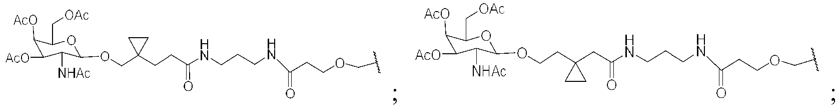 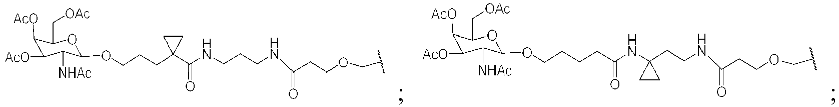 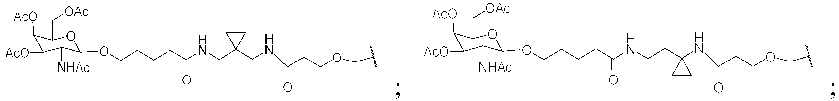 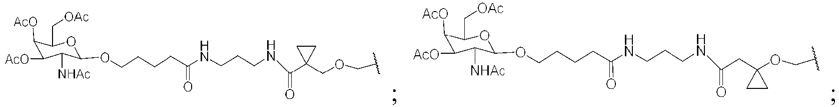 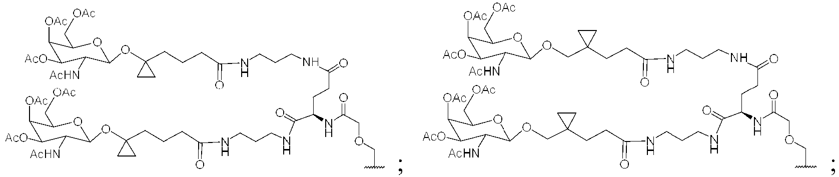 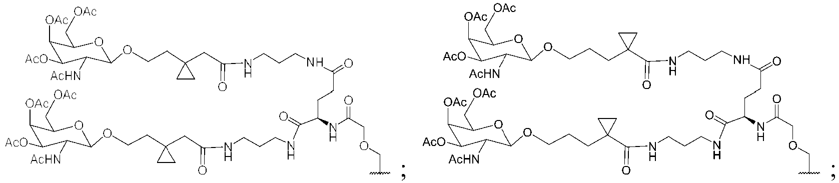 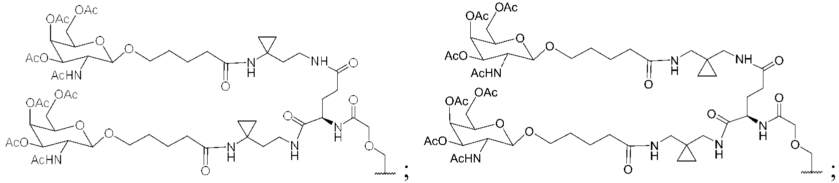 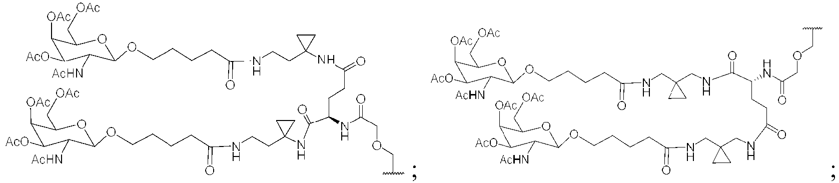 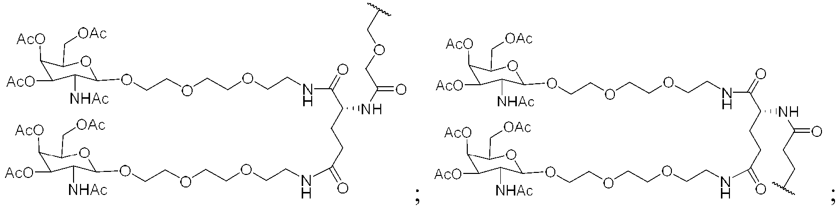 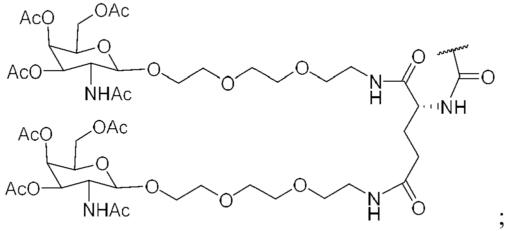 preferably, each W' is independently selected from: H;

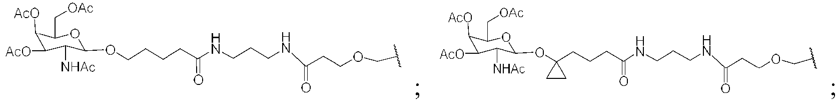

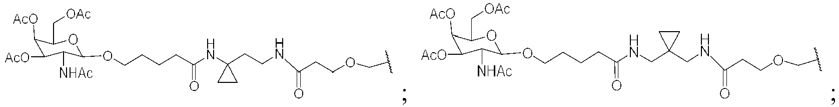

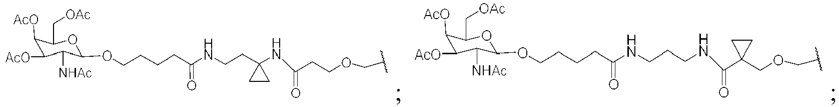

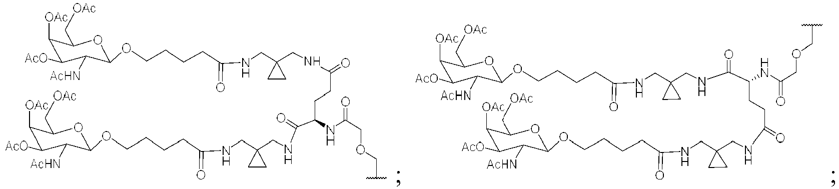

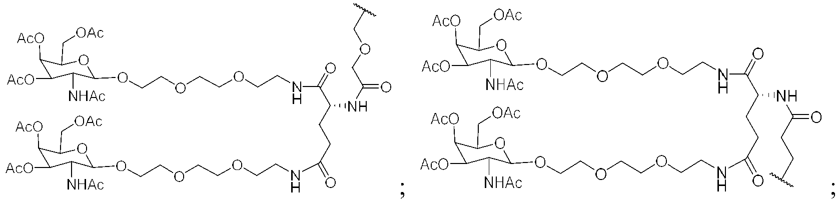

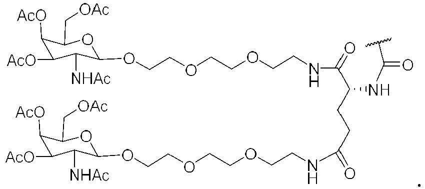

In a more specific embodiment, the present invention provides the above-mentioned compound of formula (X'), wherein R' is selected from 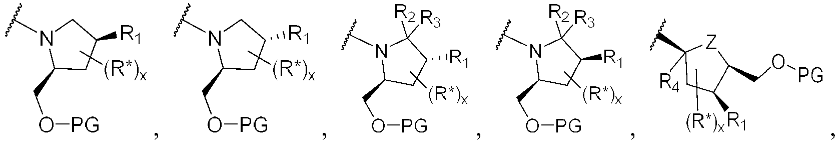 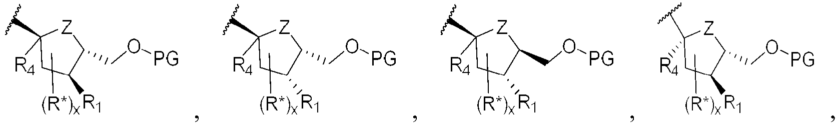 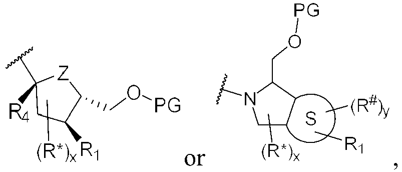
wherein PG, ring S, R₁, R₂, R₃, R₄, Z, R*, R^{#}, x and y are as defined herein;
preferably, R' is selected from 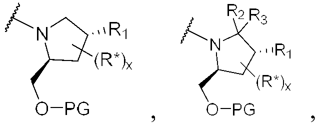 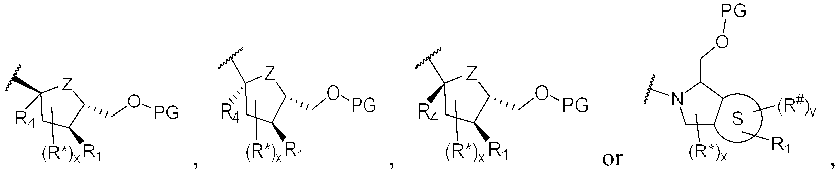 wherein PG, ring S, R₁, R₂, R₃, R₄, Z, R*, R^{#}, x and y are as defined herein;
more preferably, R' is selected from 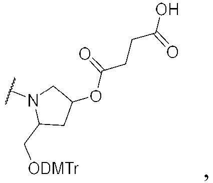 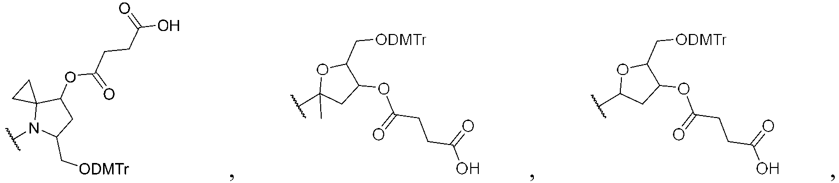 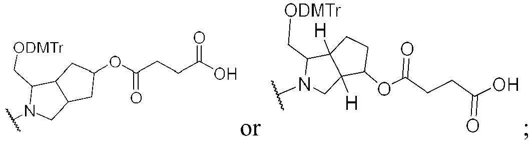
more preferably, R' is selected from 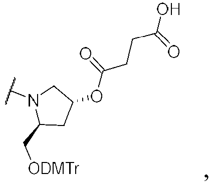 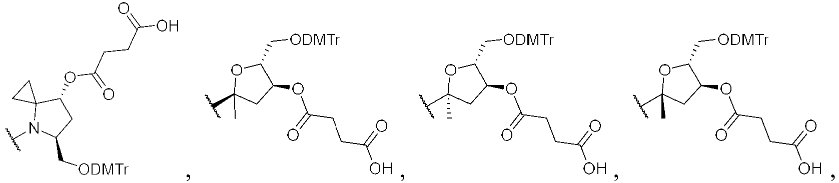 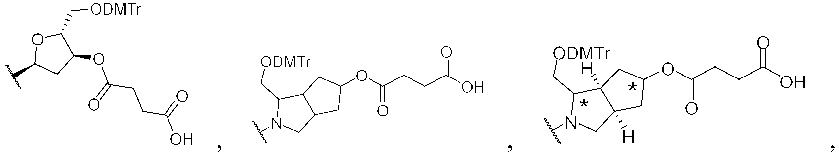 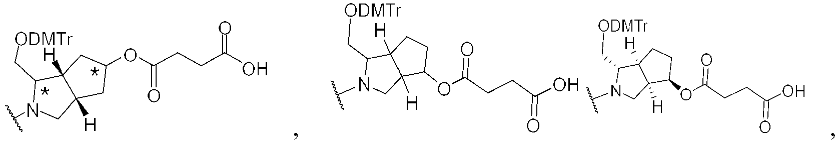 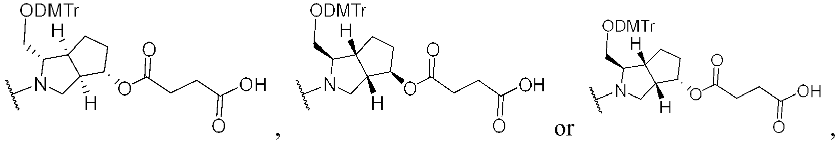 and * denotes a chiral centre, which is independently selected from (S)- or (R)-configuration, or a mixture thereof; more preferably

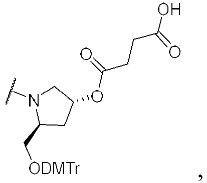

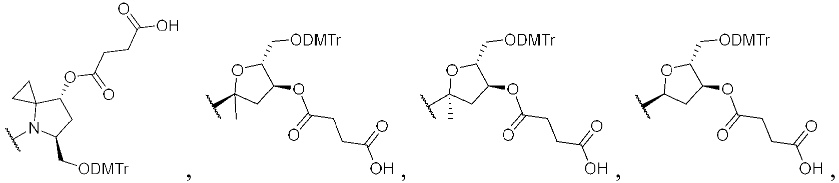

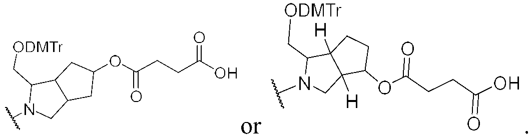

In a more specific embodiment, the present invention provides the above-mentioned compound of formula (X'), which is selected from a compound in Table B, or a pharmaceutically acceptable salt, stereoisomer, tautomer or isotopic variant thereof, or a mixture thereof:

**Table B. Representative compounds of formula (X') of the present invention**

| No. | Structural formula |
|---|---|
| B1 | 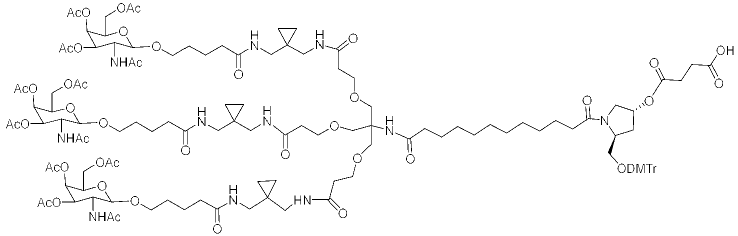 |
| B2 | 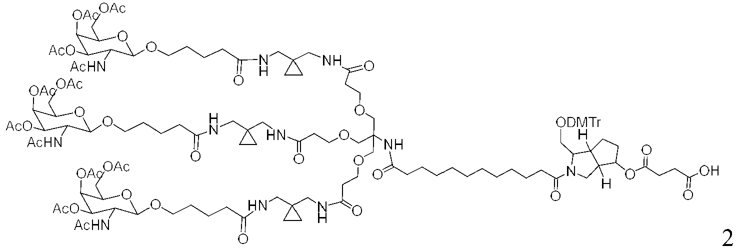 |
| | 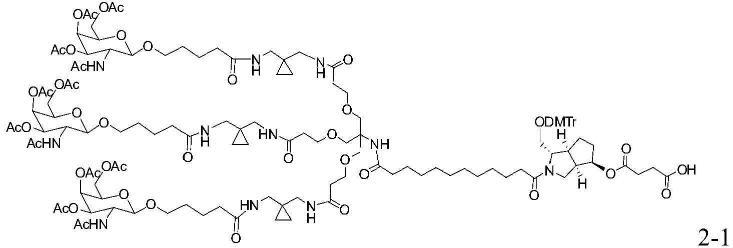 |
| | 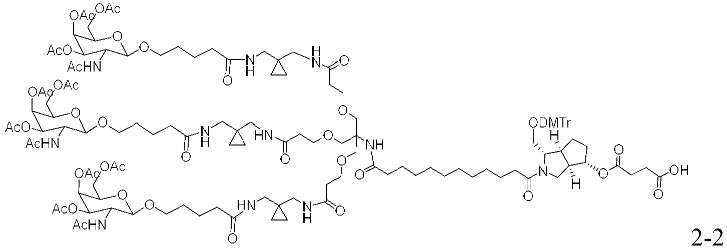 |
| | 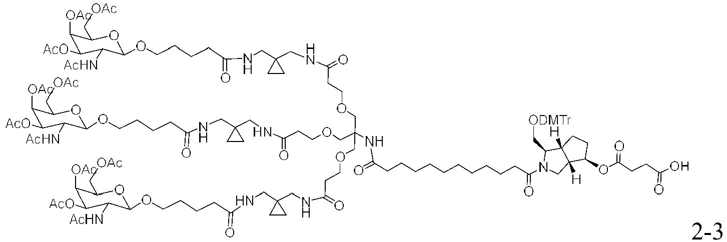 |
| | 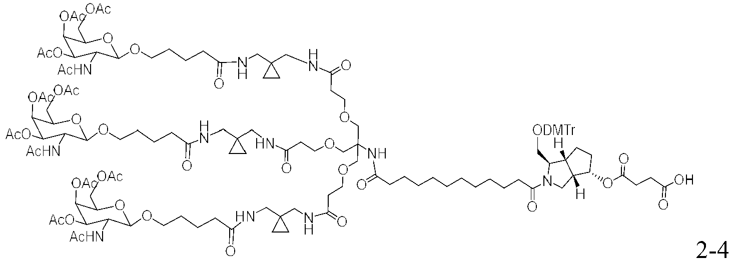 |
| B3 | 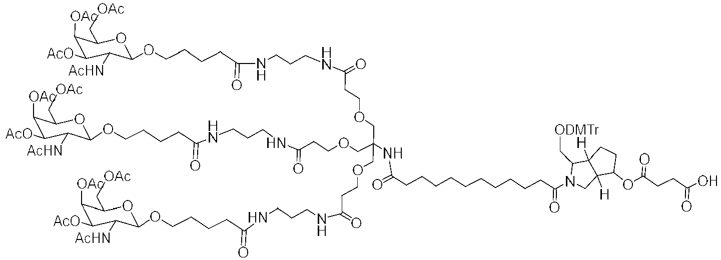 |
| B4 | 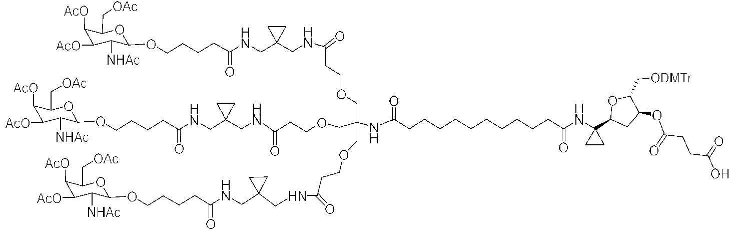 |
| B5 | 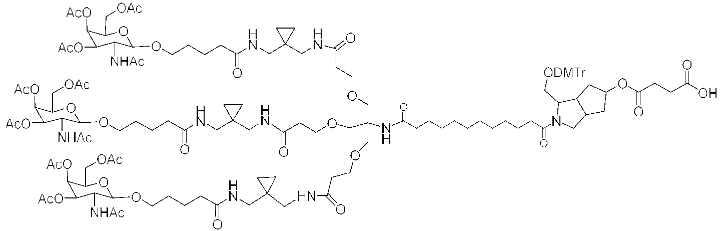 |
| | 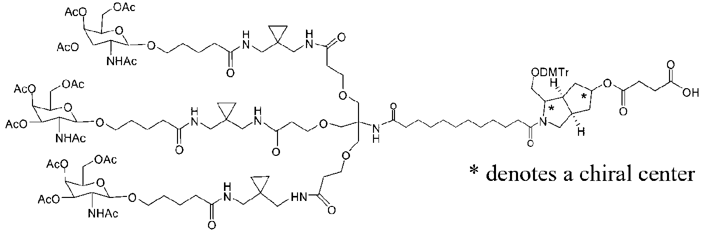 |
| | 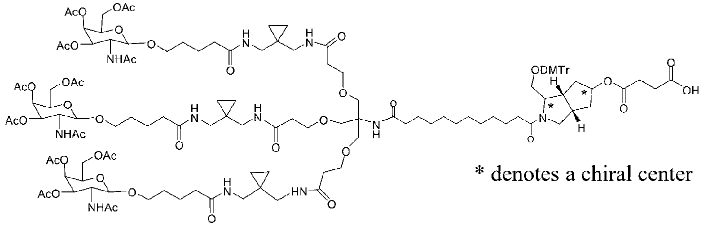 |
| B6 | 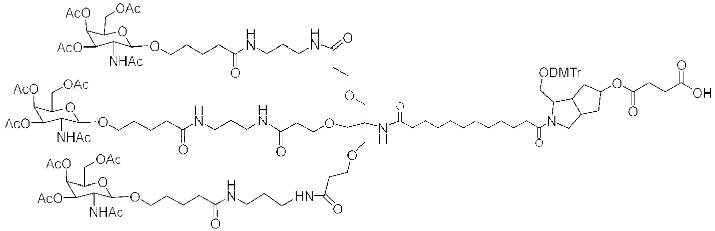 |
| B7 | 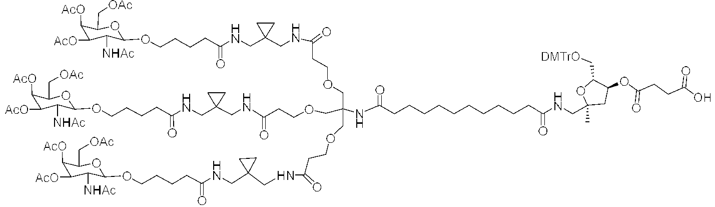 |
| B8 | 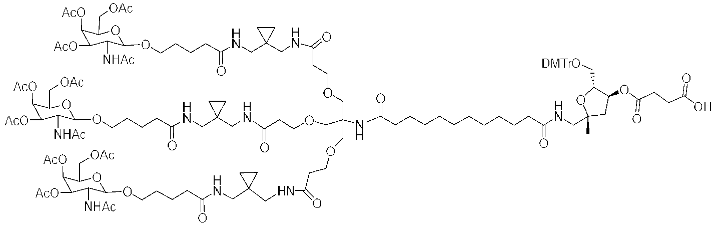 |
| B9 | 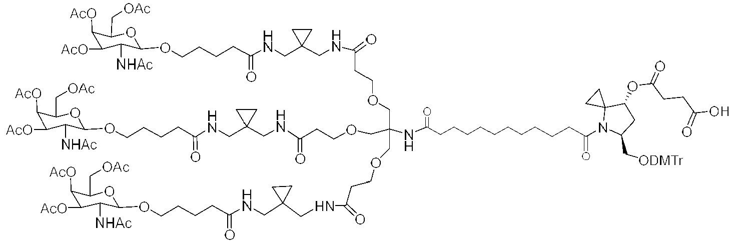 |
| B10 | 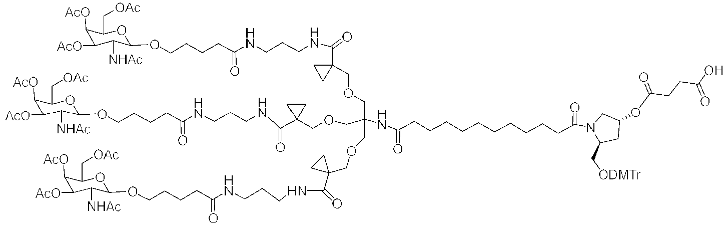 |
| B11 | 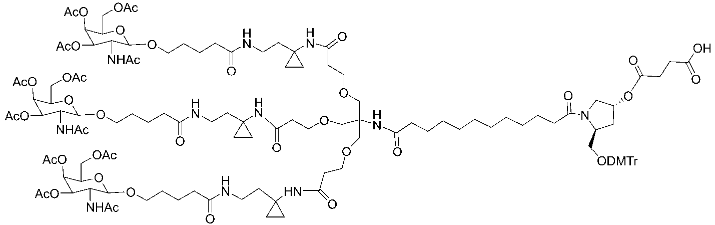 |
| B12 | 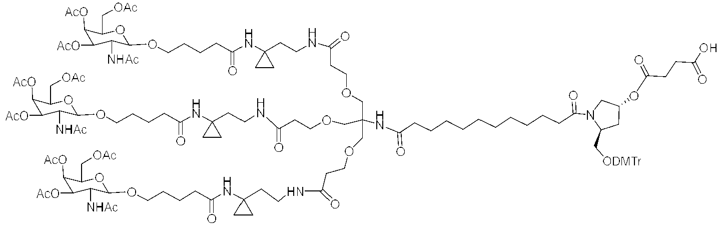 |
| B13 | 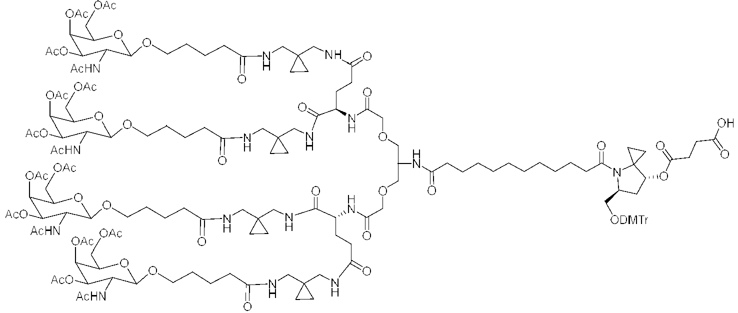 |
| B14 | 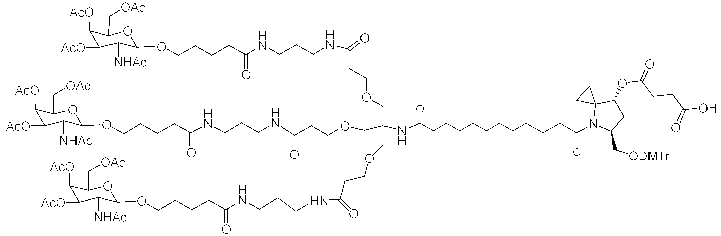 |
| B15 | 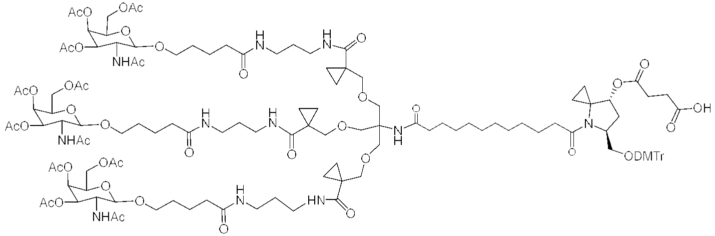 |
| B16 | 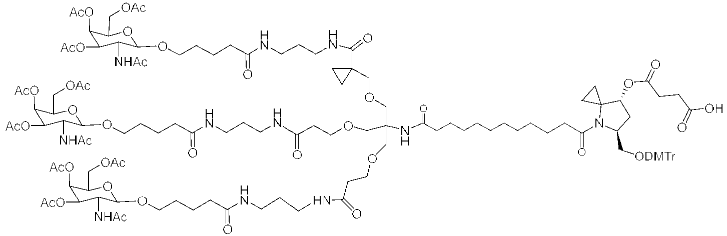 |
| B17 | 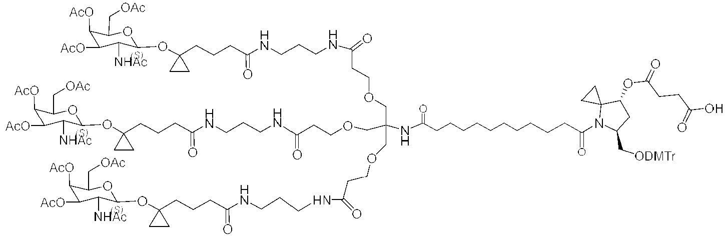 |
| B18 | 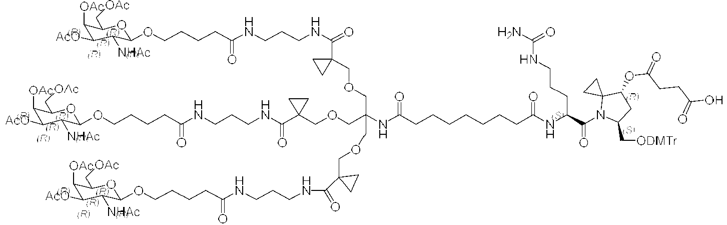 |
| B19 | 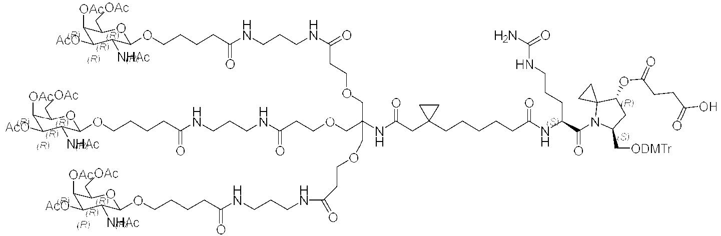 |
| B20 | 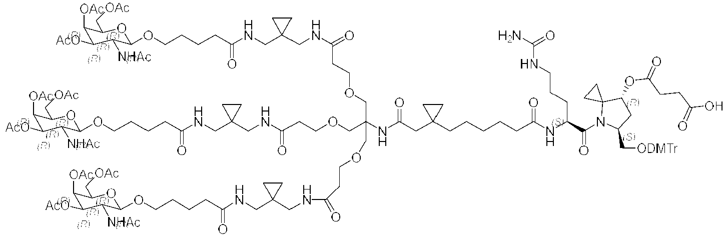 |
| B21 | 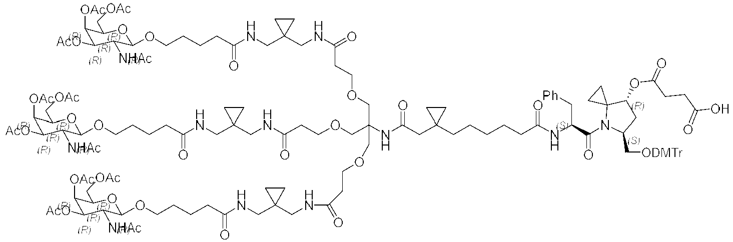 |
| B22 | 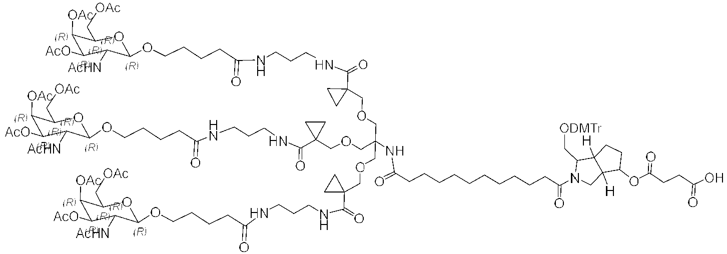 |
| B23 | 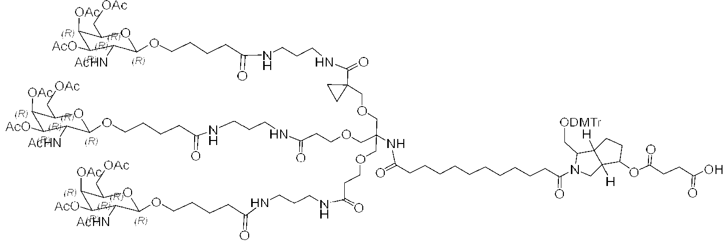 |
| B24 | 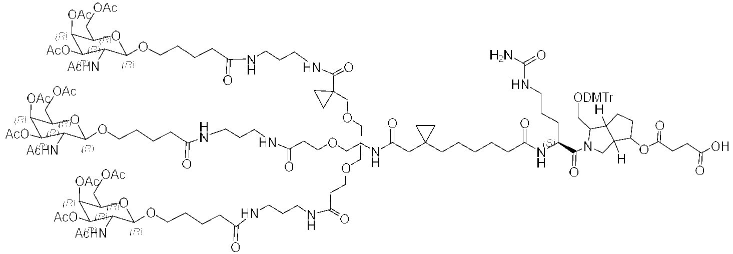 |
| B25 | 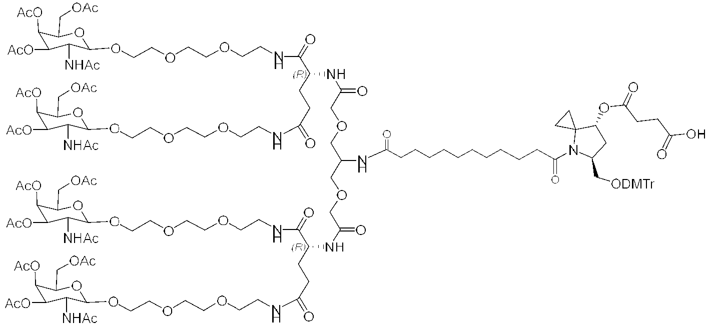 |
| B26 | 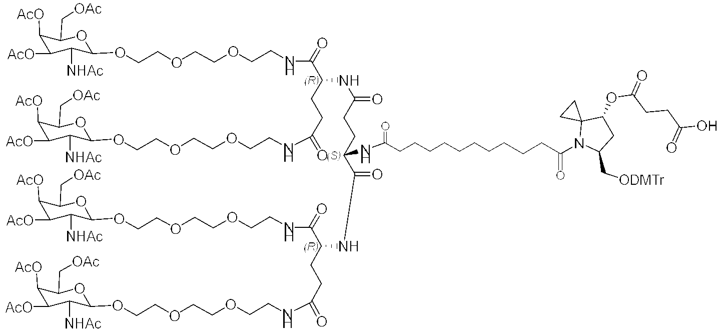 |
| B27 | 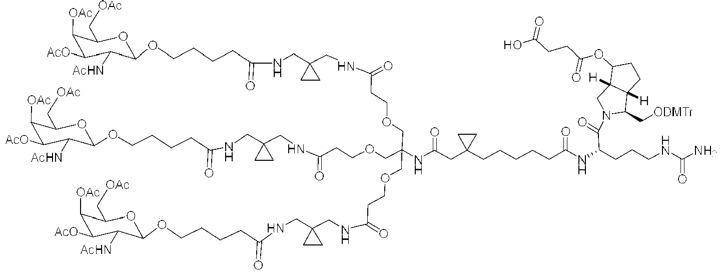 |
| B28 | 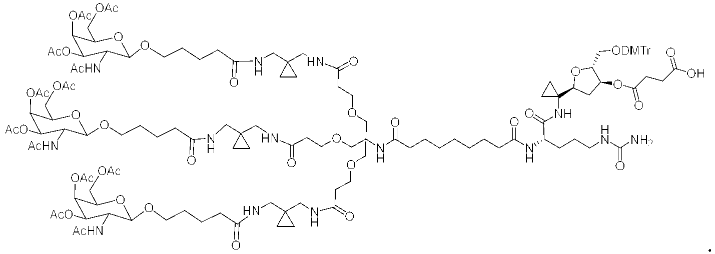 |

| | |
|---|---|
| wherein * denotes a chiral centre, which is independently selected from (S)- or (R)-configuration, or a mixture thereof. | |

In a more specific embodiment, the present invention provides a compound of formula (XI), or a pharmaceutically acceptable salt, isotopic variant, tautomer, stereoisomer, polymorph, hydrate or solvate thereof: 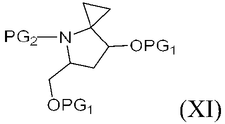 wherein
each PG₁ is independently selected from H or a hydroxyl protecting group;
PG₂ is H or an amino protecting group.

In a more specific embodiment, the present invention provides the above-mentioned compound of formula (XI), wherein the compound has a structure of formula (XI-1), (XI-2), formula (XI-3) or formula (XI-4): 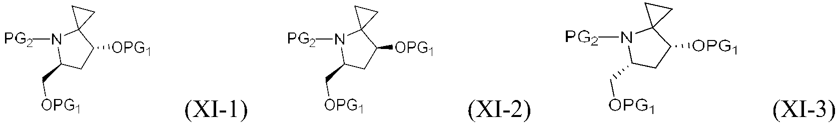 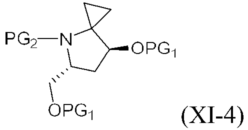 wherein PG₁ and PG₂ are as defined herein.

In a more specific embodiment, the present invention provides the above-mentioned compound of formula (XI), wherein
each PG₁ is independently selected from H, C(O)CH₂CH₂C(O)OH or 4,4'-dimethoxytrityl (DMTr);
PG₂ is selected from H, tert-butyloxycarbonyl (BOC) and 9-fluorenylmethoxycarbonyl (FMOC).

In a more specific embodiment, the present invention provides the above-mentioned compound of formula (XI), wherein the compound is selected from: 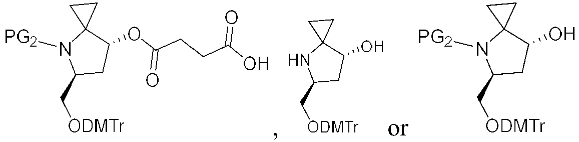 wherein
PG₂ is an amino protecting group, preferably tert-butyloxycarbonyl (BOC) or 9-fluorenylmethoxycarbonyl (FMOC).

In a more specific embodiment, the present invention provides a compound of formula (XII) or formula (XIII), or a pharmaceutically acceptable salt, isotopic variant, tautomer, stereoisomer, polymorph, hydrate or solvate thereof, or a mixture thereof: 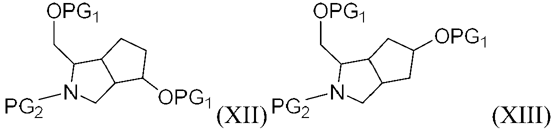 wherein
each PG₁ is independently selected from H or a hydroxyl protecting group;
PG₂ is H or an amino protecting group.

In a more specific embodiment, the present invention provides the above-mentioned compound of formula (XII) or formula (XIII), wherein the compound has a structure of formula (XII-1), (XII-2), formula (XII-3), formula (XII-4), formula (XIII-1) or (XIII-2): 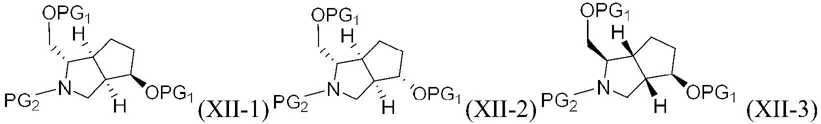 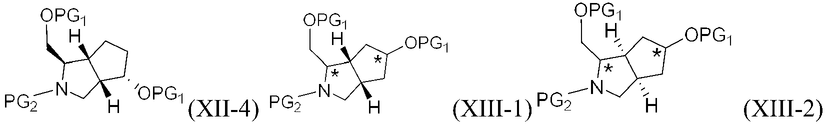
wherein * denotes a chiral centre, which is independently selected from (S)- or (R)-configuration, or a mixture thereof;
PG₁ and PG₂ are as defined herein.

In a more specific embodiment, the present invention provides the above-mentioned compound of formula (XII) or formula (XIII), wherein
each PG₁ is independently selected from H, C(O)CH₂CH₂C(O)OH or 4,4'-dimethoxytrityl (DMTr);
PG₂ is selected from H, tert-butyloxycarbonyl (BOC) and 9-fluorenylmethoxycarbonyl (FMOC).

In a more specific embodiment, the present invention provides the above-mentioned compound of formula (XII) or formula (XIII), wherein the compound is selected from: 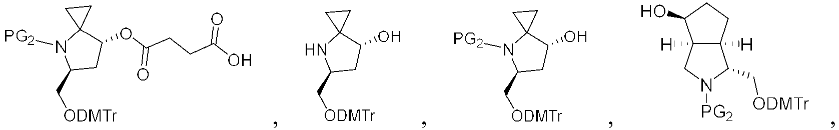 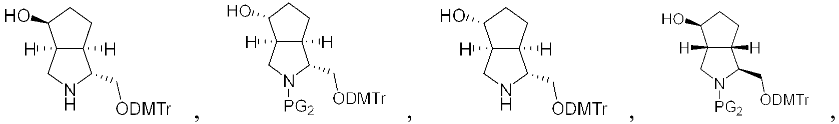 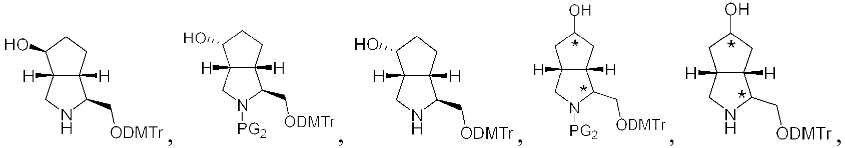 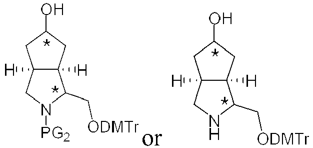
wherein * denotes a chiral centre, which is independently selected from (S)- or (R)-configuration, or a mixture thereof;
PG₂ is an amino protecting group, preferably tert-butyloxycarbonyl (BOC) or 9-fluorenylmethoxycarbonyl (FMOC).

### Examples

Unless otherwise stated, the starting materials and reagents used in the following examples are commercially available products or can be prepared by known methods.

The structures of compounds are determined by nuclear magnetic resonance (NMR) or mass spectrometry (MS). Chemical shifts (δ) are given in units of 10⁻⁶ (ppm). NMR is determined by Bruker nuclear magnetic resonance spectrometer; the solvents for determination are deuterated dimethyl sulphoxide (DMSO-d₆), deuterated chloroform (CDCl₃) and deuterated methanol (CD₃OD); and the internal standard is tetramethylsilane (TMS).

LCMS is determined by Agilent 1260 Infinity II (ESI) mass spectrometer, Waters UPLC H Class plus (ESI) or Shimadzu LCMS-2020 (ESI).

High performance liquid chromatography (HPLC) analysis is performed using Agilent 1260 or Shimadzu LC-20AD.

Preparative high performance liquid chromatography (pre-HPLC) is performed using a GILSON GX-281 or Agilent 1260 Infinity II preparative liquid chromatography system.

Chiral preparative separation is performed using supercritical fluid chromatography (SFC) on a Shimadzu LC-30Adsf or Shimadzu LC-20AD instrument.

For thin layer chromatography (TLC), the silica gel plates used are GF254 silica gel plates with acrylic binder produced by Anhui Liangchen Silicon Material Co., Ltd. The silica gel plates used for TLC analysis have a specification of 0.2 mm thickness, while those used for the separation and purification of products via TLC are of 0.5 mm thickness.

For column chromatography, silica gel (200-300 mesh) from Anhui Liangchen Silicon Material Co., Ltd. is generally used as a carrier.

The average kinase inhibition rate and IC₅₀ value are determined using SpectraMax i3X microplate reader (MD Inc., USA).

The known starting materials of the present invention can be synthesised by or in accordance with methods known in the art, or can be purchased from companies including Bide Pharmatech Co., Ltd., Leyan Biotech Co., Ltd., Accela ChemBio Inc., and Energy Chemical Co., Ltd.

Unless otherwise specified in the examples below, all reactions are carried out under argon or nitrogen atmosphere.

Argon or nitrogen atmosphere means that a reaction flask is connected to an argon or nitrogen balloon with a volume of about 1 L.

Hydrogen atmosphere means that a reaction flask is connected to a hydrogen balloon with a volume of about 1 L.

Hydrogenation reactions are typically performed by evacuating the system and refilling it with hydrogen gas, repeating this operation three times.

Oxygen atmosphere means that a reaction flask is connected to an oxygen balloon with a volume of about 1 L.

Unless otherwise specified in the examples below, the solution refers to an aqueous solution, and the reaction temperature is room temperature, ranging from 20°C to 30°C.

In the examples, the reaction progress is monitored by thin layer chromatography (TLC). The eluent system for column chromatography (used for compound purification) and the developing solvent system for TLC include: A: dichloromethane/methanol system, B: petroleum ether/ethyl acetate system. The volume ratio of the solvents is adjusted according to the polarity of compounds, and can also be adjusted by adding a small amount of basic or acidic reagents such as triethylamine and acetic acid.

### Example 1: Preparation of compound 1

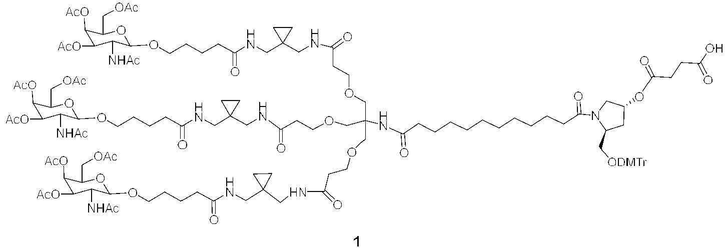

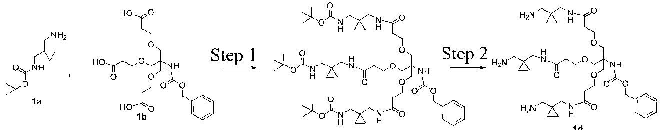

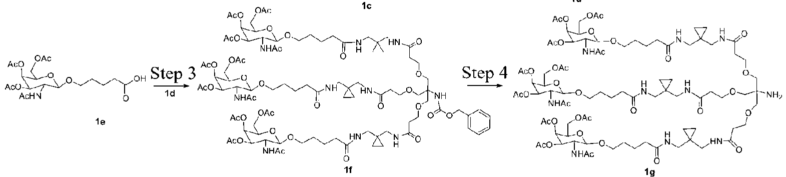

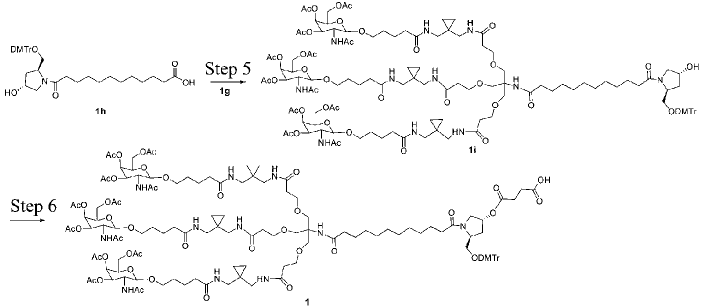

### Step 1

1a (280 mg, 1.40 mmol, prepared according to the method disclosed in Example 2 on page 24 of the patent application "WO 2016155545 A1") and 1b (200 mg, 425 µmol, prepared according to the method disclosed in Example 4 on page 512 of the patent application "WO 2014179620 A1") were dissolved in 10 mL of dichloromethane. 2-(7-Azobenzotriazole)-N,N,N',N'-tetramethyluronium hexafluorophosphate (532 mg, 1.40 mmol) and N,N-diisopropylethylamine (197 mg, 1.53 mmol) were added, and the mixture was stirred at 20°C for 2 hours. The reaction solution was concentrated by reduced-pressure distillation, and the resulting residue was purified by reversed-phase liquid chromatography (separation conditions: mobile phase: A-water (0.05% ammonium bicarbonate): B-acetonitrile, gradient elution: B%: 20%-80%), yielding title product 1c (400 mg, yield: 93%) as a white solid.

### Step 2

1c (400 mg, 393 µmol) was dissolved in 1 mL of methanol, a solution of hydrogen chloride in dioxane (4N, 20 mL) was added, and the mixture was stirred at 20°C for 6 hours. The reaction solution was concentrated by reduced-pressure distillation, and the residue was adjusted to neutral pH with a sodium carbonate solution. The resulting mixture was purified by reversed-phase liquid chromatography (separation conditions: mobile phase: A-water (0.05% ammonium bicarbonate): B-acetonitrile, gradient elution: B%: 5%-50%), yielding title product 1d (270 mg, yield: 96%) as a white solid.

MS m/z (ESI): 359.83 [1/2(M+2)].

### Step 3

1e (144 mg, 322 µmol, prepared according to the method disclosed in Example 1 on page 161 of the patent application "WO 2009073809 A2") and 1d (70 mg, 97 µmol) were dissolved in 5 mL of dichloromethane. 2-(7-Azobenzotriazole)-N,N,N',N'-tetramethyluronium hexafluorophosphate (122 mg, 322 µmol) and N,N-diisopropylethylamine (88 mg, 682 µmol) were added, and the mixture was stirred at 20°C for 2 hours. The reaction solution was concentrated by reduced-pressure distillation, and the resulting residue was purified by reversed-phase liquid chromatography (separation conditions: mobile phase: A-water (0.05% ammonium bicarbonate): B-acetonitrile, gradient elution: B%: 20%-70%), yielding title product 1f (120 mg, yield: 61%) as a white solid.

MS m/z (ESI): 1004.37 [1/2(M+2)].

### Step 4

1f (120 mg, 60 µmol) and acetic acid (17 mg, 283 µmol) were dissolved in 10 mL of methanol and 10 mL of ethyl acetate, and wet palladium on carbon (60 mg, 10%) was added. The reaction solution was purged three times with hydrogen, and stirred at 25°C under hydrogen atmosphere (15 Psi) for 6 hours. The reaction solution was filtered through Celite. The filtrate was concentrated by reduced-pressure distillation, and the resulting residue was purified by reversed-phase liquid chromatography (separation conditions: mobile phase: A-water (0.1% formic acid): B-acetonitrile, gradient elution: B%: 10%-50%), yielding title product 1g (90 mg, yield: 76%) as a white solid.

MS m/z (ESI): 937.12 [1/2(M+2)].

### Step 5

1g (15 mg, 8.01 µmol) and 1h (5.06 mg, 8.01 µmol, prepared according to the method disclosed in Example 2 on page 90 of the patent application "WO 2012037254 A1") were dissolved in 2 mL of dichloromethane. 2-(7-Azobenzotriazole)-N,N,N',N'-tetramethyluronium hexafluorophosphate (4.57 mg, 12.02 µmol) and N,N-diisopropylethylamine (5.18 mg, 40.08 µmol) were added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-70%), yielding title product 1i (12 mg, yield: 57%) as a white solid.

### Step 6

1i (10 mg, 4.02 µmol) was dissolved in 1 mL of dichloromethane. Under nitrogen atmosphere, 4-dimethylaminopyridine (0.09 mg, 0.8 µmol), succinic anhydride (2.01 mg, 20.12 µmol) and N,N-diisopropylethylamine (5.19 mg, 40.2 µmol) were added, and the mixture was stirred at 25°C for 15 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-60%), yielding title product 1 (4 mg, yield: 35%) as a white solid.

MS m/z (ESI): 1142.9 [1/2(M-301)].

¹H NMR (400 MHz, CD₃OD) δ 7.37 - 7.36 (m, 1H), 7.29 - 7.23 (m, 7H), 6.89 - 6.82 (m, 5H), 5.33 - 5.32 (m, 3H), 5.07 - 5.03 (m, 4H), 4.61 - 4.58 (m, 2H), 4.56 - 4.53 (m, 3H), 4.19 - 4.04 (m, 12H), 4.02 - 3.97 (m, 4H), 3.90 - 3.84 (m, 4H), 3.79 - 3.76 (m, 6H), 3.70 - 3.65 (m, 12H), 3.56 - 3.49 (m, 5H), 3.14 - 3.11 (m, 10H), 2.57 - 2.53 (m, 4H), 2.46 - 2.42 (m, 6H), 2.25 - 2.21 (m, 6H), 2.14 - 2.12 (m, 9H), 2.02 - 2.00 (m, 9H), 1.95 - 1.91 (m, 18H), 1.70 - 1.56 (m, 16H), 1.33 - 1.24 (m, 16H), 0.50 - 0.45 (m, 12H).

### Example 2: Preparation of compound 2

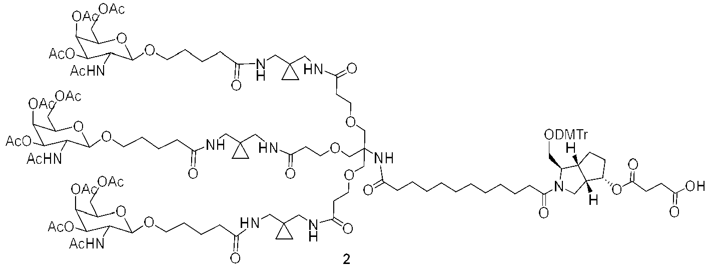

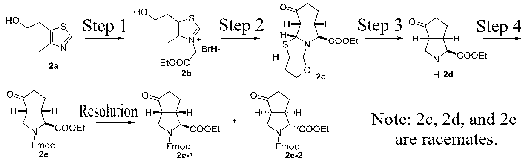

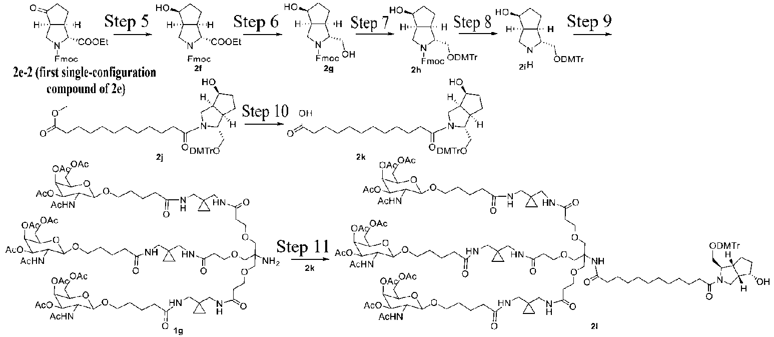

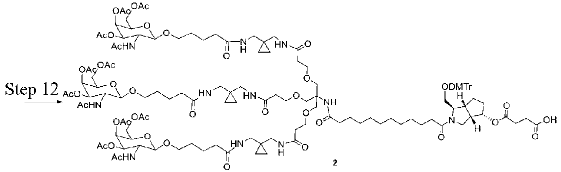

### Step 1

2a (5.00 g, 34.9 mmol) was dissolved in 20 mL of ethanol, ethyl 2-bromoacetate (5.83 g, 34.9 mmol) was added, and the mixture was stirred at 80°C for 15 hours. The reaction solution was concentrated by reduced-pressure distillation, and 25 mL of isopropanol was added to the resulting residue. Purification by recrystallisation afforded title product 2b (7.34 g, yield: 91%) as a yellow solid.

MS m/z (ESI): 230.1 [M+1].

### Step 2

2b (6.84 g, 29.7 mmol) was dissolved in 50 mL of acetonitrile. Under nitrogen atmosphere, 2-cyclopentenone (11.7 g, 142 mmol) and triethylamine (3.31 g, 32.6 mmol) were added. Under nitrogen atmosphere, the mixture was stirred at 25°C for 24 hours. 50 mL of water was added to the reaction solution, and the resulting reaction solution was extracted with ethyl acetate (80 mL × 3). The organic phase was washed with a saturated sodium chloride solution (60 mL × 2), and then dried over anhydrous sodium sulphate, and filtered. The filtrate was concentrated by reduced-pressure distillation, and the resulting residue was purified by silica gel column chromatography using developing solvent system B, yielding title product 2c (5.81 g, yield: 61%) as a yellow solid.

MS m/z (ESI): 312.1 [M+1].

### Step 3

2c (5.31 g, 16.5 mmol) was dissolved in 60 mL of toluene. Under nitrogen atmosphere, tributyltin hydride (11.9 g, 40.9 mmol) and azobisisobutyronitrile (543 mg, 3.31 mmol) were added. Under nitrogen atmosphere, the mixture was reacted at 120°C for 6 hours. The reaction solution was concentrated by reduced-pressure distillation, and 80 mL of ethyl acetate and hydrochloric acid (1N, 40 mL) were added to the resulting residue. The resulting solution was allowed to stand at 25°C for 14 hours. The organic phase was separated, and the remaining aqueous phase was washed with ethyl acetate (100 mL × 3). The resulting aqueous phase was carried forward without purification, yielding crude title product 2d (3.27 g) as a yellow liquid, which was directly used in the next reaction without purification.

### Step 4

To 2d (3.27 g, 16.6 mmol) were added a sodium bicarbonate solution (25 mL), acetonitrile (25 mL), and 9-fluorenylmethyl-N-succinimidyl carbonate (6.71 g, 19.9 mmol). Under nitrogen atmosphere, the mixture was reacted at 25°C for 2 hours. The reaction solution was extracted with ethyl acetate (80 mL × 3), and the organic phase was washed with a saturated sodium chloride solution (100 mL × 2), dried over anhydrous sodium sulphate, and filtered. The filtrate was concentrated by reduced-pressure distillation, and the resulting residue was purified by silica gel column chromatography using developing solvent system B, yielding title product 2e (2.01 g, yield: 27%) as a yellow oil.

2e (2.01 g, 4.80 mmol) was separated by SFC (separation conditions: chromatographic column: DAICEL CHIRALPAK AD (250 mm × 30 mm, 10 µm); mobile phase: A-carbon dioxide: B-methanol, isocratic elution: B: 35%), yielding the single-configuration compounds 2e-1 and 2e-2.

### 2e-2 (first single-configuration compound of 2e with a shorter retention time)

Yellow gum, 775 mg, yield: 39%. SFC analysis: retention time: 1.830 minutes. (chromatographic column: Chiralpak AD-3 50 × 4.6 mm I.D., 3 µm, mobile phase: A-carbon dioxide, B-methanol (0.05% diethylamine), gradient elution: B: 5%-40%, flow rate: 3 mL/min, instrument: Shimadzu LC-30ADsf).

MS m/z (ESI): 420.1 [M+1].

### 2e-1 (second single-configuration compound of 2e with a longer retention time)

Yellow gum, 750 mg, yield: 37%. SFC analysis: retention time: 2.193 minutes. (chromatographic column: Chiralpak AD-3 50 × 4.6 mm I.D., 3 µm, mobile phase: A-carbon dioxide, B-methanol (0.05% diethylamine), gradient elution: B: 5%-40%, flow rate: 3 mL/min, instrument: Shimadzu LC-30ADsf).

MS m/z (ESI): 420.1 [M+1].

### Step 5

(R)-5,5-Diphenyl-2-methyl-3,4-propanol-1,3,2-oxazaborolidine (79.3 mg, 286 µmol) was dissolved in 2 mL of toluene. Under nitrogen atmosphere, the mixture was cooled to 0°C. Borane dimethyl sulphide complex (10 M, 171 µL) was added dropwise, and the mixture was reacted at 0°C for 15 minutes. Subsequently, a solution of 2e-2 (first single-configuration compound of 2e) (600 mg, 1.43 mmol) in toluene (8 mL) was added, and the resulting mixture was reacted at 0°C for 30 minutes. At 0°C, 5 mL of methanol and 8 mL of water were added to the reaction solution, and the resulting reaction solution was extracted with dichloromethane (10 mL × 3). The organic phase was dried over anhydrous sodium sulphate and filtered. The filtrate was concentrated by reduced-pressure distillation, and the resulting residue was purified by silica gel column chromatography using developing solvent system A, yielding title product 2f (500 mg, yield: 81%) as a yellow oil.

MS m/z (ESI): 422.0 [M+1].

### Step 6

2f (500 mg, 1.15 mmol) was dissolved in 10 mL of tetrahydrofuran. Under nitrogen atmosphere, the mixture was cooled to 0°C. A solution of lithium borohydride in tetrahydrofuran (2 M, 864 µL) was added dropwise, and the mixture was stirred at 25°C for 1 hour. At 0°C, 1 mL of ammonium chloride solution and 5 mL of water were added, and the reaction solution was extracted with dichloromethane (8 mL × 3). The organic phase was dried over anhydrous sodium sulphate and filtered. The filtrate was concentrated by reduced-pressure distillation, and the resulting residue was purified by silica gel column chromatography using developing solvent system A, yielding title product 2g (189 mg, yield: 63%) as a white solid.

MS m/z (ESI): 380.0 [M+1].

### Step 7

2g (25 mg, 64.57 µmol) was dissolved in 3 mL of dry pyridine. Under nitrogen atmosphere, the mixture was cooled to below 5°C. Subsequently, 4,4'-dimethoxytrityl chloride (21.88 mg, 64.57 µmol) was dissolved in 5 mL of dichloromethane, and the mixture was slowly added dropwise to the reaction solution. The resulting mixture was naturally warmed to 25°C and stirred for 12 hours. 1 mL of methanol was added to quench the reaction solution. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 2h (27 mg, yield: 58%) as a white solid.

### Step 8

2h (26 mg, 38 µmol) and hexahydropyridine (6 mg, 73 µmol) were dissolved in 3 mL of N,N-dimethylformamide, and the mixture was reacted at 25°C for 12 hours. The reaction solution was purified by reversed-phase liquid chromatography without prior purification (mobile phase: A-water (0.05% ammonium bicarbonate), B-acetonitrile; gradient elution: B%: 10%-80%), yielding title product 2i (16 mg, yield: 91%) as a white solid.

### Step 9

2i (15 mg, 31.00 µmol) and monomethyl dodecanedioate (7.58 mg, 31.02 µmol) were dissolved in 2 mL of dichloromethane. 2-(7-Azobenzotriazole)-N,N,N',N'-tetramethyluronium hexafluorophosphate (14.15 mg, 37.21 µmol) and N,N-diisopropylethylamine (20.03 mg, 155.0 µmol) were added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 30%-100%), yielding title product 2j (14 mg, yield: 62%) as a white solid.

### Step 10

2j (12 mg, 16.62 µmol) was dissolved in 2 mL of tetrahydrofuran and 2 mL of water. Lithium hydroxide (1.99 mg, 83.09 µmol) was added, and the mixture was reacted at 25°C for 12 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-70%), yielding title product 2k (10 mg, yield: 85%) as a white solid.

### Step 11

2k (5 mg, 7.07 µmol) and 1g (14.71 mg, 7.07 µmol) were dissolved in 2 mL of dichloromethane. 2-(7-Azobenzotriazole)-N,N,N',N'-tetramethyluronium hexafluorophosphate (4.03 mg, 10.6 µmol) and N,N-diisopropylethylamine (4.57 mg, 35.36 µmol) were added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-70%), yielding title product 21 (11 mg, yield: 55%) as a white solid.

### Step 12

21 (10 mg, 3.94 µmol) was dissolved in 3 mL of dichloromethane. Under nitrogen atmosphere, 4-dimethylaminopyridine (0.1 mg, 0.79 µmol), succinic anhydride (1.97 mg, 19.7 µmol) and N,N-diisopropylethylamine (5.09 mg, 39.4 µmol) were added, and the mixture was stirred at 25°C for 15 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-90%), yielding title product 2 (4 mg, yield: 36%) as an off-white solid.

MS m/z (ESI): 1162.9 [1/2(M-301)].

¹H NMR (400 MHz, CD₃OD) δ 7.39 - 7.36 (m, 2H), 7.30 - 7.28 (m, 1H), 7.27 - 7.24 (m, 5H), 7.21 - 7.18 (m, 1H), 6.87 - 6.82 (m, 4H), 5.33 - 5.31 (m, 3H), 5.08 - 5.03 (m, 4H), 4.57 - 4.54 (m, 3H), 4.17 - 4.04 (m, 12H), 4.02 - 3.97 (m, 4H), 3.89 - 3.84 (m, 4H), 3.78 - 3.77 (m, 6H), 3.70 - 3.65 (m, 12H), 3.56 - 3.50 (m, 4H), 3.48 - 3.46 (m, 1H), 3.33 - 3.31 (m, 2H), 3.15 - 3.11 (m, 12H), 2.46 - 2.42 (m, 8H), 2.25 - 2.21 (m, 6H), 2.13 - 2.11 (m, 9H), 2.02 - 2.00 (m, 9H), 1.94 - 1.91 (m, 18H), 1.71 - 1.49 (m, 22H), 1.48 - 1.42 (m, 2H), 1.31 - 1.25 (m, 12H), 0.50 - 0.46 (m, 12H).

### Example 3: Preparation of compound 3

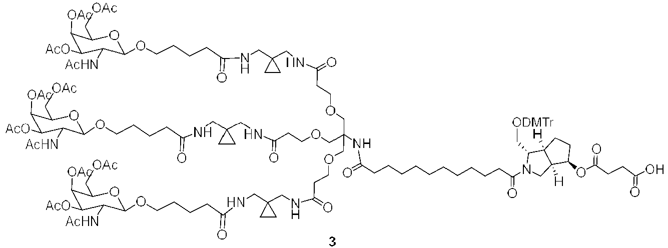

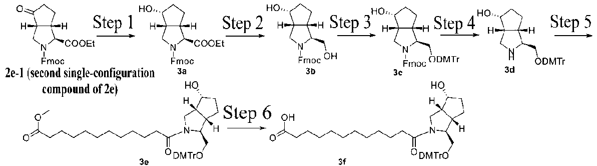

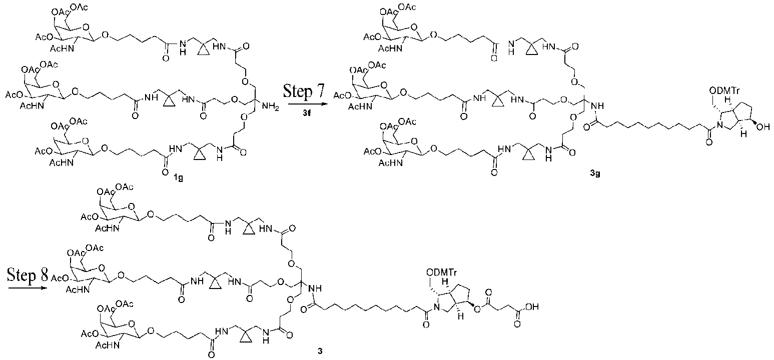

### Step 1

(S)-5,5-Diphenyl-2-methyl-3,4-propanol-1,3,2-oxazaborolidine (85.9 mg, 310 µmol) was dissolved in 10 mL of toluene. Under nitrogen atmosphere, the mixture was cooled to 0°C. Borane dimethyl sulphide complex (10 M, 186 µL) was added dropwise, and the mixture was reacted at 0°C for 15 minutes. Subsequently, 2e-1 (second single-configuration compound of 2e) (650 mg, 1.55 mmol) was added, and the resulting mixture was reacted at 0°C for 30 minutes. At 0°C, 5 mL of methanol and 8 mL of water were added to the reaction solution, and the resulting reaction solution was extracted with dichloromethane (10 mL × 3). The organic phase was dried over anhydrous sodium sulphate and filtered. The filtrate was concentrated by reduced-pressure distillation, and the resulting residue was purified by silica gel column chromatography using developing solvent system A, yielding title product 3a (503 mg, yield: 74%) as a yellow oil.

MS m/z (ESI): 422.1 [M+1].

### Step 2

3a (503 mg, 1.15 mmol) was dissolved in 8 mL of tetrahydrofuran. Under nitrogen atmosphere, the mixture was cooled to 0°C. A solution of lithium borohydride in tetrahydrofuran (2 M, 862 µL) was added dropwise, and the mixture was stirred at 25°C for 1 hour. At 0°C, 1 mL of ammonium chloride solution and 5 mL of water were added, and the reaction solution was extracted with dichloromethane (8 mL × 3). The organic phase was dried over anhydrous sodium sulphate and filtered. The filtrate was concentrated by reduced-pressure distillation, and the resulting residue was purified by silica gel column chromatography using developing solvent system A, yielding title product 3b (51.83 mg, yield: 38%) as a white solid.

MS m/z (ESI): 380.1 [M+1].

### Step 3

3b (20 mg, 52.71 µmol) was dissolved in 1 mL of dry pyridine. Under nitrogen atmosphere, the mixture was cooled to below 5°C. Subsequently, 4,4'-dimethoxytrityl chloride (27 mg, 79.07 µmol) was dissolved in 1 mL of dichloromethane, and the mixture was slowly added dropwise to the reaction solution. The resulting mixture was naturally warmed to room temperature and stirred for 15 hours. 1 mL of methanol was added to quench the reaction solution. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 3c (21 mg, yield: 58%) as a light yellow oil.

### Step 4

3c (21 mg, 30.8 µmol) was dissolved in 1 mL of N,N-dimethylformamide, piperidine (13 mg, 153.96 µmol) was then added, and the mixture was stirred and reacted at room temperature for 1 hour. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 3d (12 mg, yield: 85%) as a light yellow oil.

### Step 5

3d (12 mg, 26.11 µmol) and monomethyl dodecanedioate (7.02 mg, 28.73 µmol) were dissolved in 1 mL of dichloromethane. 2-(7-Azobenzotriazole)-N,N,N',N'-tetramethyluronium hexafluorophosphate (11.91 mg, 31.31 µmol) and N,N-diisopropylethylamine (13.50 mg, 104.46 µmol) were added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 3e (15 mg, yield: 84%) as a light yellow oil.

### Step 6

3e (15 mg, 21.87 µmol) was dissolved in 1 mL of methanol and 0.3 mL of water. Lithium hydroxide (2.62 mg, 109.39 µmol) was added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 5%-80%), yielding title product 3f (9 mg, yield: 61%) as an off-white solid.

### Step 7

3f (8 mg, 11.9 µmol) and 1g (22.48 mg, 12.0 µmol) were dissolved in 1 mL of dichloromethane. 2-(7-Azobenzotriazole)-N,N,N',N'-tetramethyluronium hexafluorophosphate (5.43 mg, 14.28 µmol) and N,N-diisopropylethylamine (6.16 mg, 47.66 µmol) were added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 5%-90%), yielding title product 3g (17 mg, yield: 56%) as a white solid.

MS m/z (ESI): 2224.8 [M-301].

### Step 8

3g (17 mg, 6.73 µmol) was dissolved in 1 mL of dichloromethane. Under nitrogen atmosphere, 4-dimethylaminopyridine (0.16 mg, 1.35 µmol), succinic anhydride (3.37 mg, 33.68 µmol) and N,N-diisopropylethylamine (8.7 mg, 67.31 µmol) were added, and the mixture was stirred at 25°C for 15 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-90%), yielding title product 3 (16 mg, yield: 90%) as a white solid.

MS m/z (ESI): 2324.8 [M-301].

¹H NMR (400 MHz, CDCl₃) δ 7.51 - 7.49 (m, 1H), 7.32 - 7.30 (m, 1H), 7.30 - 7.28 (m, 3H), 7.20 - 7.16 (m, 4H), 6.86 - 6.84 (m, 2H), 6.83 - 6.82 (m, 2H), 5.39 - 5.34 (m, 3H), 5.21 - 5.17 (m, 3H), 5.09 - 4.97 (m, 1H), 4.66 - 4.58 (m, 3H), 4.55 - 4.44 (m, 1H), 4.21 - 4.05 (m, 10H), 3.99 - 3.90 (m, 6H), 3.82 - 3.80 (m, 6H), 3.72 - 3.67 (m, 12H), 3.55 - 3.49 (m, 4H), 3.19 - 3.05 (m, 12H), 2.63 - 2.55 (m, 4H), 2.50 - 2.45 (m, 6H), 2.38 - 2.21 (m, 10H), 2.20 - 2.17 (m, 2H), 2.17 - 2.15 (m, 9H), 2.13 - 2.08 (m, 2H), 2.07 - 2.05 (m, 9H), 2.01 - 1.99 (m, 9H), 1.98 - 1.95 (m, 9H), 1.85 - 1.77 (m, 4H), 1.76 - 1.67 (m, 6H), 1.66 - 1.61 (m, 6H), 1.57 - 1.53 (m, 2H), 1.39 - 1.33 (m, 2H), 1.31 - 1.29 (m, 2H), 1.28 - 1.24 (m, 8H), 0.92 - 0.82 (m, 2H), 0.54 - 0.47 (m, 12H).

### Example 4: Preparation of compound 4

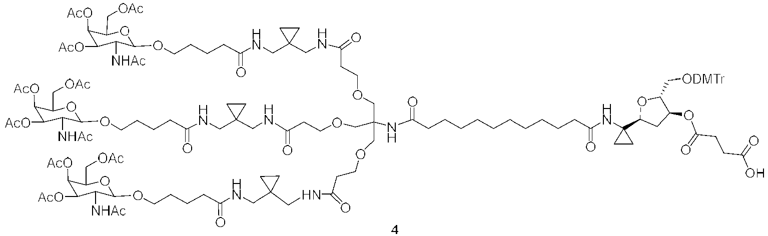

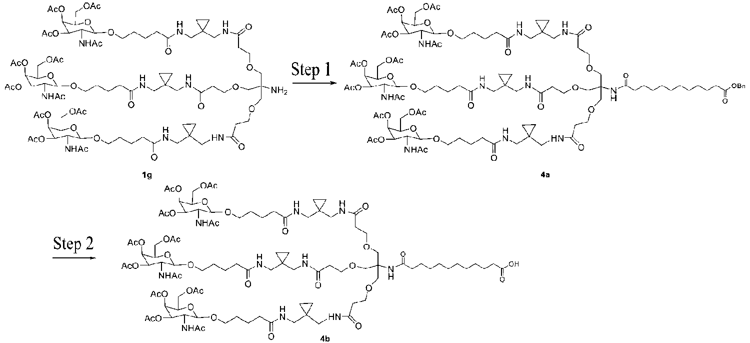

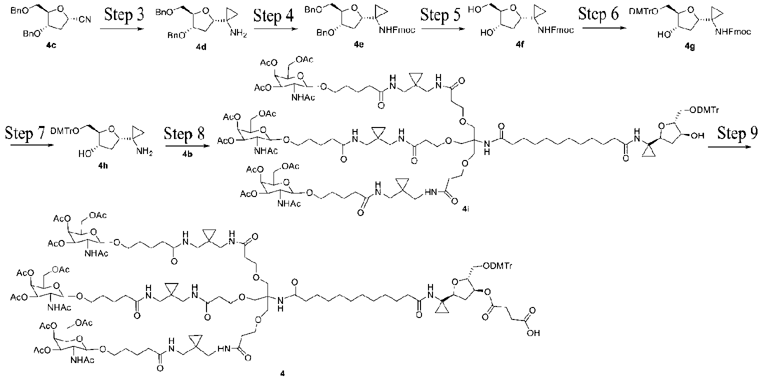

### Step 1

1g (200 mg, 0.11 mmol) and monobenzyl dodecanedioate (41.08 mg, 1.28 µmol) were dissolved in 10 mL of dichloromethane. 2-(7-Azobenzotriazole)-N,N,N',N'-tetramethyluronium hexafluorophosphate (60.93 mg, 0.16 mmol) and N,N-diisopropylethylamine (41.42 mg, 0.32 mmol) were added, and the mixture was reacted at 25°C for 2 hours. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 4a (115 mg, yield: 48%) as a white solid.

MS m/z (ESI): 1088.3 [1/2M+1].

### Step 2

4a (115 mg, 52.89 µmol) was dissolved in 10 mL of methanol and 10 mL of ethyl acetate, and a single drop of acetic acid was added dropwise, followed by the addition of wet palladium on carbon (100 mg, 10%). The reaction solution was purged three times with hydrogen, and stirred at 25°C under hydrogen atmosphere (15 Psi) for 3 hours. The reaction solution was filtered through Celite. The filtrate was concentrated by reduced-pressure distillation, and the resulting residue was purified by preparative high performance liquid chromatography (separation conditions: chromatographic column: Phenomenex luna C18 150 × 25 mm × 4 µm; mobile phase: A-water (0.1% formic acid): B-acetonitrile, gradient elution: B%: 20%-55%), yielding title product 4b (85 mg, yield: 77%) as a white solid.

MS m/z (ESI): 1043.4 [1/2M+1].

### Step 3

4c (500 mg, 1.55 mmol, prepared according to the method disclosed in Example 6 on page 99 of the patent application "WO 2023109938 A1") and tetraisopropyl titanate (659.16 mg, 2.32 mmol) were dissolved in 20 mL of super-dry tetrahydrofuran. Under nitrogen atmosphere, the mixture was cooled to below - 78°C. Subsequently, ethylmagnesium bromide (2M, 2.32 mL) was slowly added dropwise to the reaction solution, and the resulting mixture was naturally warmed to room temperature and stirred for 1 hour. Then, boron trifluoride diethyl etherate (614.43 mg, 4.33 mmol) was slowly added dropwise to the reaction solution, and the resulting mixture was stirred at 25°C for 0.5 hours. The reaction solution was quenched with 10 mL of saturated ammonium chloride, then diluted with 100 mL of water, and extracted with ethyl acetate (100 mL × 3). The organic phase was washed with a saturated sodium chloride solution (100 mL × 2), and then dried over anhydrous sodium sulphate, and filtered. The filtrate was concentrated by reduced-pressure distillation, and the resulting residue was purified by preparative high performance liquid chromatography (separation conditions: chromatographic column: Phenomenex luna C18 150 × 25 mm × 4 µm; mobile phase: A-water (0.1% formic acid): B-acetonitrile, gradient elution: B%: 5%-80%), yielding title product 4d (350 mg, yield: 64%) as an off-white solid.

MS m/z (ESI): 354.3[M+1].

### Step 4

4d (350 mg, 0.99 mmol) was dissolved in 10 mL of 1,4-dioxane and 10 mL of water. Sodium bicarbonate (415.94 mg, 4.95 mmol) and 9-fluorenylmethyl-N-succinimidyl carbonate (434.24 mg, 1.29 mmol) were added, and the mixture was reacted at 25°C for 1 hour. 50 mL of water was added to the reaction solution, and the resulting reaction solution was extracted with ethyl acetate (50 mL × 3). The organic phase was washed with a saturated sodium chloride solution (50 mL × 2), and then dried over anhydrous sodium sulphate, and filtered. The filtrate was concentrated by reduced-pressure distillation, and the resulting residue was purified by silica gel column chromatography using developing solvent system B, yielding title product 4e (437 mg, yield: 76%) as a white solid.

MS m/z (ESI): 576.5[M+1].

### Step 5

4e (425 mg, 0.74 mmol) was dissolved in 2 mL of super-dry dichloromethane. Under nitrogen atmosphere, the mixture was cooled to below - 70°C. Subsequently, boron trichloride (1M, 7.38 mL) was slowly added dropwise to the reaction solution, and the resulting mixture was stirred at a temperature maintained below -70°C for 1 hour. At -70°C, 2 mL of methanol was slowly added to the reaction solution. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 4f (160 mg, yield: 52%) as a colourless oil.

MS m/z (ESI): 396.3 [M+1].

### Step 6

4f (160 mg, 0.4 mmol) was dissolved in 3 mL of dry pyridine. Under nitrogen atmosphere, the mixture was cooled to below 5°C. Subsequently, 4,4'-dimethoxytrityl chloride (165 mg, 0.49 mmol) was dissolved in 3 mL of dichloromethane, and the mixture was slowly added dropwise to the reaction solution. The resulting mixture was naturally warmed to room temperature and stirred for 2 hours. 1 mL of methanol was added to quench the reaction solution. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 4g (210 mg, yield: 74%) as a colourless oil.

### Step 7

4g (210 mg, 0.3 mmol) was dissolved in 2 mL of N,N-dimethylformamide, 1,5-diazabicyclo[5.4.0]undec-5-ene (46.89 mg, 0.3 mmol) was added, and the mixture was stirred at 25°C for 1 hour. The reaction solution was directly purified by reversed-phase liquid chromatography without prior treatment (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 4h (141 mg, yield: 98%) as a colourless oil.

MS m/z (ESI): 476.4 [M+1].

### Step 8

4h (3.01 mg, 6.33 µmol) and 4b (11 mg, 5.28 µmol) were dissolved in 1 mL of N,N-dimethylformamide. 2-(7-Azobenzotriazole)-N,N,N',N'-tetramethyluronium hexafluorophosphate (2.41 mg, 6.34 µmol) and N,N-diisopropylethylamine (2.72 mg, 21.12 µmol) were added, and the mixture was reacted at 25°C for 0.5 hours. The reaction solution was directly purified by preparative high performance liquid chromatography without prior treatment (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 4i (8 mg, yield: 56%) as a white solid.

MS m/z (ESI): 2240.7 [M-301].

### Step 9

4i (8 mg, 3.15 µmol) was dissolved in 1 mL of dichloromethane. Under nitrogen atmosphere, 4-dimethylaminopyridine (0.08 mg, 0.63 µmol), succinic anhydride (1.57 mg, 15.69 µmol) and N,N-diisopropylethylamine (4.07 mg, 31.49 µmol) were added, and the mixture was stirred at 25°C for 15 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-90%), yielding title product 4 (6.75 mg, yield: 80%) as an off-white solid.

MS m/z (ESI): 2339.9 [M-301].

¹H NMR (400 MHz, CDCl₃) δ 7.59 - 7.57 (m, 1H), 7.34 - 7.33 (m, 1H), 7.32 - 7.31 (m, 1H), 7.30 - 7.29 (m, 1H), 7.29 - 7.29 (m, 1H), 7.20 - 7.16 (m, 4H), 6.95 - 6.89 (m, 2H), 6.86 - 6.82 (m, 4H), 6.74 - 6.71 (m, 1H), 6.64 - 6.59 (m, 1H), 6.57 - 6.54 (m, 1H), 5.37 - 5.35 (m, 3H), 5.23 - 5.18 (m, 3H), 5.16 - 5.12 (m, 1H), 5.09 - 5.02 (m, 1H), 4.66 - 4.61 (m, 3H), 4.56 - 4.49 (m, 1H), 4.21 - 4.08 (m, 10H), 4.07 - 4.01 (m, 2H), 4.00 - 3.90 (m, 8H), 3.82 - 3.80 (m, 6H), 3.73 - 3.72 (m, 2H), 3.71 - 3.69 (m, 6H), 3.67 - 3.64 (m, 2H), 3.56 - 3.49 (m, 4H), 3.20 - 3.14 (m, 4H), 3.13 - 3.11 (m, 4H), 3.10 - 3.06 (m, 2H), 2.67 - 2.64 (m, 2H), 2.61 - 2.57 (m, 2H), 2.50 - 2.46 (m, 6H), 2.31 - 2.27 (m, 4H), 2.25 - 2.22 (m, 2H), 2.19 - 2.18 (m, 2H), 2.17 - 2.15 (m, 9H), 2.12 - 2.10 (m, 2H), 2.06 - 2.05 (m, 9H), 2.02 - 2.00 (m, 9H), 1.98 - 1.96 (m, 9H), 1.83 - 1.80 (m, 2H), 1.80 - 1.77 (m, 2H), 1.76 - 1.73 (m, 2H), 1.72 - 1.70 (m, 2H), 1.69 - 1.67 (m, 2H), 1.65 - 1.62 (m, 4H), 1.61 - 1.55 (m, 4H), 1.30 - 1.24 (m, 12H), 0.99 - 0.76 (m, 6H), 0.53 - 0.50 (m, 10H).

### Example 5: Preparation of compound 5

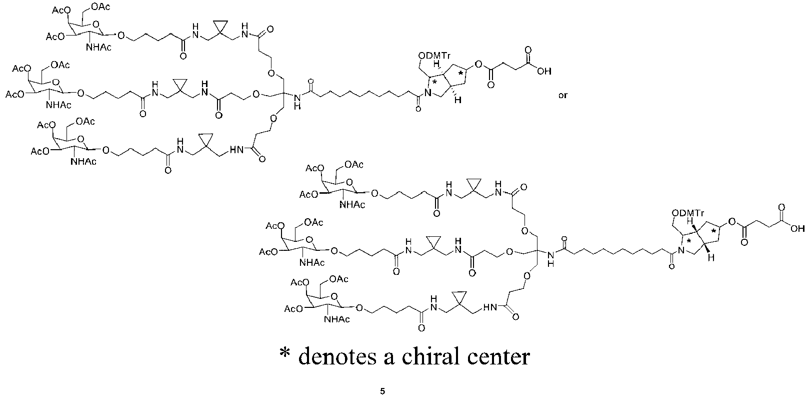

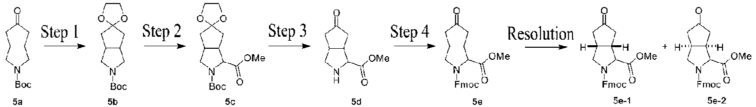

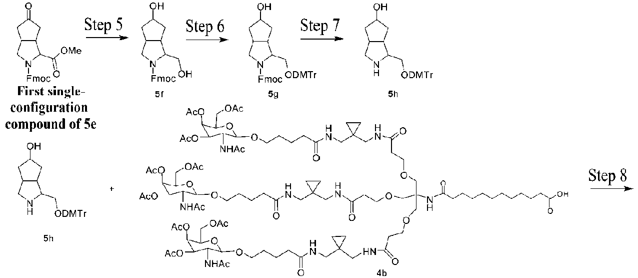

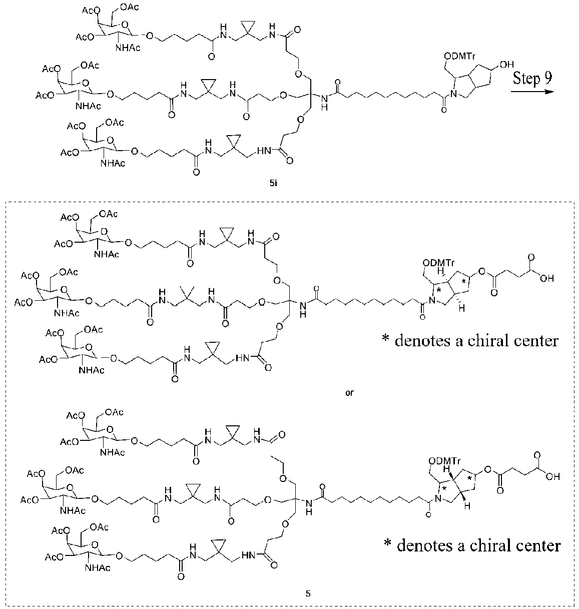

### Step 1

5a (10.2 g, 44.8 mmol) was dissolved in 360 mL of toluene, p-toluenesulphonic acid monohydrate (853 mg, 4.48 mmol) and ethylene glycol (5.57 g, 89.7 mmol) were added, and the mixture was stirred at 135°C for 12 hours. 200 mL of saturated sodium bicarbonate solution was added to the reaction solution, and the resulting reaction solution was extracted with dichloromethane (200 mL × 2). The organic phase was washed with a saturated sodium chloride solution (200 mL × 2), dried over anhydrous sodium sulphate, and filtered. The filtrate was concentrated by reduced-pressure distillation, and the resulting residue was purified by silica gel column chromatography using developing solvent system B, yielding title product 5b (3.67 g, yield: 30%) as a yellow oil.

MS m/z (ESI): 214.0 [M-55].

### Step 2

5b (9.5 g, 35.3 mmol) and (+)-sparteine (8.27 g, 35.3 mmol) were dissolved in 190 mL of tetrahydrofuran. Under nitrogen atmosphere, the mixture was cooled to below -78°C. Subsequently, sec-butyllithium (1.3M, 40.7 mL) was slowly added dropwise to the reaction solution, and the resulting mixture was stirred at -78°C for 1.5 hours. Then, methyl chloroformate (4.01 g, 42.4 mmol) was slowly added dropwise to the reaction solution. The mixture was stirred at -78 °C for 1 hour, and then naturally warmed to room temperature and stirred for 12 hours. At 0°C, the reaction solution was slowly poured into 200 mL of saturated ammonium chloride solution, and the resulting reaction solution was extracted with dichloromethane (100 mL × 2). The organic phase was washed with a saturated sodium chloride solution (100 mL × 2), dried over anhydrous sodium sulphate, and filtered. The filtrate was concentrated by reduced-pressure distillation, and the resulting residue was purified by silica gel column chromatography using developing solvent system B, yielding title product 5c (2.54 g, yield: 16%) as a yellow oil.

MS m/z (ESI): 228.1 [M-99].

### Step 3

5c (2.54 g, 5.65 mmol) was dissolved in 12 mL of tetrahydrofuran, hydrochloric acid (4M, 12 mL) was added, and the mixture was stirred at 25°C for 2 hours. The reaction solution was concentrated by reduced-pressure distillation, and the resulting residue was carried forward without purification, yielding crude title product 5d (1.04 g) as a yellow oil, which was directly used in the next reaction without purification.

MS m/z (ESI): 184.0 [M+1].

### Step 4

5d (1.04 g, 5.68 mmol) was dissolved in 10 mL of tetrahydrofuran and 10 mL of saturated sodium bicarbonate solution, 9-fluorenylmethyl-N-succinimidyl carbonate (2.3 g, 6.81 mmol) was added, and the mixture was stirred at 25°C for 1 hour. 10 mL of water was added to the reaction solution, and the resulting reaction solution was extracted with dichloromethane (20 mL × 3). The organic phase was washed with a saturated sodium chloride solution (20 mL × 2), and then dried over anhydrous sodium sulphate, and filtered. The filtrate was concentrated by reduced-pressure distillation, and the resulting residue was purified by silica gel column chromatography using developing solvent system B, yielding title product 5e (1.24 g, yield: 51%) as a yellow oil.

5e (1.24 g, 2.91 mmol) was separated by SFC (separation conditions: chromatographic column: DAICEL CHIRALCEL OJ (250 mm*30 mm, 10 µm); mobile phase: A-carbon dioxide: B-ethanol, isocratic elution: B: 35%), yielding the single-configuration compounds 5e-1 and 5e-2.

### First single-configuration compound of 5e with a shorter retention time

Colourless oil, 201 mg, yield: 52%. SFC analysis: retention time: 1.636 minutes. (chromatographic column: Chiralpak AD-3 50 × 4.6 mm I.D., 3 µm, mobile phase: A-carbon dioxide, B-ethanol (0.05% diethylamine), gradient elution: B: 5%-40%, flow rate: 3 mL/min, instrument: Shimadzu LC-30ADsf).

MS m/z (ESI): 406.0 [M+1].

### Second single-configuration compound of 5e with a longer retention time

Colourless oil, 197 mg, yield: 16%. SFC analysis: retention time: 1.830 minutes. (chromatographic column: Chiralpak AD-3 50 × 4.6 mm I.D., 3 µm, mobile phase: A-carbon dioxide, B-ethanol (0.05% diethylamine), gradient elution: B: 5%-40%, flow rate: 3 mL/min, instrument: Shimadzu LC-30ADsf).

MS m/z (ESI): 406.0 [M+1].

### Step 5

First single-configuration compound of 5e (472 mg, 1.16 mmol) was dissolved in 5 mL of tetrahydrofuran. Under nitrogen atmosphere, the mixture was cooled to below 0°C. A solution of lithium borohydride in tetrahydrofuran (2M, 2.32 mL) was added dropwise, and the mixture was stirred at 25°C for 1 hour. At 0°C, 10 mL of saturated ammonium chloride solution was added, and the reaction solution was extracted with dichloromethane (10 mL × 3). The organic phase was washed with a saturated sodium chloride solution (10 mL × 2), and then dried over anhydrous sodium sulphate, and filtered. The filtrate was concentrated by reduced-pressure distillation, and the resulting residue was purified by silica gel column chromatography using developing solvent system A, yielding title product 5f (297 mg, yield: 66%) as a colourless viscous substance.

MS m/z (ESI): 380.0 [M+1].

### Step 6

5f (15 mg, 38.73 µmol) was dissolved in 3 mL of pyridine. Under nitrogen atmosphere, 4,4'-dimethoxytrityl chloride (13.13 mg, 38.75 µmol) was dissolved in 3 mL of dichloromethane, and the mixture was added dropwise to the reaction solution at 0°C. The resulting mixture was stirred at 25°C for 12 hours. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 5g (9 mg, yield: 30%) as a white solid.

### Step 7

5g (9 mg, 11.88 µmol ) and piperidine (3.03 mg, 35.65 µmol) were dissolved in 1 mL of N,N-dimethylformamide, and the mixture was stirred and reacted at 25°C for 6 hours. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 5h (5 mg, yield: 87%) as a white solid.

### Step 8

5h (3.03 mg, 5.93 µmol) and 4b (13 mg, 5.93 µmol) were dissolved in 2 mL of dichloromethane. 2-(7-Azobenzotriazole)-N,N,N',N'-tetramethyluronium hexafluorophosphate (4.51 mg, 11.86 µmol) and N,N-diisopropylethylamine (3.83 mg, 29.63 µmol) were added, and the mixture was reacted at 25°C for 0.5 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-70%), yielding title product 5i (10 mg, yield: 60%) as a white solid.

### Step 9

5i (8 mg, 3.01 µmol) was dissolved in 1 mL of dichloromethane. Under nitrogen atmosphere, 4-dimethylaminopyridine (0.07 mg, 0.57 µmol), succinic anhydride (1.51 mg, 15.09 µmol) and N,N-diisopropylethylamine (3.88 mg, 30.02 µmol) were added, and the mixture was stirred at 25°C for 15 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-60%), yielding title product 5 (3.8 mg, yield: 43%) as a white solid.

MS m/z (ESI): 2325.1 [M-301].

¹H NMR (400 MHz, CD₃OD) δ 7.48 - 7.45 (m, 1H), 7.39 - 7.33 (m, 4H), 7.27 - 7.26 (m, 2H), 7.25 - 7.24 (m, 2H), 6.88 - 6.86 (m, 1H), 6.86 - 6.84 (m, 2H), 6.83 - 6.82 (m, 1H), 5.33 - 5.31 (m, 3H), 5.07 - 5.03 (m, 4H), 4.57 - 4.54 (m, 3H), 4.17 - 4.05 (m, 12H), 4.02 - 3.97 (m, 4H), 3.90 - 3.84 (m, 5H), 3.78 - 3.77 (m, 6H), 3.70 - 3.68 (m, 6H), 3.67 - 3.65 (m, 4H), 3.55 - 3.50 (m, 4H), 3.48 - 3.45 (m, 2H), 3.15 - 3.11 (m, 12H), 2.48 - 2.42 (m, 10H), 2.26 - 2.20 (m, 8H), 2.14 - 2.11 (m, 9H), 2.02 - 1.99 (m, 9H), 1.96 - 1.90 (m, 18H), 1.72 - 1.64 (m, 8H), 1.62 - 1.57 (m, 8H), 1.32 - 1.25 (m, 18H), 0.50 - 0.45 (m, 12H).

### Example 6: Preparation of compound 6

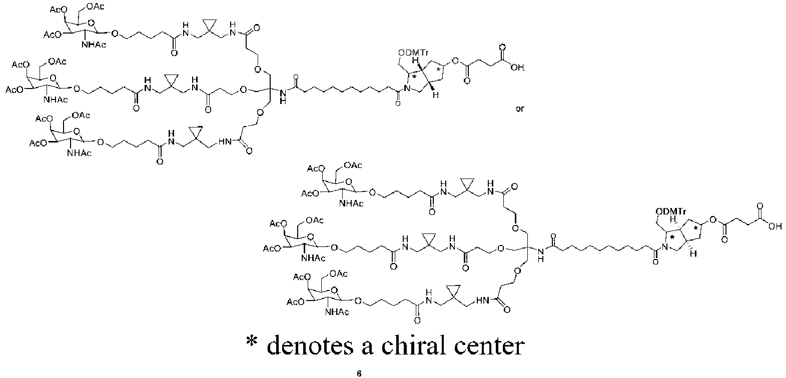

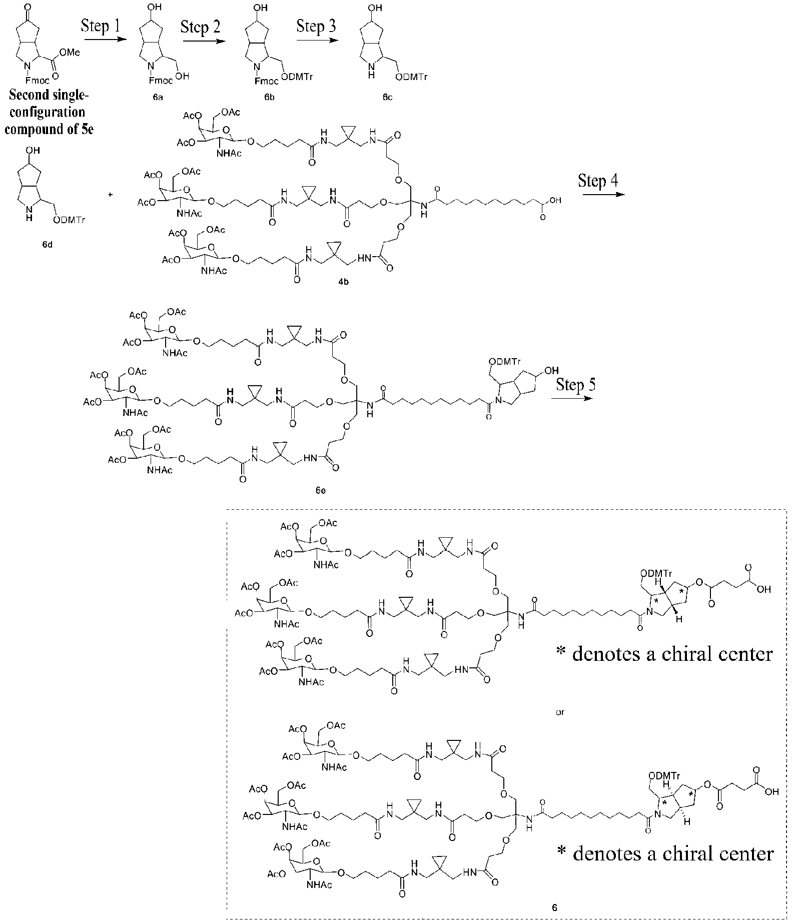

### Step 1

Second single-configuration compound of 5e (469 mg, 1.09 mmol) was dissolved in 5 mL of tetrahydrofuran. Under nitrogen atmosphere, the mixture was cooled to below 0°C. A solution of lithium borohydride in tetrahydrofuran (2M, 2.19 mL) was added dropwise, and the mixture was stirred at 25°C for 1 hour. At 0°C, 10 mL of saturated ammonium chloride solution was added, and the reaction solution was extracted with dichloromethane (10 mL × 3). The organic phase was washed with a saturated sodium chloride solution (10 mL × 2), and then dried over anhydrous sodium sulphate, and filtered. The filtrate was concentrated by reduced-pressure distillation, and the resulting residue was purified by silica gel column chromatography using developing solvent system A, yielding title product 6a (278 mg, yield: 66%) as a colourless viscous substance.

MS m/z (ESI): 380.0 [M+1].

### Step 2

6a (46 mg, 0.12 mmol) and 2,4,6-trimethylpyridine (58.76 mg, 0.48 mmol) were dissolved in 3 mL of dichloromethane. Under nitrogen atmosphere, 4,4'-dimethoxytrityl chloride (61.61 mg, 0.18 mmol) and silver nitrate (41.18 mg, 0.24 mmol) were added, and the mixture was stirred at 25°C for 0.5 hours. 1 mL of methanol was added to quench the reaction solution. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 6b (47 mg, yield: 55%) as a light yellow oil.

MS m/z (ESI): 704.3 [M+23].

### Step 3

6b (47 mg, 68.93 µmol) was dissolved in 1 mL of N,N-dimethylformamide, 1,5-diazabicyclo[5.4.0]undec-5-ene (10.49 mg, 68.90 µmol) was then added, and the mixture was stirred and reacted at room temperature for 1 hour. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-90%), yielding title product 6c (22 mg, yield: 68%) as a colourless oil.

MS m/z (ESI): 460.4 [M+1].

### Step 4

6c (3.83 mg, 8.33 µmol) and 4b (14 mg, 6.72 µmol) were dissolved in 1 mL of N,N-dimethylformamide. 2-(7-Azobenzotriazole)-N,N,N',N'-tetramethyluronium hexafluorophosphate (3.06 mg, 8.05 µmol) and N,N-diisopropylethylamine (3.47 mg, 26.85 µmol) were added, and the mixture was reacted at 25°C for 0.5 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 6d (11 mg, yield: 50%) as a white solid.

MS m/z (ESI): 2224.8 [M-301].

### Step 5

6d (11 mg, 4.35 µmol) was dissolved in 1 mL of dichloromethane. Under nitrogen atmosphere, 4-dimethylaminopyridine (0.53 mg, 4.34 µmol), succinic anhydride (4.36 mg, 43.57 µmol) and N,N-diisopropylethylamine (11.26 mg, 87.12 µmol) were added, and the mixture was stirred at 25°C for 15 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-90%), yielding title product 6 (10.64 mg, yield: 92%) as a white solid.

MS m/z (ESI): 2324.8 [M-301].

¹H NMR (400 MHz, CDCl₃) δ 7.56 - 7.54 (m, 1H), 7.34 - 7.31 (m, 2H), 7.30 - 7.30 (m, 1H), 7.29 - 7.29 (m, 1H), 7.20 - 7.18 (m, 2H), 7.17 - 7.16 (m, 2H), 6.94 - 6.91 (m, 1H), 6.91 - 6.89 (m, 1H), 6.86 - 6.84 (m, 2H), 6.84 - 6.82 (m, 2H), 6.74 - 6.70 (m, 1H), 5.37 - 5.34 (m, 3H), 5.22 - 5.17 (m, 3H), 4.66 - 4.60 (m, 3H), 4.26 - 4.22 (m, 1H), 4.21 - 4.18 (m, 2H), 4.17 - 4.15 (m, 2H), 4.13 - 4.12 (m, 2H), 4.11 - 4.08 (m, 2H), 4.00 - 3.97 (m, 2H), 3.96 - 3.94 (m, 2H), 3.93 - 3.91 (m, 2H), 3.82 - 3.80 (m, 6H), 3.73 - 3.72 (m, 2H), 3.71 - 3.70 (m, 4H), 3.69 - 3.67 (m, 4H), 3.64 - 3.59 (m, 2H), 3.57 - 3.50 (m, 4H), 3.22 - 3.17 (m, 2H), 3.16 - 3.13 (m, 2H), 3.13 - 3.10 (m, 4H), 3.09 - 3.05 (m, 2H), 2.62 - 2.59 (m, 2H), 2.57 - 2.54 (m, 2H), 2.50 - 2.46 (m, 6H), 2.38 - 2.34 (m, 2H), 2.33 - 2.31 (m, 2H), 2.31 - 2.28 (m, 4H), 2.27 - 2.25 (m, 2H), 2.25 - 2.21 (m, 2H), 2.20 - 2.17 (m, 2H), 2.17 - 2.15 (m, 9H), 2.12 - 2.10 (m, 2H), 2.06 - 2.05 (m, 9H), 2.02 - 2.00 (m, 9H), 1.98 - 1.95 (m, 9H), 1.91 - 1.87 (m, 2H), 1.86 - 1.82 (m, 2H), 1.82 - 1.76 (m, 4H), 1.74 - 1.69 (m, 4H), 1.67 - 1.61 (m, 8H), 1.57 - 1.53 (m, 2H), 1.33 - 1.31 (m, 2H), 1.29 - 1.24 (m, 10H), 0.54 - 0.48 (m, 12H).

### Example 7: Preparation of compound 7

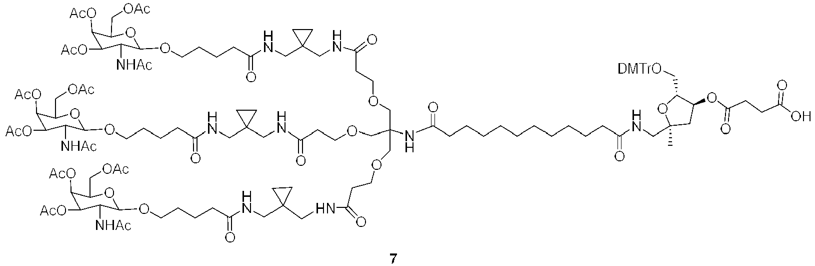

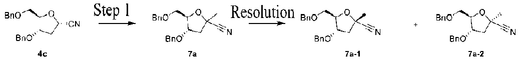

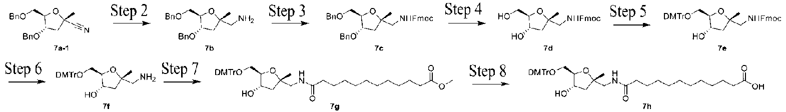

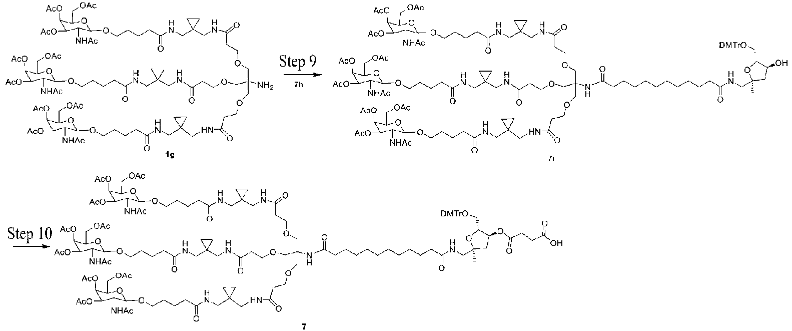

### Step 1

4c (2.2 g, 6.8 mmol) and iodomethane (1.4 g, 9.86 mmol) were dissolved in 12 mL of tetrahydrofuran. Under nitrogen atmosphere, potassium bis(trimethylsilyl)amide (1 M, 33 mL) was slowly added dropwise, and the mixture was naturally warmed to 25°C and stirred for 0.5 hours. At 0°C, 200 mL of saturated ammonium chloride solution was added to quench the reaction solution. The reaction solution was extracted with ethyl acetate (70 mL × 3), and the organic phase was washed with a saturated sodium chloride solution (50 mL × 2), dried over anhydrous sodium sulphate, and filtered. The filtrate was concentrated by reduced-pressure distillation, and title product 7a (998 mg) was obtained as a white solid. 7a (998 mg, 2.8 mmol) was purified by silica gel column chromatography using developing solvent system B, yielding title products 7a-1 (570 mg, yield: 57%) and 7a-2 (420 mg, yield: 42%) respectively as white solids.

### Step 2

7a-1 (500 mg, 1.4 mmol) was dissolved in 35 mL of tetrahydrofuran. Under nitrogen atmosphere, lithium aluminium hydride (225.52 mg, 5.94 mmol) was slowly added, and the mixture was stirred at 70°C for 2 hours. 225 µL of water, 225 µL of 15% sodium hydroxide aqueous solution, and 675 µL of water were added sequentially to quench the reaction solution. The reaction solution was dried over anhydrous sodium sulphate and filtered through Celite. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 7b (380 mg, yield: 78%) as a white solid.

MS m/z (ESI): 342.2 [M+1].

### Step 3

7b (360 mg, 1.0 mmol) was dissolved in 5 mL of tetrahydrofuran and 5 mL of water, 9-fluorenylmethyl-N-succinimidyl carbonate (426.93 mg, 1.27 mmol) and sodium bicarbonate (319.25 mg, 3.8 mmol) were added, and the mixture was stirred at 25°C for 1 hour. 20 mL of water was added to the reaction solution, and the resulting reaction solution was extracted with ethyl acetate (30 mL × 3). The organic phase was washed with a saturated sodium chloride solution (50 mL × 2), and then dried over anhydrous sodium sulphate, and filtered. The filtrate was concentrated by reduced-pressure distillation, and the resulting residue was purified by silica gel column chromatography using developing solvent system B, yielding title product 7c (398 mg, yield: 69%) as a yellow solid.

MS m/z (ESI): 564.5 [M+1].

### Step 4

7c (338 mg, 0.59 mmol) was dissolved in 35 mL of super-dry dichloromethane. Under nitrogen atmosphere, the mixture was cooled to below - 70°C. Subsequently, boron trichloride (1M, 6.89 mL) was slowly added dropwise to the reaction solution, and the resulting mixture was stirred at a temperature maintained below -70°C for 15 minutes. 30 mL of methanol was slowly added to the reaction solution. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 7d (67 mg, yield: 29%) as a colourless oil.

MS m/z (ESI): 384.3 [M+1].

### Step 5

7d (48 mg, 125.18 µmol) and 2,4,6-trimethylpyridine (37.91 mg, 312.84 µmol) were dissolved in 5 mL of super-dry dichloromethane. Under nitrogen atmosphere, 4,4'-dimethoxytrityl chloride (38.21 mg, 112.77 µmol) and silver nitrate (21.28 mg, 125.27 µmol) were added, and the mixture was stirred at 25°C for 0.5 hours. 20 mL of methanol was added to quench the reaction solution, and the reaction solution was filtered. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 7e (54 mg, yield: 61%) as a white solid.

### Step 6

7e (53 mg, 77.29 µmol) was dissolved in 1 mL of N,N-dimethylformamide, 1,5-diazabicyclo[5.4.0]undec-5-ene (11.83 mg, 77.71 µmol) was then added, and the mixture was stirred and reacted at room temperature for 1 hour. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 7f (30 mg, yield: 84%) as a colourless oil.

MS m/z (ESI): 464.4 [M+1].

### Step 7

7f (30 mg, 64.72 µmol) and monomethyl dodecanedioate (17.39 mg, 71.09 µmol) were dissolved in 2 mL of N,N-dimethylformamide. 2-(7-Azobenzotriazole)-N,N,N',N'-tetramethyluronium hexafluorophosphate (29.53 mg, 77.66 µmol) and N,N-diisopropylethylamine (33.46 mg, 258.9 µmol) were added, and the mixture was reacted at 25°C for 0.5 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 30%-100%), yielding title product 7g (36 mg, yield: 77%) as a white solid.

MS m/z (ESI): 712.7 [M+23].

### Step 8

7g (36 mg, 49.87 µmol) was dissolved in 2 mL of methanol and 1 mL of water. Lithium hydroxide (12.5 mg, 521.92 µmol) was added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 5%-80%), yielding title product 7h (26 mg, yield: 76%) as a white solid.

MS m/z (ESI): 698.7 [M+23].

### Step 9

7h (12 mg, 17.76 µmol) and 1g (33.24 mg, 17.76 µmol) were dissolved in 1 mL of N,N-dimethylformamide. 2-(7-Azobenzotriazole)-N,N,N',N'-tetramethyluronium hexafluorophosphate (8.1 mg, 21.3 µmol) and N,N-diisopropylethylamine (9.18 mg, 71.03 µmol) were added, and the mixture was reacted at 25°C for 0.5 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-90%), yielding title product 7i (16 mg, yield: 35%) as a white solid.

MS m/z (ESI): 2228.8 [M-301].

### Step 10

7i (16 mg, 6.31 µmol) was dissolved in 2 mL of dichloromethane. Under nitrogen atmosphere, 4-dimethylaminopyridine (0.77 mg, 6.3 µmol), succinic anhydride (6.33 mg, 63.26 µmol) and N,N-diisopropylethylamine (16.35 mg, 126.51 µmol) were added, and the mixture was stirred at 25°C for 15 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-90%), yielding title product 7 (16 mg, yield: 96%) as an off-white solid.

MS m/z (ESI): 2329.0 [M-301].

¹H NMR (400 MHz, CDCl₃) δ 7.57 - 7.55 (m, 1H), 7.34 - 7.32 (m, 1H), 7.32 - 7.31 (m, 1H), 7.30 - 7.30 (m, 1H), 7.29 - 7.29 (m, 1H), 7.20 - 7.16 (m, 4H), 6.97 - 6.91 (m, 1H), 6.90 - 6.88 (m, 1H), 6.86 - 6.85 (m, 2H), 6.84 - 6.82 (m, 2H), 6.71 - 6.67 (m, 1H), 5.37 - 5.34 (m, 3H), 5.22 - 5.18 (m, 3H), 4.65 - 4.61 (m, 3H), 4.22 - 4.09 (m, 10H), 4.08 - 4.03 (m, 2H), 4.01 - 3.87 (m, 8H), 3.82 - 3.80 (m, 6H), 3.74 - 3.72 (m, 2H), 3.71 - 3.69 (m, 6H), 3.68 - 3.66 (m, 2H), 3.55 - 3.49 (m, 4H), 3.38 - 3.30 (m, 2H), 3.21 - 3.16 (m, 2H), 3.16 - 3.14 (m, 2H), 3.13 - 3.11 (m, 4H), 3.09 - 3.06 (m, 2H), 2.66 - 2.60 (m, 4H), 2.50 - 2.46 (m, 6H), 2.37 - 2.32 (m, 2H), 2.32 - 2.28 (m, 4H), 2.27 - 2.25 (m, 2H), 2.25 - 2.23 (m, 2H), 2.19 - 2.18 (m, 2H), 2.17 - 2.15 (m, 9H), 2.12 - 2.10 (m, 2H), 2.06 - 2.05 (m, 9H), 2.02 - 2.00 (m, 9H), 1.98 - 1.96 (m, 9H), 1.83 - 1.80 (m, 2H), 1.79 - 1.77 (m, 2H), 1.76 - 1.73 (m, 2H), 1.72 - 1.70 (m, 2H), 1.69 - 1.67 (m, 2H), 1.66 - 1.61 (m, 6H), 1.59 - 1.52 (m, 4H), 1.29 - 1.28 (m, 3H), 1.28 - 1.25 (m, 8H), 0.54 - 0.48 (m, 12H).

### Example 8: Preparation of compound 8

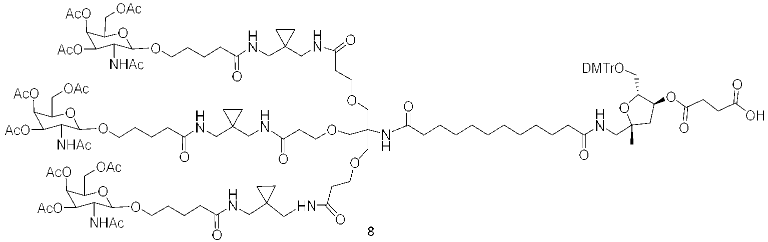

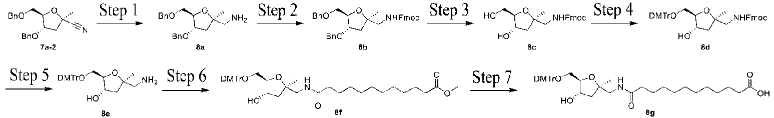

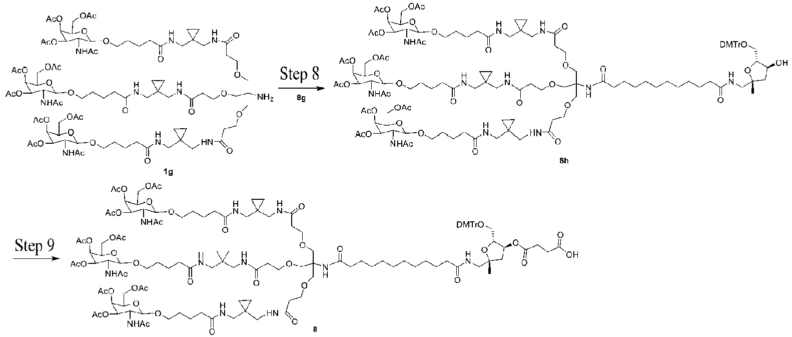

### Step 1

7a-2 (420 mg, 1.24 mmol) was dissolved in 30 mL of tetrahydrofuran. Under nitrogen atmosphere, lithium aluminium hydride (189.44 mg, 4.99 mmol) was slowly added, and the mixture was stirred at 70°C for 2 hours. At 0°C, 193 µL of water, 193 µL of 15% sodium hydroxide aqueous solution, and 579 µL of water were added sequentially to quench the reaction solution. The reaction solution was dried over anhydrous sodium sulphate and filtered through Celite. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 8a (357 mg, yield: 82%) as a white solid.

MS m/z (ESI): 342.3 [M+1].

### Step 2

8a (337 mg, 0.99 mmol) was dissolved in 5 mL of tetrahydrofuran and 5 mL of water, 9-fluorenylmethyl-N-succinimidyl carbonate (399.66 mg, 1.18 mmol) and sodium bicarbonate (249.04 mg, 2.96 mmol) were added, and the mixture was stirred at 25°C for 1 hour. 20 mL of water was added to the reaction solution, and the resulting reaction solution was extracted with ethyl acetate (30 mL × 3). The organic phase was washed with a saturated sodium chloride solution (50 mL × 2), and then dried over anhydrous sodium sulphate, and filtered. The filtrate was concentrated by reduced-pressure distillation, and the resulting residue was purified by silica gel column chromatography using developing solvent system B, yielding title product 8b (420 mg, yield: 74%) as a yellow solid.

MS m/z (ESI): 564.5 [M+1].

### Step 3

8b (410 mg, 0.71 mmol) was dissolved in 35 mL of super-dry dichloromethane. Under nitrogen atmosphere, the mixture was cooled to below - 70°C. Subsequently, boron trichloride (1M, 7.28 mL) was slowly added dropwise to the reaction solution, and the resulting mixture was stirred at a temperature maintained below -70°C for 15 minutes. 20 mL of methanol was slowly added to the reaction solution. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 8c (120 mg, yield: 43%) as a colourless oil.

MS m/z (ESI): 384.3 [M+1].

### Step 4

8c (120 mg, 0.31 mmol) was dissolved in 2 mL of super-dry pyridine. Subsequently, 4,4'-dimethoxytrityl chloride (159.23 mg, 0.47 mmol) was dissolved in 2 mL of dichloromethane, and the mixture was slowly added dropwise to the reaction solution under nitrogen atmosphere. The resulting mixture was stirred at 25°C for 4 hours. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 8d (70 mg, yield: 32%) as a white solid.

### Step 5

8d (62 mg, 89.5 µmol) was dissolved in 1 mL of N,N-dimethylformamide, 1,5-diazabicyclo[5.4.0]undec-5-ene (6.8 mg, 44.67 µmol) was then added, and the mixture was stirred and reacted at room temperature for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-70%), yielding title product 8e (30 mg, yield: 71%) as a white solid.

### Step 6

8e (6 mg, 12.81 µmol) and monomethyl dodecanedioate (4.7 mg, 19.24 µmol) were dissolved in 3.5 mL of N,N-dimethylformamide. 2-(7-Azobenzotriazole)-N,N,N',N'-tetramethyluronium hexafluorophosphate (7.31 mg, 19.22 µmol) and N,N-diisopropylethylamine (8.29 mg, 64.14 µmol) were added, and the mixture was reacted at 25°C for 0.5 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-95%), yielding title product 8f (7 mg, yield: 75%) as a white solid.

### Step 7

8f (7 mg, 9.64 µmol) was dissolved in 1.5 mL of methanol and 0.5 mL of water. Lithium hydroxide (20 mg, 0.84 mmol) was added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-95%), yielding title product 8g (5 mg, yield: 73%) as a white solid.

MS m/z (ESI): 675.6 [M+1].

### Step 8

8g (5 mg, 7.03 µmol) and 1g (13.16 mg, 7.03 µmol) were dissolved in 1 mL of N,N-dimethylformamide. 2-(7-Azobenzotriazole)-N,N,N',N'-tetramethyluronium hexafluorophosphate (2.94 mg, 7.73 µmol) and N,N-diisopropylethylamine (5.45 mg, 42.17 µmol) were added, and the mixture was reacted at 25°C for 0.5 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 15%-95%), yielding title product 8h (5 mg, yield: 26%) as a white solid.

MS m/z (ESI): 2228.8 [M-301].

### Step 9

8h (5 mg, 1.88 µmol) was dissolved in 1 mL of dichloromethane. Under nitrogen atmosphere, 4-dimethylaminopyridine (2.29 mg, 18.74 µmol), succinic anhydride (3.76 mg, 37.57 µmol) and N,N-diisopropylethylamine (7.28 mg, 56.33 µmol) were added, and the mixture was stirred at 25°C for 16 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 15%-95%), yielding title product 8 (2.9 mg, yield: 58%) as an off-white solid.

MS m/z (ESI): 2328.9 [M-301].

### Example 9: Preparation of compound 9

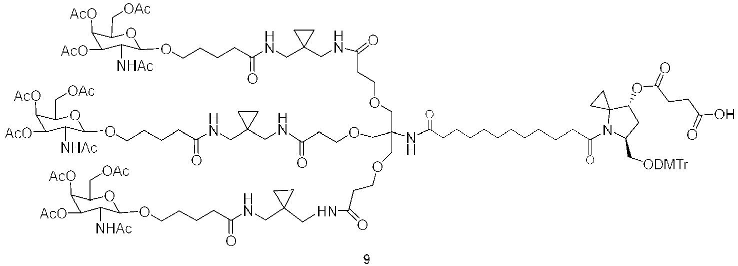

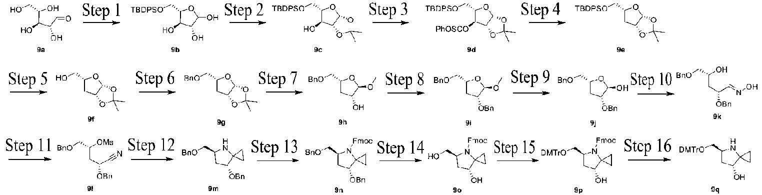

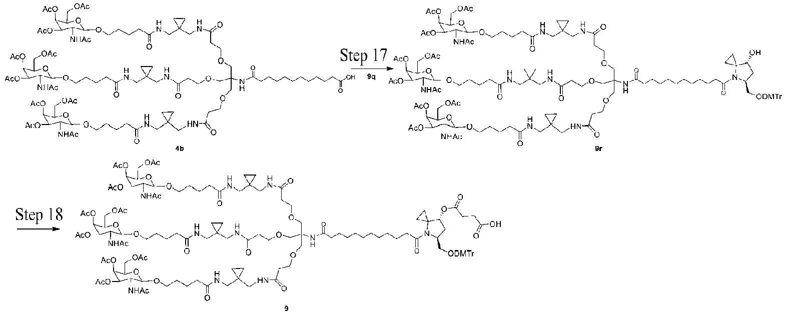

### Step 1

9a (20 g, 133.22 mmol) was dissolved in 400 mL of N,N-dimethylformamide. Under nitrogen atmosphere, imidazole (10.88 g, 159.86 mmol) and tert-butyldiphenylchlorosilane (43.94 g, 159.86 mmol) were slowly added, and the mixture was stirred at 25°C for 12 hours. The reaction solution was concentrated by reduced-pressure distillation, and 200 mL of water was added to the resulting residue. The resulting reaction solution was extracted with ethyl acetate (200 mL × 3), and the organic phase was washed with a saturated sodium chloride solution (200 mL × 2), dried over anhydrous sodium sulphate, and filtered. The filtrate was concentrated by reduced-pressure distillation, and the resulting residue was purified by silica gel column chromatography using developing solvent system A, yielding title product 9b (35 g, yield: 60%) as a colourless oil.

### Step 2

9b (35 g, 81.07 mmol) was dissolved in 400 mL of acetone. Under nitrogen atmosphere, copper sulphate (19.41 g, 121.61 mmol) and sulphuric acid (12 M, 2.16 mL) were slowly added, and the mixture was stirred at 25°C for 12 hours. The reaction solution was filtered, and 50 mL of saturated sodium bicarbonate solution was added to the reaction solution. The mixture was concentrated by reduced-pressure distillation to remove acetone, and 200 mL of water was added to the resulting residue. The resulting reaction solution was extracted with ethyl acetate (200 mL × 3), and the organic phase was washed with a saturated sodium chloride solution (200 mL × 2), dried over anhydrous sodium sulphate, and filtered. The filtrate was concentrated by reduced-pressure distillation, and the resulting residue was purified by silica gel column chromatography using developing solvent system B, yielding title product 9c (20 g, yield: 52%) as a colourless oil.

### Step 3

9c (35 g, 73.49 mmol) was dissolved in 200 mL of dichloromethane. Under nitrogen atmosphere, pyridine (15.12 g, 191.09 mmol) and N,N-dimethylaniline (890.64 mg, 7.35 mmol) were added. Phenyl chlorothionoformate (19.03 g, 110.24 mmol) was dissolved in 50 mL of super-dry dichloromethane, and the mixture was slowly added dropwise to the reaction solution. The resulting mixture was stirred at 25°C for 12 hours. 100 mL of ice water was added to the reaction solution, and the resulting reaction solution was extracted with dichloromethane (200 mL × 3). The organic phase was washed with a saturated sodium chloride solution (200 mL × 2), dried over anhydrous sodium sulphate, and filtered. The filtrate was concentrated by reduced-pressure distillation, and the resulting residue was purified by silica gel column chromatography using developing solvent system B, yielding title product 9d (22 g, yield: 52%) as a colourless oil.

### Step 4

9d (12 g, 19.12 mmol) was dissolved in 100 mL of toluene. Under nitrogen atmosphere, azobisisobutyronitrile (314.02 mg, 1.91 mmol) was added, and the mixture was stirred at 25°C for 15 minutes. Subsequently, tributyltin hydride (1M, 5.66 mL) was slowly added dropwise, and the mixture was stirred at 80°C for 1 hour. The reaction solution was concentrated by reduced-pressure distillation, and the resulting residue was purified by silica gel column chromatography using developing solvent system B, yielding title product 9e (7.95 g, yield: 90%) as a colourless oil.

### Step 5

9e (7.95 g, 17.34 mmol) was dissolved in 100 mL of super-dry tetrahydrofuran. Under nitrogen atmosphere, tetrabutylammonium fluoride (1 M, 52.02 mL) was slowly added, and the mixture was stirred at 25°C for 12 hours. The reaction solution was concentrated by reduced-pressure distillation, and the resulting residue was purified by silica gel column chromatography using developing solvent system B, yielding title product 9f (2.56 g, yield: 76%) as a colourless oil.

### Step 6

9f (2.56 g, 13.23 mmol) was dissolved in 30 mL of super-dry N,N-dimethylformamide. Under nitrogen atmosphere, sodium hydride (793.52 mg, 19.84 mmol, 60%) was added, and the mixture was stirred at 0°C for 0.5 hours. Benzyl bromide (2.71 g, 15.87 mmol) was then added, and the resulting mixture was stirred at 25°C for 2 hours. 50 mL of ice water was added to the reaction solution, and the resulting reaction solution was extracted with ethyl acetate (50 mL × 3). The organic phase was washed with a saturated sodium chloride solution (50 mL × 3), dried over anhydrous sodium sulphate, and filtered. The filtrate was concentrated by reduced-pressure distillation, and the resulting residue was purified by silica gel column chromatography using developing solvent system B, yielding title product 9g (2.86 g, yield: 74%) as a colourless oil.

### Step 7

9g (2.86 g, 9.74 mmol) was dissolved in 30 mL of anhydrous methanol, 1 mL of concentrated hydrochloric acid was added, and the mixture was stirred at 68°C for 2 hours. The reaction solution was concentrated by reduced-pressure distillation, and the resulting residue was diluted with 50 mL of water. A saturated sodium bicarbonate solution was added to adjust the pH of the reaction solution to above 7, and the resulting reaction solution was extracted with ethyl acetate (50 mL × 3). The organic phase was washed with a saturated sodium chloride solution (50 mL × 3), dried over anhydrous sodium sulphate, and filtered. The filtrate was concentrated by reduced-pressure distillation, and the resulting residue was purified by silica gel column chromatography using developing solvent system B, yielding title product 9h (2.16 g, yield: 75%) as a colourless oil.

### Step 8

9h (2.16 g, 8.16 mmol) was dissolved in 10 mL of super-dry N,N-dimethylformamide. Under nitrogen atmosphere, sodium hydride (489.46 mg, 12.24 mmol, 60%) was added, and the mixture was stirred at 0°C for 0.5 hours. Benzyl bromide (1.67 g, 9.79 mmol) was then added, and the resulting mixture was stirred at 25°C for 2 hours. 30 mL of ice water was added to the reaction solution, and the resulting reaction solution was extracted with ethyl acetate (50 mL × 3). The organic phase was washed with a saturated sodium chloride solution (50 mL × 3), dried over anhydrous sodium sulphate, and filtered. The filtrate was concentrated by reduced-pressure distillation, and the resulting residue was purified by silica gel column chromatography using developing solvent system B, yielding title product 9i (2.4 g, yield: 80%) as a colourless oil.

### Step 9

9i (2.4 g, 6.58 mmol) was dissolved in 12 mL of acetic acid and 8 mL of water, concentrated sulphuric acid (12 M, 100 µL) was added, and the mixture was stirred at 70°C for 12 hours. 100 mL of saturated sodium bicarbonate solution was added to the reaction solution, and the resulting reaction solution was extracted with ethyl acetate (100 mL × 3). The organic phase was washed with a saturated sodium chloride solution (100 mL × 2), dried over anhydrous sodium sulphate, and filtered. The filtrate was concentrated by reduced-pressure distillation, and the resulting residue was purified by silica gel column chromatography using developing solvent system B, yielding title product 9j (1.59 g, yield: 69%) as a colourless oil.

### Step 10

Hydroxylamine hydrochloride (91.51 mg, 1.32 mmol) and sodium bicarbonate (96.2 mg, 1.15 mmol) were dissolved in 4 mL of ethanol and 4 mL of water, and the mixture was stirred at 25°C for 15 minutes. 9j (100 mg, 0.29 mmol) was then added, and the resulting mixture was stirred at 25°C for 2 hours. 10 mL of water was added to the reaction solution, and the resulting reaction solution was extracted with ethyl acetate (50 mL × 3). The organic phase was washed with a saturated sodium chloride solution (50 mL × 2), dried over anhydrous sodium sulphate, and filtered. The filtrate was concentrated by reduced-pressure distillation, and the resulting residue was carried forward without purification, yielding crude title product 9k (90 mg) as a colourless oil, which was directly used in the next reaction without purification.

### Step 11

Under nitrogen atmosphere, methylsulphonyl chloride (281.79 mg, 2.46 mmol) was dissolved in 2 mL of pyridine. 9k (90 mg, 245.9 µmol) was dissolved in 2 mL of pyridine, and the mixture was added dropwise to the reaction solution at 0°C. The resulting mixture was stirred at 25°C for 3 hours. 10 mL of water was added to the reaction solution, and the resulting reaction solution was extracted with ethyl acetate (30 mL × 3). The organic phase was washed with a saturated sodium chloride solution (30 mL × 2), dried over anhydrous sodium sulphate, and filtered. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% sodium bicarbonate): B-acetonitrile, gradient elution, flow rate: 30 mL/min, instrument: Biotage), yielding title product 9l (83 mg, yield: 78%) as a colourless oil.

### Step 12

9l (300 mg, 0.77 mmol) and tetraisopropyl titanate (328.39 mg, 1.16 mmol) were dissolved in 7 mL of super-dry tetrahydrofuran. Under nitrogen atmosphere, the mixture was cooled to below -78°C. Subsequently, ethylmagnesium bromide (2M, 1.16 mL) was slowly added dropwise to the reaction solution, and the resulting mixture was naturally warmed to 25°C and stirred for 1 hour. Then, boron trifluoride diethyl etherate (306.11 mg, 2.31 mmol) was slowly added dropwise to the reaction solution, and the resulting mixture was stirred at 25°C for 0.5 hours. The reaction solution was quenched with 3 mL of saturated ammonium chloride, then diluted with 30 mL of water, and extracted with ethyl acetate (30 mL × 3). The organic phase was washed with a saturated sodium chloride solution (30 mL × 2), and then dried over anhydrous sodium sulphate, and filtered. The filtrate was concentrated by reduced-pressure distillation, and the resulting residue was purified by preparative high performance liquid chromatography (separation conditions: chromatographic column: Phenomenex luna C18 150 × 25 mm × 4 µm; mobile phase: A-water (0.1% formic acid): B-acetonitrile, gradient elution: B%: 20%-80%), yielding title product 9m (50 mg, yield: 20%) as a colourless oil.

MS m/z (ESI):324.3 [M+1].

### Step 13

9m (50 mg, 154.59 µmol) was dissolved in 3 mL of 1,4-dioxane and 3 mL of water, sodium bicarbonate (64.93 mg, 772.88 µmol) and 9-fluorenylmethyl-N-succinimidyl carbonate (67.79 mg, 200.96 µmol) were added, and the mixture was stirred at 25°C for 1 hour. The reaction solution was diluted with 20 mL of water and extracted with ethyl acetate (20 mL × 3). The organic phase was washed with a saturated sodium chloride solution (20 mL × 2), then dried over anhydrous sodium sulphate, and filtered. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% sodium bicarbonate): B-acetonitrile, gradient elution, flow rate: 30 mL/min, instrument: Biotage), yielding title product 9n (74 mg, yield: 86%) as a colourless oil.

MS m/z (ESI):546.5 [M+1].

### Step 14

9n (74 mg, 135.61 µmol) was dissolved in 3 mL of super-dry dichloromethane. Under nitrogen atmosphere, the mixture was cooled to below - 70°C. Subsequently, boron trichloride (1 M, 1.36 mL) was slowly added dropwise to the reaction solution, and the resulting mixture was stirred at a temperature maintained below -70°C for 1 hour. At -70°C, 2 mL of methanol was slowly added to the reaction solution. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 9o (15 mg, yield: 29%) as a colourless oil.

MS m/z (ESI): 366.3 [M+1].

### Step 15

9o (15 mg, 41.05 µmol) and 2,4,6-trimethylpyridine (19.9 mg, 164.22 µmol) were dissolved in 2 mL of dichloromethane. Under nitrogen atmosphere, 4,4'-dimethoxytrityl chloride (20.86 mg, 61.56 µmol) and silver nitrate (13.95 mg, 82.12 µmol) were added, and the mixture was stirred at 25°C for 0.5 hours. 1 mL methanol was added to quench the reaction solution, and the mixture was filtered. The filtrate was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 9p (11 mg, yield: 40%) as a light yellow oil.

MS m/z (ESI): 690.6 [M+23].

### Step 16

9p (11 mg, 16.47 µmol) was dissolved in 1 mL of N,N-dimethylformamide, 1,5-diazabicyclo[5.4.0]undec-5-ene (2.51 mg, 16.49 µmol) was added, and the mixture was stirred at 25°C for 1 hour. The reaction solution was directly purified by reversed-phase liquid chromatography without prior treatment (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 9q (6.2 mg, yield: 81%) as a colourless oil.

MS m/z (ESI): 446.3 [M+1].

### Step 17

9q (1.28 mg, 2.87 µmol) and 4b (6 mg, 2.87 µmol) were dissolved in 1 mL of N,N-dimethylformamide. 2-(7-Azobenzotriazole)-N,N,N',N'-tetramethyluronium hexafluorophosphate (1.64 mg, 4.31 µmol) and N,N-diisopropylethylamine (1.49 mg, 11.53 µmol) were added, and the mixture was reacted at 25°C for 0.5 hours. The reaction solution was directly purified by preparative high performance liquid chromatography without prior treatment (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-100%), yielding title product 9r (5 mg, yield: 69%) as a white solid.

MS m/z (ESI): 2210.8 [M-301].

### Step 18

9r (5 mg, 1.99 µmol) was dissolved in 1 mL of dichloromethane. Under nitrogen atmosphere, 4-dimethylaminopyridine (0.24 mg, 1.96 µmol), succinic anhydride (3.98 mg, 39.77 µmol) and N,N-diisopropylethylamine (7.72 mg, 59.73 µmol) were added, and the mixture was stirred at 25°C for 15 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 10%-90%), yielding title product 9 (4.85 mg, yield: 92%) as a white solid.

MS m/z (ESI): 2310.9[M-301].

### Example 10: Preparation of compound 10

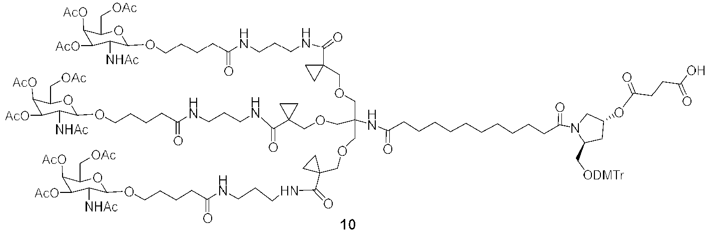

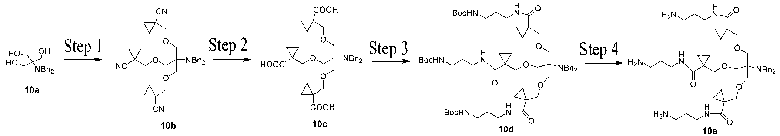

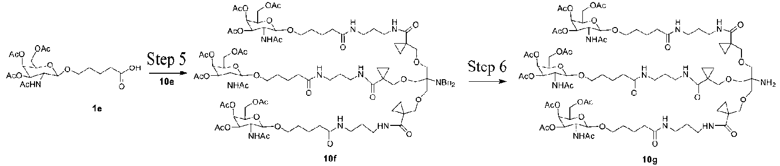

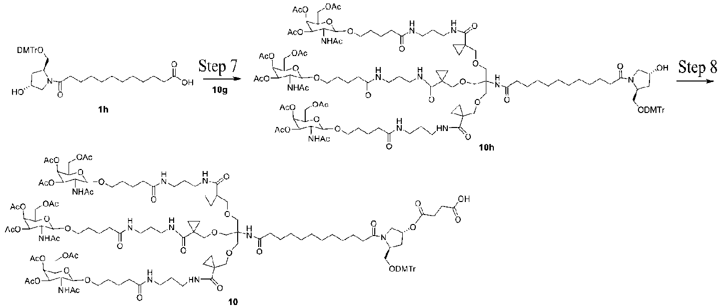

### Step 1

10a (300 mg, 0.996 mmol, prepared according to the method disclosed in Example 4 on page 65 of the patent application "WO 2022161452 A1") was dissolved in 20 mL of tetrahydrofuran. 1-(Bromomethyl)cyclopropanecarbonitrile (1.1 g, 6.97 mmol) and potassium tert-butoxide (1.01 g, 8.96 mmol) were added, and the mixture was stirred at 25°C for 12 hours. The reaction system was concentrated by reduced-pressure distillation to remove tetrahydrofuran, and 20 mL of water was added to the resulting residue. The reaction solution was extracted with ethyl acetate (50 mL × 3), and the organic phase was washed with a saturated sodium chloride solution (60 mL × 2), then dried over anhydrous sodium sulphate, and filtered. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 10b (453 mg, yield: 83%) as a yellow oil.

MS m/z (ESI): 539.5 [M+1].

### Step 2

10b (453 mg, 0.83 mmol) was dissolved in 7 mL of hydrochloric acid and 3.5 mL of water, and the mixture was stirred at 100°C for 12 hours. 20 mL of water was added to the reaction solution, and the resulting reaction solution was extracted with ethyl acetate (50 mL × 3). The organic phase was washed with a saturated sodium chloride solution (60 mL × 2), then dried over anhydrous sodium sulphate, and filtered. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 10c (283 mg, yield: 55%) as a white solid.

MS m/z (ESI): 596.5 [M+1].

### Step 3

10c (129 mg, 0.21 mmol) was dissolved in 1 mL of dichloromethane, N-tert-butyloxycarbonyl-1,3-propanediamine (292.84 mg, 1.68 mmol), 2-(7-azobenzotriazole)-N,N,N',N'-tetramethyluronium hexafluorophosphate (559.49 mg, 1.47 mmol) and N,N-diisopropylethylamine (190.18 mg, 1.47 mmol) were added, and the mixture was reacted at 25°C for 2 hours. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 10d (60 mg, yield: 24%) as a white solid.

MS m/z (ESI): 1065.1 [M+1].

### Step 4

10d (50 mg, 42.31 µmol) was dissolved in 0.5 mL of methanol, 5 mL of hydrogen chloride ethyl acetate solution was added, and the mixture was reacted at 25°C for 1 hour. The reaction solution was concentrated by reduced-pressure distillation, and the resulting residue was carried forward without purification, yielding crude title product 10e (50 mg) as a colourless oil, which was directly used in the next reaction without purification.

MS m/z (ESI): 764.8 [M+1].

### Step 5

10e (50 mg, 65.48 µmol) was dissolved in 1 mL of dichloromethane, N,N-diisopropylethylamine (59.25 mg, 0.46 mmol) was added, and the mixture was reacted for 2 minutes. The pH of the reaction solution was monitored and adjusted to be 8, and then 1e (117.14 mg, 0.26 mmol) and 2-(7-azobenzotriazole)-N,N,N',N'-tetramethyluronium hexafluorophosphate (224.11 mg, 0.59 mmol) were added, and the mixture was reacted at 25°C for 2 hours. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 10f (56 mg, yield: 42%) as a white solid.

MS m/z (ESI): 1027.4 [1/2M+1].

### Step 6

10f (50 mg, 23.96 µmol) was dissolved in 2 mL of methanol, and a single drop of acetic acid was added dropwise, followed by the addition of wet palladium on carbon (50 mg, 10%). The reaction solution was purged three times with hydrogen, and stirred at 25°C under hydrogen atmosphere (15 Psi) for 1 hour. The reaction solution was filtered through Celite. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 10g (30 mg, yield: 66%) as a white solid.

MS m/z (ESI): 937.2 [1/2M+1].

### Step 7

10g (30 mg, 16.03 µmol) was dissolved in 1 mL of N,N-dimethylformamide, 1h (15.19 mg, 24.04 µmol), 2-(7-azobenzotriazole)-N,N,N',N'-tetramethyluronium hexafluorophosphate (13.72 mg, 24.04 µmol) and N,N-diisopropylethylamine (13.99 mg, 48,08 µmol) were added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-80%), yielding title product 10h (29 mg, yield: 73%) as a white solid.

MS m/z (ESI): 1244.6 [1/2M+1].

### Step 8

10h (29 mg, 11.67 µmol) was dissolved in 1 mL of dichloromethane. Under nitrogen atmosphere, 4-dimethylaminopyridine (0.29 mg, 2.33 µmol), succinic anhydride (5.84 mg, 58.33 µmol) and N,N-diisopropylethylamine (15.09 mg, 116.66 µmol) were added, and the mixture was stirred at 25°C for 36 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-80%), yielding title product 10 (15 mg, yield: 49%) as a white solid.

MS m/z (ESI): 1294.1 [1/2M+1].

¹H NMR (400 MHz, CDCl₃) δ 7.41 - 7.40 (m, 1H), 7.32 - 7.30 (m, 2H), 7.29 - 7.28 (m, 2H), 7.20 - 7.18 (m, 2H), 7.17 - 7.16 (m, 2H), 6.86 - 6.85 (m, 2H), 6.84 - 6.82 (m, 2H), 5.38 - 5.36 (m, 3H), 5.27 - 5.21 (m, 4H), 4.68 - 4.65 (m, 3H), 4.22 - 4.16 (m, 4H), 4.15 - 4.09 (m, 4H), 4.09 - 4.01 (m, 4H), 4.00 - 3.89 (m, 8H), 3.81 - 3.80 (m, 6H), 3.79 - 3.77 (m, 4H), 3.74 - 3.66 (m, 4H), 3.55 - 3.48 (m, 10H), 3.33 - 3.25 (m, 12H), 2.66 - 2.58 (m, 5H), 2.30 - 2.28 (m, 2H), 2.27 - 2.24 (m, 4H), 2.19 - 2.18 (m, 2H), 2.17 - 2.15 (m, 9H), 2.12 - 2.09 (m, 2H), 2.07 - 2.05 (m, 9H), 2.02 - 2.00 (m, 9H), 1.98 - 1.95 (m, 9H), 1.69 - 1.59 (m, 20H), 1.29 - 1.21 (m, 18H), 0.71 - 0.67 (m, 6H).

### Example 11: Preparation of compound 11

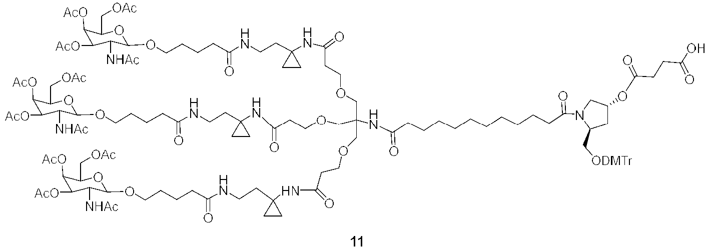

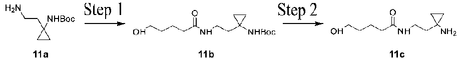

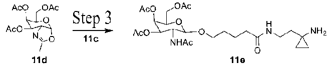

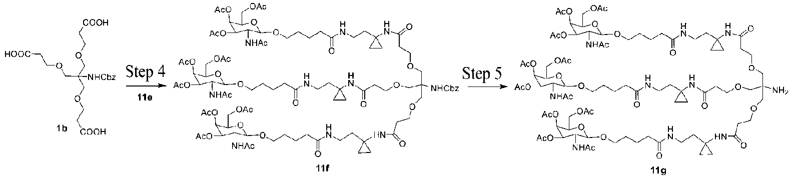

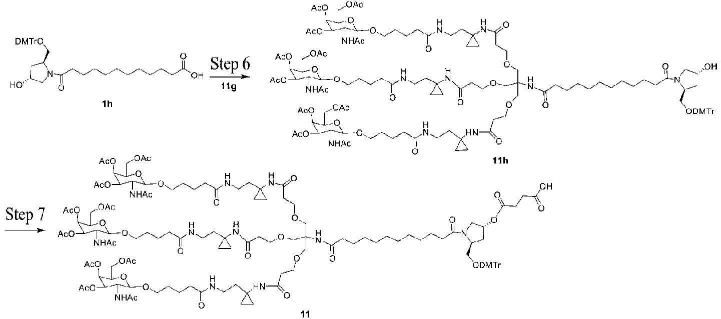

### Step 1

11a (0.92 g, 9.2 mmol, prepared according to the method disclosed in Example 16 on page 40 of the patent application "WO 2019154261 A1") was dissolved in 30 mL of dichloromethane, 5-pentanolide (0.92 g, 4.6 mmol) was added, and the mixture was stirred at 25°C for 12 hours. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 11b (900 mg, yield: 64%) as a white solid.

MS m/z (ESI): 301.2 [M+1].

### Step 2

11b (350 mg, 1.05 mmol) was dissolved in 20 mL of hydrogen chloride methanol, and the mixture was stirred at 25°C for 2 hours. The reaction solution was concentrated by reduced-pressure distillation, and the resulting residue was carried forward without purification, yielding crude title product 11c (239 mg) as a white solid, which was directly used in the next reaction without purification.

MS m/z (ESI): 201.1 [M+1].

### Step 3

11c (133 mg, 0.53 mmol) and 11d (700 mg, 2.12 mmol, prepared according to the method disclosed in Example 1 on page 96 of the patent application "WO 2009082607 A2") were dissolved in 5 mL of 1,2-dichloroethane, and 4A molecular sieve (140 mg) was added. Under nitrogen atmosphere, the mixture was stirred at 25°C for 0.5 hours. Trimethylsilyl trifluoromethanesulphonate (118.11 mg, 0.53 mmol) was added, and the resulting mixture was stirred at 25°C for 12 hours. The reaction solution was filtered, and 20 mL of saturated sodium bicarbonate solution was added to the filtrate. The reaction solution was extracted with dichloromethane (50 mL × 3), and the organic phase was washed with a saturated sodium chloride solution (60 mL × 2), then dried over anhydrous sodium sulphate, and filtered. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 11e (140 mg, yield: 40%) as a yellow solid. MS m/z (ESI): 530.5 [M+1].

### Step 4

11e (140 mg, 0.21 mmol) and 1b (35.61 mg, 0.06 mmol, prepared according to the method disclosed in Example 4 on page 182 of the patent application "CN106255755 A") were dissolved in 5 mL of dichloromethane. 2-(7-Azobenzotriazole)-N,N,N',N'-tetramethyluronium hexafluorophosphate (114.81 mg, 0.3 mmol) and N,N-diisopropylethylamine (187.07 mg, 1.45 mmol) were added, and the mixture was reacted at 25°C for 2 hours. The reaction solution was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 11f (48 mg, yield: 36%) as a white solid.

MS m/z (ESI): 1004.6 [1/2M+1].

### Step 5

11f (20 mg, 9.98 µmol) was dissolved in 0.5 mL of methanol and 0.5 mL of ethyl acetate, and a single drop of acetic acid was added dropwise, followed by the addition of wet palladium on carbon (20 mg, 10%). The reaction solution was purged three times with hydrogen, and stirred at 25°C under hydrogen atmosphere (15 Psi) for 1 hour. The reaction solution was filtered through Celite. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 11g (10 mg, yield: 53%) as a white solid.

MS m/z (ESI): 937.2 [1/2M+1].

### Step 6

11g (10 mg, 5.34 µmol) was dissolved in 1 mL of N,N-dimethylformamide, 1h (5.06 mg, 8.01 µmol), 2-(7-azobenzotriazole)-N,N,N',N'-tetramethyluronium hexafluorophosphate (3.05 mg, 8.02 µmol) and N,N-diisopropylethylamine (2.07 mg, 16.02 µmol) were added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-80%), yielding title product 11h (10 mg, yield: 75%) as a white solid.

MS m/z (ESI): 2183.7 [M-301].

### Step 7

11h (10 mg, 3.98 µmol) was dissolved in 1 mL of dichloromethane. Under nitrogen atmosphere, 4-dimethylaminopyridine (0.1 mg, 0.80 µmol), succinic anhydride (2.01 mg, 20.09 µmol) and N,N-diisopropylethylamine (5.2 mg, 40.23 µmol) were added, and the mixture was stirred at 25°C for 36 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-65%), yielding title product 11 (4 mg, yield: 34%) as a white solid.

MS m/z (ESI): 2284.4 [M-301].

¹H NMR (400 MHz, CDCl₃) δ 7.41 - 7.40 (m, 1H), 7.32 - 7.31 (m, 1H), 7.30 - 7.30 (m, 1H), 7.29 - 7.28 (m, 2H), 7.20 - 7.18 (m, 2H), 7.18 - 7.16 (m, 2H), 6.86 - 6.84 (m, 2H), 6.84 - 6.82 (m, 2H), 5.38 - 5.35 (m, 3H), 5.26 - 5.21 (m, 3H), 4.69 - 4.64 (m, 3H), 4.21 - 4.08 (m, 8H), 4.07 - 4.01 (m, 3H), 3.99 - 3.92 (m, 6H), 3.82 - 3.80 (m, 6H), 3.79 - 3.77 (m, 3H), 3.73 - 3.69 (m, 2H), 3.61 - 3.56 (m, 2H), 3.54 - 3.51 (m, 6H), 3.50 - 3.47 (m, 2H), 3.32 - 3.25 (m, 10H), 2.66 - 2.58 (m, 4H), 2.31 - 2.23 (m, 8H), 2.20 - 2.18 (m, 2H), 2.18 - 2.14 (m, 9H), 2.12 - 2.09 (m, 2H), 2.07 - 2.04 (m, 9H), 2.02 - 2.00 (m, 9H), 1.98 - 1.95 (m, 9H), 1.86 - 1.75 (m, 10H), 1.69 - 1.55 (m, 22H), 1.28 - 1.22 (m, 14H), 0.71 - 0.66 (m, 6H).

### Example 12: Preparation of compound 12

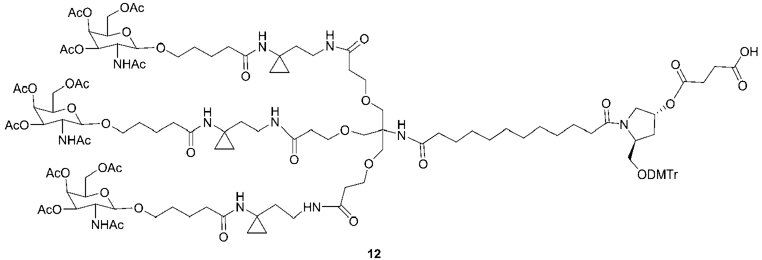

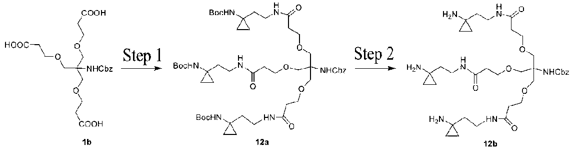

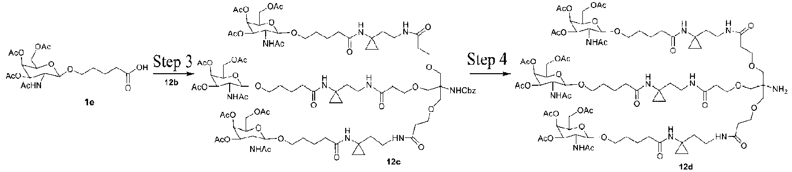

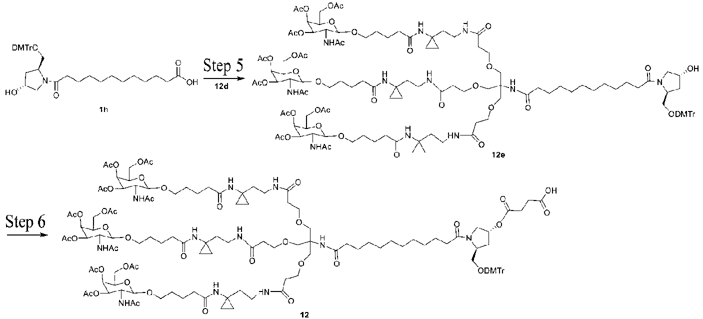

### Step 1

1b (200 mg, 0.41 mmol) and tert-butyl N-[1-(2-aminoethyl)cyclopropyl]carbamate (347.25 mg, 1.65 mmol) were dissolved in 8 mL of dichloromethane. 2-(7-Azobenzotriazole)-N,N,N',N'-tetramethyluronium hexafluorophosphate (635.91 mg, 1.65 mmol) and N,N-diisopropylethylamine (1.06 g, 8.24 mmol) were added, and the mixture was reacted at 25°C for 2 hours. 10 mL of water was added to the reaction solution, and the resulting reaction solution was extracted with dichloromethane (20 mL × 3). The organic phase was washed with a saturated sodium chloride solution (50 mL × 2), then dried over anhydrous sodium sulphate, and filtered. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 12a (310 mg, yield: 76%) as a white solid.

MS m/z (ESI): 1019.0 [M+1].

### Step 2

12a (320 mg, 0.31 mmol) was dissolved in 10 mL of hydrogen chloride methanol solution, and the mixture was reacted at 25°C for 2 hours. The reaction solution was concentrated by reduced-pressure distillation, and the resulting residue was carried forward without purification, yielding crude title product 12b (500 mg) as a yellow oil, which was directly used in the next reaction without purification.

MS m/z (ESI): 718.9 [M+1].

### Step 3

12b (165 mg, 0.19 mmol) and 1e (350 mg, 0.76 mmol) were dissolved in 5 mL of dichloromethane. 2-(7-Azobenzotriazole)-N,N,N',N'-tetramethyluronium hexafluorophosphate (360.67 mg, 0.95 mmol) and N,N-diisopropylethylamine (587.7 mg, 4.56 mmol) were added, and the mixture was reacted at 25°C for 2 hours. 10 mL of water was added to the reaction solution, and the resulting reaction solution was extracted with dichloromethane (20 mL × 3). The organic phase was washed with a saturated sodium chloride solution (50 mL × 2), then dried over anhydrous sodium sulphate, and filtered. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.05% ammonium bicarbonate): B-acetonitrile, gradient elution, flow rate: 30 mL/min, instrument: Biotage), yielding title product 12c (197 mg, yield: 46%) as a white solid.

MS m/z (ESI): 1004.3 [1/2M+1].

### Step 4

12c (50 mg, 23.93 µmol) was dissolved in 0.5 mL of methanol and 0.5 mL of ethyl acetate, and a single drop of acetic acid was added dropwise, followed by the addition of palladium on carbon (50 mg, 10%). The reaction solution was purged three times with hydrogen, and stirred at 25°C under hydrogen atmosphere (15 Psi) for 1 hour. The reaction solution was filtered through Celite. The filtrate was concentrated by reduced-pressure distillation, and the resulting residue was purified by reversed-phase liquid chromatography (separation conditions: chromatographic column: 40 g Flash Column Welch Ultimate XB_C18 20-40 µm; mobile phase: A-water (0.1% formic acid): B-acetonitrile, gradient elution, flow rate: 30 mL/min, instrument: Biotage), yielding title product 12d (40 mg, yield: 80%) as a white solid.

MS m/z (ESI): 937.2 [1/2M+1].

### Step 5

12d (20 mg, 10.68 µmol) was dissolved in 1 mL of dichloromethane, 1h (6.75 mg, 10.68 µmol), 2-(7-azobenzotriazole)-N,N,N',N'-tetramethyluronium hexafluorophosphate (6.09 mg, 16.02 µmol) and N,N-diisopropylethylamine (6.89 mg, 53.31 µmol) were added, and the mixture was reacted at 25°C for 2 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-70%), yielding title product 12e (13 mg, yield: 44%) as a white solid.

### Step 6

12e (10 mg, 4.02 µmol) was dissolved in 1 mL of dichloromethane. Under nitrogen atmosphere, 4-dimethylaminopyridine (0.1 mg, 0.81 µmol), succinic anhydride (2.01 mg, 20.1 µmol) and N,N-diisopropylethylamine (5.2 mg, 40.23 µmol) were added, and the mixture was stirred at 25°C for 36 hours. The reaction solution was concentrated by reduced-pressure distillation, and the resulting residue was purified by preparative high performance liquid chromatography (separation conditions: chromatographic column: InfinityLab Poroshell 120 SB-C18 21.2 × 250 mm, 4 µm; mobile phase: A-water (0.05% ammonium bicarbonate): B-acetonitrile, gradient elution: B%: 20%-60%), yielding title product 12 (5.2 mg, yield: 41%) as a white solid.

MS m/z (ESI): 1142.8 [1/2(M-301)].

¹H NMR (400 MHz, CD₃OD) δ 7.42 - 7.38 (m, 2H), 7.34 - 7.31 (m, 1H), 7.30 - 7.26 (m, 5H), 7.25 - 7.20 (m, 1H), 6.91 - 6.85 (m, 4H), 5.59 - 5.48 (m, 2H), 5.37 - 5.34 (m, 3H), 5.10 - 5.06 (m, 3H), 4.65 - 4.60 (m, 2H), 4.59 - 4.55 (m, 3H), 4.36 - 4.28 (m, 2H), 4.20 - 4.07 (m, 10H), 4.06 - 3.99 (m, 4H), 3.96 - 3.86 (m, 5H), 3.82 - 3.79 (m, 6H), 3.72 - 3.66 (m, 12H), 3.64 - 3.49 (m, 8H), 2.59 - 2.53 (m, 4H), 2.46 - 2.41 (m, 6H), 2.40 - 2.33 (m, 3H), 2.22 - 2.16 (m, 6H), 2.16 - 2.15 (m, 9H), 2.15 - 2.14 (m, 2H), 2.05 - 2.02 (m, 9H), 1.99 - 1.92 (m, 18H), 1.75 - 1.56 (m, 22H), 1.35 - 1.29 (m, 10H), 0.76 - 0.66 (m, 12H).

### Example 13: Preparation of compound 13

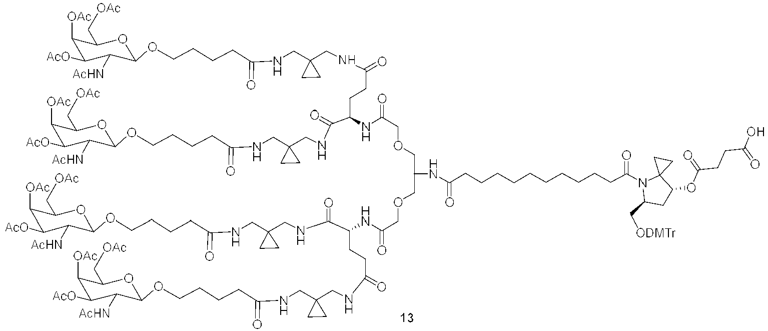

Compound 13 was prepared by a method similar to that described above.

### Example 14: Preparation of compound 14

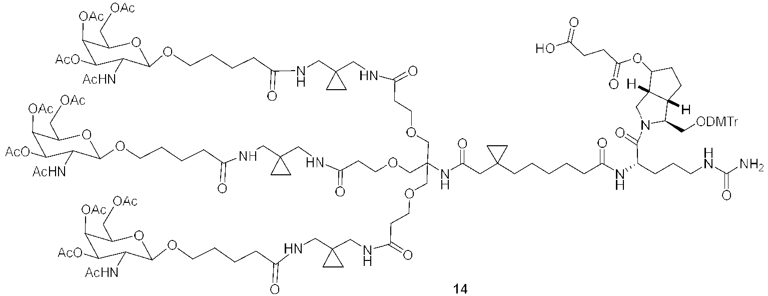

Compound 14 was prepared by a method similar to that described above.

### Example 15: Preparation of compound 15

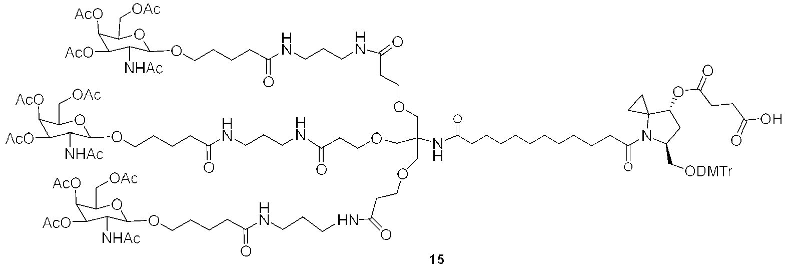

Compound 15 was prepared by a method similar to that described above.

MS m/z (ESI): 2232.9 [M-301].

### Example 16: Preparation of compound 16

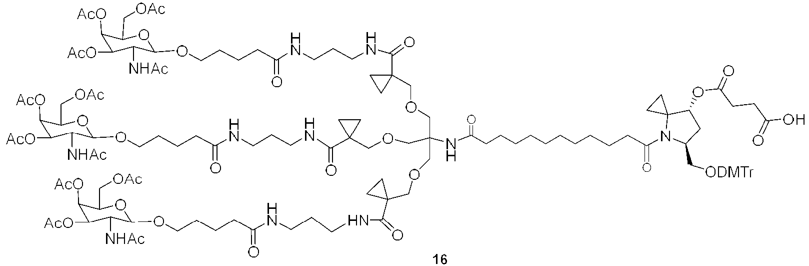

Compound 16 was prepared by a method similar to that described above.

MS m/z (ESI): 2310.0 [M-301].

### Example 17: Preparation of compound 17

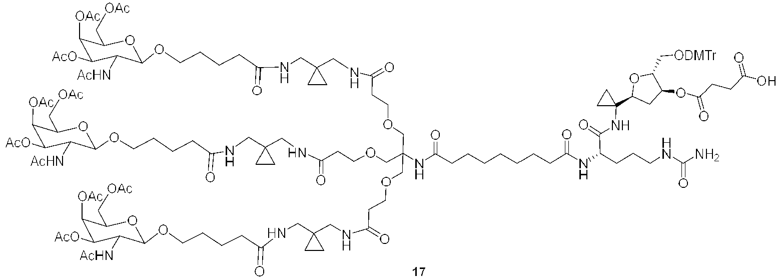

Compound 17 was prepared by a method similar to that described above.

MS m/z (ESI): 2455.9 [M-301].

### Example 18: Preparation of compound 18

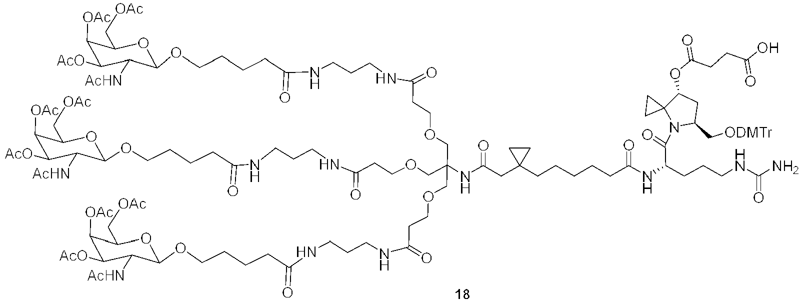

Compound 18 was prepared by a method similar to that described above.

### Example 19: Preparation of compound 19

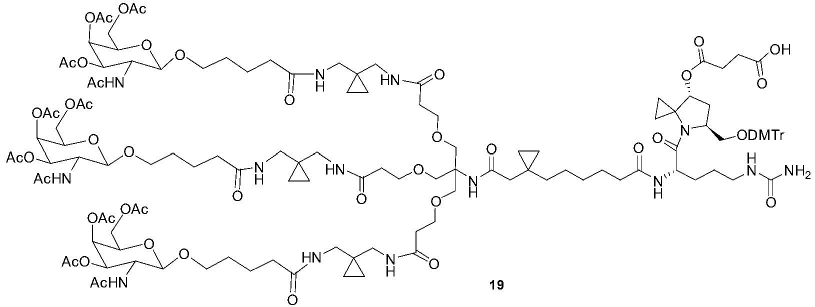

Compound 19 was prepared by a method similar to that described above.

### Synthesis of nucleic acid delivery ligand conjugates

### 1. Preparation of solid-phase carrier loaded with customised GalNAc

N,N-Diisopropylethylamine (3 eq) was weighed and dissolved in anhydrous N,N-dimethylformamide to prepare an N,N-diisopropylethylamine solution with a concentration of 8.1 mg/mL. 2-(7-Azobenzotriazole)-N,N,N',N'-tetramethyluronium hexafluorophosphate (2 eq) was weighed and dissolved in anhydrous acetonitrile to prepare a 2-(7-azobenzotriazole)-N,N,N',N'-tetramethyluronium hexafluorophosphate solution with a concentration of 14.6 mg/mL. The GalNAc compounds (corresponding to compounds 1-19 prepared in the above-mentioned Examples 1-19) were dissolved in the above-mentioned solution of N,N-diisopropylethylamine in N,N-dimethylformamide (3 eq), followed by the addition of a solution of 2-(7-azobenzotriazole)-N,N,N',N'-tetramethyluronium hexafluorophosphate in acetonitrile (2 eq). CPG (with a blank loading capacity of 180 µmol/g and a target loading capacity of 40 µmol/g) was taken and uniformly added dropwise to the above-mentioned mixed solution containing the GalNAc compounds. The mixture was gently shaken to homogenise, and the reaction solution was placed on a shaker (temperature: 25°C, rotation speed: 200 rpm) and shaken overnight. Dichloromethane was added to the reaction solution to rinse the CPG adhering to the vial wall down to the bottom. The CPG was then washed sequentially 3 times with dichloromethane and twice with anhydrous acetonitrile. After each wash, the mixture was allowed to stand until the CPG settled to the bottom, followed by careful aspiration of the supernatant. The solid obtained from the final wash was subjected to vacuum drying for 1 hour. 1 mg of 4-dimethylaminopyridine was weighed and 1000 µL of anhydrous acetonitrile was added to prepare a 1 mg/mL solution of 4-dimethylaminopyridine in acetonitrile; 20 µL of N-methylimidazole was weighed and 80 µL of anhydrous acetonitrile was added to prepare a CapA solution; 40 µL of acetic anhydride was weighed and 60 µL of anhydrous acetonitrile was added to prepare a CapB1 solution; 60 µL of anhydrous pyridine was weighed and 40 µL of anhydrous acetonitrile was added to prepare a CapB2 solution; 65.4 µL of the above-mentioned solution of 4-dimethylaminopyridine in acetonitrile, 12.95 µL of CapA, 56.95 µL of CapB1 and 6.33 µL of CapB2 were added sequentially to the CPG carriers conjugated with different GalNAc compounds. The reaction solution was then placed on a shaker (temperature: 25°C, rotation speed: 200 rpm) and shaken for 2 hours. Anhydrous acetonitrile was added to the reaction solution to rinse the CPG adhering to the vial wall down to the bottom. The CPG was then washed with anhydrous acetonitrile 3 times, followed by solid collection and vacuum drying overnight, yielding the CPG bearing GalNAc compounds (the CPG bearing GalNAc compounds prepared from the compounds of Examples 1-19 were labelled as CPG-1, CPG-2, CPG-3, CPG-4, CPG-5, CPG-6, CPG-7, CPG-8, CPG-9, CPG-10, CPG-11, CPG-12, CPG-13, CPG-14, CPG-15, CPG-16, CPG-17, CPG-18 and CPG-19, respectively). The loading capacity was subsequently determined.

The structure of CPG-1 is exemplarily listed as follows:

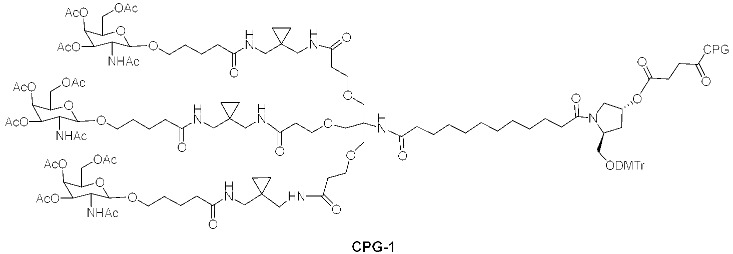

### 2. Synthesis of GalNAc-conjugated 3'-siRNA

The solid-phase carriers loaded with customised GalNAc (i.e., the compounds CPG-1 to CPG-19 prepared in Step 1 above) were weighed and packed into empty columns fitted with bottom frits, followed by covering with top frits. Nucleoside monomers were coupled sequentially in the 3'-to-5' direction in accordance with the predetermined nucleotide sequence. Each round of nucleoside monomer coupling consisted of four sequential reactions: deprotection, coupling, capping, and oxidation or sulphurisation. These operations followed standard procedures in the art.

The auxiliary synthetic reagents and 2-cyanoethyl phosphoramidite monomers (2'-deoxy-2'-fluoro, 2'-O-methyl, RNA, DNA) were obtained from Wuhu Huaren Science and Technology Co., Ltd. or Beijing Dina Xingke Biological Technology Co., Ltd. Additional phosphoramidite monomers were obtained via commercial procurement, in-house preparation or custom synthesis. In the synthesis, 50 mM solutions of the phosphoramidite monomers in acetonitrile or in a 1 : 1 mixture of acetonitrile and dichloromethane were provided. The deprotection reagent was a 3% trichloroacetic acid/dichloromethane solution, with a reaction time of 30 seconds, and the deprotection process repeated 4 times. The activator employed for the coupling reaction was a 0.25 M solution of 5-benzylthiotetrazole (BTT) in acetonitrile, with a reaction time of 160 seconds, and the coupling process repeated 3 times. Phosphorothioate linkages were generated using a 50 mM solution of N,N-dimethyl-N'-(3-thio-3H-1,2,4-dithiazol-5-yl)formamidine in a 1 : 1 mixture of acetonitrile : pyridine, with an oxidation time of 120 seconds, and the oxidation process repeated twice. The capping reagents were a 10% acetic anhydride/tetrahydrofuran solution and an N-methylimidazole/pyridine/tetrahydrofuran solution, with a capping time of 20 seconds, and the capping process repeated twice. The DMT group was removed at the final step for all sequences ("DMT Off').

After completion of the solid-phase synthesis, the solid-supported oligonucleotides were treated with 500 µL of ammonia/ethanol (3 : 1) in cryovials at 45°C for 16 hours to cleave them from the solid-phase carrier, followed by removal of the additional labile protective groups. Taking compound CPG-1 as an example, it was conjugated to the sequence via the above-mentioned solid-phase synthesis process, and then subjected to aminolysis to remove partial functional groups, yielding NGN-1. Other compounds were similarly labelled as NGN-2, ... NGN-19, etc. 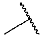 indicates the site of attachment to siRNA via a phosphate group or a phosphorothioate group.

The structures of NGN-2 and NGN-3 are exemplarily listed as follows:

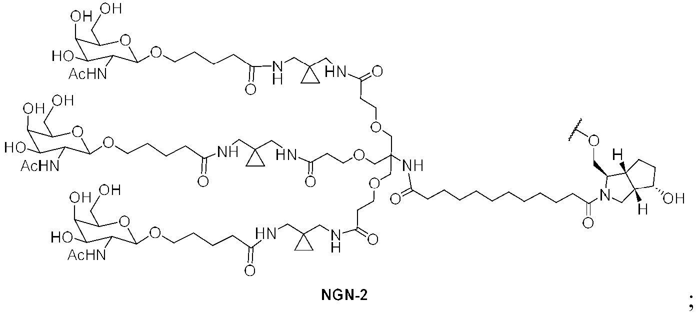

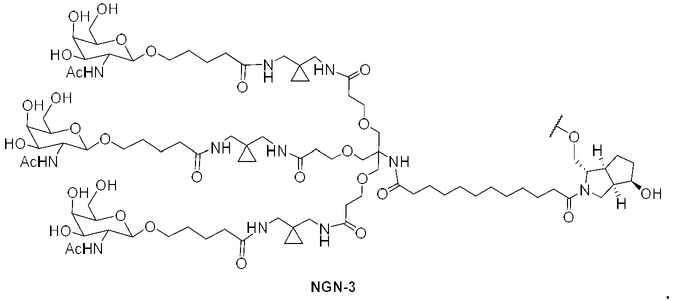

The oligonucleotides were precipitated using a 10% v/v solution of 5 M sodium chloride and 3 volumes of anhydrous ethanol, followed by centrifugation at 4°C and removal of the supernatant. The oligonucleotides were redissolved in dd-H₂O, and subjected to preparative purification using Agilent 1260 HPLC. After freeze-drying of the preparative solution, salt exchange and desalting treatments were carried out. Subsequently, quantitative analysis was conducted via LC-MS and UV spectroscopy. The concentration of the oligonucleotide solution was determined using a micro-spectrophotometer at OD₂₆₀.

The sense strands and antisense strands were mixed at an equimolar ratio according to Table 1, incubated in a water bath at 95°C for 5 min, and then slowly returned to room temperature over a period of 2-3 hours. The concentration and information of each double strand were verified, after which the double strands were subjected to in vitro screening assays.

**Table 1: Nucleic acid sequence information of the present invention**

| No. | Sequence (5'->3') | |
|---|---|---|
| dsRNA-n | Sense strand | AfsGmsCfAmAfCmAfUmGfUfGfUmUfCmAfAmA fGmUfCmAf-NGN-n (SEQ ID NO: 1) |
| | Antisense strand | UmsGfAmsCfUmUfUmGfAmAfCmAmCmAfUmGf UmUfGmCfUmsCmsAm (SEQ ID NO: 2) |
| Reference 1 | Sense strand | AmsAmsCmAmGmUmGfUmUfCfUfUmGmCmUm CmUmAmUmAmAm-L96 (SEQ ID NO: 3) |
| | Antisense strand | UmsUfsAmUmAmGfAmGfCfAmAmGmAmAfCm AfCmUmGmUmUmsUmsUm (SEQ ID NO: 4) |
| 2-dsRNA-n | Sense strand | AmsAmsCmAmGmUmGfUmUfCfUfUmGmCmUm CmUmAmUmAmAm- NGN-n (SEQ ID NO: 5) |
| | Antisense strand | UmsUfsAmUmAmGfAmGfCfAmAmGmAmAfCm AfCmUmGmUmUmsUmsUm (SEQ ID NO: 4) |
| Reference 2 (Inclisiran) | Sense strand | CmsUmsAmGmAmCmCfUmGfUmdTUmUmGmC mUmUmUmUmGmUm-L96 (SEQ ID NO: 6) |
| | Antisense strand | AmsCfsAmAfAfAfGmCfAmAfAmAfCmAfGmGfU mCfUmAmGmsAmsAm (SEQ ID NO: 7) |
| 3-dsRNA-n | Sense strand | CmsUmsAmGmAmCmCfUmGfUmdTUmUmGmC mUmUmUmUmGmUm-NGN-n (SEQ ID NO: 8) |
| | Antisense strand | AmsCfsAmAfAfAfGmCfAmAfAmAfCmAfGmGfU mCfUmAmGmsAmsAm (SEQ ID NO: 7) |

In the above table, G, A, C and U represent the nucleotides containing guanine, adenine, cytosine and uracil, respectively; m indicates that the nucleotide immediately adjacent to its left side is a 2'-O-methyl-modified nucleotide; f indicates that the nucleotide immediately adjacent to its left side is a 2'-fluoro-modified nucleotide; s indicates that the two nucleotides adjacent to its left and right sides are linked via a phosphorothioate diester group.

For the above-mentioned numbering system, when the delivery ligand fragment NGN-n contained in the sense strand is NGN-1, the corresponding siRNA is designated as dsRNA-1. Likewise, the other sequences are assigned correspondingly as dsRNA-2, ..., dsRNA-19. L96 refers to a GalNAc delivery vehicle with the following structure well known in the art, wherein 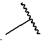 indicates the site of attachment to siRNA via a phosphate group or a phosphorothioate group. For reference, see, for example, PCT Publication Nos. WO 2009073809 and WO 2009082607.

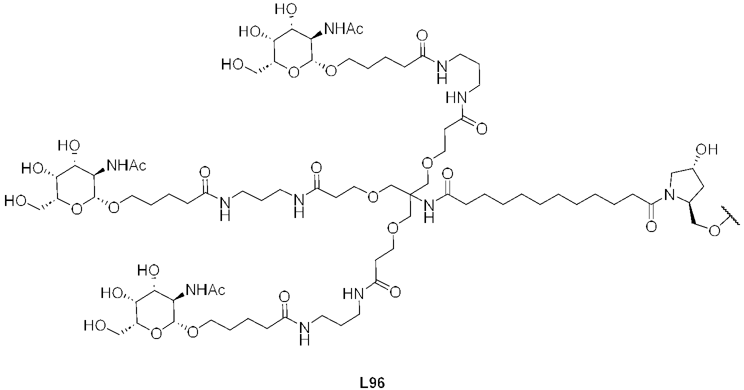

### III. Test examples

### Biological Evaluation

Test example 1: In vivo inhibitory efficiency of siRNA conjugates of the present invention on mRNA expression level of CFB target gene.

### 1. Cell culture and transfection

Primary mouse hepatocytes (PMH) and primary rat hepatocytes (PRH) were freshly isolated from female C57BL/6 mice/SD rats (Chengdu Dashuo Experimental Animal Co., Ltd.), respectively. These cells were then resuspended in DMEM medium (Shanghai BasalMedia Technologies Co., Ltd.) at a density of 2.3 × 10⁵ cells/mL. During transfection, the primary cells were seeded at a density of 23,000 cells per well into 96-well plates pre-coated with poly-L-lysine (SIGMA-ALDRICH, Cat. No. P4832).

### 2. Spontaneous uptake in primary hepatocytes

Freshly isolated primary mouse and rat hepatocytes were seeded at a density of 23,000 cells per well in 96-well plates pre-coated with poly-L-lysine (SIGMA-ALDRICH, Cat. No. P4832), respectively. Subsequently, siRNAs were added at final concentrations of 250 nM, 50 nM, 10 nM, 2 nM, 0.4 nM, 0.08 nM, and 0.016 nM. After co-incubation for 48 hours, the knockdown efficiency via spontaneous siRNA uptake was determined.

### 3. Total RNA extraction and reverse transcription

Total RNA extraction and isolation were performed using the Magnetic Bead-Based Tissue/Cell/Blood Total RNA Extraction Kit (Tiangen Biotech Co., Ltd., Cat. No. DP761) on a Thermo KingFisher^{™} Flex instrument. Subsequently, cDNA synthesis was carried out using the cDNA Reverse Transcription Kit (Nanjing Vazyme Biotech Co., Ltd., Cat. No. 4368813). Specifically, cells were harvested and lysed in 115 µL of lysis buffer at room temperature for 10 minutes. The lysed cells were then transferred to a deep-well plate pre-loaded with a mixture of 10 µL of magnetic beads and 100 µL of isopropanol. The KingFisher instrument used a magnetic rod to capture and continuously agitate the beads. Subsequently, the instrument transferred the captured magnetic beads into a well containing 300 µL of Protein Removal Buffer RD and mixed for 5 minutes, after which the beads were moved to the next deep-well plate for DNaseI digestion. Following a 10-min digestion, the beads were washed once with 300 µL of Protein Removal Buffer RD and twice with 280 µL of Wash Buffer RW. Finally, the magnetic rod captured the beads again, and the RNA was eluted by heating at 60°C with 50 µL of RNase-Free H₂O. Preparation of the cDNA synthesis system: 2 µL of 5x HiScript III RT SuperMix (containing buffer, dNTPs, reverse transcriptase, RNase inhibitor, and random primers) and 8 µL of the RNA extracted as described above for in vitro screening were added to each reaction tube. cDNA was then generated using an ETC 811 PCR instrument (Beijing Dongsheng Innovation Technology Co., Ltd.) with the following program: 50°C for 15 min, 85°C for 5 s, and hold at 4°C.

### 4. Real-time fluorescent quantitative PCR

In each well of a 96-well plate (Applied Biosystems, Cat. No. 4483354), 4 µL of cDNA was added to a premix solution containing: 5 µL of AceQ Universal Probe Mix (Nanjing Vazyme Biotech Co., Ltd., Nanjing, Cat. No. Q513), 0.1 µL of GAPDH TaqMan Probe (Genewiz Inc. (SuZhou), Cat. No. 83080101316R), 0.1 µL of CFB TaqMan Probe (Genewiz Inc. (SuZhou)), and 0.2 µL each of the GAPDH-F/R and CFB-F/R primers. Real-time fluorescent quantitative PCR was performed on a Biosystems Quant Studio 5 system (Applied Biosystems), with each sample run in technical duplicate. The real-time data were analysed using the ΔΔCt method to calculate the remaining expression level of the target gene. Using siRNA concentration as the x-axis and the remaining expression level of the target gene as the y-axis, GraphPad Prism software was employed for fitting using the "log(inhibitor) vs. response--Variable slope (four parameters)" model to calculate the IC₅₀ values.

The results from the above assays are summarised in Table 2 and Table 3.

**Table 2: IC₅₀ values of siRNA conjugates of the present invention against mRNA expression level of the target gene in primary mouse hepatocytes.**

| Compound No. | IC₅₀ (nM) |
|---|---|
| dsRNA-1 | 0.094 |
| dsRNA-2 | 0.454 |
| dsRNA-3 | 0.568 |
| dsRNA-9 | 0.880 |
| dsRNA-12 | 0.146 |

Conclusions: the siRNA conjugates of the present invention exhibit significant inhibitory activity against the mRNA expression level of the target gene in primary mouse hepatocytes in vitro.

**Table 3: IC₅₀ values of siRNA conjugates of the present invention against mRNA expression level of the target gene in primary rat hepatocytes.**

| Compound No. | IC₅₀ (nM) |
|---|---|
| dsRNA-1 | 2.636 |
| dsRNA-2 | 1.568 |
| dsRNA-3 | 2.16 |
| dsRNA-12 | 4.04 |

Conclusions: the siRNA conjugates of the present invention exhibit significant inhibitory activity against the mRNA expression level of the target gene in primary rat hepatocytes in vitro.

### Test example 2: Pharmacodynamic evaluation of siRNA conjugates of the present invention in C57BL/6 mouse model

Female C57BL/6 mice (18-22 g, 6-8 weeks old) were randomly divided into groups with 4 animals per group. The dose for each animal was calculated based on its body weight and administered as a single subcutaneous injection. The siRNA compounds were administered as a 0.1 mg/mL stock solution, with a 0.9% sodium chloride solution for injection as the solvent. Specifically, prior to the experiment, the siRNA conjugates were dissolved and diluted to the required concentration and volume with a 0.9% sodium chloride aqueous solution for injection. Both the physiological saline (control group) and the siRNA conjugates were administered at a volume of 5 mL/kg.

Blood samples were collected from the orbital venous plexus of the mice prior to administration (designated as Day 0) and on Days 7, 14, 21, and 28 post-administration, respectively. At each time point, the serum TTR protein expression levels in the mice were determined using an ELISA kit (Abcam, ab282297). Upon completion of the experiment, liver tissues were harvested, and total RNA was extracted using Trizol (Cat. No. 15596026CN, Thermo Fisher). Following reverse transcription, the TTR mRNA levels in the mouse liver were determined via real-time fluorescent quantitative PCR (qPCR), with primer information provided in Table 4. The experimental results are shown in Table 6.

**Table 4: Primer information**

| Primer name | Sequence information | Fluorophore |
|---|---|---|
| mTTR-F | CTGCTGTAGACGTGGCTGTAA (SEQ ID NO: 9) | - |
| mTTR-R | CTTCCAGTACGATTTGGTGTCC (SEQ ID NO: 10) | - |
| mTTR-P | AAGACCTCTGAGGGATCCTGGGAGCC (SEQ ID NO: 11) | 5'6-FAM, 3'BHQ1 |
| mGAPDH-F | CTGCACCACCAACTGCTTAG (SEQ ID NO: 12) | - |
| mGAPDH-R | GGATGCAGGGATGATGTTCT (SEQ ID NO: 13) | - |
| mGAPDH-p | TGGCCAAGGTCATCCATGACAACTTTGGC (SEQ ID NO: 14) | 5'VIC, 3'BHQ1 |

### Data statistics and analysis

The 2^{-ΔΔα} values were calculated and converted into percentages to obtain the residual inhibition rate. 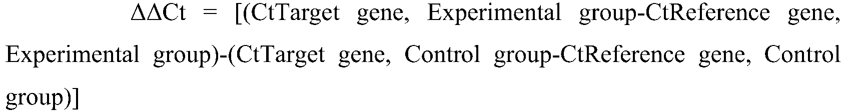 ΔΔCt = [(CtTarget gene, Experimental group-CtReference gene, Experimental group)-(CtTarget gene, Control group-CtReference gene, Control group)]

In the formula, the target gene is mTTR and the reference gene is mGAPDH.

**Table 5: Compound information**

| | Compound No. | Administration dose |
|---|---|---|
| 1 | Physiological saline | / |
| 2 | Reference 1 | 0.5 mg/kg |
| 3 | 2-dsRNA-2 | 0.5 mg/kg |
| 4 | 2-dsRNA-3 | 0.5 mg/kg |

**Table 6: Pharmacodynamic experimental results of compounds in the C57BL/6 mouse model**

| | | Day 0 | | Day 7 | | Day 14 | | Day 21 | | Day 28 | |
|---|---|---|---|---|---|---|---|---|---|---|---|
| | Test compound | Targe t gene mRN A residu al | SD | Target gene mRN A residu al | SD | Target gene mRN A residu al | SD | Targe t gene mRN A residu al | SD | Target gene mRN A residu al | SD |
| 1 | Physiologi cal saline | 100% | 11.48 % | 104.45 % | 8.48 % | 105.34 % | 16.33 % | 95.38 % | 5.60 % | 116.47 % | 17.11 % |
| 2 | Reference 1 | 100% | 9.39 % | 12.48 % | 4.25 % | 9.42% | 4.96 % | 23.35 % | 4.89 % | 42.05 % | 5.80 % |
| 3 | 2-dsRNA-2 | 100% | 12.07 % | 9.13% | 3.95 % | 8.14% | 4.22 % | 20.07 % | 4.49 % | 33.29 % | 9.33 % |
| 4 | 2-dsRNA-3 | 100% | 5.16 % | 0.96% | 7.97 % | - 2.75% | 7.64 % | 18.51 % | 5.29 % | 31.75 % | 9.51 % |

The results from the above assays indicate that the tested siRNA conjugates can effectively knock down the TTR mRNA level in mouse liver, and at the same dose, compounds 2-dsRNA-2 and 2-dsRNA-3 have a better knockdown effect than Reference 1.

### Test example 3: Rat pharmacokinetic experiments of siRNA conjugates of the present invention

### 1. Test objective:

The objective of this experiment was to investigate the pharmacokinetic characteristics of the siRNA conjugates targeting the PCSK9 gene, so as to provide a reference for subsequent studies.

### 2. Test drugs and materials:

(1) Test drugs: siRNA conjugates targeting the PCSK9 gene: 5 mg/kg.
(2) Experimental animals: SD rats (6-8 weeks old, Sichuan Vital River Laboratory Animal Technology Co., Ltd.) were housed in a sterile animal room at 25°C under a 12-hour light/dark cycle, with free access to food and water.

### 3. Experimental method:

SD rats were randomly divided into two groups, with 6 rats per group (half male and half female), and subcutaneous administration was performed on each group, respectively.

Sampling time: blood samples were collected once at 5 min prior to administration, as well as at 5 min, 30 min, 1 h and 4 h post-administration. Sampling method: blood was collected from the jugular vein of the rats.

Blood sample processing: after blood collection, the samples were placed in EDTA anticoagulant tubes and gently inverted several times to ensure thorough mixing. The tubes were then incubated in a constant-temperature oven at 37°C for 30 min, followed by centrifugation at 4500 rpm and 4°C for 12 min. The plasma was aspirated and stored in a refrigerator at -20°C; after 24 hours, it was transferred to an ultra-low-temperature freezer (≤ -60°C). Sample analysis: the plasma concentrations of the siRNA conjugates were analysed using the LC-MS/MS method. The experimental results are shown in Table 7.

**Table 7: Rat pharmacokinetic experimental results**

| Test compound | Tₘₐₓ (h) | Cₘₐₓ (ng/ml) | AUCₗₐₛₜ (h*ng/ml) | MRTₗₐₛₜ (h) |
|---|---|---|---|---|
| Reference 2 | 1 | 652 | 1832.69 | 1.83 |
| 3-dsRNA-2 | 1 | 930 | 2606.31 | 1.74 |

The results from the above experiments indicate that both the maximum plasma concentration (Cₘₐₓ) and the AUC of compound 3-dsRNA-2 were higher than those of Reference 2.

### Test example 4: Rat toxicity experiment of siRNA conjugates of the present invention

### 1. Test objective:

The objective of this experiment was to investigate the toxicity characteristics of the siRNA conjugates targeting the PCSK9 gene, so as to provide a reference for subsequent studies.

### 2. Test drugs and materials:

(1) Test drugs: siRNA conjugate 3-dsRNA-2 targeting the PCSK9 gene: 30 mg/kg, 100 mg/kg, 300 mg/kg.
(2) Experimental animals: SD rats (6-8 weeks old, weighing 200-300 g, Sichuan Vital River Laboratory Animal Technology Co., Ltd.) were housed in a sterile animal room at 25°C under a 12-hour light/dark cycle, with free access to food and water.

### 3. Experimental method:

Each group consisted of 4 rats, half male and half female, and they were divided into 3 groups in total. Different doses of compound 3-dsRNA-2 were administered via subcutaneous injection once a week. For the phosphate-buffered saline (PBS) group, PBS was administered via subcutaneous injection once a week.

Subsequently, the rats were observed every other day for survival status, body weight changes, and endpoint blood biochemical indicators.

4. Experimental results: the results of body weight change are shown in Figures 1 and 2, and the endpoint blood biochemical results are shown in Table 8. The results show that all rats survived for more than 30 days at all doses, with normal body weight changes and endpoint blood biochemical indicators.

5. Experimental conclusion: based on the results of this test example, the maximum tolerated dose of the siRNA conjugate 3-dsRNA-2 of the present invention in rats exceeds 300 mg/kg, demonstrating high safety profile.

**Table 8: Rat blood biochemical indicator data**

| **Group** | **Dose** | **Ite m** | **Hepatic function** | | | | | | **Renal function** | | **Blood lipid** | | **Electrolyte and myocardial enzyme** | | |
|---|---|---|---|---|---|---|---|---|---|---|---|---|---|---|---|
| | | | **Glutamic-pyruvic transaminase** | **Glutamic-oxalacetic transaminase** | **Total bilirubin** | **Direct bilirubin** | **Total protein** | **γ-Glutamyl transferase** | **Urea** | **Creati nine** | **Triglyc eride** | **Total cholestol ol** | **Inorganic phosphorus** | **Creatine kinase** | **Alkaline phosphatase** |
| | | | **ALT** | **AST** | **T-bil-D II** | **D-Bil** | **TP** | **γ-GT** | **URE A** | **CRE A-S** | **TG** | **TC** | **P** | **CK** | **ALP** |
| | | | **(U/L)** | **(U/L)** | **(umol/L)** | **(umol/L)** | **(g/L)** | **(U/L)** | **(mm ol/L)** | **(umol /L)** | **(mmol/ L)** | **(mmol/L)** | **(mmol/L)** | **(U/L)** | **(U/L)** |
| **PBS** ♀ | / | 1 | 46.7 | 88.3 | 2.9 | 1.32 | 77.8 | 1.1 | 4.88 | 29 | 1.2 | 1.72 | 2.19 | 138.6 | 30.1 |
| | | 2 | 38.9 | 98.9 | 2.58 | 2.06 | 91 | 0 | 6.07 | 38.4 | 1.6 | 2.69 | 2.73 | 286.2 | 40.6 |
| 3-dsRNA-2 ♀ | 30 mg/kg | 1 | 42 | 121.6 | 2.84 | 1.66 | 76 | -0.6 | 5.25 | 31.6 | 1.42 | 1.48 | 1.9 | 86.5 | 53.2 |
| | | 2 | 56 | 131.4 | 1.82 | 1.62 | 79.3 | 1.2 | 5.26 | 29.8 | 2.06 | 1.5 | 1.65 | 75.1 | 77.7 |
| | 100 mg/kg | 1 | 43.2 | 130.2 | 1.55 | 1.43 | 65.9 | 3 | 5.15 | 37.9 | 0.36 | 0.7 | 2.23 | 548.2 | 71.2 |
| | | 2 | 38.3 | 155.4 | 2.58 | 0.78 | 74.3 | 1.8 | 5.67 | 30.1 | 1.32 | 1.39 | 2.06 | 122 | 43.9 |
| | 300 mg/kg | 1 | 48.5 | 136 | 1.42 | 1.01 | 76 | 1.6 | 5.28 | 26.1 | 1.05 | 2.29 | 1.96 | 95.2 | 70.2 |
| | | 2 | 44 | 132.5 | 2.23 | 1.89 | 75.1 | 1.4 | 4.28 | 29.5 | 0.45 | 1 | 1.98 | 124.6 | 59.9 |
| PBS ♂ | / | 1 | 41.9 | 105.2 | 0.05 | 1.46 | 66.7 | -1.2 | 4.92 | 28.1 | 2.14 | 1.63 | 2.47 | 971 | 68.7 |
| | | 2 | 61.8 | 130.1 | 0.51 | 1.5 | 66 | 1.1 | 5.82 | 29.4 | 2.18 | 1.85 | 2.89 | 1644.7 | 103.2 |
| 3-dsRNA-2 ♂ | 30 mg/kg | 1 | 51.4 | 123.6 | 0.68 | 1.22 | 65 | 2 | 4.28 | 28.4 | 2.29 | 0.79 | 2.59 | 327.3 | 153.8 |
| | | 2 | 37.2 | 94 | 0.25 | 1.22 | 67.9 | 1.8 | 5.42 | 28.5 | 3.15 | 0.97 | 2.32 | 274 | 94 |
| | 100 mg/kg | 1 | 41.9 | 123.6 | 0.56 | 1.95 | 63.7 | 1.7 | 5.33 | 27.5 | 1.93 | 0.69 | 2.34 | 438.9 | 71 |
| | | 2 | 40.8 | 98.5 | 1.99 | 0.8 | 68.3 | 0.3 | 4.47 | 26.7 | 1.71 | 0.76 | 2.36 | 179.6 | 91 |
| | 300 mg/kg | 1 | 42.1 | 113.1 | 1.3 | 1.44 | 66.7 | 0.5 | 4.44 | 27.5 | 1.39 | 0.91 | 2.68 | 182.3 | 143.7 |
| | | 2 | 64.8 | 142.9 | 0.53 | 2.15 | 62.3 | 1.8 | 4.53 | 29.2 | 1.41 | 0.77 | 2.44 | 668.7 | 111.9 |

Test example 5: Determination of plasma stability of siRNA conjugates of the present invention

### 1. Test objective:

To test the stability of siRNA targeting gene TTR following incubation for a specified period in plasma from four different species, namely human plasma (collected from volunteers), monkey plasma (Beijing Iphase Biosciences Co, Ltd.; 032B11.21), mouse plasma (Beijing Iphase Biosciences Co, Ltd.; 032E21.21), and rat plasma (Beijing Iphase Biosciences Co, Ltd.; 033D11.21). The siRNA samples were diluted to the same concentration using plasma from different species, incubated in an incubator at 37°C for a specified period, then removed, and determined for concentration using mass spectrometry (SCIEX, Triple Quad 5500 plus).

### 2. Experimental method:

(1) Sample dilution: The siRNA samples were diluted to 30 µg/mL with plasma from different species, with a volume of 2 mL.
(2) Filtration sterilisation: The 2 mL siRNA sample solutions were filtered through 0.22 µm Syringe Driven Filters (Biofil, 220509-051-A), ensuring a post-filtration volume of > 1.8 mL.
(3) Sample aliquoting: The filtered siRNA sample solutions were aliquoted into 1.5 mL EP tubes (Axygen, MCT-150-C-S) at 100 µl per tube, followed by labelling and sealing with parafilm.
(4) Sample incubation: The aliquoted samples were incubated in an incubator at 37°C for 0, 2, 4, 8, and 24 hours. Samples at the 0-hour time point were taken directly for mass spectrometry analysis.
(5) Sample submission and analysis: After incubation, the samples were submitted for mass spectrometry analysis. The concentration of the siRNA samples was measured using a mass spectrometer. The results are summarised in Table 9.

**Table 9: Plasma stability experimental results**

| Plasma type | Compound No. | Incubation time | Relative content |
|---|---|---|---|
| Human plasma | 2-dsRNA-2 | 0 h | 100.0% |
| | | 2 h | 100.0% |
| | | 4 h | 95.3% |
| | | 8 h | 94.9% |
| | | 24 h | 97.1% |
| | 2-dsRNA-3 | 0 h | 100.0% |
| | | 2 h | 104.8% |
| | | 4 h | 106.2% |
| | | 8 h | 97.6% |
| | | 24 h | 102.5% |
| Cynomolgus monkey plasma | 2-dsRNA-2 | 0 h | 100.0% |
| | | 2 h | 99.7% |
| | | 4 h | 97.5% |
| | | 8 h | 94.8% |
| | | 24 h | 94.0% |
| | 2-dsRNA-3 | 0 h | 100.0% |
| | | 2 h | 104.2% |
| | | 4 h | 103.6% |
| | | 8 h | 97.7% |
| | | 24 h | 96.6% |
| Rat plasma | 2-dsRNA-2 | 0 h | 100.0% |
| | | 2 h | 98.6% |
| | | 4 h | 100.8% |
| | | 8 h | 99.1% |
| | | 24 h | 92.2% |
| | 2-dsRNA-3 | 0 h | 100.0% |
| | | 2 h | 103.2% |
| | | 4 h | 104.6% |
| | | 8 h | 106.0% |
| | | 24 h | 101.1% |
| Mouse plasma | 2-dsRNA-2 | 0 h | 100.0% |
| | | 2 h | 100.7% |
| | | 4 h | 98.4% |
| | | 8 h | 101.2% |
| | | 24 h | 100.8% |
| | 2-dsRNA-3 | 0 h | 100.0% |
| | | 2 h | 105.0% |
| | | 4h | 110.6% |
| | | 8 h | 103.0% |
| | | 24 h | 93.7% |

The results from the above experiments indicate that compounds 2-dsRNA-2 and 2-dsRNA-3 have good stability in the four types of plasma within 24 hours.

Test example 6: In vitro inhibitory efficiency of siRNA conjugates of the present invention on mRNA expression level of PCSK9 target gene

### 1. Cell culture and transfection

Primary cynomolgus monkey hepatocytes (Milecell Biotechnology Inc., Shanghai) were retrieved from a liquid nitrogen tank and resuspended at a density of 3.3 × 10⁵ cells/mL in a 1 : 1 mixture of hepatocyte plating medium and hepatocyte maintenance medium (Milecell Biotechnology Inc., Shanghai). During transfection, the primary monkey cells were seeded at a density of 33,000 cells per well into 96-well plates pre-coated with poly-L-lysine (SIGMA-ALDRICH, Cat. No. P4832).

### 2. Spontaneous uptake in primary hepatocytes

After 6 h of culture, siRNAs were added at final concentrations of 100 nM, 25 nM, 6.25 nM, 1.563 nM, 0.391 nM, 0.098 nM, 0.024 nM, 0.006 nM, and 0.0015 nM. After co-incubation for 72 hours, the knockdown efficiency via spontaneous siRNA uptake was determined.

### 3. Total RNA extraction and reverse transcription

Total RNA extraction and isolation were performed using the Magnetic Bead-Based Tissue/Cell/Blood Total RNA Extraction Kit (Tiangen Biotech Co., Ltd., Cat. No. DP761) on a Thermo KingFisher^{™} Flex instrument. Subsequently, cDNA synthesis was carried out using the cDNA Reverse Transcription Kit (Nanjing Vazyme Biotech Co., Ltd., Cat. No. 4368813). Specifically, cells were harvested and lysed in 115 µL of lysis buffer at room temperature for 10 minutes. The lysed cells were then transferred to a deep-well plate pre-loaded with a mixture of 10 µL of magnetic beads and 100 µL of isopropanol. The KingFisher instrument used a magnetic rod to capture and continuously agitate the beads. Subsequently, the instrument transferred the captured magnetic beads into a well containing 300 µL of Protein Removal Buffer RD and mixed for 5 minutes, after which the beads were moved to the next deep-well plate for DNaseI digestion. Following a 10-min digestion, the beads were washed once with 300 µL of Protein Removal Buffer RD and twice with 280 µL of Wash Buffer RW. Finally, the magnetic rod captured the beads again, and the RNA was eluted by heating at 60°C with 50 µL of RNase-Free H₂O. Preparation of the cDNA synthesis system: 2 µL of 5x HiScript III RT SuperMix (containing buffer, dNTPs, reverse transcriptase, RNase inhibitor, and random primers) and 8 µL of the RNA extracted as described above for in vitro screening were added to each reaction tube. cDNA was then generated using an ETC 811 PCR instrument (Beijing Dongsheng Innovation Technology Co., Ltd.) with the following program: 50°C for 15 min, 85°C for 5 s, and hold at 4°C.

### 4. Real-time fluorescent quantitative PCR

In each well of a 96-well plate (Applied Biosystems, Cat. No. 4483354), 4 µL of cDNA was added to a premix solution containing: 5 µL of AceQ Universal Probe Mix (Nanjing Vazyme Biotech Co., Ltd., Nanjing, Cat. No. Q513), 0.1 µL of GAPDH TaqMan Probe (Genewiz Inc. (SuZhou), Cat. No. 83080101316R), 0.1 µL of cynoPCSK9 TaqMan Probe (Genewiz Inc. (SuZhou)), and 0.2 µL each of the GAPDH-F/R and cynoPCSK9-F/R primers. Real-time fluorescent quantitative PCR was performed on a Biosystems Quant Studio 5 system (Applied Biosystems), with each sample run in technical triplicate. The real-time data were analysed using the ΔΔCt method to calculate the remaining expression level of the target gene. Using siRNA concentration as the x-axis and the remaining expression level of the target gene as the y-axis, GraphPad Prism software was employed for fitting using the "log(inhibitor) vs. response--Variable slope (four parameters)" model to calculate the IC₅₀ values.

The results from the above assays are summarised in Table 10.

**Table 10: IC₅₀ values of siRNA conjugates of the present invention against mRNA expression level of the target gene in primary cynomolgus monkey hepatocytes.**

| Compound No. | IC₅₀ (nM) |
|---|---|
| Reference 2 | 0.463 |
| 3-dsRNA-2 | 0.121 |

Conclusions: compound 3-dsRNA-2 exhibits significant inhibitory activity against the mRNA expression level of the target gene in primary cynomolgus monkey hepatocytes in vitro, and is superior to Reference 2.

The embodiments of the technical solutions of the present invention are exemplarily explained above. It should be understood that the scope of protection of the present invention is not limited to the above-described embodiments. Any modifications, equivalent substitutions, or improvements made by those skilled in the art within the spirit and principles of the present invention shall be included within the scope of protection of the claims of the present application.

## Claims

1. A delivery ligand, **characterised in that** the delivery ligand comprises a conjugated group as shown in formula (X), or an isotopic variant, tautomer, or stereoisomer thereof, or a mixture thereof: 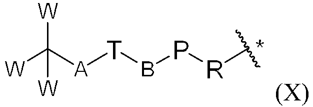 wherein
the R is attached to a biomolecule via 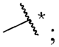
each W is independently selected from H, D, 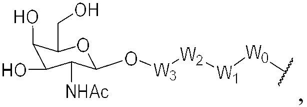 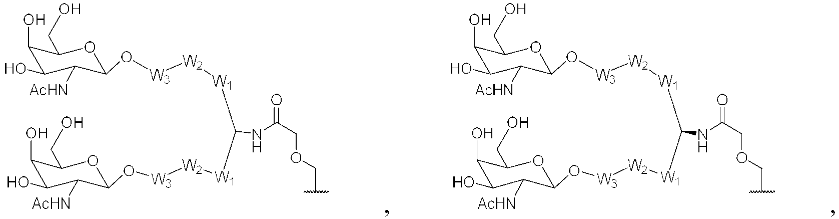 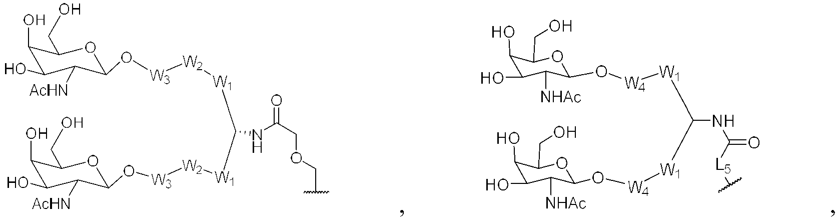 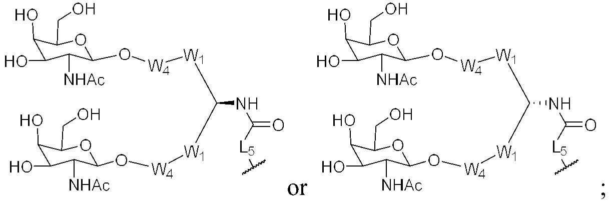
W₀ is selected from a chemical bond, -OCH₂-, -OCH₂CH₂-, -OCH₂OCH₂-, - OCH₂CH₂CH₂-, -OCH₂CH₂CH₂CH₂-, -CH₂OCH₂CH₂CH₂-, -CH₂CH₂OCH₂CH₂-, - CH₂CH₂CH₂OCH₂-, -CH₂CH₂OCH₂CH₂O-, C₁₋₆ alkylene, C₁₋₆ haloalkylene, C₁₋₆ alkenylene or C₁₋₆ alkynylene, which is optionally deuterated, up to fully deuterated;
W₁ is selected from a chemical bond, -O-, -O-L₁-, -C(O)-, -C(O)-Li-, -C(O)O- or -C(O)O-L₁-, which is optionally deuterated, up to fully deuterated;
L₁ is -(CRₐR_{b})ₘ-, and 1, 2, 3, 4 or 5 non-adjacent CRₐR_{b} in the L₁ may be replaced by a heteroatom selected from -O-, -S- and -NRₐ-;
Rₐ and R_{b} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₐ and R_{b} on any one or more carbon atoms in L₁, together with the same or different carbon atoms to which they are attached, form C₃₋₁₀ cycloalkyl, 3- to 10-membered heterocyclyl, C₆₋₁₀ aryl or 5-to 10-membered heteroaryl;
W₂ is selected from -L₂-NH-, -L₂-O-, -L₂-C(O)- or -L₂-OC(O)-, which is optionally deuterated, up to fully deuterated;
L₂ is -(CR_{c}R_{d})ₙ-, and 1, 2, 3, 4 or 5 non-adjacent CR_{c}R_{d} in the L₂ may be replaced by a heteroatom selected from -O-, -S- and -NR_{c}-;
R_{c} and R_{d} are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{c} and R_{d} on any one or more carbon atoms in L₂, together with the same or different carbon atoms to which they are attached, form C₃₋₁₀ cycloalkyl, 3- to 10-membered heterocyclyl, C₆₋₁₀ aryl or 5-to 10-membered heteroaryl;
W₃ is selected from -L₃-C(O)NH-, -L₃-NHC(O)-, -L₃-OC(O)NH- or -L₃-NHC(O)O-, which is optionally deuterated, up to fully deuterated;
L₃ is -(CRₑR_{f})ₕ-, and 1, 2, 3, 4 or 5 non-adjacent CRₑR_{f} in the L₃ may be replaced by a heteroatom selected from -O-, -S- and -NRₑ-;
Rₑ and R_{f} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₑ and R_{f} on any one or more carbon atoms in L₃, together with the same or different carbon atoms to which they are attached, form C₃₋₁₀ cycloalkyl, 3- to 10-membered heterocyclyl, C₆₋₁₀ aryl or 5-to 10-membered heteroaryl;
W₄ is selected from -L₄-NHC(O)-, -L₄-NHC(O)-(CH₂)₁₋₆-, -L₄-NHC(O)O-, - L₄-NHC(O)O-(CH₂)₁₋₆-, -L₄-C(O)NH-, -L₄-C(O)NH-(CH₂)₁₋₆-, -L₄-OC(O)NH- or - L₄-OC(O)NH-(CH₂)₁₋₆-, which is optionally deuterated, up to fully deuterated;
L₄ is selected from -(CR_{g}Rₕ)ₖ-, -(CR_{g}Rₕ)₁₋₆-(OCR_{g}Rₕ-CR_{g}Rₕ)ₖ- or -(CR_{g}Rₕ)₁₋₆-(NH-CR_{g}Rₕ-CR_{g}Rₕ)ₖ-, and 1, 2, 3, 4 or 5 non-adjacent CR_{g}Rₕ in the L₄ may be replaced by a heteroatom selected from -O-, -S- and -NR_{g}-;
R_{g} and Rₕ are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{g} and Rₕ on any one or more carbon atoms in L₄, together with the same or different carbon atoms to which they are attached, form C₃₋₁₀ cycloalkyl, 3- to 10-membered heterocyclyl, C₆₋₁₀ aryl or 5-to 10-membered heteroaryl;
m, n, h and k are independently selected from 0, 1, 2, 3, 4, 5, 6, 7, 8, 9 or 10; L₅ is selected from a chemical bond, C₁₋₆ alkylene, C₁₋₆ alkenylene or -C₁₋₆ alkylene-O-C₁₋₆ alkylene-, which is optionally deuterated, up to fully deuterated;
A is selected from a chemical bond, -C(O)- or -NHC(O)-;
T is -(CRᵢRⱼ)_{q}-, and 1, 2, 3, 4 or 5 non-adjacent CRᵢRⱼ in the T may be replaced by a heteroatom selected from -O-, -S- and -NRᵢ-; T is optionally deuterated, up to fully deuterated;
Rᵢ and Rⱼ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rᵢ and Rⱼ on any one or more carbon atoms in T, together with the same or different carbon atoms to which they are attached, form C₃₋₁₀ cycloalkyl, 3- to 10-membered heterocyclyl, C₆₋₁₀ aryl or 5-to 10-membered heteroaryl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-, and 1, 2 or 3 non-adjacent CRₖRₘ in the B may be replaced by a heteroatom selected from -O-, -S- and -NRₖ-; the B is optionally deuterated, up to fully deuterated;
Rₖ and Rₘ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₖ and Rₘ on any one or more carbon atoms in B, together with the same or different carbon atoms to which they are attached, form C₃₋₁₀ cycloalkyl, 3- to 10-membered heterocyclyl, C₆₋₁₀ aryl or 5-to 10-membered heteroaryl;
P is selected from a chemical bond or 1 to 3 amino acid residues;
R is selected from 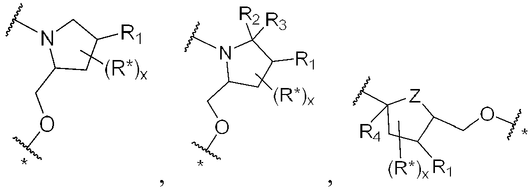 or 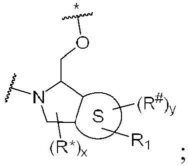
ring S is selected from C₃₋₁₀ cycloalkyl, 3- to 10-membered heterocyclyl, C₆₋₁₀ aryl or 5- to 10-membered heteroaryl;
R₁ is selected from a hydroxyl-containing group, such as -OR₀, -C₁₋₆ alkylene-OR₀, -C₁₋₆ alkenylene-OR₀ or -C₁₋₆ alkynylene-OR₀, wherein R₀ is H, D, a hydroxyl protecting group or a solid-phase carrier;
the hydroxyl protecting group is selected from trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), - C(O)CH₂CH₂C(O)OH or 4,4'-dimethoxytrityl (DMTr), preferably - C(O)CH₂CH₂C(O)OH or 4,4'-dimethoxytrityl (DMTr);
R₂ and R₃ are independently selected from H, D, halogen, C₁₋₆ alkyl, C₁₋₆ alkoxy, C₁₋₆ haloalkyl, C₁₋₆ alkenyl or C₁₋₆ alkynyl; alternatively, R₂ and R₃, together with the same carbon atom to which they are attached, form C₃₋₁₀ cycloalkyl, 3- to 10-membered heterocyclyl, C₆₋₁₀ aryl or 5- to 10-membered heteroaryl; R₂, R₃ and the ring formed thereby are optionally deuterated, up to fully deuterated;
R₄ is selected from H, D, halogen, C₁₋₆ alkyl, C₁₋₆ alkoxy, C₁₋₆ haloalkyl, C₁₋₆ alkenyl or C₁₋₆ alkynyl, and R₄ is optionally deuterated, up to fully deuterated;
Z is selected from O, NH or CH₂;
R* and R^{#} are independently selected from H, D, halogen, C₁₋₆ alkyl, C₁₋₆ haloalkyl, C₁₋₆ alkoxy, C₃₋₁₀ cycloalkyl, 3- to 10-membered heterocyclyl, C₆₋₁₀ aryl or 5- to 10-membered heteroaryl;
x and y are independently selected from 0, 1, 2, 3, 4 or 5;
the above-mentioned W, A, T, B, P and R are each optionally further substituted with 1, 2, 3, 4, 5, 6, 7, 8 or more substituents selected from the following: H, D, OH, CN, NH₂, NO₂, oxo, thio, halogen, C₁₋₆ alkyl, C₁₋₆ haloalkyl, C₁₋₆ alkoxy, C₃₋₁₀ cycloalkyl, 3- to 10-membered heterocyclyl, C₆₋₁₀ aryl or 5- to 10-membered heteroaryl;
provided that, when R is 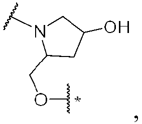 at least one pair among Rₐ and R_{b}, R_{c} and R_{d}, Rₑ and R_{f}, R_{g} and Rₕ, Rᵢ and Rⱼ, and Rₖ and Rₘ, together with the same or different carbon atoms to which they are attached, forms C₃₋₁₀ cycloalkyl, 3- to 10-membered heterocyclyl, C₆₋₁₀ aryl or 5- to 10-membered heteroaryl.

2. The delivery ligand according to claim 1, **characterised in that** at least one pair among Rₐ and R_{b}, R_{c} and R_{d}, Rₑ and R_{f}, R_{g} and Rₕ, Rᵢ and Rⱼ, and Rₖ and Rₘ, together with the same or different carbon atoms to which they are attached, forms C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

3. The delivery ligand according to claim 1 or 2, **characterised in that** Rₐ and R_{b} on any one or more carbon atoms in L₁, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

4. The delivery ligand according to any one of claims 1-3, **characterised in that** R_{c} and R_{d} on any one or more carbon atoms in L₂, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

5. The delivery ligand according to any one of claims 1-4, **characterised in that** Rₑ and R_{f} on any one or more carbon atoms in L₃, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

6. The delivery ligand according to any one of claims 1-5, **characterised in that** R_{g} and Rₕ on any one or more carbon atoms in L₄, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

7. The delivery ligand according to any one of claims 1-6, **characterised in that** Rᵢ and Rⱼ on any one or more carbon atoms in T, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

8. The delivery ligand according to any one of claims 1-7, **characterised in that** Rₖ and Rₘ on any one or more carbon atoms in B, together with the same carbon atom to which they are attached, form C₃₋₁₀ cycloalkyl, 3- to 10-membered heterocyclyl, C₆₋₁₀ aryl or 5- to 10-membered heteroaryl, preferably C₃₋₇ cycloalkyl or 3- to 7-membered heterocyclyl.

9. The delivery ligand according to any one of claims 1-8, **characterised in that** R is selected from 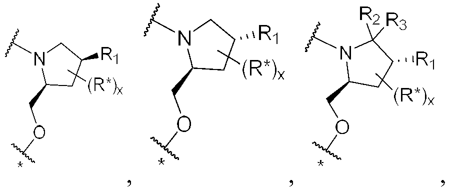 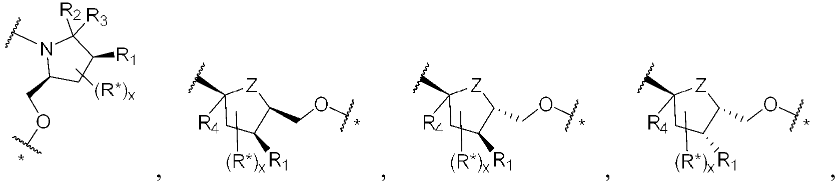 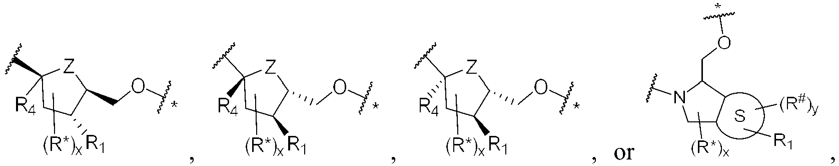 wherein ring S, R₁, R₂, R₃, R₄, Z, R*, R#, x and y are as defined in any one of claims 1-8;
preferably, R is selected from 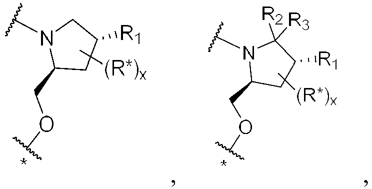 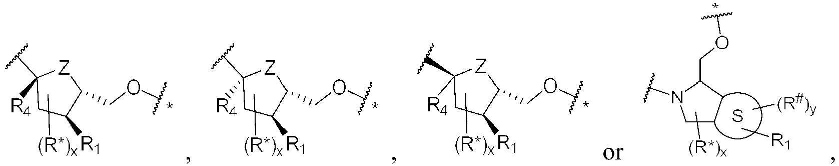 wherein ring S, R₁, R₂, R₃, R₄, Z, R*, R#, x and y are as defined in any one of claims 1-8;
more preferably, R is selected from 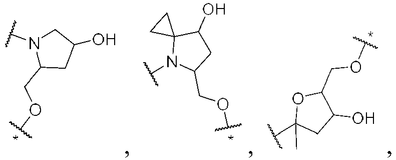 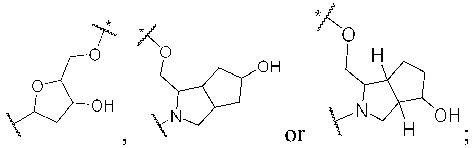
more preferably, R is selected from 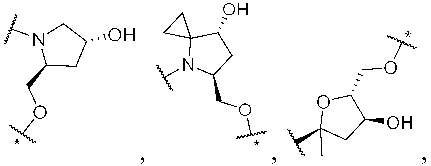 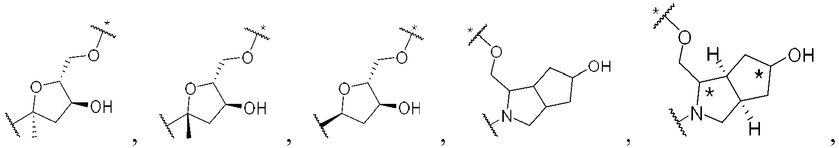 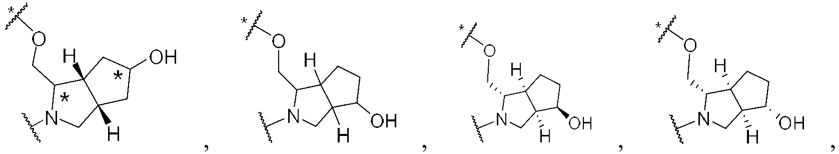 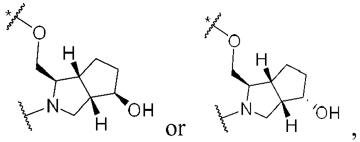 and * denotes a chiral centre, which is independently selected from (S)- or (R)-configuration, or a mixture thereof.

10. The delivery ligand according to any one of claims 1-9, **characterised in that**
W₀ is selected from -OCH₂-, -OCH₂CH₂-, -OCH₂CH₂CH₂- or - OCH₂CH₂CH₂CH₂-;
W₁ is selected from a chemical bond, -O-, -O-Li-, -C(O)-Li- or -C(O)-;
W₂ is selected from -L₂-NH- or -L₂-O-;
W₃ is selected from -L₃-C(O)NH- or -L₃-NHC(O)-;
W₄ is selected from -L₄-NHC(O)-, -L₄-NHC(O)-(CH₂)₁₋₆-, -L₄-C(O)NH- or - L₄-C(O)NH-(CH₂)₁₋₆-;
preferably,
W₀ is -OCH₂-;
W₁ is selected from a chemical bond, -C(O)-Li- or -C(O)-;
W₂ is -L₂-NH-;
W₃ is -L₃-C(O)NH-;
W₄ is selected from -L₄-NHC(O)- or -L₄-NHC(O)-(CH₂)₁₋₆-.

11. The delivery ligand according to any one of claims 1-10, **characterised in that**
the R is attached to a biomolecule via 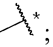
each W is independently selected from H, D, 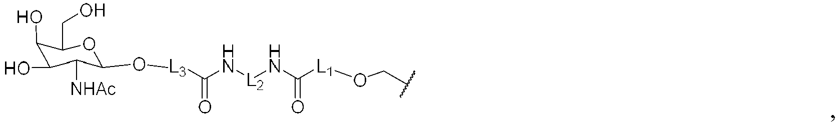 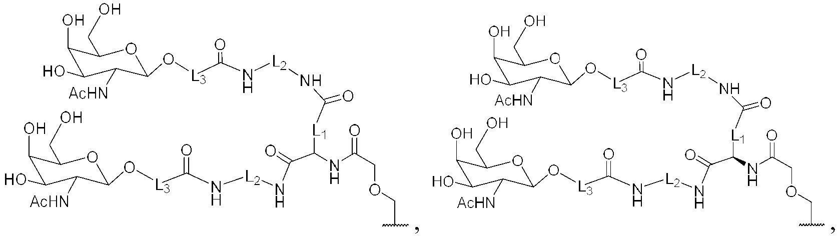 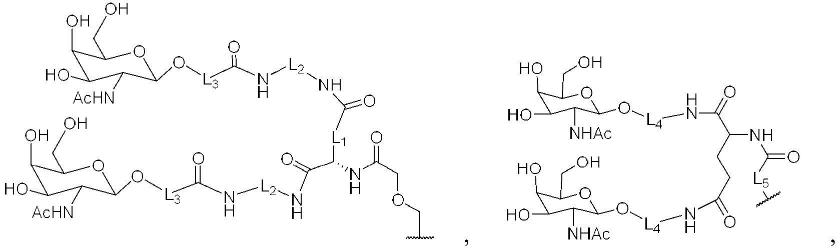 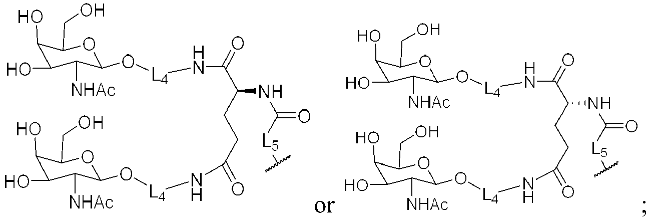
L₁ is -(CRₐR_{b})ₘ-;
Rₐ and R_{b} are each independently H, D, C₁₋₆ alkyl, C₁₋₆ alkoxy or C₁₋₆ haloalkyl; alternatively, Rₐ and R_{b} on any one or more carbon atoms in L₁, together with the carbon atom to which they are attached, form C₃₋₇ cycloalkyl or 3- to 7-membered heterocyclyl;
L₂ is -(CR_{c}R_{d})ₙ-;
R_{c} and R_{d} are each independently H, D, C₁₋₆ alkyl, C₁₋₆ alkoxy or C₁₋₆ haloalkyl; alternatively, R_{c} and R_{d} on any one or more carbon atoms in L₂, together with the carbon atom to which they are attached, form C₃₋₇ cycloalkyl or 3- to 7-membered heterocyclyl;
L₃ is -(CRₑR_{f})ₕ-;
Rₑ and R_{f} are each independently H, D, C₁₋₆ alkyl, C₁₋₆ alkoxy or C₁₋₆ haloalkyl; alternatively, Rₑ and R_{f} on any one or more carbon atoms in L₃, together with the carbon atom to which they are attached, form C₃₋₇ cycloalkyl or 3- to 7-membered heterocyclyl;
L₄ is -(CR_{g}Rₕ)₁₋₆-(OCR_{g}Rₕ-CR_{g}Rₕ)ₖ-;
R_{g} and Rₕ are each independently H, D, C₁₋₆ alkyl, C₁₋₆ alkoxy or C₁₋₆ haloalkyl; alternatively, R_{g} and Rₕ on any one or more carbon atoms in L₄, together with the carbon atom to which they are attached, form C₃₋₇ cycloalkyl or 3- to 7-membered heterocyclyl;
m, n, h and k are independently selected from 1, 2, 3, 4, 5, 6, 7 or 8;
L₅ is selected from a chemical bond, C₁₋₆ alkylene, C₁₋₆ alkenylene or -C₁₋₆ alkylene-O-C₁₋₆ alkylene-;
A is selected from -C(O)- or -NHC(O)-;
T is -(CRᵢRⱼ)_{q}-;
Rᵢ and Rⱼ are each independently H, D, C₁₋₆ alkyl, C₁₋₆ alkoxy or C₁₋₆ haloalkyl; alternatively, Rᵢ and Rⱼ on any one or more carbon atoms in T, together with the carbon atom to which they are attached, form C₃₋₇ cycloalkyl or 3- to 7-membered heterocyclyl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-;
Rₖ and Rₘ are each independently H, D, C₁₋₆ alkyl, C₁₋₆ alkoxy or C₁₋₆ haloalkyl; alternatively, Rₖ and Rₘ on any one or more carbon atoms in B, together with the carbon atom to which they are attached, form C₃₋₇ cycloalkyl or 3- to 7-membered heterocyclyl;
P is selected from a chemical bond or 1 to 3 amino acid residues;
R is selected from 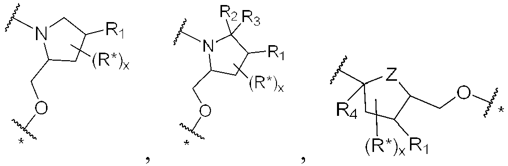 or 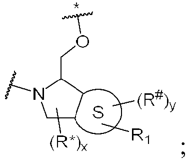
ring S is selected from C₃₋₁₀ cycloalkyl or 3- to 10-membered heterocyclyl;
R₁ is selected from -OR₀, -CH₂OR₀ or -CH₂CH₂OR₀, wherein R₀ is H, D, a hydroxyl protecting group or a solid-phase carrier;
the hydroxyl protecting group is selected from trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), - C(O)CH₂CH₂C(O)OH or 4,4'-dimethoxytrityl (DMTr), preferably - C(O)CH₂CH₂C(O)OH or 4,4'-dimethoxytrityl (DMTr);
R₂ and R₃ are independently selected from H, D, C₁₋₆ alkyl, C₁₋₆ alkoxy or C₁₋₆ haloalkyl; alternatively, R₂ and R₃, together with the carbon atom to which they are attached, form C₃₋₇ cycloalkyl or 3- to 7-membered heterocyclyl;
R₄ is selected from H, D, C₁₋₆ alkyl, C₁₋₆ alkoxy or C₁₋₆ haloalkyl;
Z is selected from O, NH or CH₂;
R* and R^{#} are independently selected from H, D, C₁₋₆ alkyl or C₁₋₆ haloalkyl;
x and y are independently selected from 0, 1, 2, 3, 4 or 5;
provided that, when R is 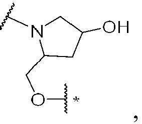
at least one pair among Rₐ and R_{b}, R_{c} and R_{d}, Rₑ and R_{f}, R_{g} and Rₕ, Rᵢ and Rⱼ, and Rₖ and Rₘ, together with the carbon atom to which they are attached, forms C₃₋₇ cycloalkyl or 3- to 7-membered heterocyclyl.

12. The delivery ligand according to any one of claims 1-11, **characterised in that** the conjugated group is selected from a structure as shown in formula (I), formula (I-1), formula (I-2), formula (I'), formula (I'-1), formula (I'-2), formula (II), formula (II-1), formula (II-2), formula (II'), formula (II'-1), formula (II'-2), formula (III), formula (III-1), formula (III-2), formula (III-3), formula (III-4), formula (III-5), formula (III-6), formula (III'), formula (III'-1), formula (III'-2), formula (III'-3), formula (III'-4), formula (III'-5), formula (III'-6), formula (IV), formula (IV-1), formula (IV-2), formula (IV'), formula (IV'-1), formula (IV'-2), formula (V), formula (V-1), formula (V-2), formula (V-3), formula (V-4), formula (V'), formula (V'-1), formula (V'-2), formula (V'-3) or formula (V'-4), or an isotopic variant, tautomer, or stereoisomer thereof, or a mixture thereof: 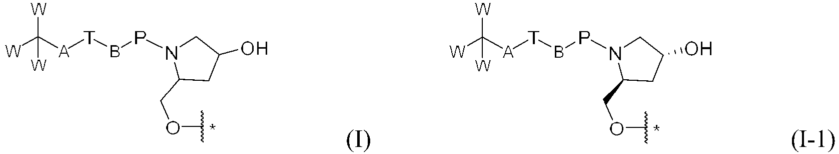 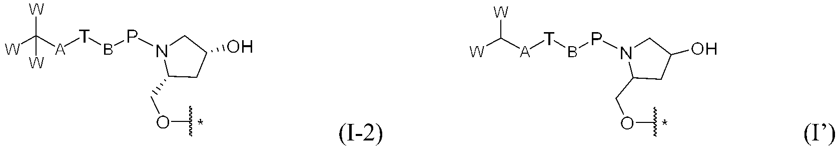 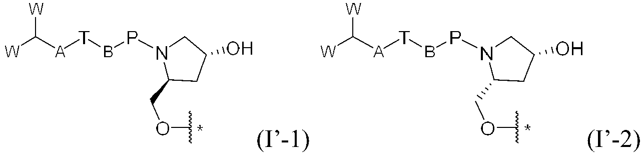 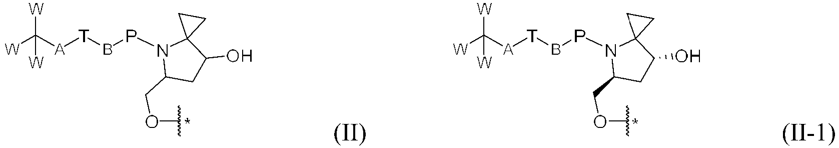 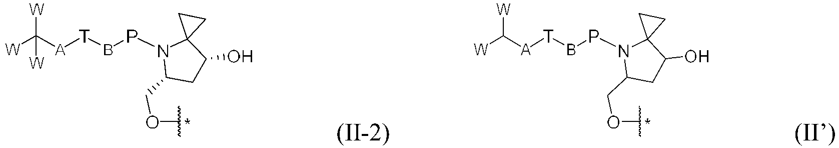 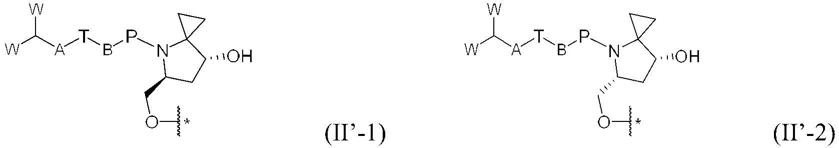 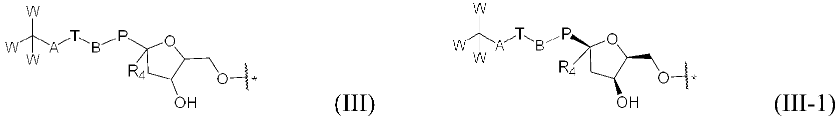 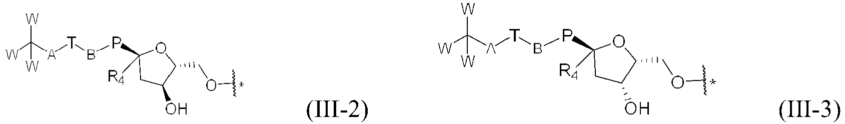 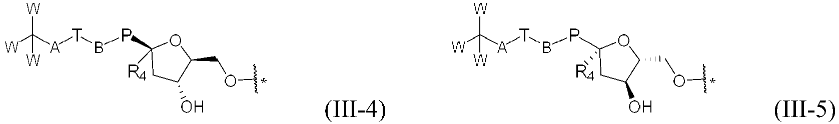 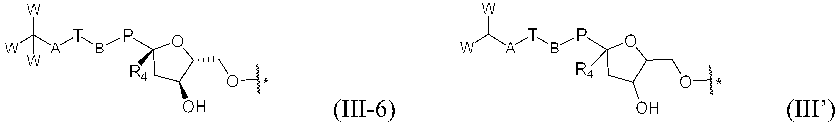 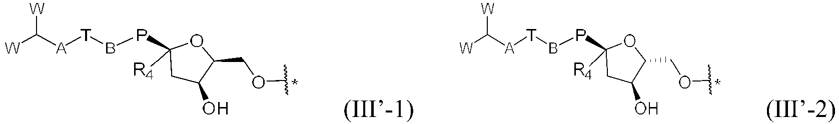 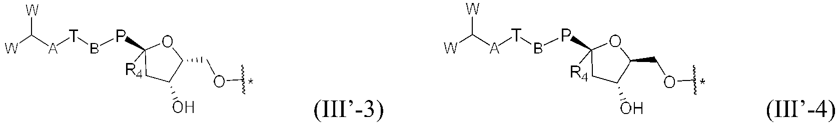 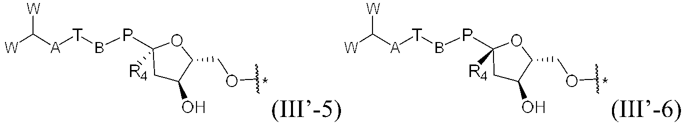 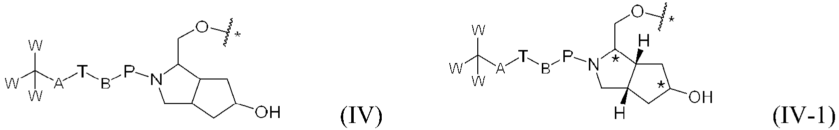 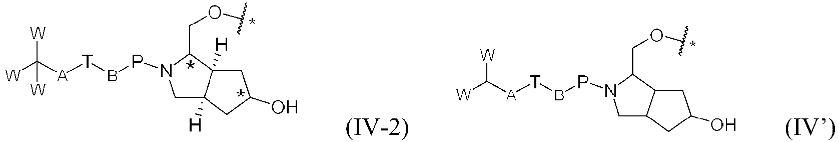 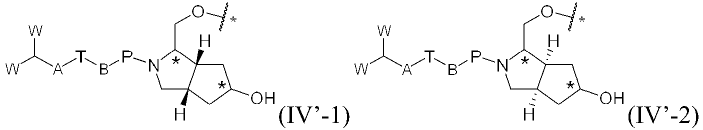 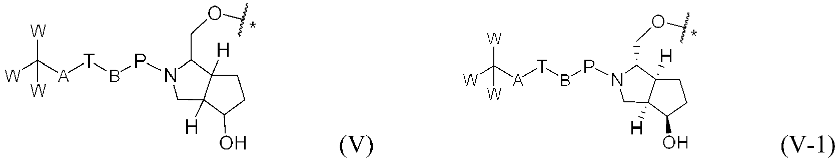 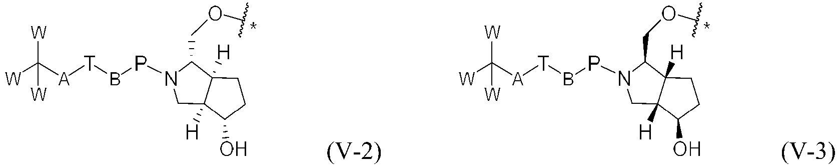 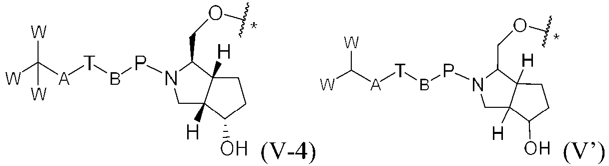 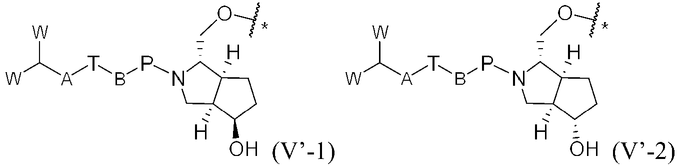 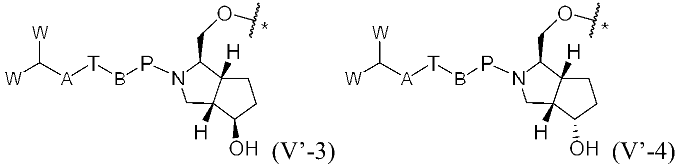
wherein * denotes a chiral centre, which is independently selected from (S)- or (R)-configuration, or a mixture thereof;
each variable is as defined in any one of claims 1-11.

13. The delivery ligand according to any one of claims 1-12, **characterised in that** the conjugated group is selected from a structure as shown in formula (I-1), formula (I'-1), formula (II-1), formula (II'-1), formula (III-2), formula (III-5), formula (III-6), formula (III'-2), formula (III'-5), formula (III'-6), formula (IV), formula (IV-1), formula (IV-2), formula (IV'), formula (IV'-1), formula (IV'-2), formula (V), formula (V-1), formula (V-4), formula (V'), formula (V'-1) or formula (V'-4), or an isotopic variant, tautomer, or stereoisomer thereof, or a mixture thereof: 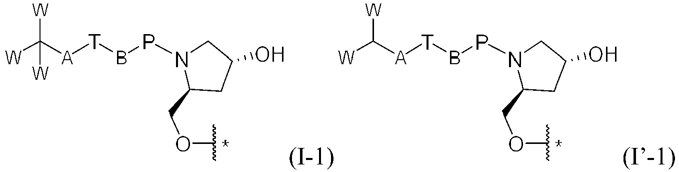 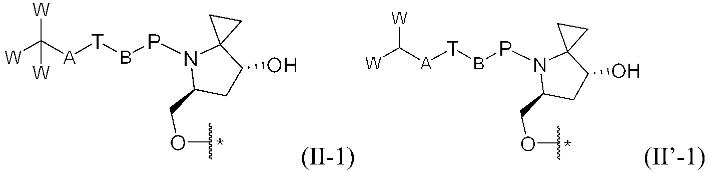 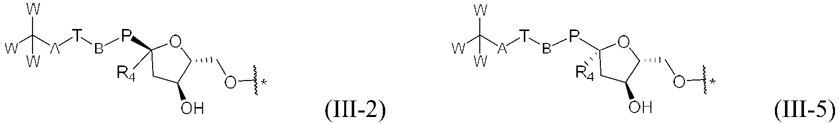 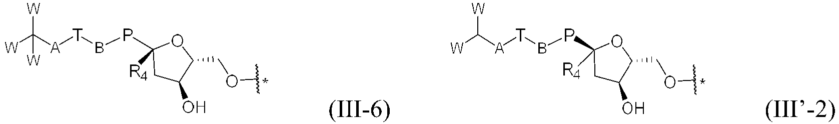 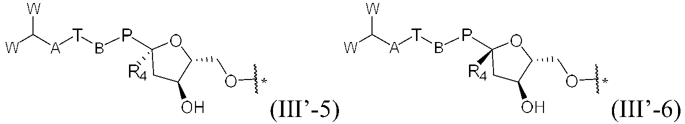 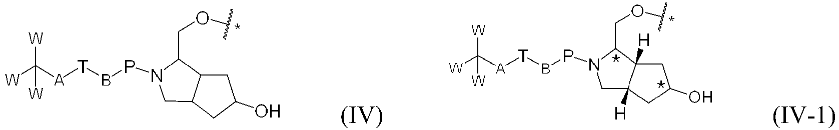 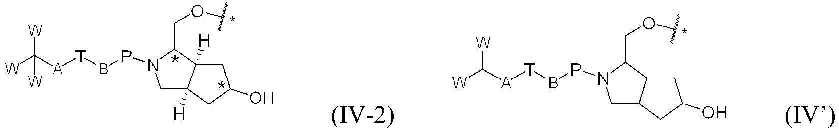 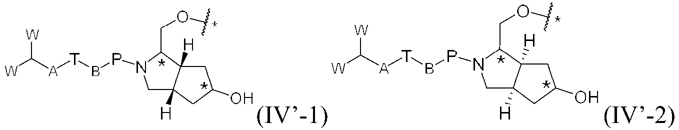 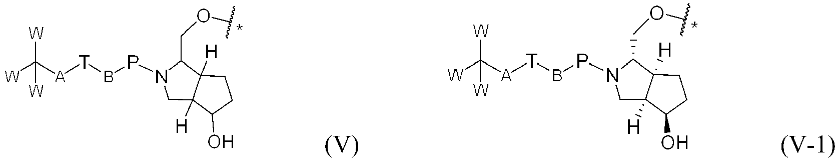 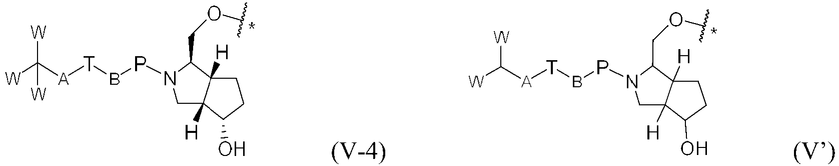 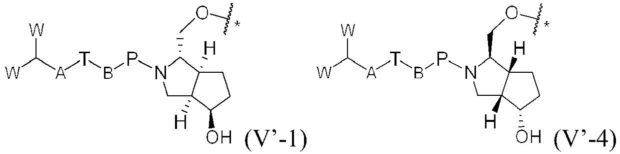 wherein
* denotes a chiral centre, which is independently selected from (S)- or (R)-configuration, or a mixture thereof;
each W is independently selected from H, 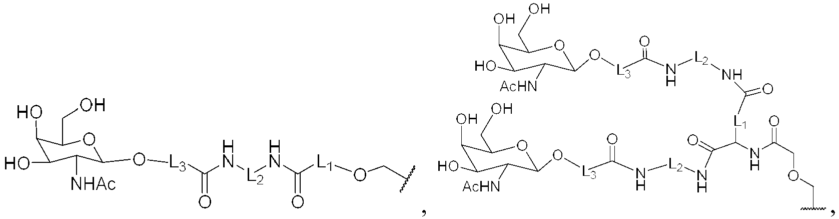 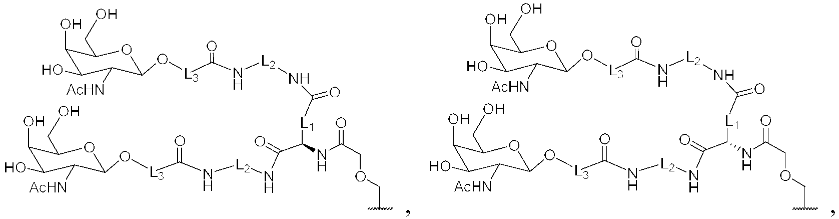 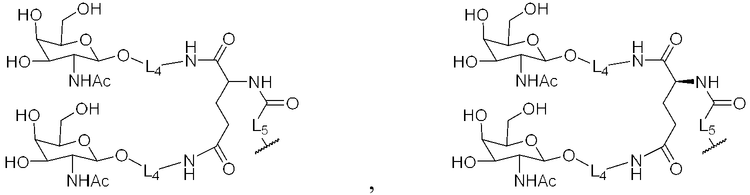 or 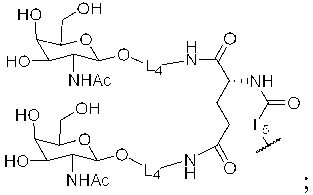
L₁ is -(CRₐR_{b})ₘ-, and 1, 2, 3, 4 or 5 non-adjacent CRₐR_{b} in the L₁ may be replaced by a heteroatom selected from -O-, -S- and -NRₐ-;
Rₐ and R_{b} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₐ and R_{b} on any one or more carbon atoms in L₁, together with the same or different carbon atoms to which they are attached, form C₃₋₁₀ cycloalkyl or 3- to 10-membered heterocyclyl;
L₂ is -(CR_{c}R_{d})ₙ-, and 1, 2, 3, 4 or 5 non-adjacent CR_{c}R_{d} in the L₂ may be replaced by a heteroatom selected from -O-, -S- and
R_{c} and R_{d} are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{c} and R_{d} on any one or more carbon atoms in L₂, together with the same or different carbon atoms to which they are attached, form C₃₋₁₀ cycloalkyl or 3- to 10-membered heterocyclyl;
L₃ is -(CRₑR_{f})ₕ-, and 1, 2, 3, 4 or 5 non-adjacent CRₑR_{f} in the L₃ may be replaced by a heteroatom selected from -O-, -S- and
Rₑ and R_{f} are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₑ and R_{f} on any one or more carbon atoms in L₃, together with the same or different carbon atoms to which they are attached, form C₃₋₁₀ cycloalkyl or 3- to 10-membered heterocyclyl;
L₄ is selected from -(CR_{g}Rₕ)ₖ-, -(CR_{g}Rₕ)₁₋₆-(OCR_{g}Rₕ-CR_{g}Rₕ)ₖ- or -(CR_{g}Rₕ)₁₋₆-(NH-CR_{g}Rₕ-CR_{g}Rₕ)ₖ-, and 1, 2, 3, 4 or 5 non-adjacent CR_{g}Rₕ in the L₄ may be replaced by a heteroatom selected from -O-, -S- and -NR_{g}-;
R_{g} and Rₕ are each independently H, D, halogen, C₁₋₆ alkyl, C₁₋₆ alkoxy, C₁₋₆ haloalkyl, C₁₋₆ alkenyl or C₁₋₆ alkynyl; alternatively, R_{g} and Rₕ on any one or more carbon atoms in L₄, together with the same or different carbon atoms to which they are attached, form C₃₋₁₀ cycloalkyl or 3- to 10-membered heterocyclyl;
m, n, h and k are independently selected from 1, 2, 3, 4, 5, 6, 7, 8, 9 or 10;
L₅ is selected from a chemical bond, C₁₋₆ alkylene, C₁₋₆ alkenylene or -C₁₋₆ alkylene-O-C₁₋₆ alkylene-;
A is selected from -C(O)- or -NHC(O)-;
T is -(CRᵢRⱼ)_{q}-, and 1, 2, 3, 4 or 5 non-adjacent CRᵢRⱼ in the T may be replaced by a heteroatom selected from -O-, -S- and -NRᵢ-;
Rᵢ and Rⱼ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rᵢ and Rⱼ on any one or more carbon atoms in T, together with the same or different carbon atoms to which they are attached, form C₃₋₁₀ cycloalkyl or 3- to 10-membered heterocyclyl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-, and 1, 2, 3, 4 or 5 non-adjacent CRₖRₘ in the B may be replaced by a heteroatom selected from -O-, -S- and -NRᵢ-;
Rₖ and Rₘ are each independently H, D, halogen, C₁₋₆ alkyl, C₁₋₆ alkoxy, C₁₋₆ haloalkyl, C₁₋₆ alkenyl or C₁₋₆ alkynyl; alternatively, Rₖ and Rₘ in CRₖRₘ, together with the carbon atom to which they are attached, form C₃₋₁₀ cycloalkyl or 3- to 10-membered heterocyclyl;
P is selected from a chemical bond or 1 to 3 amino acid residues;
L₁-L₅, T and B are optionally deuterated, up to fully deuterated;
provided that, when the conjugated group has a structure of formula (I), at least one pair among Rₐ and R_{b}, R_{c} and R_{d}, Rₑ and R_{f}, R_{g} and Rₕ, Rᵢ and Rⱼ, and Rₖ and Rₘ, together with the carbon atom to which they are attached, forms C₃₋₁₀ cycloalkyl or 3- to 10-membered heterocyclyl.

14. The delivery ligand according to claim 13, **characterised in that**
L₁ is -(CRₐR_{b})ₘ-;
Rₐ and R_{b} are each independently H, D, C₁₋₆ alkyl, C₁₋₆ alkoxy or C₁₋₆ haloalkyl; alternatively, Rₐ and R_{b} on any one or more carbon atoms in L₁, together with the carbon atom to which they are attached, form C₃₋₇ cycloalkyl or 3- to 7-membered heterocyclyl;
L₂ is -(CR_{c}R_{d})ₙ-;
R_{c} and R_{d} are each independently H, D, C₁₋₆ alkyl, C₁₋₆ alkoxy or C₁₋₆ haloalkyl; alternatively, R_{c} and R_{d} on any one or more carbon atoms in L₂, together with the carbon atom to which they are attached, form C₃₋₇ cycloalkyl or 3- to 7-membered heterocyclyl;
L₃ is -(CRₑR_{f})ₕ-;
Rₑ and R_{f} are each independently H, D, C₁₋₆ alkyl, C₁₋₆ alkoxy or C₁₋₆ haloalkyl; alternatively, Rₑ and R_{f} on any one or more carbon atoms in L₃, together with the carbon atom to which they are attached, form C₃₋₇ cycloalkyl or 3- to 7-membered heterocyclyl;
L₄ is -(CR_{g}Rₕ)₁₋₆-(OCR_{g}Rₕ-CR_{g}Rₕ)ₖ-;
R_{g} and Rₕ are each independently H, D, C₁₋₆ alkyl, C₁₋₆ alkoxy or C₁₋₆ haloalkyl; alternatively, R_{g} and Rₕ on any one or more carbon atoms in L₄, together with the carbon atom to which they are attached, form C₃₋₇ cycloalkyl or 3- to 7-membered heterocyclyl;
m, n, h and k are independently selected from 1, 2, 3, 4 or 5;
L₅ is selected from a chemical bond, C₁₋₆ alkylene or -C₁₋₄ alkylene-O-C₁₋₄ alkylene-;
A is selected from -C(O)- or -NHC(O)-;
T is -(CRᵢRⱼ)_{q}-;
Rᵢ and Rⱼ are each independently H, D, C₁₋₆ alkyl, C₁₋₆ alkoxy or C₁₋₆ haloalkyl; alternatively, Rᵢ and Rⱼ on any one or more carbon atoms in T, together with the carbon atom to which they are attached, form C₃₋₇ cycloalkyl or 3- to 7-membered heterocyclyl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-;
Rₖ and Rₘ are each independently H, D, C₁₋₆ alkyl, C₁₋₆ alkoxy or C₁₋₆ haloalkyl; alternatively, Rₖ and Rₘ on any one or more carbon atoms in B, together with the carbon atom to which they are attached, form C₃₋₇ cycloalkyl or 3- to 7-membered heterocyclyl;
P is selected from a chemical bond or 1 amino acid residue.

15. The delivery ligand according to claim 13 or 14, **characterised in that**
L₁ is -(CRₐR_{b})ₘ-;
Rₐ and R_{b} are each independently H, D, C₁₋₆ alkyl or C₁₋₆ haloalkyl; alternatively, Rₐ and R_{b} on any one or more carbon atoms in L₁, together with the carbon atom to which they are attached, form C₃₋₇ cycloalkyl;
m is selected from 1, 2, 3 or 4;
L₂ is -(CR_{c}R_{d})ₙ-;
R_{c} and R_{d} are each independently H, D, C₁₋₆ alkyl or C₁₋₆ haloalkyl; alternatively, R_{c} and R_{d} on any one or more carbon atoms in L₂, together with the carbon atom to which they are attached, form C₃₋₇ cycloalkyl;
n is selected from 1, 2, 3 or 4;
L₃ is -(CRₑR_{f})ₕ-;
Rₑ and R_{f} are each independently H, D, C₁₋₆ alkyl or C₁₋₆ haloalkyl; alternatively, Rₑ and R_{f} on any one or more carbon atoms in L₃, together with the carbon atom to which they are attached, form C₃₋₇ cycloalkyl;
h is selected from 2, 3, 4 or 5;
L₄ is -(CR_{g}Rₕ)₁₋₆-(OCR_{g}Rₕ-CR_{g}Rₕ)ₖ-;
R_{g} and Rₕ are each independently H, D, C₁₋₆ alkyl or C₁₋₆ haloalkyl; alternatively, R_{g} and Rₕ on any one or more carbon atoms in L₄, together with the carbon atom to which they are attached, form C₃₋₇ cycloalkyl;
k is selected from 1, 2, 3 or 4;
L₅ is selected from a chemical bond, C₁₋₆ alkylene or -C₁₋₄ alkylene-O-C₁₋₄ alkylene-;
A is selected from -C(O)- or -NHC(O)-;
T is -(CRᵢRⱼ)_{q}-;
Rᵢ and Rⱼ are each independently H, D, C₁₋₆ alkyl or C₁₋₆ haloalkyl; alternatively, Rᵢ and Rⱼ on any one or more carbon atoms in T, together with the carbon atom to which they are attached, form C₃₋₇ cycloalkyl;
q is selected from 5, 6, 7, 8, 9, 10, 11 or 12;
B is selected from -C(O)-, -OC(O)-, -NH-, -NHC(O)-, -C(O)-(CRₖRₘ)₀₋₄- or - C(O)NH-(CRₖRₘ)₀₋₄-;
Rₖ and Rₘ are each independently H, D, C₁₋₆ alkyl or C₁₋₆ haloalkyl; alternatively, Rₖ and Rₘ on any one or more carbon atoms in B, together with the carbon atom to which they are attached, form C₃₋₇ cycloalkyl;
P is selected from a chemical bond or 1 amino acid residue.

16. The delivery ligand according to any one of claims 13-15, **characterised in that**
L₁ is -(CRₐR_{b})ₘ-;
Rₐ and R_{b} are each independently H or C₁₋₄ alkyl; alternatively, Rₐ and R_{b} on any one or more carbon atoms in L₁, together with the carbon atom to which they are attached, form C₃₋₅ cycloalkyl;
m is selected from 1, 2 or 3;
L₂ is -(CR_{c}R_{d})ₙ-;
R_{c} and R_{d} are each independently H or C₁₋₄ alkyl; alternatively, R_{c} and R_{d} on any one or more carbon atoms in L₂, together with the carbon atom to which they are attached, form C₃₋₅ cycloalkyl;
n is selected from 2, 3 or 4;
L₃ is -(CRₑR_{f})ₕ-;
Rₑ and R_{f} are each independently H or C₁₋₄ alkyl; alternatively, Rₑ and R_{f} on any one or more carbon atoms in L₃, together with the carbon atom to which they are attached, form C₃₋₅ cycloalkyl;
h is selected from 3, 4 or 5;
L₄ is -(CR_{g}Rₕ)₁₋₆-(OCR_{g}Rₕ-CR_{g}Rₕ)ₖ-;
R_{g} and Rₕ are each independently H or C₁₋₄ alkyl; alternatively, R_{g} and Rₕ on any one or more carbon atoms in L₄, together with the carbon atom to which they are attached, form C₃₋₅ cycloalkyl;
k is selected from 2, 3 or 4;
L₅ is selected from a chemical bond, -(CH₂)₁₋₄- or -(CH₂)₁₋₂O(CH₂)₁₋₂-;
A is selected from -C(O)- or -NHC(O)-;
T is -(CRᵢRⱼ)_{q}-;
Rᵢ and Rⱼ are each independently H or C₁₋₄ alkyl; alternatively, Rᵢ and Rⱼ on any one or more carbon atoms in T, together with the carbon atom to which they are attached, form C₃₋₅ cycloalkyl;
q is selected from 5, 6, 7, 8, 9 or 10;
B is selected from -C(O)-, -C(O)NH- or -C(O)NH-(CRₖRₘ)₁₋₃-;
Rₖ and Rₘ are each independently H or C₁₋₄ alkyl; alternatively, Rₖ and Rₘ on any one or more carbon atoms in B, together with the carbon atom to which they are attached, form cyclopropyl;
P is selected from a chemical bond, -Cit-, -Arg- or -Phe-.

17. The delivery ligand according to any one of claims 13-16, **characterised in that**
L₁ is -(CRₐR_{b})₂-, preferably -CH₂CH₂- or 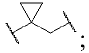
Rₐ and R_{b} are each independently H; alternatively, Rₐ and R_{b} on any one or more carbon atoms in L₁, together with the carbon atom to which they are attached, form cyclopropyl;
L₂ is -(CR_{c}R_{d})₃-, preferably -CH₂CH₂CH₂-, 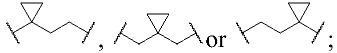
R_{c} and R_{d} are each independently H; alternatively, R_{c} and R_{d} on any one or more carbon atoms in L₂, together with the carbon atom to which they are attached, form cyclopropyl;
L₃ is -(CRₑR_{f})₄-, preferably -CH₂CH₂CH₂CH₂- or 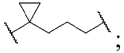
Rₑ and R_{f} are each independently H; alternatively, Rₑ and R_{f} on any one or more carbon atoms in L₃, together with the carbon atom to which they are attached, form cyclopropyl;
L₄ is -(CR_{g}Rₕ)₂-(OCR_{g}Rₕ-CR_{g}Rₕ)₂-, preferably 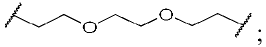
R_{g} and Rₕ are each independently H; alternatively, R_{g} and Rₕ on any one or more carbon atoms in L₄, together with the carbon atom to which they are attached, form cyclopropyl;
A is -C(O)- or -NHC(O)-;
L₅ is selected from a chemical bond, -CH₂CH₂- or -CH₂OCH₂-;
T is -(CRᵢRⱼ)₇₋₁₀-;
Rᵢ and Rⱼ are each independently H; alternatively, Rᵢ and Rⱼ on any one or more carbon atoms in T, together with the carbon atom to which they are attached, form cyclopropyl;
B is selected from -C(O)-, -C(O)NH- or -C(O)NH-CRₖRₘ-;
Rₖ and Rₘ are independently H; alternatively, Rₖ and Rₘ on any one or more carbon atoms in B, together with the carbon atom to which they are attached, form cyclopropyl;
P is selected from a chemical bond, -Cit- or -Phe-.

18. The delivery ligand according to any one of claims 1-17, **characterised in that**
each W is the same or different, and is independently selected from H, 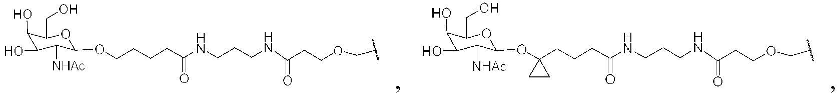 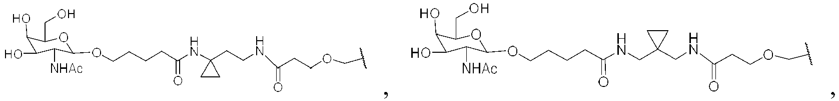 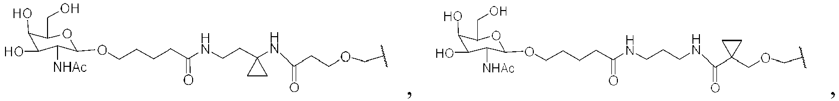 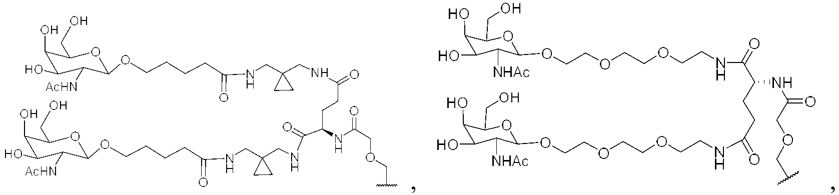 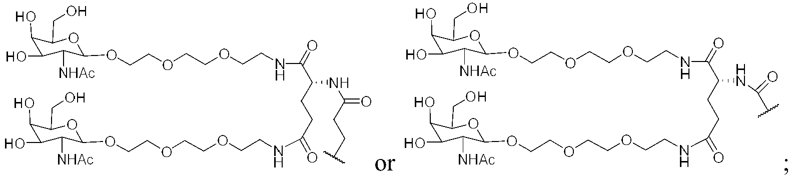
A is -NHC(O)-;
T is selected from 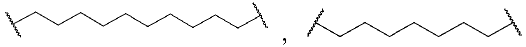 or 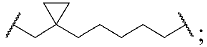
B is selected from -C(O)-, 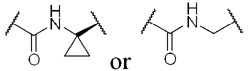
P is selected from a chemical bond,

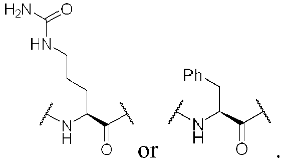

19. The delivery ligand according to any one of claims 1-18, **characterised in that** the conjugated group is selected from a structure as shown in formula (VI), formula (VI-1), formula (VI-2), formula (VI'), formula (VI'-1), formula (VI'-2), formula (VII), formula (VII-1), formula (VII-2), formula (VII'), formula (VII'-1), formula (VII'-2), formula (VIII), formula (VIII-1), formula (VIII-2), formula (VIII-3), formula (VIII-4), formula (VIII'), formula (VIII-5), formula (VIII-6), formula (VIII'-1), formula (VIII'-2), formula (VIII'-3), formula (VIII'-4), formula (VIII'-5), formula (VIII'-6), formula (IX), formula (IX-1), formula (IX-2), formula (IX'), formula (IX'-1) or formula (IX'-2), or an isotopic variant, tautomer, or stereoisomer thereof, or a mixture thereof: 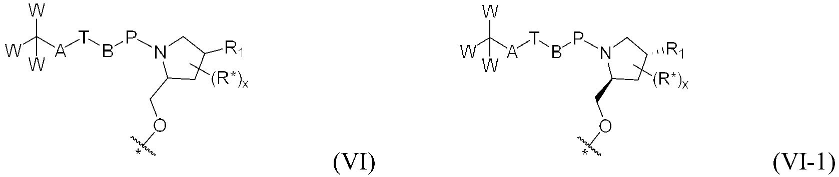 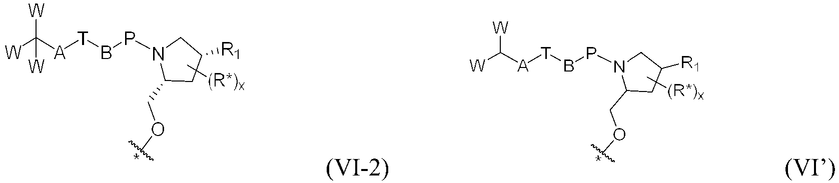 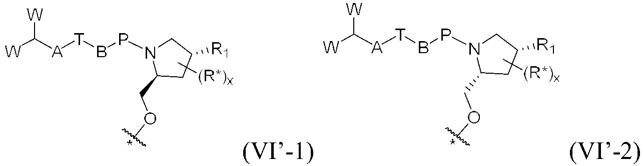 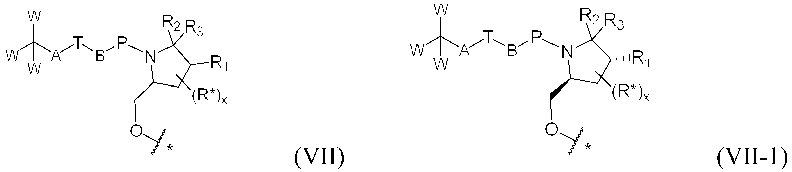 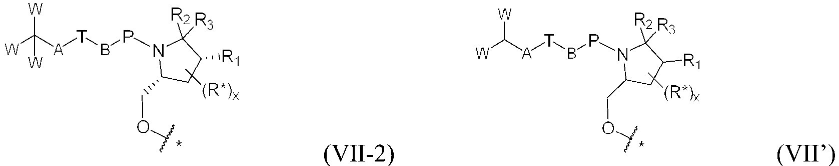 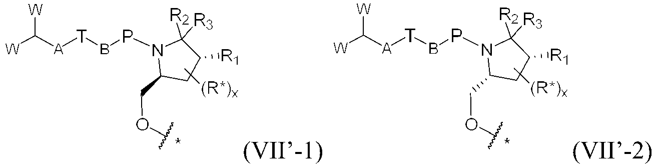 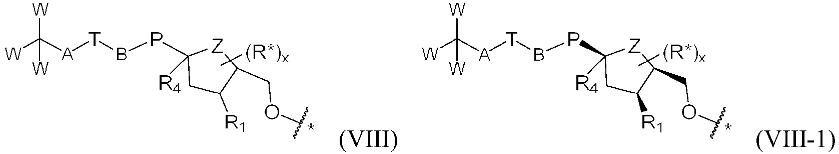 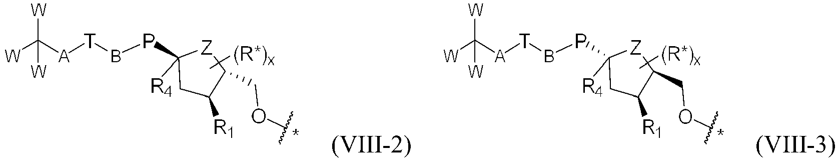 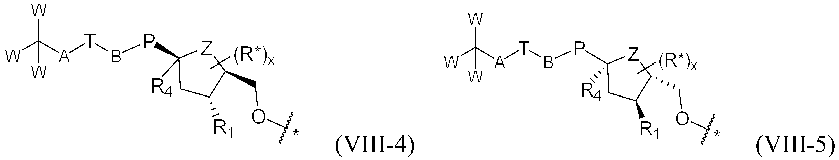 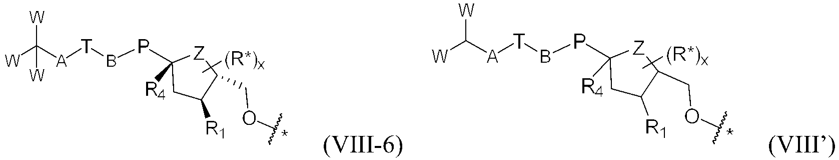 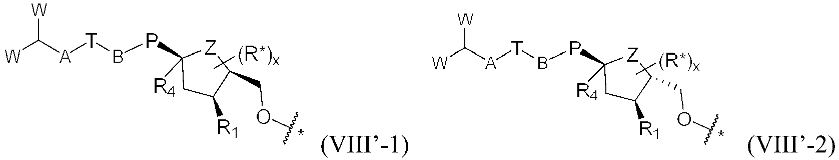 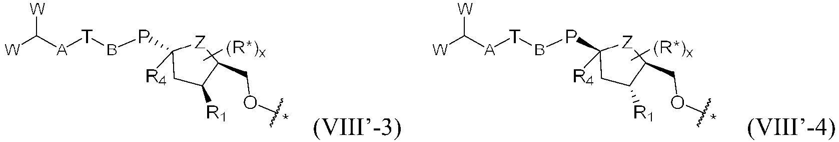 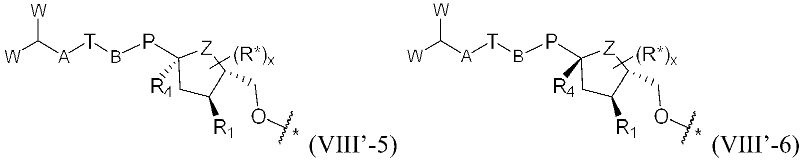 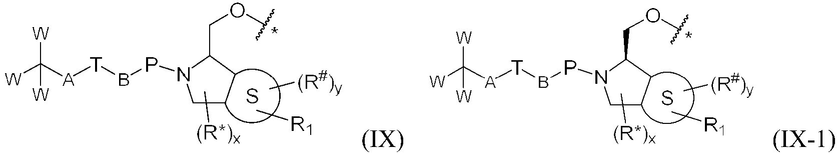 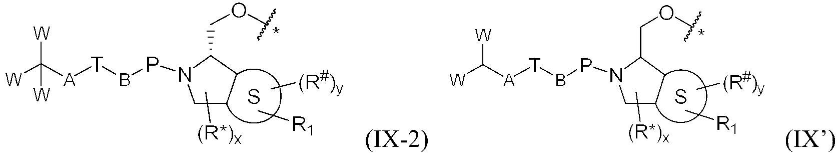 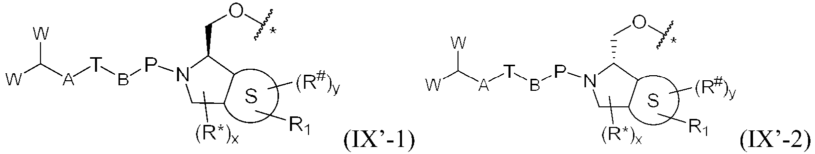 wherein each variable is as defined in any one of claims 1-18.

20. The delivery ligand according to any one of claims 1-18, **characterised in that** the conjugated group is selected from a structure as shown in formula (VI-1) or formula (VI'-1), or an isotopic variant, tautomer, or stereoisomer thereof, or a mixture thereof: 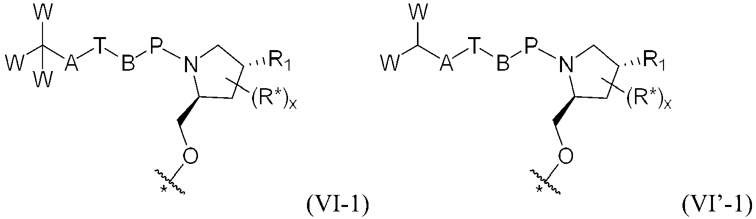
each W is independently selected from H, 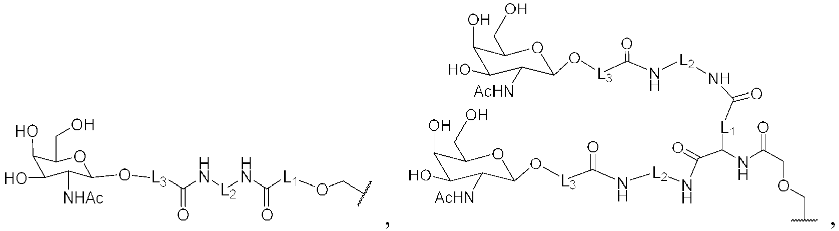 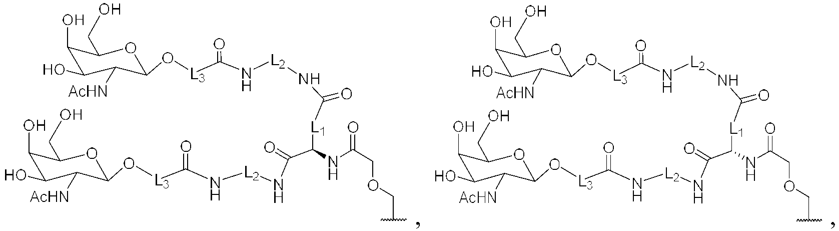 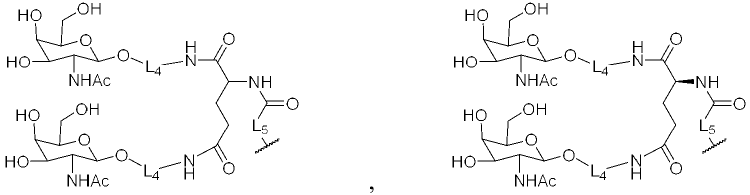 or 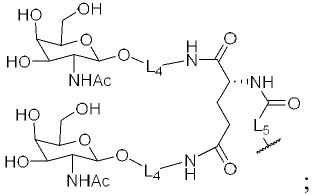
L₁, L₂, L₃, L₄, L₅, A, B, T and P are as defined in any one of claims 1-18;
provided that, at least one pair among Rₐ and R_{b}, R_{c} and R_{d}, Rₑ and R_{f}, R_{g} and Rₕ, Rᵢ and Rⱼ, and Rₖ and Rₘ, together with the carbon atom to which they are attached, forms a ring;
R₁ is selected from -OR₀, -CH₂OR₀ or -CH₂CH₂OR₀, wherein R₀ is H, D, a hydroxyl protecting group or a solid-phase carrier;
the hydroxyl protecting group is preferably -C(O)CH₂CH₂C(O)OH or 4,4'-dimethoxytrityl (DMTr);
R* is selected from H, D, C₁₋₆ alkyl or C₁₋₆ haloalkyl;
x is selected from 0, 1, 2, 3, 4, 5 or 6;
preferably,
R₁ is -OR₀, wherein R₀ is H or a hydroxyl protecting group, preferably H;
the hydroxyl protecting group is preferably -C(O)CH₂CH₂C(O)OH;
R* is selected from H or C₁₋₆ alkyl;
x is selected from 0, 1, 2, 3 or 4;
more preferably,
R₁ is OH;
R* is selected from H or C₁₋₄ alkyl, preferably H;
x is 0, 1, 2 or 3.

21. The delivery ligand according to any one of claims 1-18, **characterised in that** the conjugated group is selected from a structure as shown in formula (VII-1) or formula (VII'-1), or an isotopic variant, tautomer, or stereoisomer thereof, or a mixture thereof: 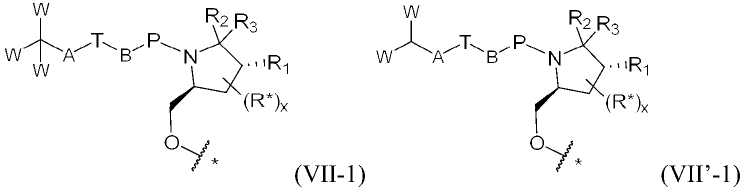 wherein
each W is independently selected from H, 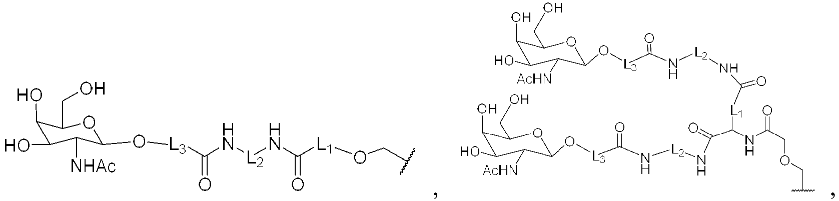 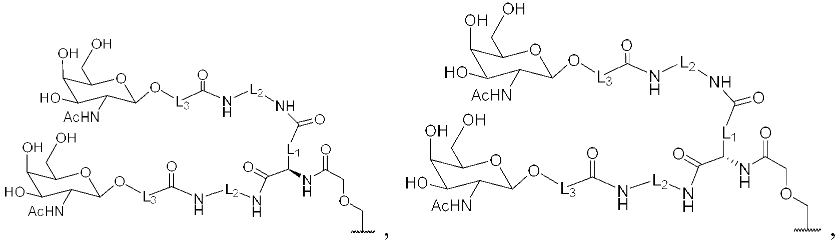 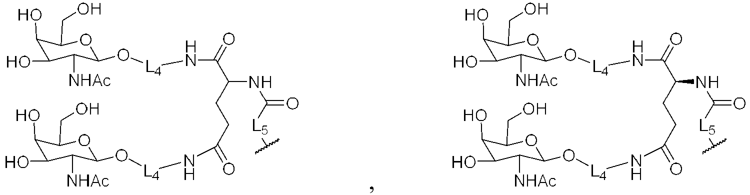 or 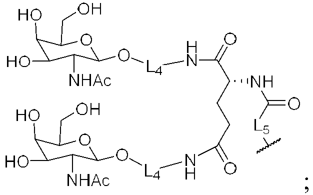
L₁, L₂, L₃, L₄, L₅, A, B, T and P are as defined in any one of claims 1-18;
R₁ is selected from -OR₀, -CH₂OR₀ or -CH₂CH₂OR₀, wherein R₀ is H, D, a hydroxyl protecting group or a solid-phase carrier;
the hydroxyl protecting group is preferably -C(O)CH₂CH₂C(O)OH or 4,4'-dimethoxytrityl (DMTr);
R₂ and R₃ are independently selected from H, D, C₁₋₆ alkyl, C₁₋₆ alkoxy or C₁₋₆ haloalkyl; alternatively, R₂ and R₃, together with the carbon atom to which they are attached, form C₃₋₇ cycloalkyl or 3- to 7-membered heterocyclyl;
R* is selected from H, D, C₁₋₆ alkyl or C₁₋₆ haloalkyl;
x is selected from 0, 1, 2, 3 or 4;
preferably,
R₁ is -OR₀, wherein R₀ is H or a hydroxyl protecting group, preferably H;
the hydroxyl protecting group is preferably -C(O)CH₂CH₂C(O)OH;
R₂ and R₃ are independently selected from H, D, C₁₋₆ alkyl or C₁₋₆ haloalkyl; alternatively, R₂ and R₃, together with the carbon atom to which they are attached, form C₃₋₅ cycloalkyl;
R* is selected from H or C₁₋₆ alkyl;
x is selected from 0, 1, 2, 3 or 4;
more preferably,
R₁ is OH;
R₂ and R₃ are independently selected from H or C₁₋₄ alkyl; alternatively, R₂ and R₃, together with the carbon atom to which they are attached, form C₃₋₅ cycloalkyl, such as cyclopropyl;
R* is selected from H or C₁₋₄ alkyl, preferably H;
x is 0, 1, 2 or 3.

22. The delivery ligand according to any one of claims 1-18, **characterised in that** the conjugated group is selected from a structure as shown in formula (VIII-2), formula (VIII-5), formula (VIII-6), formula (VIII'-2), formula (VIII'-5) or formula (VIII'-6), or an isotopic variant, tautomer, or stereoisomer thereof, or a mixture thereof: 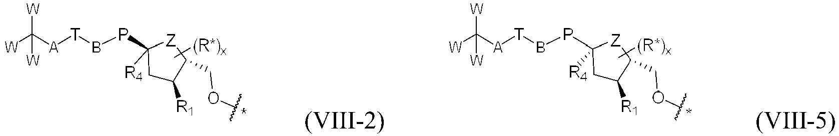 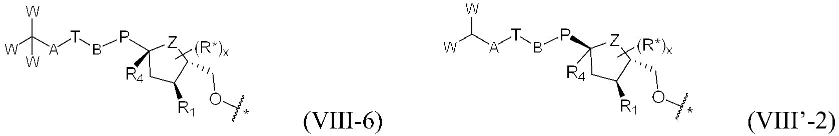 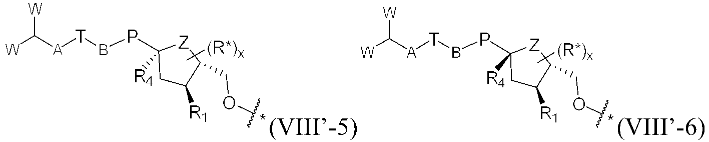 wherein
each W is independently selected from H, 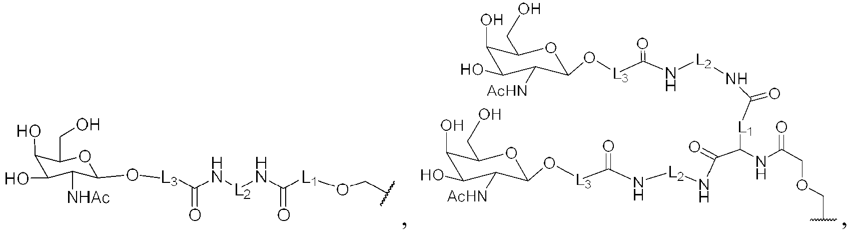 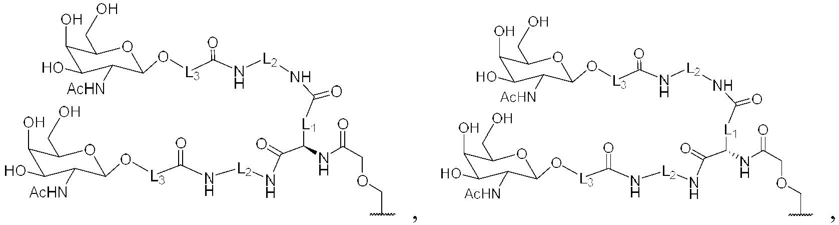 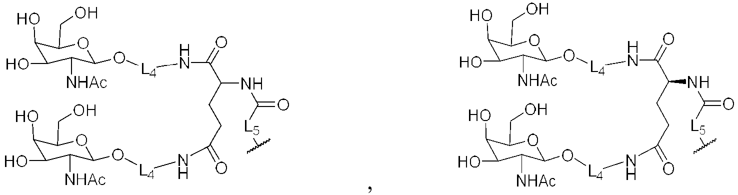 or 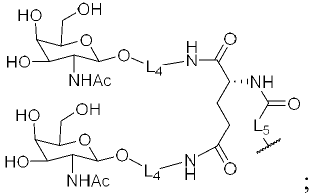
L₁, L₂, L₃, L₄, L₅, A, B, T and P are as defined in any one of claims 1-18;
Z is selected from O, NH or CH₂;
R₁ is selected from -OR₀, -CH₂OR₀ or -CH₂CH₂OR₀, wherein R₀ is H, D, a hydroxyl protecting group or a solid-phase carrier;
the hydroxyl protecting group is preferably -C(O)CH₂CH₂C(O)OH or 4,4'-dimethoxytrityl (DMTr);
R₄ is selected from H, D, C₁₋₆ alkyl or C₁₋₆ haloalkyl;
R* is selected from H, D, C₁₋₆ alkyl or C₁₋₆ haloalkyl;
x is selected from 0, 1, 2, 3 or 4;
preferably,
Z is selected from O, NH or CH₂;
R₁ is -OR₀, wherein R₀ is H or a hydroxyl protecting group, preferably H;
the hydroxyl protecting group is preferably -C(O)CH₂CH₂C(O)OH;
R₄ is selected from H or C₁₋₆ alkyl;
R* is selected from H or C₁₋₆ alkyl;
x is selected from 0, 1, 2, 3 or 4;
more preferably,
Z is selected from O or NH, preferably O;
R₁ is OH;
R₄ is selected from H or C₁₋₄ alkyl, preferably methyl;
R* is selected from H or C₁₋₄ alkyl, preferably H;
x is 0, 1, 2 or 3.

23. The delivery ligand according to any one of claims 1-18, **characterised in that** the conjugated group is selected from a structure as shown in formula (IX) or formula (IX'), or an isotopic variant, tautomer, or stereoisomer thereof, or a mixture thereof: 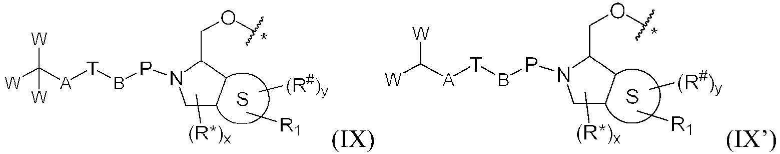
each W is independently selected from H, 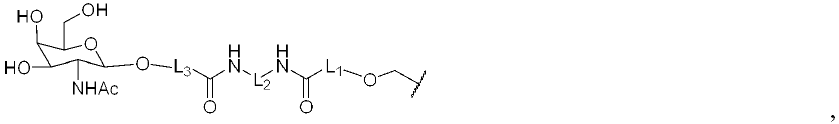 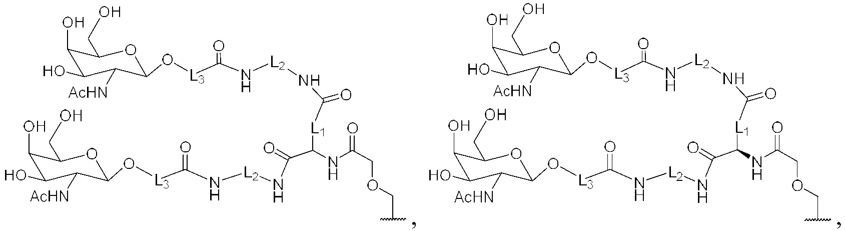 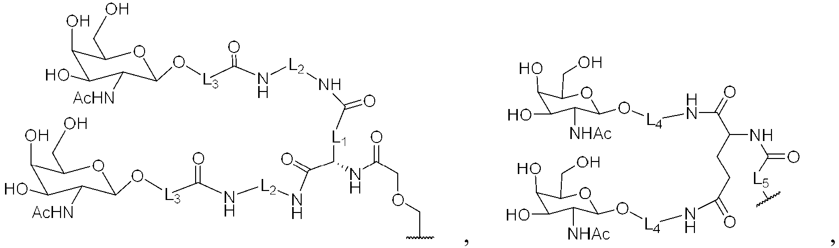 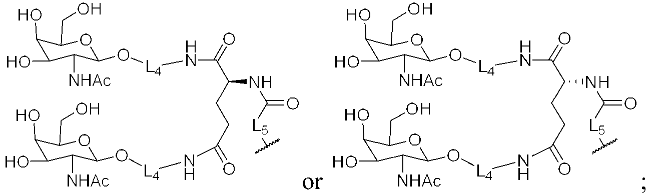
L₁, L₂, L₃, L₄, L₅, A, B, T and P are as defined in any one of claims 1-18;
ring S is selected from C₃₋₁₀ cycloalkyl or 3- to 10-membered heterocyclyl;
R₁ is selected from -OR₀, -CH₂OR₀ or -CH₂CH₂OR₀, wherein R₀ is H, D, a hydroxyl protecting group or a solid-phase carrier;
the hydroxyl protecting group is preferably -C(O)CH₂CH₂C(O)OH or 4,4'-dimethoxytrityl (DMTr);
R* and R^{#} are independently selected from H, D, C₁₋₆ alkyl or C₁₋₆ haloalkyl;
x and y are independently selected from 0, 1, 2, 3 or 4;
preferably,
ring S is selected from C₃₋₇ cycloalkyl or 3- to 7-membered heterocyclyl;
R₁ is -OR₀, wherein R₀ is H or a hydroxyl protecting group, preferably H;
the hydroxyl protecting group is preferably -C(O)CH₂CH₂C(O)OH;
R₄ is selected from H or C₁₋₆ alkyl;
R* and R^{#} are independently selected from H or C₁₋₆ alkyl;
x and y are independently selected from 0, 1, 2, 3 or 4;
more preferably,
ring S is C₅₋₆ cycloalkyl, such as cyclopentyl;
R₁ is OH;
R* and R^{#} are independently selected from H or C₁₋₄ alkyl, preferably H;
x and y are independently selected from 0, 1, 2 or 3.

24. The delivery ligand according to any one of claims 1-18, **characterised in that** the conjugated group is selected from a structure as shown in formula (V-1) or formula (V-4), or an isotopic variant, tautomer, or stereoisomer thereof, or a mixture thereof: 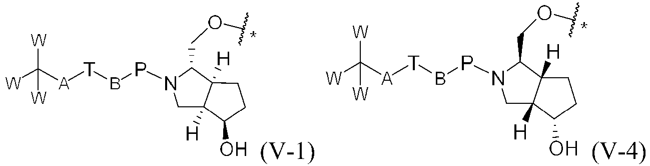
each W is independently selected from H, 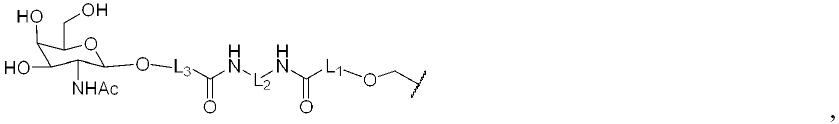 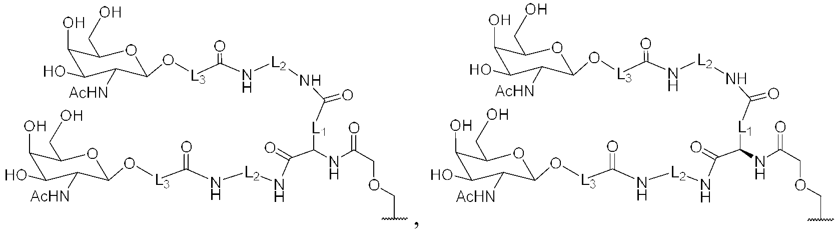 or 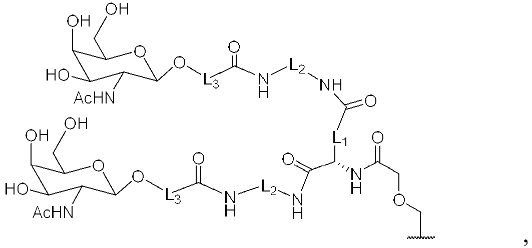 preferably 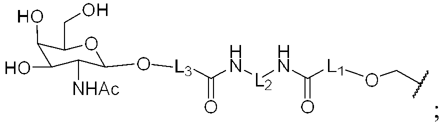
L₁ is -(CRₐR_{b})ₘ-;
Rₐ and R_{b} are each independently H, D, C₁₋₆ alkyl, C₁₋₆ alkoxy or C₁₋₆ haloalkyl; alternatively, Rₐ and R_{b} on any one or more carbon atoms in L₁, together with the carbon atom to which they are attached, form C₃₋₇ cycloalkyl or 3- to 7-membered heterocyclyl;
L₂ is -(CR_{c}R_{d})ₙ-;
R_{c} and R_{d} are each independently H, D, C₁₋₆ alkyl, C₁₋₆ alkoxy or C₁₋₆ haloalkyl; alternatively, R_{c} and R_{d} on any one or more carbon atoms in L₂, together with the carbon atom to which they are attached, form C₃₋₇ cycloalkyl or 3- to 7-membered heterocyclyl;
L₃ is -(CRₑR_{f})ₕ-;
Rₑ and R_{f} are each independently H, D, C₁₋₆ alkyl, C₁₋₆ alkoxy or C₁₋₆ haloalkyl; alternatively, Rₑ and R_{f} on any one or more carbon atoms in L₃, together with the carbon atom to which they are attached, form C₃₋₇ cycloalkyl or 3- to 7-membered heterocyclyl;
m, n and h are independently selected from 1, 2, 3, 4 or 5;
A is selected from -C(O)- or -NHC(O)-;
T is -(CRᵢRⱼ)_{q}-;
Rᵢ and Rⱼ are each independently H, D, C₁₋₆ alkyl, C₁₋₆ alkoxy or C₁₋₆ haloalkyl; alternatively, Rᵢ and Rⱼ on any one or more carbon atoms in T, together with the carbon atom to which they are attached, form C₃₋₇ cycloalkyl or 3- to 7-membered heterocyclyl;
q is selected from 3, 4, 5, 6, 7, 8, 9, 10, 11, 12, 13, 14 or 15;
B is selected from -C(O)-(CRₖRₘ)₀₋₆-, -NH-(CRₖRₘ)₀₋₆-, -OC(O)-(CRₖRₘ)₀₋₆-, -NHC(O)-(CRₖRₘ)₀₋₆- or -C(O)NH-(CRₖRₘ)₀₋₆-;
Rₖ and Rₘ are each independently H, D, C₁₋₆ alkyl, C₁₋₆ alkoxy or C₁₋₆ haloalkyl; alternatively, Rₖ and Rₘ on any one or more carbon atoms in B, together with the carbon atom to which they are attached, form C₃₋₇ cycloalkyl or 3- to 7-membered heterocyclyl;
P is selected from a chemical bond or 1 amino acid residue;
preferably,
each W is independently 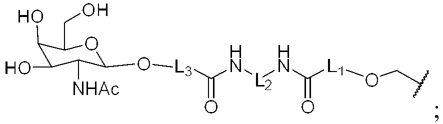
L₁ is -(CRₐR_{b})ₘ-;
Rₐ and R_{b} are each independently H, D, C₁₋₆ alkyl or C₁₋₆ alkoxy; alternatively, Rₐ and R_{b} on any one or more carbon atoms in L₁, together with the carbon atom to which they are attached, form C₃₋₇ cycloalkyl;
L₂ is -(CR_{c}R_{d})ₙ-;
R_{c} and R_{d} are each independently H, D, C₁₋₆ alkyl or C₁₋₆ alkoxy; alternatively, R_{c} and R_{d} on any one or more carbon atoms in L₂, together with the carbon atom to which they are attached, form C₃₋₇ cycloalkyl;
L₃ is -(CRₑR_{f})ₕ-;
Rₑ and R_{f} are each independently H, D, C₁₋₆ alkyl or C₁₋₆ alkoxy; alternatively, Rₑ and R_{f} on any one or more carbon atoms in L₃, together with the carbon atom to which they are attached, form C₃₋₇ cycloalkyl;
m, n and h are independently selected from 1, 2, 3, 4 or 5;
A is selected from -C(O)- or -NHC(O)-;
T is -(CRᵢRⱼ)_{q}-;
Rᵢ and Rⱼ are each independently H, D, C₁₋₆ alkyl or C₁₋₆ alkoxy;
q is selected from 4, 5, 6, 7, 8, 9, 10, 11 or 12;
B is selected from -C(O)-(CRₖRₘ)₀₋₆-, -NH-(CRₖRₘ)₀₋₆- or -OC(O)-(CRₖRₘ)₀₋₆-;
Rₖ and Rₘ are each independently H, D, C₁₋₆ alkyl or C₁₋₆ alkoxy;
P is selected from a chemical bond or 1 amino acid residue, preferably a chemical bond;
more preferably,
each W is independently 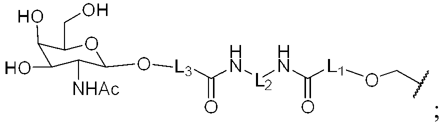
L₁ is -(CRₐR_{b})ₘ-;
Rₐ and R_{b} are each independently H, C₁₋₆ alkyl or C₁₋₆ alkoxy, and Rₐ and R_{b} on at least one carbon atom in L₁, together with the carbon atom to which they are attached, form C₃₋₅ cycloalkyl;
L₂ is -(CR_{c}R_{d})ₙ-;
R_{c} and R_{d} are each independently H, C₁₋₆ alkyl or C₁₋₆ alkoxy;
L₃ is -(CRₑR_{f})ₕ-;
Rₑ and R_{f} are each independently H, C₁₋₆ alkyl or C₁₋₆ alkoxy;
m, n and h are independently selected from 1, 2, 3, 4 or 5;
A is selected from -C(O)- or -NHC(O)-, preferably -NHC(O)-;
T is -(CRᵢRⱼ)_{q}-;
Rᵢ and Rⱼ are each independently H, C₁₋₆ alkyl or C₁₋₆ alkoxy;
q is selected from 4, 5, 6, 7, 8, 9, 10, 11 or 12;
B is -C(O)-(CRₖRₘ)₀₋₆-;
Rₖ and Rₘ are each independently H, C₁₋₆ alkyl or C₁₋₆ alkoxy;
P is a chemical bond.

25. The delivery ligand according to any one of claims 1-24, **characterised in that** each W is the same or different, and is independently selected from: H; 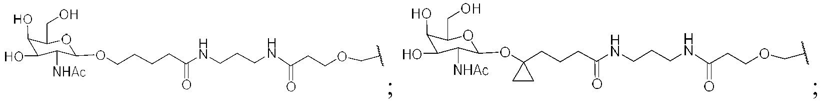 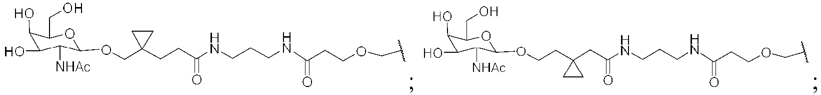 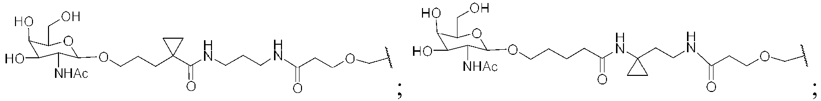 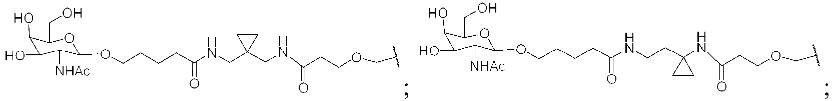 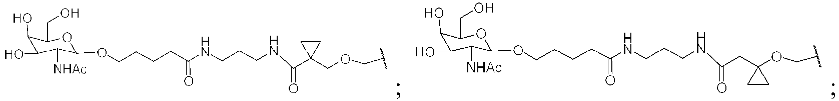 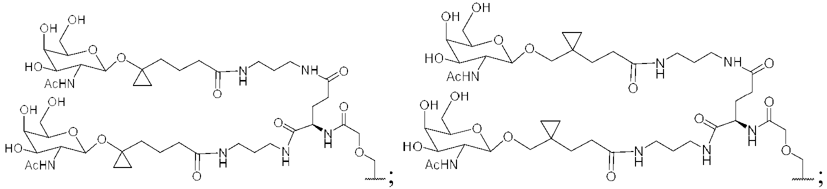 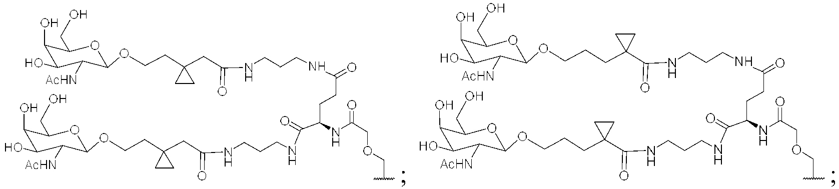 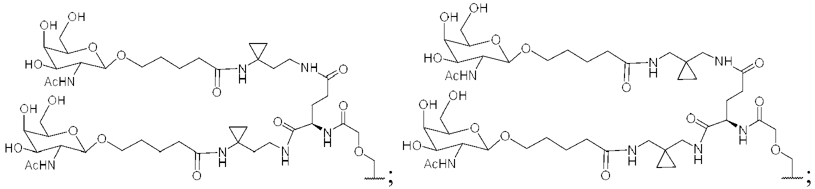 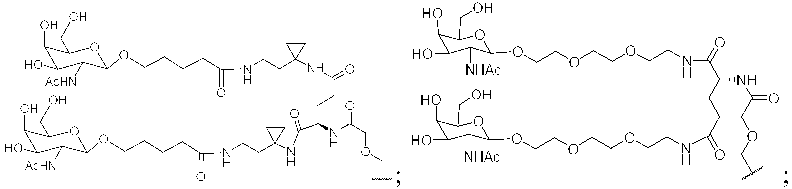 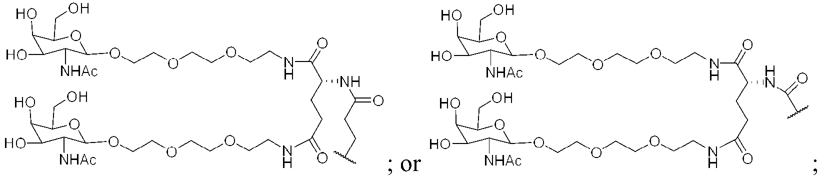
preferably, each W is independently selected from: H; 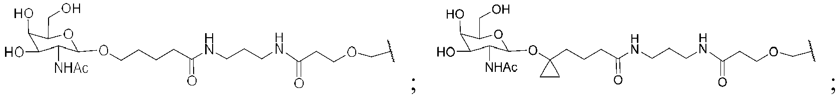 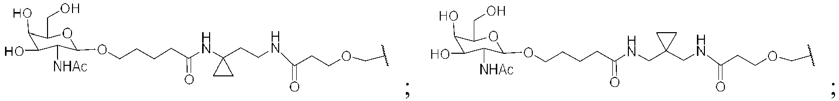 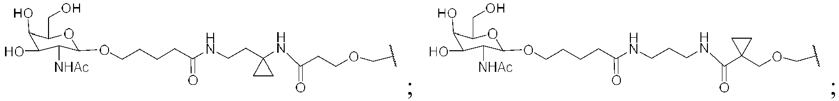 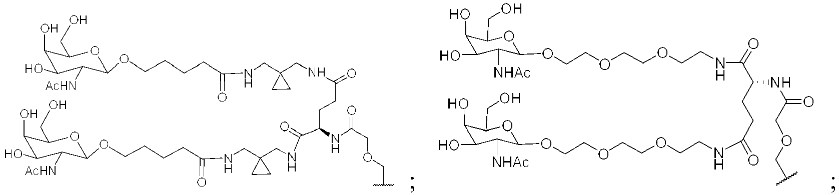 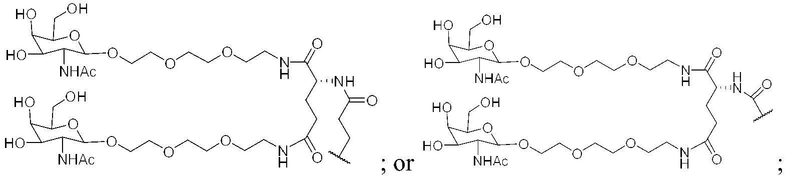
more preferably, 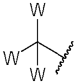 is selected from: H,

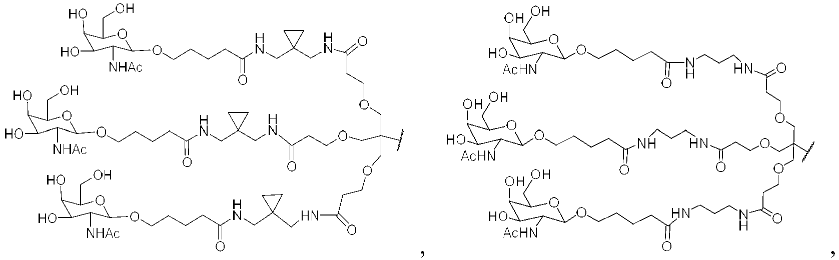

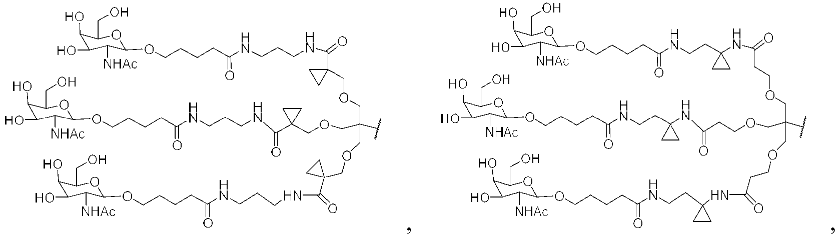

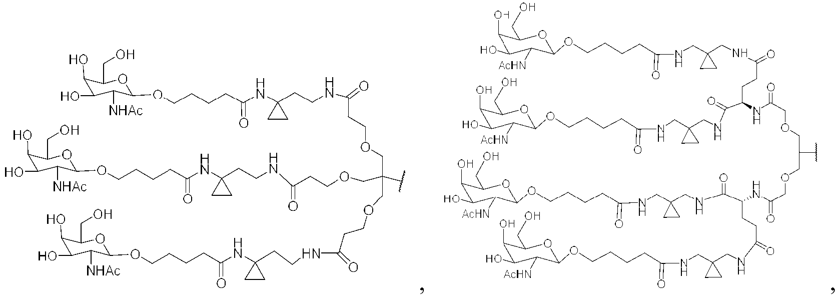

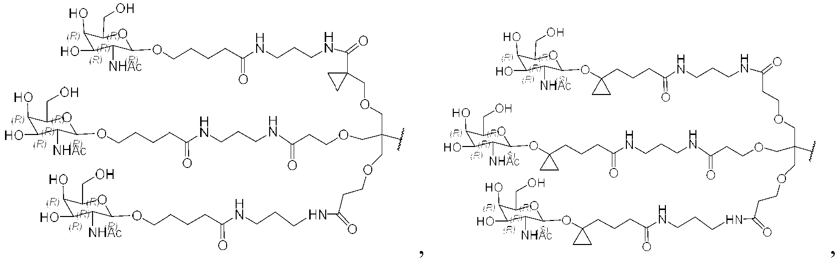

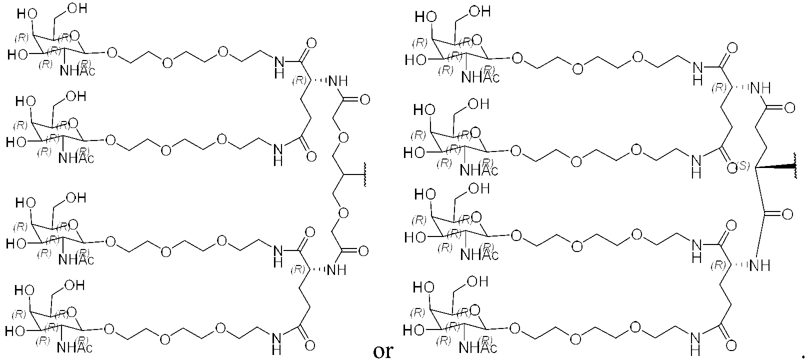

26. The delivery ligand according to any one of claims 1-25, **characterised in that** the -A-T-B-P- is selected from the following: 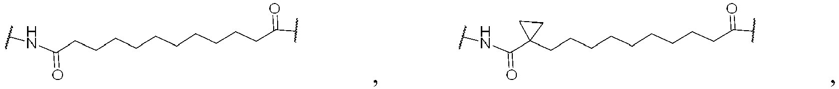 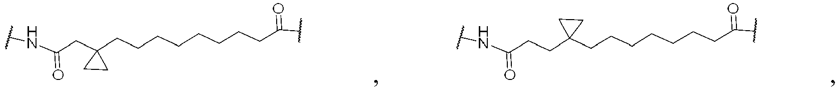 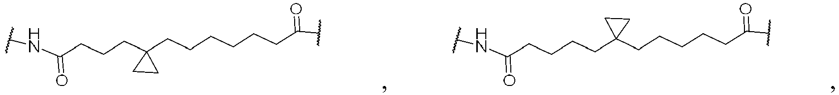 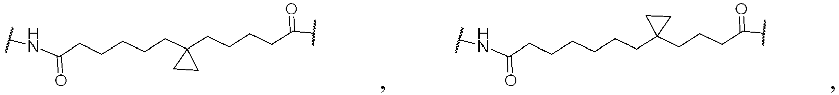 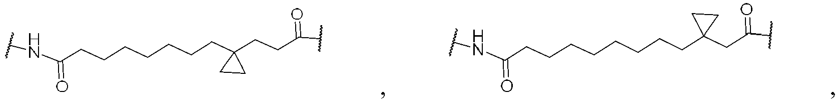 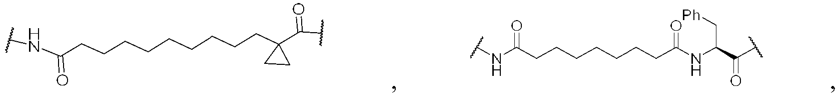 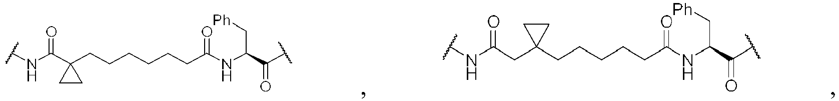 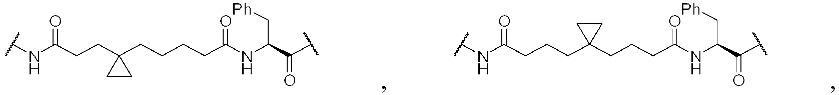 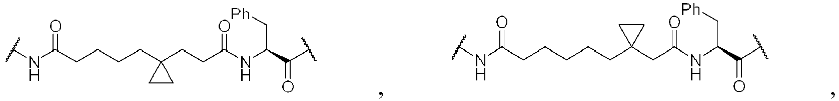 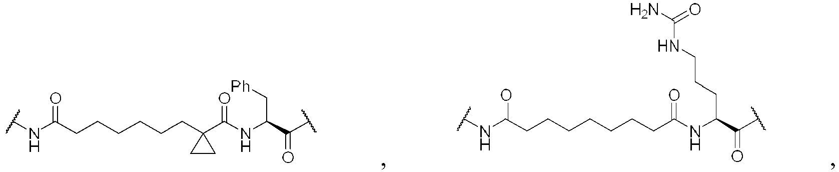 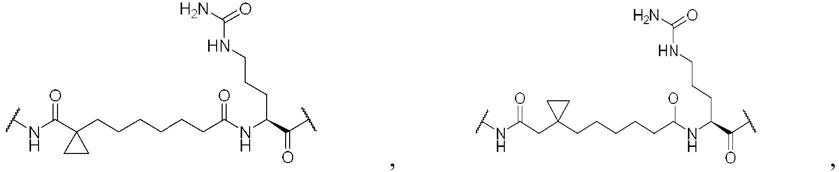 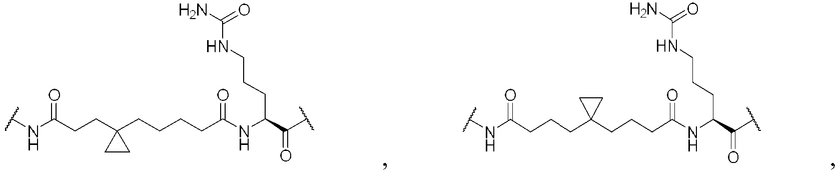 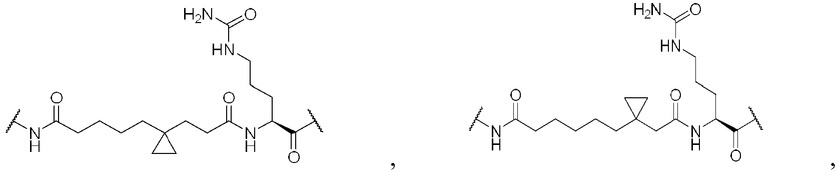 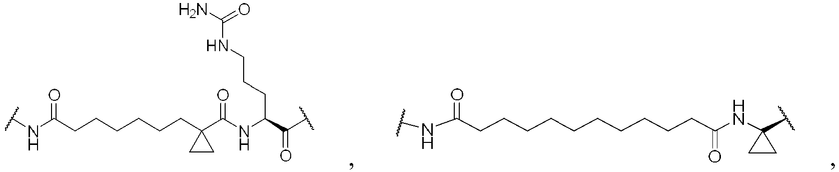 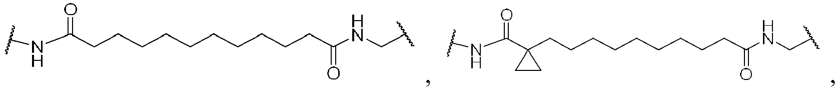 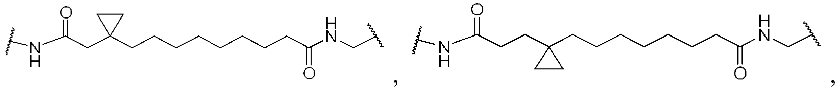 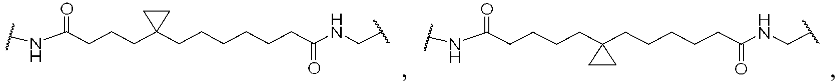 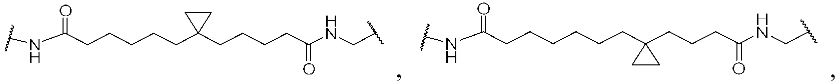 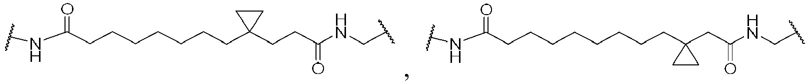 or 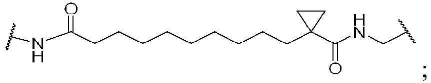 preferably, the -A-T-B-P- is selected from the following:

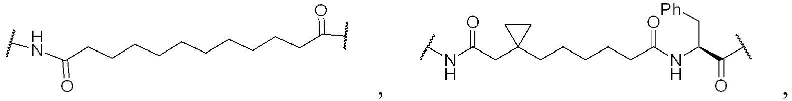

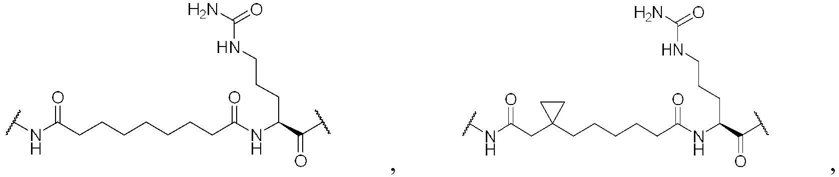

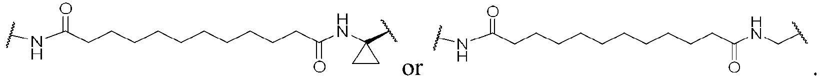

27. The delivery ligand according to any one of claims 1-26, **characterised in that** the conjugated group is selected from a structure as shown in Table A, or an isotopic variant, tautomer, or stereoisomer thereof, or a mixture thereof.

28. The delivery ligand according to any one of claims 1-27, **characterised in that** the biomolecule is a nucleic acid.

29. The delivery ligand according to any one of claims 1-28, **characterised by** targeting an asialoglycoprotein receptor (ASGPR) on the surface of hepatocytes.

30. A nucleic acid molecule, **characterised by** comprising a nucleic acid and the delivery ligand according to any one of claims 1-29 attached thereto.

31. The nucleic acid molecule according to claim 30, **characterised in that** the nucleic acid is selected from DNA, RNA and a DNA/RNA hybrid.

32. The nucleic acid molecule according to claim 30 or 31, **characterised in that** the nucleic acid molecule is single-stranded or double-stranded.

33. The nucleic acid molecule according to any one of claims 30-32, **characterised in that** the nucleic acid molecule is selected from small interfering RNA (siRNA) and short hairpin RNA (shRNA).

34. The nucleic acid molecule according to any one of claims 30-33, **characterised by** specifically binding to the asialoglycoprotein receptor (ASGPR) on the surface of hepatocytes.

35. A double-stranded RNA molecule, **characterised by** comprising a sense strand and an antisense strand, wherein the double-stranded RNA molecule contains one or more delivery ligands according to any one of claims 1-29.

36. The double-stranded RNA molecule according to claim 35, **characterised in that** the conjugated group is attached to the 3'-end of the sense strand of the double-stranded RNA molecule.

37. The double-stranded RNA molecule according to claim 35, **characterised in that** the conjugated group is attached to the 5'-end of the sense strand of the double-stranded RNA molecule.

38. The double-stranded RNA molecule according to claim 35, **characterised in that** the conjugated group is attached to the 3'-end and 5'-end of the sense strand of the double-stranded RNA molecule.

39. The double-stranded RNA molecule according to claim 35, **characterised in that** the conjugated group is attached to the 3'-end of the antisense strand of the double-stranded RNA molecule.

40. The double-stranded RNA molecule according to claim 35, **characterised in that** the conjugated group is attached to the 5'-end of the antisense strand of the double-stranded RNA molecule.

41. The double-stranded RNA molecule according to claim 35, **characterised in that** the conjugated group is attached to the 3'-end and 5'-end of the antisense strand of the double-stranded RNA molecule.

42. The double-stranded RNA molecule according to any one of claims 35-41, **characterised in that** each of the sense strand and the antisense strand of the double-stranded RNA molecule has 10 to 30 nucleotides, preferably 15 to 25 nucleotides, more preferably 20 to 25 nucleotides.

43. The double-stranded RNA molecule according to any one of claims 35-42, **characterised in that** one or more nucleotides in the sense strand and/or the antisense strand of the double-stranded RNA molecule comprise a chemical modification.

44. A pharmaceutical composition, **characterised by** comprising the nucleic acid molecule according to any one of claims 30-34 or the double-stranded RNA molecule according to any one of claims 35-43, and a pharmaceutically acceptable carrier or excipient.

45. A kit, **characterised by** comprising the nucleic acid molecule according to any one of claims 30-34 or the double-stranded RNA molecule according to any one of claims 35-43.

46. A compound of formula (X'), or a pharmaceutically acceptable salt, stereoisomer, tautomer or isotopic variant thereof, or a mixture thereof: 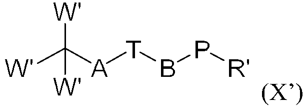
characterised in thatR' is selected from 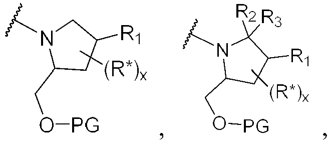 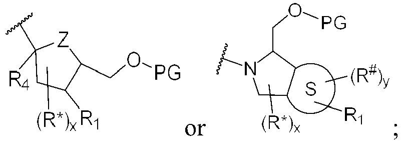
PG is a hydroxyl protecting group, such as trimethylsilyl (TMS), triethylsilyl (TES), dimethylisopropylsilyl (DMIPS), diethylisopropylsilyl (DEIPS), tert-butyl dimethylsilyl (TBDMS), tert-butyl diphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butyloxycarbonyl (Boc), benzyl (Bn), p-methoxybenzyl (PMB), allyl, trityl (Tr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), -C(O)CH₂CH₂C(O)OH or 4,4'-dimethoxytrityl (DMTr);
preferably, PG is selected from 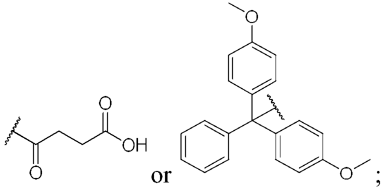
each W' is independently selected from H, D, 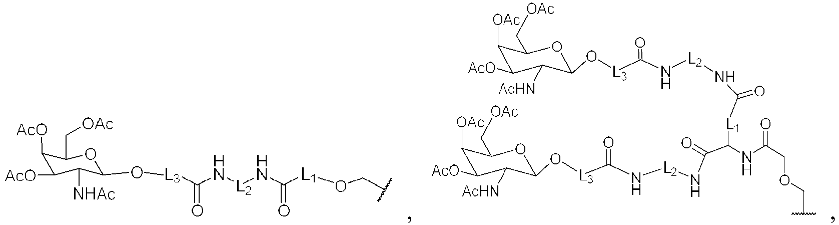 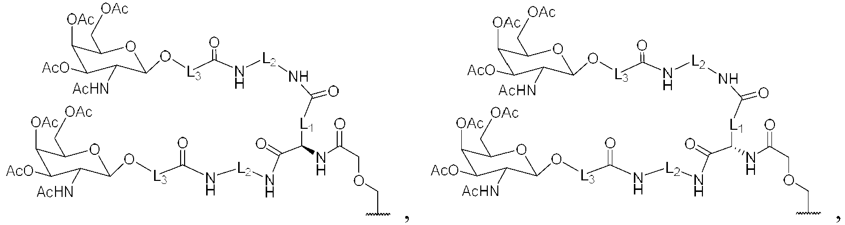 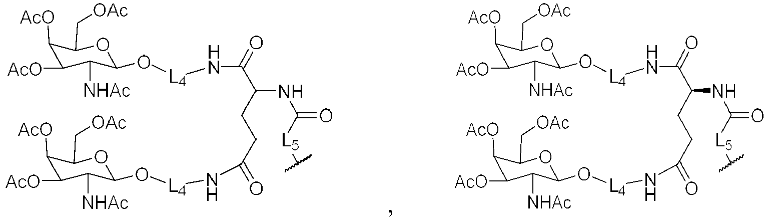 or 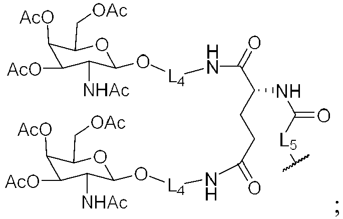
provided that, when R' is 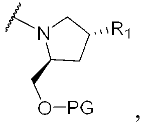 at least one pair among Rₐ and R_{b}, R_{c} and R_{d}, Rₑ and R_{f}, R_{g} and Rₕ, Rᵢ and Rⱼ, and Rₖ and Rₘ in W', together with the carbon atom to which they are attached, forms C₃₋₇ cycloalkyl or 3- to 7-membered heterocyclyl;
each of the other variables is as defined in any one of claims 1-26.

47. The compound according to claim 46, **characterised in that** each W' is the same or different, and is independently selected from: H; 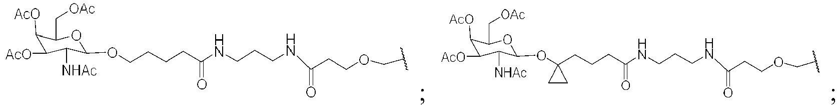 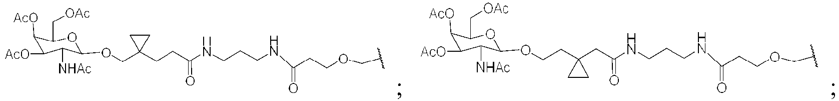 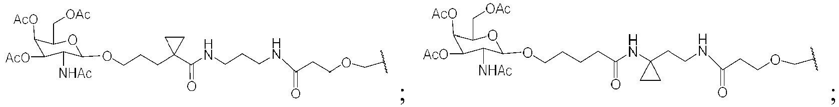 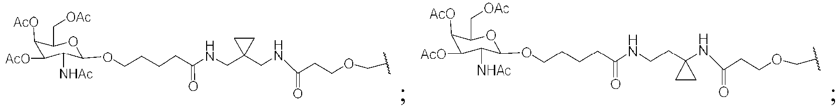 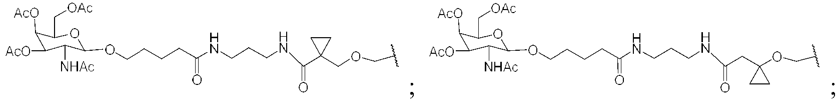 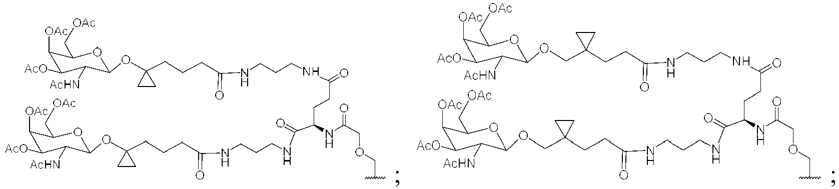 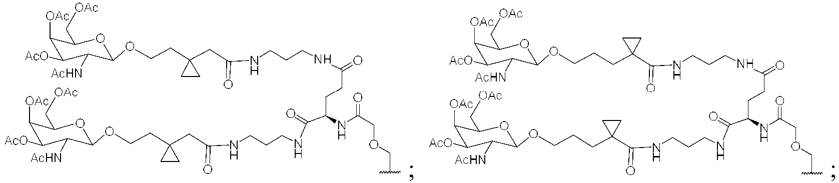 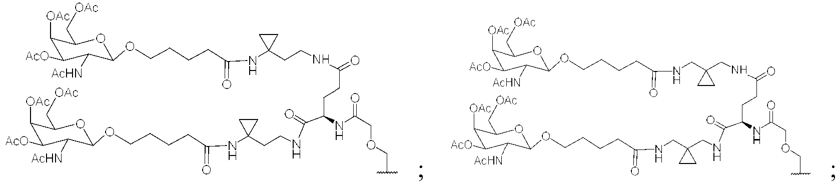 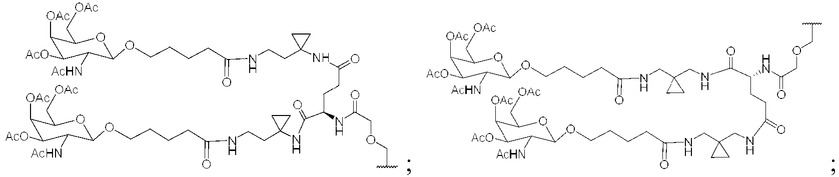 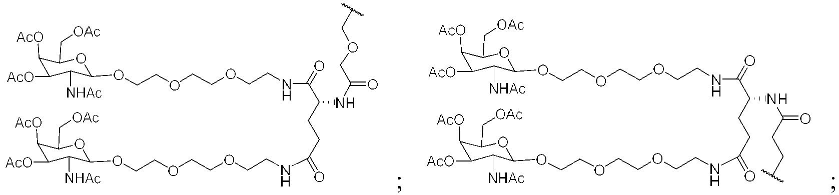 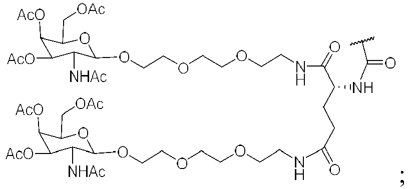 preferably, each W' is independently selected from: H;

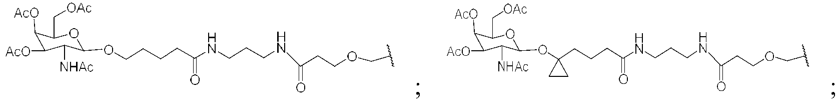

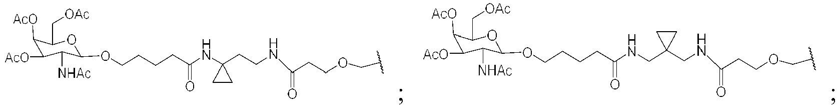

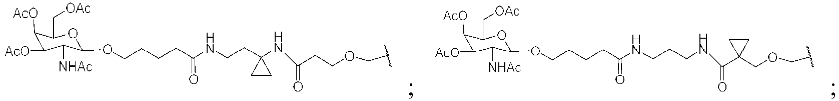

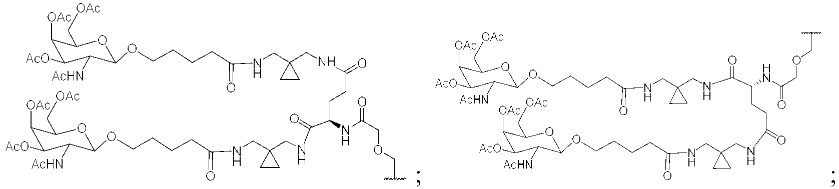

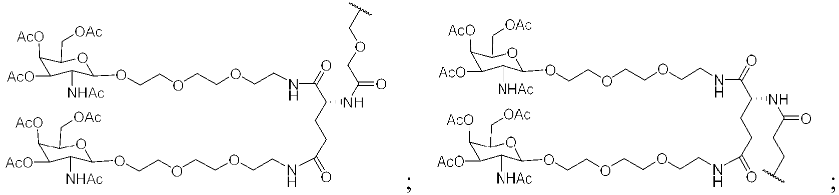

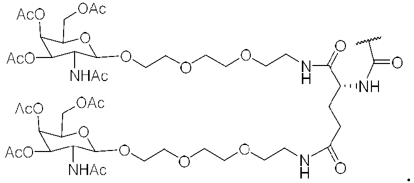

48. The compound according to claim 46 or 47, **characterised in that** R' is selected from 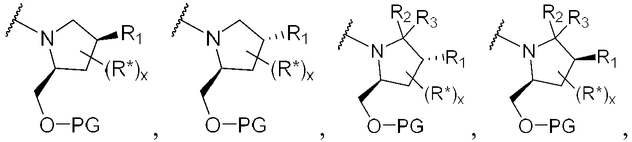 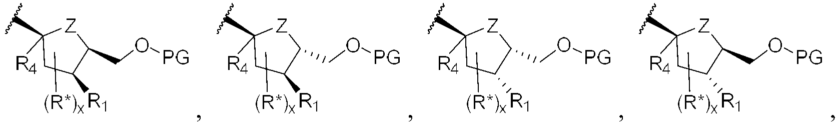 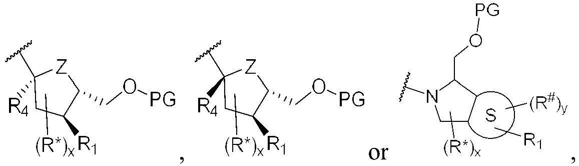 wherein PG is as defined in claim 46, and ring S, R₁, R₂, R₃, R₄, Z, R*, R#, x and y are as defined in any one of claims 1-26;
preferably, R' is selected from 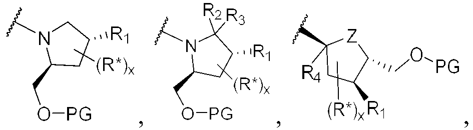 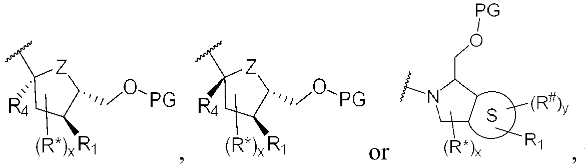 wherein PG is as defined in claim 46, and ring S, R₁, R₂, R₃, R₄, Z, R*, R#, x and y are as defined in any one of claims 1-26;
more preferably, R' is selected from 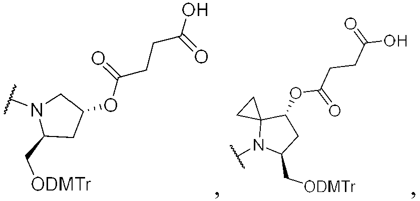 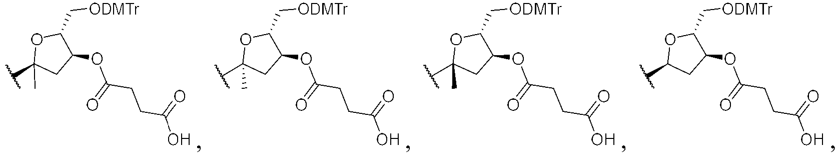 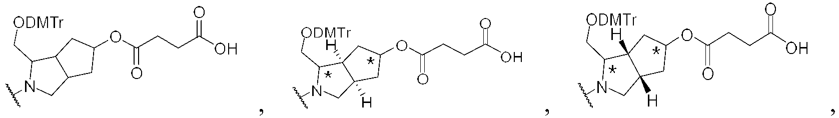 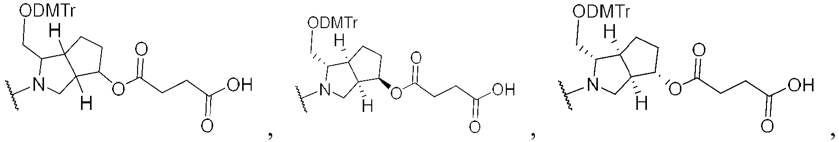 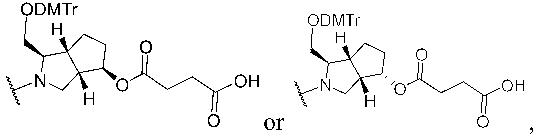 and * denotes a chiral centre, which is independently selected from (S)- or (R)-configuration, or a mixture thereof.

49. The compound according to any one of claims 46-48, which is selected from a compound in Table B, or a pharmaceutically acceptable salt, stereoisomer, tautomer or isotopic variant thereof, or a mixture thereof.

50. A compound of formula (XI), formula (XII) or formula (XIII), or a pharmaceutically acceptable salt, isotopic variant, tautomer, stereoisomer, polymorph, hydrate or solvate thereof, or a mixture thereof: 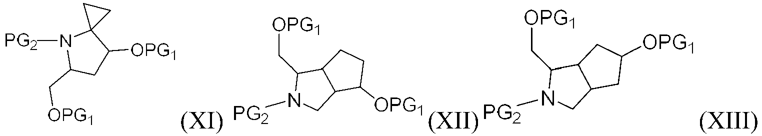
**characterised in that** each PG₁ is independently selected from H or a hydroxyl protecting group;
PG₂ is H or an amino protecting group.

51. The compound according to claim 50, **characterised in that** the compound has a structure of formula (XI-1), formula (XI-2), formula (XI-3), formula (XI-4), formula (XII-1), formula (XII-2), formula (XII-3), formula (XII-4), formula (XIII-1) or formula (XIII-2): 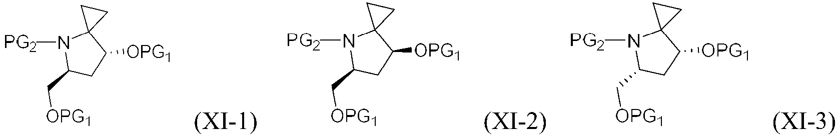 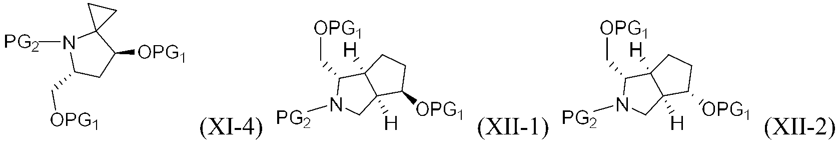 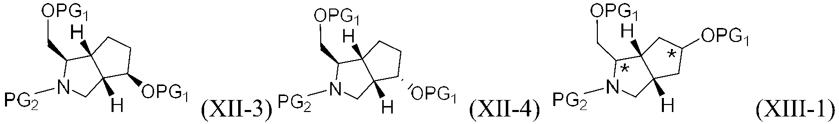 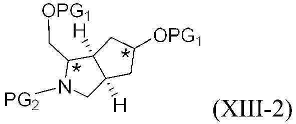
wherein * denotes a chiral centre, which is independently selected from (S)- or (R)-configuration, or a mixture thereof;
PG₁ and PG₂ are as defined in claim 50.

52. The compound according to claim 50 or 51, **characterised in that**
each PG₁ is independently selected from H, C(O)CH₂CH₂C(O)OH or 4,4'-dimethoxytrityl (DMTr);
PG₂ is selected from H, tert-butyloxycarbonyl (BOC) and 9-fluorenylmethoxycarbonyl (FMOC).

53. The compound according to any one of claims 50-52, **characterised in that** the compound is selected from: 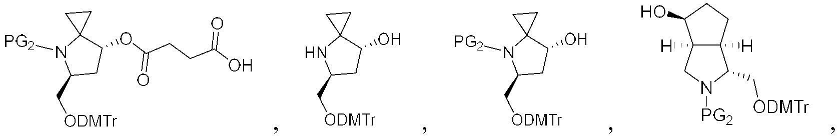 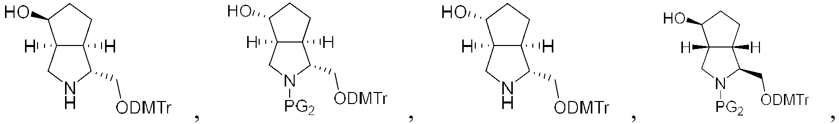 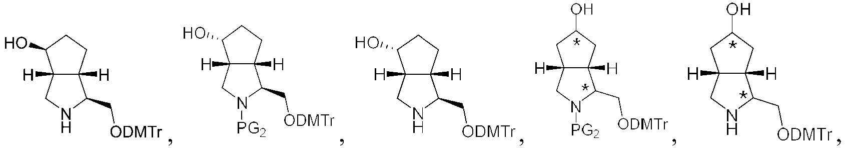 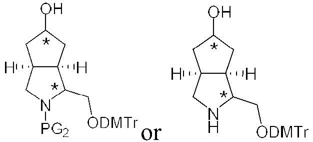
wherein * denotes a chiral centre, which is independently selected from (S)- or (R)-configuration, or a mixture thereof;
PG₂ is an amino protecting group, preferably tert-butyloxycarbonyl (BOC) or 9-fluorenylmethoxycarbonyl (FMOC).
